(19) Europäisches Patentamt
European Patent Office
Office européen des brevets

(11) Veröffentlichungsnummer: **0 532 466 A2**

# (12) EUROPÄISCHE PATENTANMELDUNG

(21) Anmeldenummer: **92810678.0**

(22) Anmeldetag: **03.09.92**

(51) Int. Cl.[5]: **C07K 5/04**, C07D 295/16, A61K 37/64

(30) Priorität: **12.09.91 CH 2689/91**
**27.03.92 CH 980/92**
**25.06.92 CH 2007/92**

(43) Veröffentlichungstag der Anmeldung:
**17.03.93 Patentblatt 93/11**

(84) Benannte Vertragsstaaten:
**AT BE CH DE DK ES FR GB GR IE IT LI LU MC NL PT SE**

(71) Anmelder: **CIBA-GEIGY AG**
**Klybeckstrasse 141**
**CH-4002 Basel (CH)**

(72) Erfinder: **Lang, Marc, Dr.**
**Rue de Valdoie 24**
**F-68200 Mulhouse (FR)**
Erfinder: **Bold, Guido, Dr.**
**Bleumatthöhe 16**
**CH-5264 Gipf-Oberfrick (CH)**
Erfinder: **Fässler, Alexander, Dr.**
**Hallenstrasse 10**
**CH-4104 Oberwil (CH)**
Erfinder: **Schneider, Peter, Dr.**
**Bäumliackerstrasse 8**
**CH-4103 Bottmingen (CH)**
Erfinder: **van Hoogevest, Peter, Dr.**
**Burgstrasse 5**
**CH-4125 Riehen (CH)**

(54) **5-Amino-4-Hydroxyhexansäurederivate als Therapeutika.**

(57) Beschrieben sind Verbindungen der Formel

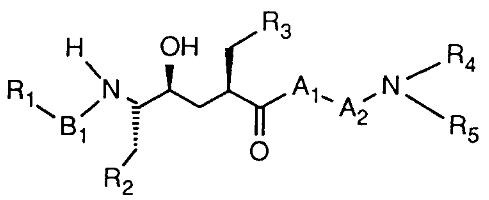

(I),

worin $R_1$ Wasserstoff, Niederalkoxycarbonyl, Heterocyclylcarbonyl, Benzyloxycarbonyl, welches unsubstituiert oder durch bis zu drei unabhängig voneinander aus Fluor, Haloniederalkyl, Niederalkanoyl, Sulfo, Niederalkylsulfonyl und Cyano ausgewählte Reste substituiert ist, Heterocyclyloxycarbonyl, worin Heterocyclyl über ein Kohlenstoffatom gebunden ist, einen der genannten Carbonylreste, worin die bindende Carbonylgruppe durch eine Thiocarbonylgruppe ersetzt ist, Heterocyclylsulfonyl, Niederalkylsulfonyl oder N-(Heterocyclylniederalkyl)-N-niederalkyl-aminocarbonyl bedeutet, $B_1$ eine Bindung oder ein bivalentes Radikal einer $\alpha$-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl, wobei diese Reste unsubstituiert oder durch ein bis drei unabhängig aus Hydroxy, Niederalkoxy, Halo, Haloniederalkyl, Sulfo, Niederalkylsulfonyl, Cyano und Nitro ausgewählte Reste substituiert sind, bedeuten, $A_1$ eine Bindung zwischen -C=O und $A_2$ oder ein bivalentes Radikal einer $\alpha$-Aminosäure bedeutet, welches N-terminal mit der Gruppe -C=O und C-terminal mit $A_2$ verbunden ist, $A_2$ ein bivalentes Radikal einer $\alpha$-Aminosäure bedeutet, welches N-terminal mit $A_1$ und C-terminal mit der Gruppe $NR_4R_5$ verbunden ist, oder $A_1$ und $A_2$ zusammen ein bivalentes Radikal eines Dipeptides bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom unsubstituiertes oder substituiertes Thiomorpholino oder Morpholino bedeuten, und die Salze dieser Verbindungen, sofern salzbildende Gruppen vorliegen, oder hydroxygeschützte Derivate davon. Diese HIV-Proteaseinhibitoren dienen zur Behandlung von AIDS.

EP 0 532 466 A2

Die Erfindung betrifft nicht hydrolysierbare Analoga für durch Aspartatproteasen spaltbare Peptide, nämlich 5-Amino-4-hydroxy-hexansäurederivate, Verfahren zu ihrer Herstellung, pharmazeutische Präparate, die diese Peptidanaloga enthalten, und deren Verwendung als Arzneimittel oder zur Herstellung pharmazeutischer Präparate zur Bekämpfung retroviral bedingter Erkrankungen.

AIDS ist nach bisherigem Wissen eine durch das Retrovirus HIV ("Human Immunodeficiency Virus") hervorgerufene Erkrankung des Immunsystems. Diese Erkrankung betrifft nach Schätzungen der WHO rund 10 Millionen Menschen und breitet sich immer weiter aus. Die Erkrankung führt praktisch stets zum Tod des Patienten.

Bisher konnten die Retroviren HIV-1 und HIV-2 (HIV steht für "Human Immunodeficiency Virus") als Ursache für die Erkrankung identifiziert und molekularbiologisch charakterisiert werden. Für die Therapie ist über die bisherigen begrenzten Möglichkeiten zur Linderung der AIDS-Symptome und gewisse präventive Möglichkeiten hinaus insbesondere die Suche nach Präparaten interessant, welche die Vermehrung des Virus selbst beeinträchtigen, ohne die intakten Zellen und Gewebe der Patienten zu schädigen.

Ein interessanter Ansatz sind Verbindungen, die eine Vermehrung des Virus blockieren, indem sie den Zusammenbau infektiöser Viruspartikel behindern.

HIV-1 und HIV-2 weisen jeweils in ihrem Genom einen Bereich auf, der für eine "gag-Protease" kodiert. Diese "gag-Protease" ist für die korrekte proteolytische Spaltung der Vorläuferproteine verantwortlich, die aus den für die "Group Specific Antigens " (gag) kodierenden Genomabschnitten hervorgehen. Hierbei werden die Strukturproteine des Viruskerns, englisch "Core", freigesetzt. Die "gag-Protease" selbst ist Bestandteil eines durch den pol-Genomabschnitt von HIV-1 und HIV-2 kodierten Vorläuferproteines, das auch die Abschnitte für die "Reverse Transcriptase" und die "Integrase" enthält und vermutlich autoproteolytisch gespalten wird.

Die "gag-Protease" spaltet das Hauptkernprotein ("Major Core Protein") p24 von HIV-1 und HIV-2 bevorzugt N-terminal von Prolinresten, z. B. in den bivalenten Radikalen Phe-Pro, Leu-Pro oder Tyr-Pro. Es handelt sich um eine Protease mit einem katalytisch aktiven Aspartatrest im aktiven Zentrum, eine sogenannte Aspartatprotease.

Aufgrund der zentralen Rolle der "gag-Protease" bei der Prozessierung der genannten "Core-Proteine" wird davon ausgegangen, dass eine wirksame Inhibierung dieses Enzyms in vivo den Zusammenbau reifer Virionen unterbindet, so dass entsprechende Inhibitoren therapeutisch eingesetzt werden können.

Voraussetzung für die therapeutische Wirksamkeit in vivo ist das Erreichen guter Bioverfügbarkeit, z. B. eines hohen Blutspiegels, um so an den infizierten Zellen ausreichend hohe Konzentrationen zu erreichen.

Es sind bereits eine Reihe von "gag-Protease"-Hemmern synthetisiert worden, welche zentrale Gruppen enthalten, die nicht proteolytisch spaltbare Peptidisostere darstellen. Bisher ist es trotz intensiver Forschung jedoch noch nicht gelungen, zur Anwendung am Menschen geeignete Aspartatproteasehemmer zur Bekämpfung von AIDS bei einem Grossteil der Infizierten zum Einsatz zu bringen. Hierfür sind vor allem pharmakodynamische Probleme ausschlaggebend. Das Ziel der hier vorliegenden Erfindung ist, neue Hemmstoffe der HIV-I-Aspartatprotease zugänglich zu machen.

Bei den erfindungsgemässen Verbindungen handelt es sich um Verbindungen der Formel

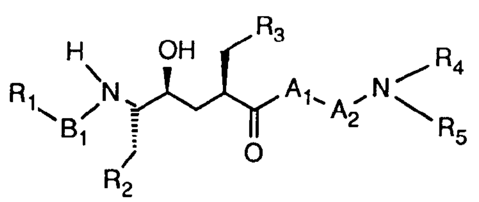

(I),

worin $R_1$ Wasserstoff, Niederalkoxycarbonyl, Heterocyclylcarbonyl, Benzyloxycarbonyl, welches unsubstituiert oder durch bis zu drei unabhängig voneinander aus fluor, Haloniederalkyl, Niederalkanoyl, Sulfo, Niederalkylsulfonyl und Cyano ausgewählte Reste substituiert ist, Heterocyclyloxycarbonyl, worin Heterocyclyl über ein Kohlenstoffatom gebunden ist, einen der genannten Carbonylreste, worin die bindende Carbonylgruppe durch eine Thiocarbonylgruppe ersetzt ist, Heterocyclylsulfonyl, Niederalkylsulfonyl oder N-(Heterocyclylniederalkyl)-N-niederalkyl-aminocarbonyl bedeutet, $B_1$ eine Bindung oder ein bivalentes Radikal einer α-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl, wobei diese Reste unsubstituiert oder durch ein bis drei unabhängig aus Hydroxy, Niederalkoxy, Halo, Haloniederalkyl, Sulfo, Niederalkylsulfonyl, Cyano und Nitro ausgewählte Reste substituiert sind, bedeuten, $A_1$ eine Bindung zwischen -C=O und $A_2$ oder ein bivalentes Radikal einer α-Aminosäure bedeutet, welches N-terminal mit der Gruppe -C=O und C-terminal mit $A_2$ verbunden ist, $A_2$ ein bivalentes Radikal einer α-Aminosäure bedeutet, welches N-ter-

minal mit $A_1$ und C-terminal mit der Gruppe $NR_4R_5$ verbunden ist, oder $A_1$ und $A_2$ zusammen ein bivalentes Radikal eines Dipeptides bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom unsubstituiertes oder substituiertes Thiomorpholino oder Morpholino bedeuten, oder Salze dieser Verbindungen, sofern salzbildende Gruppen vorliegen, oder hydroxygeschützte Derivate dieser Verbindungen oder deren Salze.

In der Beschreibung der vorliegenden Erfindung bedeutet der bei der Definition von Gruppen oder Resten, z. B. Niederalkyl, Niederalkoxycarbonyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten.

Gegebenenfalls vorhandene asymmetrische Kohlenstoffatome in den Substituenten $R_1$, $B_1$, $R_2$, $R_3$, $A_1$ und/oder $A_2$, sowie in aus $R_4$ und $R_5$ gemeinsam mit dem bindenden Stickstoffatom gebildetem substituiertem Thiomorpholino oder Morpholino, können in der (R)-, (S)- oder (R,S)-Konfiguration vorliegen. Somit können die vorliegenden Verbindungen als Isomerengemische oder als reine Isomeren, insbesondere als Diastereomerengemische, Enantiomerenpaare oder reine Enantiomere vorliegen.

Die in der Beschreibung der vorliegenden Erfindung verwendeten allgemeinen Ausdrücke und Bezeichnungen haben vorzugsweise die folgenden Bedeutungen, wobei auf den verschiedenen Definitionsebenen von den vor- oder nachstehend aufgeführten Resten beliebige Kombinationen oder Einzelreste anstelle der allgemeinen Definitionen verwendet werden können:

Niederalkoxycarbonyl $R_1$ enthält vorzugsweise einen verzweigten Niederalkylrest, insbesondere einen sec- oder tert-Niederalkylrest, und ist z. B. Butoxycarbonyl, wie tert-Butoxycarbonyl oder Isobutoxycarbonyl. Besonders bevorzugt ist tert-Butoxycarbonyl.

Heterocyclylcarbonyl $R_1$ enthält insbesondere einen 5- oder 6-gliedrigen Heterocyclus, der 1 bis 3 Heteroatome, die unabhängig voneinander aus S, O oder N ausgewählt sind, enthält, ungesättigt oder ganz oder teilweise gesättigt ist und ein- oder bis zu dreifach benzanelliert, cyclopenta-, cyclohexa- oder cyclohepta-anelliert ist, wobei die genannten anellierten Ringe ein weiteres Stickstoffatom als Heteroatom enthalten können, beispielsweise einen Heterocyclylrest ausgewählt aus Pyrrolyl, Furanyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, ß-Carbolinyl und einem benzanellierten, cyclopenta-, cyclohexa- oder cyclohepta-anellierten Derivat dieser Reste, die auch ganz oder teilweise gesättigt vorliegen können, vorzugsweise teilweise gesättigt, oder ist ausgewählt aus Pyridylcarbonyl, z. B. Pyridyl-3-carbonyl, Morpholinylcarbonyl, z. B. Morpholinocarbonyl, und Benzofuranoyl, z. B. 3-Benzofuranoyl, sowie alternativ oder ergänzend hierzu Tetrahydroisochinolylcarbonyl, z. B. Tetrahydroisochinolyl-3-carbonyl, vorzugsweise Tetrahydroisochinolyl-3(S)-carbonyl.

Benzyloxycarbonyl $R_1$ ist unsubstituiert oder durch bis zu drei unabhängig voneinander aus Fluor, Haloniederalkyl, z. B. Trifluormethyl oder Pentafluorethyl, Niederalkanoyl, wie Acetyl, Propanoyl, Butyryl oder Pivaloyl, Sulfo, Niederalkylsulfonyl, z. B. Methylsulfonyl, Ethylsulfonyl, n-Propylsulfonyl oder iso-Propylsulfonyl, und Cyano ausgewählte Reste substituiert. Bevorzugt ist unsubstituiertes oder durch einen aus fluor, Trifluormethyl, Sulfo, Methylsulfonyl, Ethylsulfonyl und Cyano ausgewählten Rest im Phenylring o-, m- oder p-substituiertes, insbesondere p-substituiertes Benzyloxycarbonyl, z. B. Benzyloxycarbonyl, Fluorphenylmethoxycarbonyl, wie p-Fluorphenylmethoxycarbonyl, Trifluormethylphenylmethoxycarbonyl, wie p-Trifluormethylphenylmethoxycarbonyl, Methylsulfonylphenylmethoxycarbonyl, wie p-Methylsulfonylphenylmethoxycarbonyl, oder Cyanophenylmethoxycarbonyl, wie p-Cyanophenylmethoxycarbonyl.

Heterocyclyloxycarbonyl $R_1$ enthält als Heterocyclyl insbesondere einen 5- oder 6-gliedrigen Heterocyclus, der 1 bis 3 Heteroatome, die unabhängig voneinander aus S, O oder N ausgewählt sind, enthält, ungesättigt oder ganz oder teilweise gesättigt ist und einoder bis zu dreifach benzanelliert, cyclopenta-, cyclohexa- oder cyclohepta-anelliert ist, wobei die genannten anellierten Ringe ein weiteres Stickstoffatom als Heteroatom enthalten können, beispielsweise einen Rest ausgewählt aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, ß-Carbolinyl und einem benzanellierten, cyclopenta-, cyclohexa- oder cyclohepta-anellierten Derivat dieser Reste, die auch ganz oder teilweise gesättigt vorliegen können, wobei die Heterocyclylreste über ein Ringkohlenstoffatom an den Sauerstoff des zugehörigen Oxycarbonylrestes gebunden sind, vorzugsweise ausgewählt aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, ß-Carbolinyl und einem ganz oder teilweise gesättigten Derivat dieser Reste, z. B. einem teilweise gesättigten Derivat dieser Reste oder Indol-3-yl-oxycarbonyl, Benzthiazol-6-yl-oxycarbonyl oder Chinol-8-yl-oxycarbonyl. In einer ganz besonders bevorzugten Variante der Definition von $R_1$ sind die unter die Definition der Substituenten Heterocyclyloxycarbonyl fallenden Reste auf allen Definitionsebenen nicht umfasst.

In den genannten Resten kann die bindende Carbonyl- auch durch eine Thiocarbonylgruppe ersetzt sein. Bevorzugt ist eine Carbonylgruppe.

Niederalkylsulfonyl $R_1$ ist vorzugsweise Methylsulfonyl, Ethylsulfonyl, n-Propylsulfonyl oder iso-Propylsulfonyl. Die Verbindungen der Formel I, worin $R_1$ Niederalkylsulfonyl bedeutet und die übrigen Reste die genannten Bedeutungen haben, können bei der Definition der Verbindungen der Formel I wegfallen, oder sie sind besonders bevorzugt.

Heterocyclylsulfonyl enthält als Heterocyclyl vorzugsweise einen der unter Heterocyclylcarbonyl $R_1$ genannten Heterocyclen, der unsubstituiert oder durch Niederalkyl, wie Methyl oder Ethyl, substituiert ist, wobei Heterocyclen bevorzugt sind, die wenigstens ein Stickstoffatom enthalten, welches an den Schwefel der Sulfonylgruppe gebunden ist, und ist insbesondere Piperidinosulfonyl, unsubstituiertes oder am nicht an den Sulfonyl-Schwefel gebundenen Stickstoff durch Niederalkyl, wie Methyl, substituiertes Piperazin-1-yl-sulfonyl, Pyrrolidin-1-yl-sulfonyl, Imidazolidin-1-yl-sulfonyl, Pyrimidin-1-yl-sulfonyl, Chinolin-1-ylsulfonyl, Morpholinosulfonyl oder Thiomorpholinosulfonyl, vor allem Thiomorpholinosulfonyl oder Morpholinosulfonyl. Die Verbindungen der Formel I, worin $R_1$ Heterocyclylsulfonyl bedeutet und die übrigen Reste die genannten Bedeutungen haben, können bei der Definition der Verbindungen der Formel I wegfallen, oder sie sind besonders bevorzugt.

N-(Heterocylylniederalkyl)-N-niederalkyl-aminocarbonyl $R_1$ enthält als Heterocyclyl vorzugsweise einen der unter Heterocyclylcarbonyl $R_1$ genannten Heterocyclen, insbesondere Pyridyl, wie 2-, 3- oder 4-Pyridyl, Pyrazinyl, Pyrimidinyl, Morpholinyl, wie Morpholino, Thiomorpholinyl, wie Thiomorpholino, oder Chinolyl, wie 2- oder 3-Chinolyl, und ist insbesondere N-(Heterocyclylmethyl)-N-methyl-aminocarbonyl, z. B. N-(Pyridylmethyl)-N-methyl-aminocarbonyl, wie N-(2-Pyridylmethyl)-N-methyl-aminocarbonyl. Die Verbindungen der Formel I, worin $R_1$ N-(Heterocylylniederalkyl)N-niederalkyl-aminocarbonyl bedeutet und die übrigen Reste die genannten Bedeutungen haben, können bei der Definition der Verbindungen der Formel I wegfallen, oder sie sind besonders bevorzugt.

Ein bivalentes Radikal $B_1$ einer α-Aminosäure, welches N-terminal mit $R_1$ und C-terminal mit der Aminogruppe am $R_2$-$CH_2$- tragenden Kohlenstoffatom verbunden ist, ist vorzugsweise ausgewählt aus Glycin (H-Gly-OH), Alanin (H-Ala-OH), Valin (H-Val-OH), Norvalin (α-Aminovaleriansäure), Leucin, (H-Leu-OH), Isoleucin (H-Ile-OH), Norleucin (α-Aminohexansäure, H-Nle-OH), Serin (H-Ser-OH), Homoserin (α-Amino-γ-hydroxybuttersäure), Threonin (H-Thr-OH), Methionin (H-Met-OH), Cystein (H-Cys-OH), Prolin (H-Pro-OH), trans-3- und trans-4-Hydroxyprolin, Phenylalanin (H-Phe-OH), p-Fluorphenylalanin (H-(p-F-Phe)-OH), Tyrosin (H-Tyr-OH), p-Methoxy-phenylalanin (H-(p-$CH_3O$-Phe)-OH), 4-Aminophenylalanin, 4-Chlorphenylalanin, 4-Carboxyphenylalanin, β-Phenylserin (β-Hydroxyphenylalanin), Phenylglycin, α-Naphthylalanin (H-Nal-OH), Cyclohexylalanin (H-Cha-OH), Cyclohexylglycin, Tryptophan (H-Trp-OH), Indolin-2-carbonsäure, 1,2,3,4-Tetrahydroisochinolin-3-carbonsäure, Aminomalonsäure, Aminomalonsäure-monoamid, Asparaginsäure (H-Asp-OH), Asparagin (H-Asn-OH), Glutaminsäure (H-Glu-OH), Glutamin (H-Gln-OH), Histidin (H-His-OH), Arginin (H-Arg-OH), Lysin (H-Lys-OH), δ-Hydroxylysin, Ornithin (α,δ-Diaminovaleriansäure), α,γ-Diaminobuttersäure und α,β-Diaminopropionsäure, oder alternativ und ergänzend hierzu 4-Cyano-phenylalanin (H-(p-CN-Phe)-OH), besonders bevorzugt der Rest einer hydrophoben Aminosäure, z. B. Prolin, Phenylalanin, p-Fluorphenylalanin, p-Methoxyphenylalanin, Tyrosin, Phenylglycin, α-Naphthylalanin, Cyclohexylalanin, Cyclohexylglycin oder eine aliphatische α-Aminosäure ausgewählt aus Glycin, Valin, Norvalin, Alanin, Leucin, Norleucin und Isoleucin, insbesondere Valin, wobei jede der genannten α-Aminosäuren in der D-, L- oder (D,L)-, vorzugsweise in der L-Form vorliegt, und insbesondere mit Resten $R_1$ ausgewählt aus Niederalkoxycarbonyl, z. B. tert-Butoxycarbonyl, oder Heterocyclylcarbonyl, z. B. Morpholinocarbonyl, verknüpft ist.

Wenn $B_1$ eine Bindung bedeutet, ist $R_1$ direkt mit dem Aminostickstoff verbunden, den das den Rest $R_2$-$CH_2$- tragende Kohlenstoffatom in Formel I bindet.

Phenyl oder Cyclohexyl $R_2$ oder $R_3$ ist unsubstituiert oder durch bis zu drei unabhängig aus Hydroxy, Niederalkoxy, wie Methoxy oder Ethoxy, Halo, z. B. Fluor, Haloniederalkyl, z. B. Trifluormethyl, Sulfo, Niederalkylsulfonyl, z. B. Methyl- oder Ethylsulfonyl, Cyano und Nitro ausgewählte Reste substituiert, vorzugsweise durch ein bis zwei dieser Reste, besonders bevorzugt ausgewählt aus Hydroxy, Methoxy, Fluor, Trifluormethyl, Sulfo, Niederalkylsulfonyl, z. B. Methyl- oder Ethylsulfonyl, und Cyano; für Phenyl ganz besonders bevorzugt aus Fluor und Cyano, für Cyclohexyl ganz besonders bevorzugt aus Fluor, Trifluormethyl, Sulfo oder Niederalkylsulfonyl, in erster Linie Fluor, wobei die genannten Substituenten in 2-, 3- oder 4- Stellung des Phenyl- oder Cyclohexylringes, insbesondere in 4-Stellung, gebunden sind, wie in Phenyl, Cyclohexyl, 4-Fluor- oder 4-Cyanophenyl oder 4-Fluorcyclohexyl, insbesondere in Phenyl, Cyclohexyl, 4-Cyano-phenyl oder 4-Fluorphenyl.

Besonders bevorzugt sind solche Kombinationen von $R_2$ und $R_3$, bei denen wenigstens einer der Reste $R_2$ oder $R_3$ durch einen oder bis zu drei aus Halo, insbesondere Fluor, Haloniederalkyl, insbesondere Trifluormethyl, Sulfo, Niederalkylsulfonyl, insbesondere Methyl oder Ethylsulfonyl, Cyano und Nitro ausgewählte Reste substituiert ist, wobei in stark bevorzugter Weise ein Substituent ausgewählt aus Fluor oder Cyano vorliegt.

Noch stärker bevorzugt ist $R_2$ ausgewählt aus Phenyl, 4-Hydroxyphenyl, 4-Methoxyphenyl, 4-Fluorphenyl,

Cyclohexyl und 4-Trifluormethylphenyl, während $R_3$ ausgewählt ist aus Phenyl, 4-Hydroxyphenyl, 4-Methoxyphenyl, Cyclohexyl, 4-Fluorphenyl, 4-Trifluormethylphenyl und 4-Cyanophenyl.

In erster Linie ist $R_2$ ausgewählt aus Phenyl, 4-Fluorphenyl und Cyclohexyl, während $R_3$ ausgewählt ist aus Phenyl, Cyclohexyl, 4-Fluorphenyl und 4-Cyanophenyl.

In allererster Linie bevorzugt sind die Kombinationen: $R_2$ Phenyl und $R_3$ Phenyl; $R_2$ Cyclohexyl und $R_3$ 4-Cyanophenyl; $R_2$ Cyclohexyl und $R_3$ 4-Fluorphenyl; und $R_2$ und $R_3$ jeweils Cyclohexyl. Alternativ oder ergänzend hierzu sind auch die Kombinationen $R_2$ Phenyl und $R_3$ 4-Fluorphenyl; $R_2$ Phenyl und $R_3$ 4-Cyanophenyl; $R_2$ 4-Fluorphenyl und $R_3$ 4-Fluorphenyl; $R_2$ 4-Fluorphenyl und $R_3$ 4-Trifluormethylphenyl; $R_2$ 4-Trifluormethylphenyl und $R_3$ Phenyl; $R_2$ 4-Trifluormethylphenyl und $R_3$ 4-Fluorphenyl; $R_2$ 4-Trifluormethylphenyl und $R_3$ 4-Trifluormethylphenyl; $R_2$ Hydroxyphenyl und $R_3$ Phenyl; $R_2$ Phenyl und $R_3$ Hydroxyphenyl; oder $R_2$ Hydroxyphenyl und $R_3$ Hydroxyphenyl in allererster Linie bevorzugt.

Hydroxygruppen, insbesondere die Hydroxygruppe in Verbindungen der Formel I an dem Kohlenstoffatom, welches dem Kohlenstoffatom benachbart ist, das den Rest $R_2$-$CH_2$- trägt, können frei oder in geschützter Form vorliegen, wobei als Hydroxyschutzgruppen die unten bei der Beschreibung der Herstellungsverfahren für Verbindungen der Formel I genannten Reste in Frage kommen, insbesondere frei oder geschützt als phyiologisch spaltbare Ester, z. B. als Niederalkanoyloxy, wie Acetyloxy.

Ein bivalentes Radikal einer α-Aminosäure $A_1$, welches N-terminal mit der Gruppe -C=O und C-terminal mit $A_2$ verbunden ist, ist beispielsweise eine der oben für $B_1$ genannten α-Aminosäuren, wobei diese Aminosäuren in der (D)-, (L)- oder (D,L)-Form, vorzugsweise der (D)- oder (L)-, insbesondere der (L)-Form, vorliegen können. Bevorzugt sind die unter $B_1$ genannten hydrophoben α-Aminosäuren, insbesondere die dort genannten aliphatischen hydrophoben α-Aminosäuren, z. B. Glycin, Valin oder Isoleucin. In den genannten α-Aminosäuren ist die an $A_2$ bindende Carboxygruppe nicht reduziert oder ferner reduziert, insbesondere zu einer Methylengruppe, z. B. in den genannten hydrophoben α-Aminosäuren, wie in den reduzierten Aminosäureradikalen Gly(red), Val(red) oder Ile(red), insbesondere in Val(red), wobei der Zusatz (red) die Reduktion der Carbonylgruppe des entsprechenden Aminosäureradikals zur Methylengruppe anzeigt.

Bedeutet $A_1$ eine Bindung, so ist $A_2$ direkt mit der Carbonylgruppe am Kohlenstoffatom, der den Rest $R_3$-$CH_2$- trägt, verbunden.

Ein bivalentes Radikal einer α-Aminosäure $A_2$, welches N-terminal mit $A_1$ und C-terminal mit der Gruppe $NR_4R_5$ verbunden ist, ist beispielsweise eine der oben für $B_1$ genannten α-Aminosäuren, wobei diese Aminosäuren in der (D)-, (L)- oder (D,L)-Form, vorzugsweise der (D)- oder (L)-, insbesondere der (L)-Form, vorliegen können. Bevorzugt sind die unter $B_1$ genannten hydrophoben α-Aminosäuren, z. B. Glycin, Valin, Phenylalanin, p-Fluorphenylalanin, Tyrosin, p-Methoxy-phenylalanin, Phenylglycin, α-Naphthylalanin, Cyclohexylalanin oder Cyclohexylglycin, vorzugsweise Glycin, Valin, Phenylalanin, p-Fluorphenylalanin, p-Methoxy-phenylalanin oder Cyclohexylalanin, wobei die genannten Reste in der (D)- oder (L)-Form, vorzugsweise, ausser Phenylalanin, das in der (L)- oder der (D)-Form vorliegt, in der (L)-Form vorliegen.

Ein aus $A_1$ und $A_2$ gebildetes bivalentes Radikal eines Dipeptides, dessen zentrale Peptidbindung reduziert ist und das N-terminal mit der Gruppe -C=O und C-terminal mit der Gruppe $NR_4R_5$ verbunden ist, besteht vorzugsweise aus 2 der oben genannten hydrophoben α-Aminosäuren, insbesondere aus einem N-terminalen Aminosäureradikal ausgewählt aus Gly(red), Val(red) und Ile(red) und einer C-terminalen Aminosäure ausgewählt aus Glycin, Phenylalanin, Tyrosin, p-Methoxyphenylalanin, Cyclohexylalanin und p-Fluorphenylalanin.

Besonders bevorzugt bilden $A_1$ und $A_2$ zusammen ein bivalentes Radikal eines Dipeptides der Formel Val-Phe, Ile-Phe, Val-Cha, Ile-Cha, Val-Gly, Val-(p-F-Phe), Val-(p-$CH_3O$-Phe), Gly-(p-F-Phe); und alternativ oder zusätzlich eines Dipeptides der Formel Val-Tyr, Ile-Tyr, Gly-Tyr, Ile-Gly oder Val-Val; worin die Aminosäuren in der (D)- oder (L)-, insbesondere der (L)-Form vorliegen mit Ausnahme von (L)-Val-Phe, in dem Phe in der (L)- oder (D)-Form vorliegt, oder eines Derivates hiervon mit reduzierter zentraler Amidbindung, z. B. mit der Formel Val(red)-Phe, das N-terminal mit der Gruppe -C=O und C-terminal mit der Gruppe $NR_4R_5$ verbunden ist.

Eine bevorzugte Ausführungsform der Erfindung bezieht sich entweder auf die Verbindungen der Formel I, in denen $B_1$ eines der genannten bivalenten Radikale einer α-Aminosäure bedeutet und einer der Reste $A_1$ oder $A_2$ eine Bindung bedeutet und der andere eine der genannten α-Aminosäuren bedeutet, oder auf diejenigen Verbindungen der Formel I, worin $B_1$ eine Bindung bedeutet und $A_1$ und $A_2$ jeweils eines der genannten bivalenten Radikale einer α-Aminosäure oder gemeinsam eines der genannten bivalenten Radikale eines Dipeptides mit reduzierter zentraler Amidbindung bedeuten.

Aus $R_4$ und $R_5$ gemeinsam mit dem bindenden Stickstoffatom gebildetes Thiomorpholino oder Morpholino ist unsubstituiert oder substituiert an einem oder mehreren der Kohlenstoffatome, bevorzugt einem Kohlenstoffatom, durch Niederalkyl, wie Ethyl, Propyl, Butyl, iso-Butyl oder tert-Butyl, durch Phenyl- oder Naphthylniederalkyl, wie Benzyl, 1- oder 2-Naphthylmethyl oder Phenyl-1- oder Phenyl-2-ethyl, insbesondere Phenyl-1- oder Phenyl-2-ethyl, durch Hydroxy, durch Niederalkoxy, wie Methoxy, Ethoxy oder tert-Butoxy,

durch Amino, durch Niederalkylamino, wie Methyl- oder Ethylamino, oder durch Diniederalkylamino, wie Dimethylamino oder Diethylamino, durch Niederalkanoyl, wie Acetyl oder Propionyl, durch Phenyl- oder Naphthyl-niederalkanoyl, wie Phenylacetyl oder 1-oder 2-Naphthylacetyl, durch Carboxy, durch Niederalkoxycarbonyl, wie iso-Propoxycarbonyl oder tert-Butoxycarbonyl, durch Phenyl-, Naphthyl- oder Fluorenylniederalkoxycarbonyl, wie Benzyloxycarbonyl, 1- oder 2-Naphthylmethoxycarbonyl oder 9-Fluorenylmethoxycarbonyl, durch Carbamoyl, durch Mono- oder Diniederalkylcarbamoyl, wie Dimethylcarbamoyl, durch Mono- oder Di-hydroxyniederalkyl-carbamoyl, wie Dihydroxymethyl-carbamoyl, durch Sulfo, durch Niederalkylsulfonyl, wie Methylsulfonyl oder Ethylsulfonyl, durch Phenyl- oder Naphthylsulfonyl, wobei Phenyl durch Niederalkyl, z. B. Methyl oder Ethyl, substituiert sein kann, z. B. Phenylsulfonyl oder Toluolsulfonyl, durch Sulfamoyl, durch Halogen, z. B. Fluor oder Chlor, durch Cyano, durch Nitro und/oder durch Oxo.

Sehr bevorzugt bilden $R_4$ und $R_5$ zusammen mit dem bindenden Stickstoffatom unsubstituiertes Thiomorpholino oder Morpholino, vor allem unsubstituiertes Morpholino.

Salze von Verbindungen der Formel I sind insbesondere Säureadditionssalze, Salze mit Basen oder bei Vorliegen mehrerer salzbildender Gruppen gegebenenfalls auch Mischsalze oder innere Salze.

Salze sind in erster Linie die pharmazeutisch verwendbaren, nichttoxischen Salze von Verbindungen der Formel I.

Solche Salze werden beispielsweise von Verbindungen der Formel I mit einer sauren Gruppe, z. B. einer Carboxy- oder Sulfogruppe, gebildet und sind beispielsweise deren Salze mit geeigneten Basen, wie nichttoxische, von Metallen der Gruppe Ia, Ib, IIa und IIb des Periodensystems der Elemente abgeleitete Metallsalze, in erster Linie geeignete Alkalimetall-, z. B. Lithium-, Natrium- oder Kalium-, oder Erdalkalimetallsalze, z. B. Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, insbesondere Mono-, Di- oder Triniederalkylaminen, oder mit quaternären Ammoniumverbindungen gebildet werden, z. B. mit N-Methyl-N-ethylamin, Diethylamin, Triethylamin, Mono-, Bis- oder Tris-(2-hydroxyniederalkyl)-aminen, wie Mono-, Bis- oder Tris-(2-hydroxyethyl)-amin, 2-Hydroxy-tert-butylamin oder Tris(hydroxymethyl)-methylamin, N,N-Diniederalkyl-N-(hydroxyniederalkyl)-aminen, wie N,N-Dimethyl-N-(2-hydroxyethyl)-amin oder Tri-(2-hydroxyethyl)amin, N-Methyl-D-glucamin oder quaternären Ammoniumsalzen, wie Tetrabutylammoniumsalzen. Die Verbindungen der Formel I mit einer basischen Gruppe, z. B. einer Aminogruppe, können Säureadditionssalze bilden, beispielsweise mit anorganischen Säuren, z. B. Halogenwasserstoffsäure, wie Salzsäure, Schwefelsäure oder Phosphorsäure, oder mit organischen Carbon-, Sulfon-, Sulfo- oder Phosphosäuren oder N-substituierter Sulfaminsäuren, wie z. B. Essigsäure, Propionsäure, Glykolsäure, Bernsteinsäure, Maleinsäure, Hydroxymaleinsäure, Methylmaleinsäure, Fumarsäure, Äpfelsäure, Weinsäure, Gluconsäure, Glucarsäure, Glucuronsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner mit Aminosäuren, wie z. B. den weiter vom genannten $\alpha$-Aminosäuren, sowie mit Methansulfonsäure, Ethansulfonsäure, 2-Hydroxyethansulfonsäure, Ethan-1,2-disulfonsäure, Benzolsulfonsäure, 4-Methylbenzolsulfonsäure, Naphthalin-2-sulfonsäure, 2- oder 3-Phosphoglycerat, Glucose-6-phosphat, N-Cyclohexylsulfaminsäure (unter Bildung der Cyclamate) oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden.

Die Ausdrücke "Verbindungen" und "Salze" schliessen ausdrücklich auch einzelne Verbindungen oder einzelne Salze ein.

Die Verbindungen der vorliegenden Erfindung zeigen Hemmwirkung auf retrovirale Aspartatproteasen, insbesondere gag-Protease-hemmende Wirkungen. In erster Linie hemmen sie in den nachfolgend beschriebenen Tests in Konzentrationen von $10^{-6}$ bis $10^{-9}$ M die Wirkung der gag-Protease von HIV-1 und sind daher geeignete Mittel gegen durch diese oder verwandte Retroviren verursachte Krankheiten, wie gegen AIDS.

Die Fähigkeit der Verbindungen der Formel I, die proteolytische Aktivität von z. B. HIV-1 Protease zu inhibieren, lässt sich beispielsweise gemäss dem von J. Hansen et al., The EMBO Journal 7, 1785-1791 (1988), beschriebenen Verfahren demonstrieren. Dabei wird die Hemmung der Wirkung der HIV-1-Protease auf ein Substrat gemessen, das ein in E. coli exprimiertes Fusionsprotein aus dem gag-Vorläuferprotein und MS-2 ist. Das Substrat und seine Spaltprodukte werden durch Polyacrylamid-Gelektrophorese getrennt und durch Immunoblotting mit monoklonalen Antikörpern gegen MS-2 sichtbar gemacht.

In einem noch einfacher zu handhabenden Test, der genaue quantitative Aussagen ermöglicht, wird als Substrat für die gag-Protease ein synthetisches Peptid eingesetzt, das einer der Spaltstellen des gag-Vorläuferproteins entspricht. Dieses Substrat und seine Spaltprodukte können durch Hochdruckflüssig-Chromatographie (HPLC) gemessen werden.

Beispielsweise wird als Substrat für eine rekombinante HIV-1-Protease (Herstellung gemäss Billich, S., et

al., J. Biol. Chem. 263(34), 17905 - 17908 (1990)) ein synthetisches chromophores Peptid (z. B. HKARVL[$NO_2$]FEANleS (Bachem, Schweiz) oder ein Icosapeptid wie RRSNQVSQNYPIVQNIQGRR (hergestellt durch Peptidsynthese nach bekannten Verfahren) eingesetzt, das einer der Spaltstellen des gag-Vorläuferproteins entspricht. Dieses Substrat und seine Spaltprodukte können durch Hochdruckflüssig-Chromatographie (HPLC) gemessen werden.

Hierzu wird ein zu testender Hemmstoff der Formel I in Dimethylsulfoxid gelöst; der Enzymtest wird durchgeführt, indem geeignete Verdünnungen des Hemmstoffes in 20 mM β-Morpholinoethansulfonsäure (MES)-Puffer pH 6,0 zum Assay-Mix aus 67,2 μM des oben genannten chromophoren Peptides in 0,3 M Natriumacetat, 0,1 M NaCl pH 7,4; oder 122 μM des oben genannten Icosapeptids in 20 mM MES-Puffer pH 6,0; zugegeben werden. Die Grösse der Ansätze beträgt 100 μl. Die Reaktion wird gestartet durch Zugabe von im ersten Fall 2 μl, im zweiten Fall 10 μl HIV-I-Protease und im ersten Fall nach 15 min durch Zugabe von 100 μl 0,3 M $HClO_4$, im zweiten Fall nach einer Stunde Inkubation bei 37 °C durch Zugabe von 10 μl 0,3 M $HClO_4$ gestoppt. Die Reaktionsprodukte werden nach Abzentrifugieren der Probe für 5 min bei 10 000 x g in 100 μl (Ansatz mit chromophorem Peptid) bzw. 20 μl (Icosapeptid-Ansatz) des erhaltenen Überstandes und nach Auftragen auf eine 125 x 4,6 mm Nucleosil® C18-5μ-HPLC-Säule (Macherey & Nagel, Düren) und Elution quantifiziert anhand der Peak-Höhe des Spaltproduktes bei 280 (Ansatz mit chromophorem Peptid) oder bei 215 nm (Ansatz mit Icosapeptid), Gradient: 100 % El.1 -> 50 % El.1 /50 % El.2 (El.1: 10 % Acetonitril, 90 % $H_2O$, 0,1 % Trifluoressigsäure (TFA); El.2: 75 % Acetonitril, 25 % $H_2O$, 0,08 % TFA) innerhalb von 15 min; Durchflussrate 1 ml/min (El. = Eluens).

Hierbei werden für Verbindungen der Formel I vorzugsweise $IC_{50}$-Werte ($IC_{50}$ = diejenige Konzentration, welche die Aktivität der HIV-1-Protease gegenüber einer Kontrolle ohne Hemmstoff um 50 % senkt) von etwa $10^{-6}$ bis $10^{-9}$ M, insbesondere von $10^{-7}$ bis $10^{-8}$ M, ermittelt.

In einem weiteren Test kann gezeigt werden, dass die Verbindungen der vorliegenden Erfindung Zellen, die normalerweise von HIV infiziert werden, vor einer solchen Infektion schützen oder zumindest eine solche Infektion verlangsamen. Dabei wird die menschliche T-Zell-Leukämie Zellinie MT-2 (Science 229, 563 (1985)), die empfindlich gegen den zytopathogenen Effekt von HIV ist, mit HIV-1 allein oder mit HIV-1 in Gegenwart einer erfindungsgemässen Verbindungen inkubiert und nach einigen Tagen die Lebensfähigkeit der so behandelten Zellen beurteilt.

Hierzu werden die MT-2-Zellen in RPMI 1640-Medium (Gibco, Schweiz; RPMI 1640 enthält ein Aminosäurengemisch ohne L-Gln), das mit 10% hitzeinaktiviertem fetalem Kälberserum, L-Glutamin, Hepes (2-[4-(2-Hydroxyethyl)-1-piperazino]-ethansulfonsäure) und Standardantibiotika supplementiert ist, bei 37 °C in befeuchteter Luft mit 5% $CO_2$ gehalten. 50 μl der jeweiligen Testverbindung in Kulturmedium und 100 μl HIV-1 in Kulturmedium (800 TCID50/ml) (TCID50 = Tissue Culture Infectious Dose 50 = Dosis, die 50% der MT-2-Zellen infiziert) werden zu $4x10^3$ exponentiell wachsenden MT-2-Zellen in 50 μl Kulturmedium pro Vertiefung auf 96-Loch-Mikrotiterplatten gegeben. Parallele Ansätze auf einer weiteren Mikrotiterplatte mit Zellen und Testverbindung erhalten 100 μl Kulturmedium ohne Virus. Nach 4 Tagen Inkubation wird in 10 μl Zellüberstand die Reverse-Transkriptase (RT)-Aktivität ermittelt. Die RT-Aktivität wird bestimmt in 50 mM Tris (α,α,α-Tris(hydroxymethyl)-methylamin, Ultra pur, Merck, Bundesrepublik Deutschland) pH 7,8; 75 mM KCl, 2 mM Dithiothreitol, 5 mM $MgCl_2$; 0,05% Nonidet P-40 (Detergens; Sigma, Schweiz); 50 μg/ml Polyadenylic Acid (Pharmacia, Schweden); 1,6 μg/ml dT(12-18) (Sigma, Schweiz). Die Mischung wird durch einen 0,45 μ Acrodisc®-Filter (Gellman Science Inc, Ann Arbor) abfiltriert und bei -20 °C aufbewahrt. Zu Aliquoten dieser Lösung werden 0,1% (v/v) [alpha-$^{32}$P]dTTP zum Erzielen einer radioaktiven Endaktivität von 10 μCi/ml zugegeben. 10 μl des Kulturüberstandes werden auf eine neue 96-Loch-Mikrotiterplatte übertragen und hierzu 30 μl des genannten RT-Cocktails gegeben. Nach Mischen wird die Platte für 1,5 bis 3 h bei 37 °C inkubiert. 5 μl dieser Reaktionsmischung werden auf Whatman DE81-Papier (Whatman) überführt. Die getrockneten Filter werden 3-mal für 5 min mit 300 mM NaCl/25 mM Tri-Natriumcitrat und 1-mal mit 95% Ethanol gewaschen und erneut luftgetrocknet. Die Auswertung erfolgt in einem Matrix Packard 96well counter (Packard). Die ED90-Werte werden errechnet und als die niedrigste Konzentration der jeweiligen Testverbindung definiert, welche die RT-Aktivität um 90% im Vergleich zu nicht mit der Testsubstanz behandelten Zellansätzen senkt. Die RT-Aktivität ist dabei ein Mass für die HIV-1-Vermehrung.

Die erfindungsgemässen Verbindungen zeigen hierbei eine ED90 von etwa $10^{-5}$ bis $10^{-8}$ M, vorzugsweise von etwa $10^{-7}$ bis $10^{-8}$ M.

Die Verbindungen der vorliegenden Erfindung zeigen vorteilhafte pharmakokinetische Eigenschaften, die erwarten lassen, dass sie in vivo die genannten Hemmwirkungen entfalten. So ist beispielsweise der Blutspiegel bei den genannten Verbindungen bei intravenöser oder intraperitonealer Applikation an Mäusen von 20 mg/kg einer Verbindung der Formel I 10 min nach der Applikation 4 μg/ml Blut und höher. Ferner ist bei peroraler (p. o.) Gabe von 120 mg/kg einer Verbindung der Formel I die Konzentration nach 90 min etwa gleich hoch oder höher als die oben erwähnte ED90 im Zellversuch.

Die Ermittlung des Blutspiegels wird beispielsweise wie folgt vorgenommen: Die zu untersuchenden Verbindungen werden in einem organischen Lösungsmittel, wie Dimethylsulfoxid (DMSO), gelöst. Eine Lösung von Hydroxypropyl-β-cyclodextrin (20 % w/v) in Wasser wird zugegeben bis zum Erhalt der gewünschten Konzentration des Wirkstoffes (beispielsweise 2 mg/ml für parenterale Applikation, 12 mg/ml für orale Applikation) bei gleichzeitiger Einstellung einer Konzentration von 5 % DMSO (v/v). Verbindungen, die unter diesen Bedingungen unlöslich sind, werden bei parenteraler Applikation nur intraperitoneal appliziert, lösliche Verbindungen zusätzlich intravenös. Nach Applikation der Verbindungen (beispielsweise 20 mg/kg intravenös oder intraperitoneal, oder 120 mg/kg peroral) wird zu verschiedenen Zeitpunkten, beispielsweise nach 10 min bei parenteraler Applikation, oder nach 90 min bei peroraler Applikation, Blut entnommen. Pro Zeitpunkt wird das Blut von drei Mäusen verwendet und entweder für jede Maus einzeln oder aus dem vereinten Blut der drei Mäuse nach Zugabe eines Lösungsmittels, z. B. Acetonitril, und anschliessender Zentrifugation der Überstand gewonnen. Die Konzentration des Wirkstoffes wird mittels HPLC bestimmt, beispielsweise auf einer Nucleosil® $5C_{18}$-Säule von 120 mm Länge und 4,6 mm Durchmesser mit entweder 60 % Acetonitril/40 % Wasser/0,05 % Trifluoressigsäure (v/v) oder 50 % Acetonitril/50 % Wasser/0,05 % Trifluoressigsäure (v/v) als Laufmittel bei einer Flussrate von 1 ml/min und Detektion und Quantifikation bei 200 nm.

Bei den im folgenden genannten Gruppen von Verbindungen der Formel I können in sinnvoller Weise, z. B. zur Ersetzung allgemeinerer durch speziellere Definitionen, Definitionen von Resten aus den oben genannten allgemeinen Definitionen eingesetzt werden oder Definitionen aus den anderen Gruppen eingefügt oder weggelassen werden.

Eine bevorzugte Variante der Erfindung bezieht sich auf die Verbindungen der Formel I, worin $R_1$ Wasserstoff, Niederalkoxycarbonyl, Heterocyclylcarbonyl, Benzyloxycarbonyl, welches unsubstituiert oder durch bis zu drei unabhängig voneinander aus fluor, Haloniederalkyl, Niederalkanoyl, Sulfo, Niederalkylsulfonyl und Cyano ausgewählte Reste substituiert ist, oder Heterocyclyloxycarbonyl, worin Heterocyclyl über ein Kohlenstoffatom gebunden ist, bedeutet, oder einen der genannten Carbonylreste, worin die bindende Carbonylgruppe durch eine Thiocarbonylgruppe ersetzt ist, $B_1$ eine Bindung oder ein bivalentes Radikal einer α-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl, wobei diese Reste unsubstituiert oder durch ein bis drei unabhängig aus Hydroxy, Methoxy, Halo, Haloniederalkyl, Sulfo, Niederalkylsulfonyl, Cyano und Nitro ausgewählte Reste substituiert sind, bedeuten, $A_1$ eine Bindung zwischen -C=O und $A_2$ oder ein bivalentes Radikal einer α-Aminosäure bedeutet, welches N-terminal mit der Gruppe -C=O und C-terminal mit $A_2$ verbunden ist, $A_2$ ein bivalentes Radikal einer α-Aminosäure bedeutet, welches N-terminal mit $A_1$ und C-terminal mit der Gruppe $NR_4R_5$ verbunden ist, oder $A_1$ und $A_2$ zusammen ein bivalentes Radikal eines Dipeptides bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom unsubstituiertes oder substituiertes Thiomorpholino oder Morpholino bedeuten; und alternativ oder ergänzend hierzu die Verbindungen der Formel I, worin $R_1$ Heterocyclylsulfonyl, Niederalkylsulfonyl oder N-(Heterocyclylniederalkyl)-N-niederalkyl-aminocarbonyl bedeutet, und die übrigen Reste die genannten Bedeutungen haben; und Salze dieser Verbindungen, sofern salzbildende Gruppen vorliegen; wobei die Hydroxygruppe in Verbindungen der Formel I an dem Kohlenstoffatom, welches dem Kohlenstoffatom benachbart ist, das den Rest $R_2$-$CH_2$- trägt, frei oder in geschützter Form vorliegt, insbesondere geschützt als physiologisch spaltbarer Ester, z. B. als Niederalkanoyloxy, wie Acetyloxy, wobei sowohl die freien Verbindungen der Formel I als auch die geschützte Form, in denen alle weiteren Reste die genannten Bedeutungen haben, oder deren Salze, besonders bevorzugt sind. Besonders hervorzuheben sind hier die Verbindungen, worin $A_1$ und $A_2$ jeweils ein bivalentes Radikal einer α-Aminosäure bedeuten und die übrigen Reste die genannten Bedeutungen haben, oder Salze davon.

Bevorzugt sind auch Verbindungen der Formel I, worin $R_1$ Wasserstoff, Niederalkoxycarbonyl, Heterocyclylcarbonyl, Benzyloxycarbonyl, welches unsubstituiert oder durch bis zu drei unabhängig voneinander aus Fluor, Haloniederalkyl, Niederalkanoyl, Sulfo, Niederalkylsulfonyl und Cyano ausgewählte Reste substituiert ist, oder Heterocyclyloxycarbonyl, worin Heterocyclyl über ein Kohlenstoffatom gebunden ist, bedeutet, oder einen der genannten Carbonylreste, worin die bindende Carbonylgruppe durch eine Thiocarbonylgruppe ersetzt ist, $B_1$ eine Bindung oder ein bivalentes Radikal einer α-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl bedeuten, wobei diese Reste unsubstituiert oder durch ein bis drei unabhängig aus Halo, Haloniederalkyl, Sulfo, Niederalkylsulfonyl, Cyano und Nitro ausgewählte Reste substituiert sind, $A_1$ eine Bindung zwischen -C=O und $A_2$ bildet oder ein bivalentes Radikal einer α-Aminosäure bedeutet, welches N-terminal mit der Gruppe -C=O und C-terminal mit $A_2$ verbunden ist, $A_2$ ein bivalentes Radikal einer α-Aminosäure bedeutet, welches N-terminal mit $A_1$ und C-terminal mit der Gruppe $NR_4R_5$ verbunden ist, oder $A_1$ und $A_2$ zusammen ein bivalentes Radikal eines Dipeptides bilden, dessen zentrale Amidbindung reduziert ist und das N-terminal mit der Gruppe -C=O und C-terminal mit der Gruppe $NR_4R_5$ verbunden ist, und und $R_4$ und $R_5$ gemeinsam mit dem bindenden Stickstoffatom unsubstituiertes oder substituiertes Morpholino bedeuten; und alternativ oder ergänzend hierzu die Verbindungen der Formel I, worin $R_1$ Heterocyclylsulfonyl, Niederalkylsulfonyl oder N-(Heterocyclylniederalkyl)-N-niederalkyl-aminocarbonyl bedeutet, und die übrigen Reste die genannten Bedeutungen haben; und Salze dieser Verbindungen, sofern salzbildende Gruppen vorliegen; wobei die Hydroxygruppe in Verbindungen der Formel I an dem Kohlenstoffatom, welches dem Kohlenstoffatom benachbart ist, das den Rest $R_2$-$CH_2$- trägt, frei oder in geschützter Form vorliegt, insbesondere geschützt als phyiologisch spaltbarer Ester, z. B. als Niederalkanoyloxy, wie Acetyloxy, wobei sowohl die freien Verbindungen der Formel I als auch die geschützte Form, in denen alle weiteren Reste die genannten Bedeutungen haben, oder deren Salze, besonders bevorzugt sind.

Bevorzugt sind auch Verbindungen der Formel I, worin wenigstens einer der Reste $R_2$ und $R_3$ durch einen bis zu drei aus Hydroxy, Methoxy, Halo, Haloniederalkyl, Sulfo, Niederalkylsulfonyl, Cyano und Nitro ausgewählte Reste substituiert ist, und die Reste $R_1$, $B_1$, $A_1$, $A_2$ und $NR_4R_5$ die in den beiden letzten Absätzen genannten Bedeutungen haben, und Salze davon, sofern salzbildende Gruppen vorliegen.

Stärker bevorzugt sind die Verbindungen der Formel I, worin $R_1$ Wasserstoff, tert-Butoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, mit bis zu drei unabhängig voneinander aus Fluor, Haloniederalkyl, Niederalkanoyl, Sulfo, Niederalkylsulfonyl und Cyano ausgewählten Resten substituiertes Benzyloxycarbonyl oder Heterocyclyloxycarbonyl bedeutet, worin Heterocyclyl über ein Kohlenstoffatom gebunden ist und ausgewählt ist aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, ß-Carbolinyl und einem ganz oder teilweise gesättigten Derivat dieser Reste, oder worin die Bedeutung Heterocyclyloxycarbonyl für $R_1$ fehlt, $B_1$ eine Bindung oder ein zweiwertiges Radikal einer $\alpha$-Aminosäure bedeutet, welches N-terminal mit $R_1$ und C-terminal mit der Aminogruppe am $R_2$-$CH_2$- tragenden Kohlenstoffatom verbunden ist, bevorzugt den Rest einer hydrophoben Aminosäure, z. B. Prolin, Phenylalanin, p-Fluorphenylalanin, Phenylglycin, $\alpha$-Naphthylalanin, Cyclohexylalanin, Cyclohexylglycin oder einer aliphatischen $\alpha$-Aminosäure ausgewählt aus Glycin, Valin, Norvalin, Alanin, Leucin, Norleucin und Isoleucin, insbesondere Valin, wobei vorzugsweise jede der genannten $\alpha$-Aminosäuren in der D-, L- oder (D,L)-, vorzugsweise in der L-Form vorliegt, bedeutet, wobei vorzugsweise jede der genannten Aminosäuren mit einem der unter $R_1$ genannten Reste ausgewählt aus Wasserstoff, N-tert-Butoxycarbonyl oder Morpholinocarbonyl substituiert ist, $R_2$ und $R_3$ unabhängig voneinander Phenyl oder Cyclohexyl, wobei diese Reste unsubstituiert oder durch ein bis zwei unabhängig voneinander aus Hydroxy, Methoxy, Fluor, Sulfo, Niederalkylsulfonyl, Trifluormethyl und Cyano ausgewählte Reste substituiert sind, wie oben in den allgemeinen Definitionen gezeigt, bedeuten, $A_1$ ein bivalentes Radikal einer hydrophoben $\alpha$-Aminosäure, wie oben unter den allgemeinen Definitionen gezeigt, bedeutet, welches N-terminal mit der Gruppe -C=O und C-terminal mit $A_2$ verbunden ist, $A_2$ ein bivalentes Radikal einer hydrophoben $\alpha$-Aminosäure, vorzugsweise wie oben unter den allgemeinen Definitionen definiert, bedeutet, welches N-terminal mit $A_1$ verbunden ist und C-terminal mit dem Rest $NR_4R_5$ verbunden ist, wobei die genannten Aminosäurereste in der (D)oder (L)-Form, vorzugsweise, ausser Phenylalanin, das in der (L)- oder der (D)-Form vorliegt, in der (L)-Form vorliegen, insbesondere $A_1$ und $A_2$ ein bivalentes Radikal eines Dipeptides der Formel Val-Phe, Ile-Phe, Val-Cha, Ile-Cha, Ile-Gly, Val-Val, Val-Gly, Val-(p-F-Phe), Val-Tyr, Val-(p-$CH_3O$-Phe) oder Gly-(p-F-Phe) bilden, worin die Aminosäuren in der (D)- oder (L)-, insbesondere der (L)-Form vorliegen mit Ausnahme von (L)-Val-Phe, in dem Phe in der (L)- oder (D)-Form vorliegt; oder $A_1$ und $A_2$ zusammen ein bivalentes Radikal eines Dipeptides aus zwei, vorzugsweise der oben unter den allgemeinen Definitionen genannten, hydrophoben $\alpha$-Aminosäuren bilden, dessen zentrale Amidbindung reduziert ist und das N-terminal mit der Gruppe -C=O und C-terminal mit der Gruppe $NR_4R_5$ verbunden ist, wie in den allgemeinen Definitionen aufgeführt, z. B. mit der Formel Val(red)-Phe, und $R_4$ und $R_5$ gemeinsam mit dem bindenden Stickstoffatom Thiomorpholino oder Morpholino, insbesondere Morpholino, bedeuten; und alternativ oder ergänzend hierzu die Verbindungen der Formel I, worin $R_1$ Morpholinosulfonyl oder N-(2-Pyridylmethyl)-N-methylaminocarbonyl bedeutet und die übrigen Reste die genannten Bedeutungen haben; und die pharmazeutisch verwendbaren Salze dieser Verbindungen, sofern salzbildende Gruppen vorliegen; wobei die Hydroxygruppe in Verbindungen der Formel I an dem Kohlenstoffatom, welches dem Kohlenstoffatom benachbart ist, das den Rest $R_2$-$CH_2$- trägt, frei oder in durch Niederalkanoyl geschützter Form vorliegt, insbesondere in freier Form; und wobei bei der Definition von $R_1$ Heterocyclyloxycarbonyl auch wegfallen kann.

Stärker bevorzugt sind auch die Verbindungen der Formel I, worin $R_1$ Wasserstoff, tert-Butoxycarbonyl, Isobutyloxycarbonyl, Pyridin-3-carbonyl, Morpholinocarbonyl, 3-Benzofuranoyl, 1,2,3,4-Tetrahydroisochinolin-3-carbonyl, mit bis zu drei unabhängig voneinander aus Fluor, Haloniederalkyl, Niederalkanoyl, Sulfo, Niederalkylsulfonyl und Cyano ausgewählten Resten substituiertes Benzyloxycarbonyl oder Heterocyclyloxycarbonyl bedeutet, worin Heterocyclyl über ein Kohlenstoffatom gebunden ist und ausgewählt ist aus Pyrrolyl, Thienyl, Imidazolyl, Pyrazolyl, Oxazolyl, Thiazolyl, Pyrazinyl, Pyrimidinyl, Indolyl, Chinolyl,

Isochinolyl, Chinoxalinyl, β-Carbolinyl und einem ganz oder teilweise gesättigten Derivat dieser Reste, oder worin die Bedeutung Heterocyclyloxycarbonyl für $R_1$ fehlt, $B_1$ eine Bindung oder ein zweiwertiges Radikal einer α-Aminosäure bedeutet, welches N-terminal mit $R_1$ und C-terminal mit der Aminogruppe am $R_2$-$CH_2$- tragenden Kohlenstoffatom verbunden ist, bevorzugt den Rest einer hydrophoben Aminosäure, z. B. Prolin, Phenylalanin, p-Fluorphenylalanin, Phenylglycin, α-Naphthylalanin, Cyclohexylalanin, Cyclohexylglycin oder einer aliphatischen α-Aminosäure ausgewählt aus Glycin, Valin, Norvalin, Alanin, Leucin, Norleucin und Isoleucin, insbesondere Valin, wobei vorzugsweise jede der genannten α-Aminosäuren in der D-, L- oder (D,L)-, vorzugsweise in der L-Form vorliegt, bedeutet, wobei vorzugsweise jede der genannten Aminosäuren mit einem der unter $R_1$ genannten Reste ausgewählt aus Wasserstoff, N-tert-Butoxycarbonyl oder Morpholinocarbonyl substituiert ist, $R_2$ und $R_3$ unabhängig voneinander Phenyl oder Cyclohexyl, wobei diese Reste unsubstituiert oder durch ein bis zwei unabhängig voneinander aus Fluor, Sulfo, Niederalkylsulfonyl und Cyano ausgewählte Reste substituiert sind, wie oben in den allgemeinen Definitionen gezeigt, bedeuten, $A_1$ ein bivalentes Radikal einer hydrophoben α-Aminosäure, wie oben unter den allgemeinen Definitionen gezeigt, bedeutet, welches N-terminal mit der Gruppe -C=O und C-terminal mit $A_2$ verbunden ist, $A_2$ ein bivalentes Radikal einer hydrophoben α-Aminosäure, vorzugsweise wie oben unter den allgemeinen Definitionen definiert, bedeutet, welches N-terminal mit $A_1$ verbunden ist und C-terminal mit dem Rest $NR_4R_5$ verbunden ist, wobei die genannten Aminosäurereste in der (D)- oder (L)-Form, vorzugsweise, ausser Phenylalanin, das in der (L)- oder der (D)-Form vorliegt, in der (L)-Form vorliegen, insbesondere $A_1$ und $A_2$ ein bivalentes Radikal eines Dipeptides der Formel Val-Phe, Ile-Phe, Val-Cha, Ile-Cha, Ile-Gly, Val-Val, Val-Gly, Val-(p-F-Phe), Val-(p-$CH_3$O-Phe) oder Gly-(p-F-Phe) bilden, worin die Aminosäuren in der (D)- oder (L)-, insbesondere der (L)-Form vorliegen mit Ausnahme von (L)-Val-Phe, in dem Phe in der (L)- oder (D)-Form vorliegt; oder $A_1$ und $A_2$ zusammen ein bivalentes Radikal eines Dipeptides, vorzugsweise aus zwei der oben unter den allgemeinen Definitionen genannten hydrophoben α-Aminosäuren, bilden, dessen zentrale Amidbindung reduziert ist und das N-terminal mit der Gruppe -C=O und C-terminal mit der Gruppe $NR_4R_5$ verbunden ist, wie in den allgemeinen Definitionen aufgeführt, z. B. mit der Formel Val(red)-Phe, und $R_4$ und $R_5$ gemeinsam mit dem bindenden Stickstoffatom Thiomorpholino oder Morpholino, insbesondere Morpholino, bedeuten; und alternativ oder ergänzend hierzu die Verbindungen der Formel I, worin $R_1$ Morpholinosulfonyl oder N-(2-Pyridylmethyl)-N-methyl-aminocarbonyl bedeutet; und die pharmazeutisch verwendbaren Salze dieser Verbindungen, sofern salzbildende Gruppen vorliegen; wobei die Hydroxygruppe in Verbindungen der Formel I an dem Kohlenstoffatom, welches dem Kohlenstoffatom benachbart ist, das den Rest $R_2$-$CH_2$- trägt, frei oder in durch Niederalkanoyl geschützter Form vorliegt, insbesondere in freier Form; und wobei bei der Definition von $R_1$ Heterocyclyloxycarbonyl auch wegfallen kann.

Sehr bevorzugt sind die Verbindungen der Formel I, worin $R_1$ Wasserstoff, tert-Butoxycarbonyl, Isobutyloxycarbonyl, Pyridin-3-carbonyl, Morpholinocarbonyl, 3-Benzofuranoyl oder 1,2,3,4-Tetrahydro-isochinolin-3-carbonyl bedeutet; oder alternativ und ergänzend hierzu Morpholinosulfonyl oder N-(2-Pyridylmethyl)-N-methyl-aminocarbonyl bedeutet, $B_1$ eine Bindung oder ein bivalentes Radikal der α-Aminosäure Valin bedeutet, welches N-terminal mit $R_1$ und C-terminal mit der Aminogruppe am $R_2$-$CH_2$-tragenden Kohlenstoffatom verbunden ist, wobei im letzteren Falle $R_1$ bevorzugt Wasserstoff, tert-Butoxycarbonyl oder Morpholinocarbonyl ist, oder alternativ oder ergänzend hierzu Morpholinosulfonyl oder N-(2-Pyridylmethyl)-N-methyl-aminocarbonyl ist, $R_2$ und $R_3$ unabhängig voneinander Phenyl oder Cyclohexyl bedeuten, wobei diese Reste unsubstituiert oder durch ein bis zwei unabhängig voneinander aus Hydroxy, Methoxy, Fluor, Sulfo, Niederalkylsulfonyl, Cyano und Trifluormethyl ausgewählte Reste subsrituiert sind, $A_1$ ein bivalentes Radikal einer der α-Aminosäuren Glycin, Valin oder Isoleucin bedeutet, welches N-terminal mit der Gruppe -C=O und C-terminal mit $A_2$ verbunden ist, $A_2$ ein bivalentes Radikal einer der α-Aminosäuren Glycin, Valin, Phenylalanin, Tyrosin, Cyclohexylalanin, p-Methoxy-phenylalanin oder p-Fluorphenylalanin bedeutet, welches N-terminal mit $A_1$ und C-terminal mit der Gruppe $NR_4R_5$ verbunden ist, oder ferner $A_1$ und $A_2$ zusammen ein bivalentes Radikal eines Dipeptides mit reduzierter zentraler Peptidbindung bedeuten, welches aus einem N-terminalen Aminosäureradikal ausgewählt aus Gly(red), Val(red) oder Ile(red) und einem C-terminalen Aminosäureradikal ausgewählt aus Glycin, Phenylalanin, Cyclohexylalanin, Tyrosin, p-Methoxy-phenylalanin oder p-Fluorphenylalanin besteht, und welches N-terminal mit der Gruppe -C=O und C-terminal mit der Gruppe $NR_4R_5$ verbunden ist, wie es oben für $A_1$ und $A_2$ definiert ist, und $R_4$ und $R_5$ gemeinsam mit dem bindenden Stickstoffatom Thiomorpholino oder Morpholino bedeutet, insbesondere Morpholino, und die pharmazeutisch verwendbaren Salze dieser Verbindungen, sofern salzbildende Gruppen vorliegen.

Sehr bevorzugt sind auch die Verbindungen der Formel I, worin $R_1$ Wasserstoff, tert-Butoxycarbonyl, Isobutyloxycarbonyl, Pyridin-3-carbonyl, Morpholinocarbonyl, 3-Benzofuranoyl oder 1,2,3,4-Tetrahydro-isochinolin-3-carbonyl bedeutet; oder alternativ und ergänzend hierzu Morpholinosulfonyl oder N-(2-Pyridylmethyl)-N-methyl-aminocarbonyl bedeutet, $B_1$ eine Bindung oder ein bivalentes Radikal der α-Aminosäure Valin bedeutet, welches N-terminal mit $R_1$ und C-terminal mit der Aminogruppe am $R_2$-$CH_2$- tragenden Kohlenstoffatom verbunden ist, wobei im letzteren Falle $R_1$ bevorzugt Wasserstoff, tert-Butoxycarbonyl oder Morpholinocarbonyl ist, oder alternativ oder ergänzend hierzu Morpholinosulfonyl oder N-(2-Pyridylmethyl)-N-methyl-aminocarbonyl ist, $R_2$ und $R_3$ unabhängig voneinander Phenyl oder Cyclohexyl bedeuten, wobei diese Reste unsubstituiert oder durch ein bis zwei unabhängig voneinander aus Fluor, Sulfo, Niederalkylsulfonyl und Cyano, und alternativ oder ergänzend hierzu aus Hydroxy, Methoxy und Trifluormethyl, ausgewählte Reste substituiert sind, $A_1$ ein bivalentes Radikal einer der α-Aminosäuren Glycin, Valin oder Isoleucin bedeutet, welches N-terminal mit der Gruppe -C=O und C-terminal mit $A_2$ verbunden ist, $A_2$ ein bivalentes Radikal einer der α-Aminosäuren Glycin, Valin, Phenylalanin, Tyrosin, Cyclohexylalanin, p-Methoxy-phenylalanin oder p-Fluorphenylalanin bedeutet, welches N-terminal mit $A_1$ und C-terminal mit der Gruppe $NR_4R_5$ verbunden ist, oder ferner $A_1$ und $A_2$ zusammen ein bivalentes Radikal eines Dipeptides mit reduzierter zentraler Peptidbindung bedeuten, welches aus einem N-terminalen Aminosäureradikal ausgewählt aus Gly(red), Val(red) oder Ile(red) und einem C-terminalen Aminosäureradikal ausgewählt aus Glycin, Phenylalanin, Tyrosin, Cyclohexylalanin, p-Methoxy-phenylalanin oder p-Fluorphenylalanin besteht, und welches N-terminal mit der Gruppe -C=O und C-terminal mit der Gruppe $NR_4R_5$ verbunden ist, wie es oben für $A_1$ und $A_2$ definiert ist, und $R_4$ und $R_5$ gemeinsam mit dem bindenden Stickstoffatom Thiomorpholino oder Morpholino bedeutet, insbesondere Morpholino, und die pharmazeutisch verwendbaren Salze dieser Verbindungen, sofern salzbildende Gruppen vorliegen.

Noch bevorzugter sind die Verbindungen der Formel I gemäss den bisher gemachten Definitionen, worin $B_1$ eines der genannten bivalenten Radikale einer α-Aminosäure bedeutet und einer der Reste $A_1$ oder $A_2$ eine Bindung bedeutet und der andere eine der genannten α-Aminosäuren bedeutet, oder Verbindungen der Formel I, worin $B_1$ eine Bindung bedeutet und $A_1$ und $A_2$ jeweils eines der genannten bivalenten Radikale einer α-Aminosäure oder gemeinsam eines der genannten bivalenten Radikale eines Dipeptides mit reduzierter zentraler Amidbindung bedeuten, wobei die übrigen Reste die genannten Bedeutungen haben.

Noch bevorzugter sind auch die Verbindungen der Formel I gemäss den bisher gemachten Definitionen, worin $B_1$ eine Bindung oder eines der genannten bivalenten Radikale einer α-Aminosäure bedeutet und $A_1$ und $A_2$ jeweils das bivalente Radikal einer der genannten Aminosäuren bedeutet, wobei die übrigen Reste die genannten Bedeutungen haben, oder die pharmazeutisch annehmbaren Salze dieser Verbindungen, sofern mindestens eine salzbildende Gruppe vorliegt.

In erster Linie betroffen sind die Verbindungen der Formel I, worin $R_1$ Wasserstoff, tert-Butoxycarbonyl, Isobutyloxycarbonyl, Pyridin-3-carbonyl, Morpholinocarbonyl, 3-Benzofuranoyl oder 1,2,3,4-Tetrahydroisochinolin-3-carbonyl bedeutet, oder alternativ oder ergänzend hierzu Morpholinosulfonyl oder N-(2-Pyridylmethyl)-N-methyl-aminocarbonyl bedeutet, $B_1$ eine Bindung oder ein bivalentes Radikal der α-Aminosäure Valin bedeutet, welches N-terminal mit $R_1$ und C-terminal mit der Aminogruppe am $R_2$-$CH_2$-tragenden Kohlenstoffatom verbunden ist, wobei $R_1$ im letzteren Falle bevorzugt Wasserstoff, tert-Butoxycarbonyl oder Morpholinocarbonyl bedeutet oder alternativ oder ergänzend hierzu Morpholinosulfonyl oder N-(2-Pyridylmethyl)-N-methyl-aminocarbonyl bedeutet, $R_2$ und $R_3$ unabhängig voneinander Phenyl oder Cyclohexyl bedeuten, wobei diese Reste unsubstituiert oder durch ein bis zwei unabhängig voneinander aus Hydroxy, Methoxy, Fluor und Cyano ausgewählte Reste substituiert sind, insbesondere durch einen der genannten Reste, vorzugsweise in 4-Stellung, z. B. in 4-Hydroxyphenyl, 4-Methoxyphenyl, 4-Fluorphenyl, 4-Cyanophenyl oder 4-Fluorcyclohexyl, wie in den Kombinationen von $R_2$ und $R_3$, die unter den allgemeinen Definitionen oben als in erster Linie bevorzugt genannt sind, oder alternativ oder ergänzend hierzu $R_2$ und $R_3$ unabhängig voneinander Phenyl oder Cyclohexyl, welches unsubstituiert oder durch ein bis zwei unabhängig voneinander aus Trifluormethyl, Cyano und Fluor ausgewählte Reste substituiert ist, insbesondere durch einen dieser Reste, vorzugsweise in 4-Stellung, z. B. in 4-Trifluormethylphenyl, 4-Cyanophenyl oder 4-Fluorphenyl, bedeuten, $A_1$ und $A_2$ zusammen ein bivalentes Radikal eines Dipeptides der Formel Val-Phe, Ile-Phe, Val-Cha, Ile-Cha, Ile-Gly, Val-Val, Val-Gly, Val-(p-F-Phe), Val-Tyr, Val-(p-$CH_3$O-Phe), Gly-(p-F-Phe) oder eines Derivates hiervon mit reduzierter zentraler Amidbindung der Formel Val(red)-Phe bilden, das N-terminal mit der Gruppe -C=O und C-terminal mit der Gruppe $NR_4R_5$ verbunden ist, und $R_4$ und $R_5$ gemeinsam mit dem bindenden Stickstoffatom Thiomorpholino oder Morpholino, insbesondere Morpholino, bedeuten, und die pharmazeutisch verwendbaren Salze dieser Verbindungen, sofern salzbildende Gruppen vorliegen, wobei die die Hydroxygruppe in Verbindungen der Formel I an dem Kohlenstoffatom, welches dem Kohlenstoffatom benachbart ist, das den Rest $R_2$-$CH_2$-trägt, frei oder in durch Acetyl geschützter Form vorliegt, wobei sowohl die freien Verbindungen der Formel I als auch die geschützte Form, worin alle weiteren Reste die genannten Bedeutungen haben, oder deren Salze, besonders bevorzugt sind.

In erster Linie betroffen sind auch die Verbindungen der Formel I, worin $R_1$ Wasserstoff, tert-Butoxycarbonyl, Isobutyloxycarbonyl, Pyridin-3-carbonyl, Morpholinocarbonyl, 3-Benzofuranoyl oder 1,2,3,4-Tetrahydro-isochinolin-3-carbonyl bedeutet, oder alternativ oder ergänzend hierzu Morpholinosulfonyl oder N-(2-Pyridylmethyl)-N-methyl-aminocarbonyl bedeutet, $B_1$ eine Bindung oder ein bivalentes Radikal der α-Aminosäure Valin bedeutet, welches N-terminal mit $R_1$ und C-terminal mit der Aminogruppe am $R_2$-$CH_2$-tragenden Kohlenstoffatom verbunden ist, wobei $R_1$ im letzteren Falle bevorzugt Wasserstoff, tert-Butoxycarbonyl oder

Morpholinocarbonyl bedeutet oder alternativ oder ergänzend hierzu Morpholinosulfonyl oder N-(2-Pyridylmethyl)-N-methyl-aminocarbonyl bedeutet, $R_2$ und $R_3$ unabhängig voneinander Phenyl oder Cyclohexyl bedeuten, wobei diese Reste unsubstituiert oder durch ein bis zwei unabhängig voneinander aus Fluor und Cyano ausgewählte Reste substituiert sind, insbesondere durch einen der genannten Reste, vorzugsweise in 4-Stellung, z. B. in 4-Fluorphenyl, 4-Cyanophenyl oder 4-Fluorcyclohexyl, wie in den Kombinationen von $R_2$ und $R_3$, die unter den allgemeinen Definitionen oben als in erster Linie bevorzugt genannt sind, oder alternativ oder ergänzend hierzu $R_2$ und $R_3$ unabhängig voneinander Phenyl oder Cyclohexyl, welches unsubstituiert oder durch ein bis zwei unabhängig voneinander aus Trifluormethyl, Cyano und fluor ausgewählte Reste substituiert ist, insbesondere durch einen dieser Reste, vorzugsweise in 4-Stellung, z. B. in 4-Trifluormethylphenyl, 4-Cyanophenyl oder 4-Fluorphenyl, bedeuten, $A_1$ und $A_2$ zusammen ein bivalentes Radikal eines Dipeptides der Formel Val-Phe, Ile-Phe, Val-Cha, Ile-Cha, Ile-Gly, Val-Val, Val-Gly, Val-(p-F-Phe), Val-(p-$CH_3O$-Phe), Gly-(p-F-Phe) oder eines Derivates hiervon mit reduzierter zentraler Amidbindung der Formel Val(red)-Phe bilden, das N-terminal mit der Gruppe -C=O und C-terminal mit der Gruppe $NR_4R_5$ verbunden ist, und $R_4$ und $R_5$ gemeinsam mit dem bindenden Stickstoffatom Thiomorpholino oder Morpholino, insbesondere Morpholino, bedeuten, und die pharmazeutisch verwendbaren Salze dieser Verbindungen, sofern salzbildende Gruppen vorliegen, wobei die die Hydroxygruppe in Verbindungen der Formel I an dem Kohlenstoffatom, welches dem Kohlenstoffatom benachbart ist, das den Rest $R_2$-$CH_2$- trägt, frei oder in durch Acetyl geschützter Form vorliegt, wobei sowohl die freien Verbindungen der Formel I als auch die geschützte Form, worin alle weiteren Reste die genannten Bedeutungen haben, oder deren Salze, besonders bevorzugt sind.

In allererster Linie betroffen sind die in den Beispielen genannten Verbindungen und die Salze dieser Verbindungen, insbesondere die pharmazeutisch verwendbaren Salze, sofern salzbildende Gruppen vorliegen. Hierzu gehören die Verbindungen der Formel I mit den Bezeichnungen
Boc-Cha[C](p-F)Phe-(L)-Ile-(L)-Phe-morpholin-4-ylamid,
Boc-(p-F)Phe[C](p-F)Phe-(L)-Val-(L)-Phe-morpholin-4-ylamid,
Boc-(p-F)Phe[C](p-F)Phe-(L)-Val-(L)-(p-F-Phe)-morpholin-4-ylamid,
Boc-(p-F)Phe[C](p-F)Phe-(L)-Val-(L)-(p-$CH_3O$-Phe)-morpholin-4-ylamid,
Boc-(p-F)Phe[C](p-F)Phe-(L)-Val-(L)-Cha-morpholin-4-ylamid,
Boc-(p-F)Phe[C](p-F)Phe-(L)-ne-(L)-Phe-morpholin-4-ylamid,
Boc-(p-F)Phe[C](p-CN)Phe-(L)-Val-(L)-Phe-morpholin-4-ylamid,
Boc-(p-F)Phe[C](p-CN)Phe-(L)-Val-(L)-(p-F-Phe)-morpholin-4-ylamid,
Boc-(p-F)Phe[C](p-CN)Phe-(L)-Val-(L)-(p-$CH_3O$-Phe)-morpholin-4-ylamid,
Boc-(p-F)Phe[C](p-CN)Phe-(L)-Val-(L)-Cha-morpholin-4-ylamid,
Boc-(p-F)Phe[C](p-CN)Phe-(L)-Ile-(L)-Phe-morpholin-4-ylamid,
Boc-Phe[C](p-F)Phe-(L)-Val-(L)-Phe-morpholin-4-ylamid,
Boc-Phe[C](p-F)Phe-(L)-Val-(L)-(p-F-Phe)-morpholin-4-ylamid,
Boc-Phe[C](p-F)Phe-(L)-Val-(L)-(p-$CH_3O$-Phe)-morpholin-4-ylamid,
Boc-Phe[C](p-F)Phe-(L)-Val-(L)-Cha-morpholin-4-ylamid,
Boc-Phe[C](p-F)Phe-(L)-Ile-(L)-Phe-morpholin-4-ylamid,
Boc-Phe[C](p-CN)Phe-(L)-Val-(L)-Phe-morpholin-4-ylamid,
Boc-Phe[C](p-CN)Phe-(L)-Val-(L)-(p-F-Phe)-morpholin-4-ylamid,
Boc-Phe[C](p-CN)Phe-(L)-Val-(L)-(p-$CH_3O$-Phe)-morpholin-4-ylamid,
Boc-Phe[C](p-CN)Phe-(L)-Val-(L)-Cha-morpholin-4-ylamid,
Boc-Phe[C](p-CN)Phe-(L)-Ile-(L)-Phe-morpholin-4-ylamid,
Boc-Phe[C](p-$CH_3O$)Phe-(L)-Val-(L)-Phe-morpholin-4-ylamid,
Boc-Phe[C](p-$CH_3O$)Phe-(L)-Val-(L)-(p-F-Phe)-morpholin-4-ylamid,
Boc-Phe[C](p-$CH_3O$)Phe-(L)-Val-(L)-(p-$CH_3O$-Phe)-morpholin-4-ylamid,
Boc-Phe[C](p-$CH_3O$)Phe-(L)-Val-(L)-Cha-morpholin-4-ylamid,
Boc-Phe[C](p-$CH_3O$)Phe-(L)-Ile-(L)-Phe-morpholin-4-ylamid,
Boc-Phe[C](p-$CF_3$)Phe-(L)-Val-(L)-Phe-morpholin-4-ylamid,
Boc-Phe[C](p-$CF_3$)Phe-(L)-Val-(L)-(p-F-Phe)-morpholin-4-ylamid,
Boc-Phe[C](p-$CF_3$)Phe-(L)-Val-(L)-(p-$CH_3O$-Phe)-morpholin-4-ylamid,
Boc-Phe[C](p-$CF_3$)Phe-(L)-Val-(L)-Cha-morpholin-4-ylamid,
Boc-Phe[C](p-$CF_3$)Phe-(L)-Ile-(L)-Phe-morpholin-4-ylamid,
Boc-Cha[C](p-CN)Phe-(L)-Val-(L)-Phe-morpholin-4-ylamid,
Boc-Cha[C](p-CN)Phe-(L)-Val-(L)-(p-F-Phe)-morpholin-4-ylamid,
Boc-Cha[C](p-CN)Phe-(L)-Val-(L)-(p-$CH_3O$-Phe)-morpholin-4-ylamid,
Boc-Cha[C](p-CN)Phe-(L)-Val-(L)-Cha-morpholin-4-ylamid,
Boc-Cha[C](p-CN)Phe-(L)-Ile-(L)-Phe-morpholin-4-ylamid,

Boc-Cha[C](p-$CH_3O$)Phe-(L)-Val-(L)-Phe-morpholin-4-ylamid,
Boc-Cha[C](p-$CH_3O$)Phe-(L)-Val-(L)-(p-F-Phe)-morpholin-4-ylamid,
Boc-Cha[C](p-$CH_3O$)Phe-(L)-Val-(L)-(p-$CH_3O$-Phe)-morpholin-4-ylamid,
Boc-Cha[C](p-$CH_3O$)Phe-(L)-Val-(L)-Cha-morpholin-4-ylamid,
Boc-Cha[C](p-$CH_3O$)Phe-(L)-Ile-(L)-Phe-morpholin-4-ylamid,
Boc-Cha[C](p-$CF_3$)Phe-(L)-Val-(L)-Phe-morpholin-4-ylamid,
Boc-Cha[C](p-$CF_3$)Phe-(L)-Val-(L)-(p-F-Phe)-morpholin-4-ylamid,
Boc-Cha[C](p-$CF_3$)Phe-(L)-Val-(L)-(p-$CH_3O$-Phe)-morpholin-4-ylamid,
Boc-Cha[C](p-$CF_3$)Phe-(L)-Val-(L)-Cha-morpholin-4-ylamid, oder
Boc-Cha[C](p-$CF_3$)Phe-(L)-Ile-(L)-Phe-morpholin-4-ylamid, oder die entsprechenden Verbindungen, worin anstelle von -morpholin-4-ylamid der Rest -thiomorpholin-4-ylamid steht.
Hierzu gehören auch die Verbindungen der Formel I gemäss Anspruch 1 mit den Bezeichnungen
Boc-(L)-Val-Phe[C]Phe-(L)-Val-(L)-Phe-morpholin-4-ylamid;
H-(L)-Val-Phe[C]Phe-(L)-Val-(L)-Phe-morpholin-4-ylamid;
Boc-Phe[C]Phe-(L)-Val-(D)-Phe-morpholin-4-ylamid;
Boc-Phe[C]Phe-(L)-Val(red)-(L)-Phe-morpholin-4-ylamid;
oder Isobutyloxycarbonyl-Phe[C]Phe-(L)-Val-(L)-Phe-morpholin-4-ylamid; oder die
entsprechenden Verbindungen, worin anstelle von -morpholin-4-ylamid der Rest -thiomorpholin-4-ylamid steht; oder Salze davon, sofern salzbilende Gruppen vorliegen;
oder die Verbindungen der Formel I gemäss Anspruch 1 mit den Bezeichnungen Boc-Cha[C] (p-CN)Phe-(L)-Val-(L)-Phe-thiomorpholin-4-ylamid;
oder Boc-Cha[C] (p-F)Phe-(L)-Val- (L)-Phe-thiomorpholin-4-ylamid; oder die Verbindungen der Formel I mit den Bezeichungen Boc-Cha[C](p-F)Phe-(L)-Ile-(L)-Phe-morpholin-4-ylamid,
Boc-Cha[C](p-F)Phe-(L)-Val-(L)-Cha-morpholin-4-ylamid,
Boc-(p-$CF_3$)Phe[C]Phe-(L)-Val-(L)-Phe-morpholin-4-ylamid,
Boc-(p-$CF_3$)Phe[C]Phe-(L)-Val-(L)-(p-F)Phe-morpholin-4-ylamid,
Boc-(p-$CF_3$)Phe[C]Phe-(L)-Val-(L)-(p-$CH_3O$)Phe-morpholin-4-ylamid,
Boc-(p-$CF_3$)Phe[C]Phe-(L)-Val-(L)-Cha-morpholin-4-ylamid,
Boc-(p-$CF_3$)Phe[C]Phe-(L)-Ile-(L)-Phe-morpholin-4-ylamid,
Boc-(p-$CF_3$)Phe[C](p-F)Phe-(L)-Val-(L)-Phe-morpholin-4-ylamid,
Boc-(p-$CF_3$)Phe[C](p-F)Phe-(L)-Val-(L)-(p-F)Phe-morpholin-4- ylamid,
Boc-(p-$CF_3$)Phe[C](p-F)Phe-(L)-Val-(L)-Cha-morpholin-4-ylamid,
Boc-(p-$CF_3$)Phe[C](p-F)Phe-(L)-Val-(L)-(p-$CH_3O$)Phe-morpholin-4-ylamid,
Boc-(p-$CF_3$)Phe[C](p-F)Phe-(L)-Ile-(L)-Phe-morpholin-4-ylamid,
Boc-(p-$CF_3$)Phe[C](p-$CF_3$)Phe-(L)-Val-(L)-Phe-morpholin-4-ylamid,
Boc-(p-$CF_3$)Phe[C](p-$CF_3$)Phe-(L)-Val-(L)-(p-F)Phe-morpholin-4-ylamid,
Boc-(p-$CF_3$)Phe[C](p-$CF_3$)Phe-(L)-Ile-(L)-Phe-morpholin-4-ylamid,
Boc-(p-$CF_3$)Phe[C](p-$CF_3$)Phe-(L)-Val-(L)-(p-$CH_3O$)Phe-morpholin-4-ylamid, oder
Boc-(p-$CF_3$)Phe[C](p-$CF_3$)Phe-(L)-Val-(L)-Cha-morpholin-4-ylamid; oder die entsprechenden Verbindungen, worin anstelle von -morpholin-4-ylamid der Rest -thiomorpholin-4-ylamid steht, oder die Verbindungen der Formel I mit den Bezeichnungen
Boc-Phe[C]Phe-(L)-Val-(L)-Tyr-morpholin-4-ylamid;
Boc-Tyr[C]Phe-(L)-Val-(L)-Phe-morpholin-4-ylamid;
Boc-Tyr[C]Phe-(L)-Val-(L)-Tyr-morpholin-4-ylamid;
Boc-Phe[C]Tyr-(L)-Val-(L)-Phe-morpholin-4-ylamid;
Boc-Phe[C]Tyr-(L)-Val-(L)-Tyr-morpholin-4-ylamid;
Boc-Tyr[C]Tyr-(L)-Val-(L)-Phe-morpholin-4-ylamid;
Boc-Tyr[C]Tyr-(L)-Val-(L)-Tyr-morpholin-4-ylamid, oder die entsprechenden Verbindungen, worin anstelle von -morpholin-4-ylamid der Rest-thiomorpholin-4-ylamid steht.

Ganz besonders wichtig ist die Verbindung der Formel I, worin $R_1$ tert-Butoxycarbonyl, $B_1$ eine Bindung, $R_2$ Cyclohexyl, $R_3$ p-Fluor-phenyl, $A_1$ Valin, $A_2$ Phenylalanin, und $R_4$ und $R_5$ zusammen mit dem bindenden Stickstoffatom Morpholino bedeuten.

Ganz besonders wichtig ist auch die Verbindung der Formel I, worin $R_1$ tert-Butoxycarbonyl, $B_1$ eine Bindung, $R_2$ und $R_3$ Phenyl, $A_1$ Valin, $A_2$ Phenylalanin, und $R_4$ und $R_5$ zusammen mit dem bindenden Stickstoffatom Morpholino bedeuten.

Ganz besonders wichtig ist auch die Verbindung der Formel I, worin $R_1$ tert-Butoxycarbonyl, $B_1$ eine Bindung, $R_2$ Cyclohexyl, $R_3$ p-Fluor-phenyl, $A_1$ Valin, $A_2$ p-Fluorphenylalanin, und $R_4$ und $R_5$ zusammen mit dem bindenden Stickstoffatom Morpholino bedeuten.

Ganz besonders wichtig ist auch die Verbindung der Formel I, worin $R_1$ tert-Butoxycarbonyl, $B_1$ eine Bindung, $R_2$ Cyclohexyl, $R_3$ p-Fluor-phenyl, $A_1$ Valin, $A_2$ p-Methoxyphenylalanin, und $R_4$ und $R_5$ zusammen mit dem bindenden Stickstoffatom Morpholino bedeuten.

Ganz besonders wichtig ist auch die Verbindung der Formel I, worin $R_1$ tert-Butoxycarbonyl, $B_1$ eine Bindung, $R_2$ Cyclohexyl, $R_3$ p-Fluor-phenyl, $A_1$ Valin, $A_2$ Cyclohexylalanin, und $R_4$ und $R_5$ zusammen mit dem bindenden Stickstoffatom Morpholino bedeuten

Ganz besonders wichtig ist auch die Verbindung der Formel I, worin $R_1$ tert-Butoxycarbonyl, $B_1$ eine Bindung, $R_2$ Cyclohexyl, $R_3$ p-Fluor-phenyl, $A_1$ Valin, $A_2$ Phenylalanin, und $R_4$ und $R_5$ zusammen mit dem bindenden Stickstoffatom Thiomorpholino bedeuten.

Ganz besonders wichtig ist auch die Verbindung der Formel I, worin $R_1$ tert-Butoxycarbonyl, $B_1$ eine Bindung, $R_2$ Cyclohexyl, $R_3$ p-Fluor-phenyl, $A_1$ Isoleucin, $A_2$ Phenylalanin, und $R_4$ und $R_5$ zusammen mit dem bindenden Stickstoffatom Morpholino bedeuten.

Ganz besonders wichtig ist auch die Verbindung der Formel I, worin $R_1$ tert-Butoxycarbonyl, $B_1$ eine Bindung, $R_2$ Phenyl, $R_3$ p-Fluor-phenyl, $A_1$ Valin, $A_2$ Phenylalanin, und $R_4$ und $R_5$ zusammen mit dem bindenden Stickstoffatom Morpholino bedeuten.

Ganz besonders wichtig ist auch die Verbindung der Formel I, worin $R_1$ tert-But-oxycarbonyl, $B_1$ eine Bindung, $R_2$ p-Fluor-phenyl, $R_3$ p-Fluor-phenyl, $A_1$ Valin, $A_2$ Phenylalanin, und $R_4$ und $R_5$ zusammen mit dem bindenden Stickstoffatom Morpholino bedeuten.

Ganz besonders wichtig ist auch die Verbindung der Formel I, worin $R_1$ tert-Butoxycarbonyl, $B_1$ eine Bindung, $R_2$ p-Fluor-phenyl, $R_3$ p-Fluor-phenyl, $A_1$ Valin, $A_2$ p-Fluorphenylalanin, und $R_4$ und $R_5$ zusammen mit dem bindenden Stickstoffatom Morpholino bedeuten.

Ganz besonders wichtig ist auch die Verbindung der Formel I, worin $R_1$ tert-Butoxycarbonyl, $B_1$ eine Bindung, $R_2$ Cyclohexyl, $R_3$ p-Cyano-phenyl, $A_1$ Valin, $A_2$ Phenylalanin und $R_4$ und $R_5$ gemeinsam mit dem bindenden Stickstoffatom Morpholino bedeuten.

Ganz besonders wichtig ist auch die Verbindung der Formel I, worin $R_1$ tertButoxycarbonyl, $B_1$ eine Bindung, $R_2$ und $R_3$ Phenyl, $A_1$ Valin, $A_2$ Phenylalanin, und $R_4$ und $R_5$ zusammen mit dem bindenden Stickstoffatom Thiomorpholino bedeuten.

Die Verbindungen der Formel I oder deren hydroxygeschützte Derivate und Salze von solchen Verbindungen mit mindestens einer salzbildenden Gruppe werden nach an sich bekannten Verfahren erhalten, z. B. indem man

a) zur Herstellung von Verbindungen der Formel

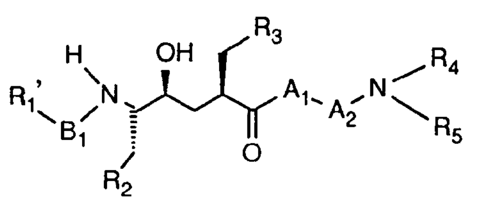

(Ib),

worin $R_1'$ die für Verbindungen der Formel I genannten Bedeutungen von $R_1$ ausser Wasserstoff hat, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I genannten Bedeutungen haben, eine Säure der Formel

$$R_1'\text{-OH} \qquad \text{(II)}$$

oder ein reaktionsfähiges Säurederivat davon, worin $R_1'$ ausser Wasserstoff dieselben Bedeutungen hat wie $R_1$ in Verbindungen der Formel I, mit einer Aminoverbindung der Formel

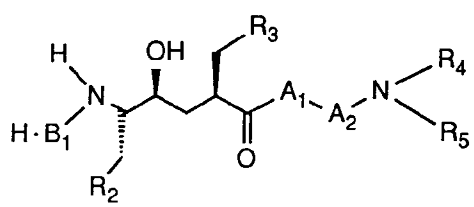

(III)

oder einem reaktionsfähigen Derivat hiervon, worin die Reste die für Verbindungen der Formel I genannten

Bedeutungen haben, kondensiert, wobei in den Ausgangsmaterialien der Formeln II und III freie funktionelle Gruppen mit Ausnahme der an der Reaktion beteiligten gegebenenfalls in geschützter Form vorliegen, und gewünschtenfalls vorhandene Schutzgruppen abspaltet, oder

b) zur Herstellung von Verbindungen der Formel

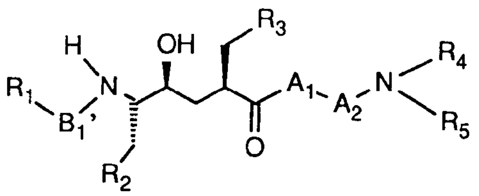

(Ic),

worin $B_1'$ dieselben Reste wie $B_1$ in Verbindungen der Formel I ausser einer Bindung bedeutet, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I genannten Bedeutungen haben, eine Carbonsäure der Formel

$$R_1\text{-}B_1'\text{-OH} \qquad \text{(IV)}$$

oder ein reaktionsfähiges Säurederivat davon, worin $R_1$ die für Verbindungen der Formel I genannten Bedeutungen hat und $B_1'$ die zuletzt genannten Bedeutungen hat, mit einer Aminoverbindung der Formel

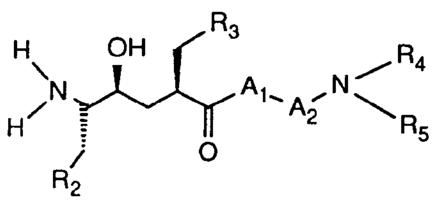

(V),

oder einem reaktionsfähigen Derivat davon, worin die Reste die für Verbindungen der Formel I genannten Bedeutungen haben, kondensiert, wobei freie funktionelle Gruppen in den Ausgangsmaterialien der Formeln IV und V mit Ausnahme der an der Reaktion beteiligten gegebenenfalls in geschützter Form vorliegen, und gewünschtenfalls vorhandene Schutzgruppen abspaltet, oder

c) eine Carbonsäure der Formel

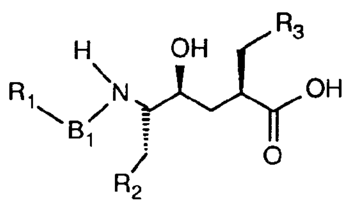

(VI),

oder ein reaktionsfähiges Derivat davon, worin die Reste die für Verbindungen der Formel I genannten Bedeutungen haben, mit einer Aminoverbindung der Formel

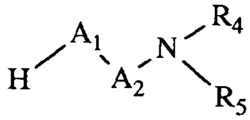

(VII)

oder einem reaktionsfähigen Derivat davon, worin die Reste die für Verbindungen der Formel I genannten Bedeutungen haben, kondensiert, wobei freie funktionelle Gruppen in den Ausgangsmaterialien der Formeln VI und VII mit Ausnahme der an der Reaktion beteiligten gegebenenfalls in geschützter Form vorliegen, und gewünschtenfalls vorhandene Schutzgruppen abspaltet, oder

d) zur Herstellung einer Verbindung der Formel

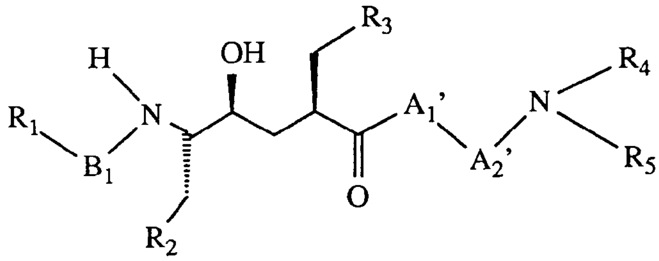

(Id),

worin $A_1'$ und $A_2'$ die Bedeutungen von $A_1$ und $A_2$ in Verbindungen der Formel I haben, wobei $A_1'$ jedoch keine Bindung bedeutet und die Peptidbindung zwischen $A_1'$ und $A_2'$ nicht in reduzierter Form vorliegt, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I genannten Bedeutungen haben, eine Carbonsäure der Formel

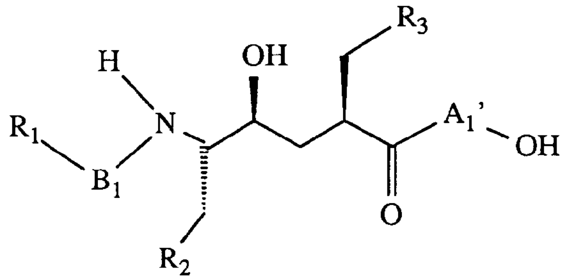

(VIII)

oder ein reaktionsfähiges Derivat davon, worin die Reste die zuletzt genannten Bedeutungen haben, mit einer Aminoverbindung der Formel

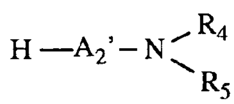

(IX)

oder einem reaktionsfähigen Derivat davon, worin die Reste die zuletzt genannten Bedeutungen haben, kondensiert, wobei freie funktionelle Gruppen in den Ausgangsmaterialien der Formeln VIII und IX mit Ausnahme der an der Reaktion beteiligten gegebenenfalls in geschützter Form vorliegen, und gewünschtenfalls vorhandene Schutzgruppen abspaltet, oder

e) eine Carbonsäure der Formel

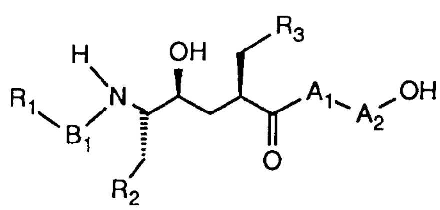

(X)

oder ein reaktionsfähiges Derivat davon, worin die Reste die für Verbindungen der Formel I genannten Bedeutung haben, mit einer Aminoverbindung der Formel

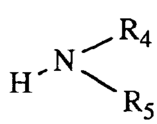

(XI)

oder einem reaktionsfähigen Derivat hiervon, wobei die Reste die für Verbindungen der Formel I genannten Bedeutungen haben, kondensiert, wobei freie funktionelle Gruppen in den Ausgangsmaterialien der Formeln X und XI mit Ausnahme der an der Reaktion beteiligten gegebenenfalls in geschützter Form vorliegen, und gewünschtenfalls vorhandene Schutzgruppen abspaltet, oder

f) in einer Verbindung der Formel I, worin die Substituenten die oben genannten Bedeutungen haben mit der Massgabe, dass in der betreffenden Verbindung der Formel I mindestens eine funktionelle Gruppe durch Schutzgruppen geschützt ist, vorhandene Schutzgruppen abspaltet,

und/oder gewünschtenfalls eine nach einem der vorstehenden Verfahren a) bis f) erhaltene Verbindung der Formel I mit mindestens einer salzbildenden Gruppe in ihr Salz und/oder ein erhältliches Salz in die freie Verbindung oder in ein anderes Salz überführt und/oder gegebenenfalls erhältliche Isomerengemische von Verbindungen der Formel I auftrennt und/oder eine erfindungsgemässe Verbindung der Formel I in eine andere erfindungsgemässe Verbindung der Formel I umwandelt.

Die oben definierten Verfahren werden nachfolgend näher beschrieben:

Verfahren a) (Herstellung einer Amidbindung)

In Ausgangsmaterialien der Formel II und III sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Zu den Schutzgruppen für funktionelle Gruppen in Ausgangsmaterialien, deren Umsetzung vermieden werden soll, insbesondere Carboxy-, Amino-, Hydroxy-, Mercapto- und Sulfogruppen, zählen insbesondere diejenigen Schutzgruppen (conventional protecting groups), die üblicherweise bei der Synthese von Peptid-Verbindungen, aber auch von Cephalosporinen und Penicillinen sowie Nucleinsäurederivaten und Zuckern verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. In bestimmten Fällen können die Schutzgruppen darüber hinaus einen selektiven, beispielsweise stereoselektiven Verlauf von Umsetzungen bewirken. Charakteristisch für Schutzgruppen ist, dass sie leicht, d. h. ohne unerwünschte Nebenreaktionen abspaltbar sind, beispielsweise solvolytisch, reduktiv, photolytisch oder auch enzymatisch, z. B. auch unter physiologischen Bedingungen. Schutzgruppen können aber auch in den Endstoffen vorhanden sein. Verbindungen der Formel I mit geschützten funktionellen Gruppen können eine höhere metabolische Stabilität oder anderweitig verbesserte pharmakodynamische Eigenschaften aufweisen als die entsprechenden Verbindungen mit freien funktionellen Gruppen. Die entsprechenden Verbindungen mit geschützten Gruppen, z. B. mit geschützten Hydroxygruppen, können auch "Prodrugs " darstellen, die in vivo durch enzymatische Spaltung aktiviert werden, beispielsweise durch Esterasen.

Der Schutz von funktionellen Gruppen durch solche Schutzgruppen, die Schutzgruppen selbst, sowie ihre Abspaltungsreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (E. Gross und J. Meienhofer, Herausg.), Academic Press, London und New York 1981, in "Methoden der organischen Chemie", Houben-Weyl, 4. Auflage, Band 15/I, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate: Monosaccharide und Derivate", Georg Thieme Verlag, Stuttgart 1974, beschrieben.

Eine Carboxygruppe ist z. B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche bevorzugt in 1-Stellung der Niederalkylgruppe verzweigt oder in 1 - oder 2-Stellung der Niederalkylgruppe durch geeignete Substituenten substituiert ist.

Eine geschützte Carboxygruppe, welche durch eine Niederalkylgruppe verestert ist, ist beispielsweise Methoxycarbonyl oder Ethoxycarbonyl.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z. B. tert-Butoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise Arylmethoxycarbonyl mit einem oder zwei Arylresten, worin Aryl unsubstituiertes oder z. B. durch Niederalkyl, z. B. tert-Niederalkyl, wie tert-Butyl, Niederalkoxy, z. B. Methoxy, Hydroxy, Halogen, z. B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z. B. 4-Nitrobenzyloxycarbonyl oder 4-Methoxybenzyloxycarbonyl, Diphenylmethoxycarbonyl

oder durch die genannten Substituenten substituiertes Diphenylmethoxycarbonyl, z. B. Di-(4-methoxyphenyl)-methoxycarbonyl, ferner durch eine Niederalkylgruppe verestertes Carboxy, wobei die Niederalkylgruppe in 1- oder 2-Stellung durch geeignete Substituenten substituiert ist, wie 1-Niederalkoxyniederalkoxycarbonyl, z. B. Methoxymethoxycarbonyl, 1-Methoxyethoxycarbonyl oder 1-Ethoxyethoxycarbonyl, 1-Niederalkylthioniederalkoxycarbonyl, z. B. 1-Methylthiomethoxycarbonyl oder 1-Ethylthioethoxycarbonyl, Aroylmethoxycarbonyl, worin die Aroylgruppe gegebenenfalls beispielsweise durch Halogen, wie Brom, substituiertes Benzoyl darstellt, z. B. Phenacyloxycarbonyl, 2-Halogenniederalkoxycarbonyl, z. B. 2,2,2-Trichlorethoxycarbonyl, 2-Bromethoxycarbonyl oder 2-Iodethoxycarbonyl, sowie 2-(trisubstituiertes Silyl)-niederalkoxycarbonyl, worin die Substituenten unabhängig voneinander je einen gegebenenfalls, z. B. durch Niederalkyl, Niederalkoxy, Aryl, Halogen, und/oder Nitro substituierten aliphatischen, araliphatischen, cycloaliphatischen oder aromatischen Kohlenwasserstoffrest bedeuten, beispielsweise gegebenenfalls wie oben substituiertes Niederalkyl, Phenylniederalkyl, Cycloalkyl oder Phenyl, z. B. 2-Triniederalkylsilylniederalkoxycarbonyl, wie 2-Triniederalkylsilylethoxycarbonyl, z. B. 2-Trimethylsilylethoxycarbonyl oder 2-(Di-n-butyl-methyl-silyl)-ethoxycarbonyl, oder 2-Triarylsilylethoxycarbonyl, wie Triphenylsilylethoxycarbonyl.

Eine Carboxygruppe wird auch als organische Silyloxycarbonylgruppe geschützt. Eine organische Silyloxycarbonylgruppe ist beispielsweise eine Triniederalkylsilyloxycarbonylgruppe, z. B. Trimethylsilyloxycarbonyl. Das Siliciumatom der Silyloxycarbonylgruppe kann auch durch zwei Niederalkyl-, z. B. Methylgruppen, und eine Amino- oder Carboxygruppe eines zweiten Moleküls der Formel I substituiert sein. Verbindungen mit solchen Schutzgruppen lassen sich z. B. mit Dimethylchlorsilan als Silylierungsmittel herstellen.

Eine Carboxygruppe wird auch in Form eines inneren Esters mit einer in geeignetem Abstand, z. B. in γ-Stellung, zur Carboxygruppe im Molekül vorliegenden Hydroxygruppe, d. h. in Form eines Lactons, vorzugsweise eines γ-Lactons, geschützt.

Eine geschützte Carboxygruppe ist bevorzugt tert-Niederalkoxycarbonyl, z. B. tert-Butoxycarbonyl, Benzyloxycarbonyl, 4-Nitrobenzyloxycarbonyl, 9-Fluorenylmethoxycarbonyl oder Diphenylmethoxycarbonyl, oder eine in Form eines Lactons, insbesondere eines y-Lactons, geschützte Carboxygruppe.

Eine geschützte Aminogruppe ist durch eine Aminoschutzgruppe geschützt, z. B. in Form einer Acylamino-, Arylmethylamino-, veretherten Mercaptoamino-, 2-Acyl-niederalk-1-enylamino oder Silylaminogruppe oder als Azidogruppe.

In einer Acylaminogruppe ist Acyl beispielsweise der Acylrest einer organischen Carbonsäure mit z. B. bis zu 18 Kohlenstoffatomen, insbesondere einer gegebenenfalls, z.B durch Halogen oder Aryl, substituierten Niederalkancarbonsäure oder gegebenenfalls, z. B. durch Halogen, Niederalkoxy oder Nitro, substituierten Benzoesäure, oder bevorzugt eines Kohlensäurehalbesters. Solche Acylgruppen sind vorzugsweise Niederalkanoyl, wie Formyl, Acetyl, Propionyl oder Pivaloyl, Halogenniederalkanoyl, z. B. 2-Halogenacetyl, wie 2-Chlor-, 2-Brom-, 2-Iod-, 2,2,2-Trifluor- oder 2,2,2-Trichloracetyl, gegebenenfalls z. B. durch Halogen, Niederalkoxy oder Nitro substituiertes Benzoyl, wie Benzoyl, 4-Chlorbenzoyl, 4-Methoxybenzoyl oder 4-Nitrobenzoyl, Niederalkoxycarbonyl, vorzugsweise in 1-Stellung des Niederalkylrestes verzweigtes oder in 1- oder 2-Stellung geeignet substituiertes Niederalkoxycarbonyl, z. B. tert-Niederalkoxycarbonyl, wie tert-Butoxycarbonyl, Arylmethoxycarbonyl mit einem, zwei oder drei Arylresten, die gegebenenfalls, z. B. durch Niederalkyl, insbesondere tert-Niederalkyl, wie tert-Butyl, Niederalkoxy, wie Methoxy, Hydroxy, Halogen, wie Chlor, und/oder Nitro mono- oder polysubstituiertes Phenyl darstellen, z. B. Benzyloxycarbonyl, 4-Nitrobenzyloxycarbonyl, Diphenylmethoxycarbonyl, 9-Fluorenylmethoxycarbonyl oder Di- (4-methoxyphenyl)methoxycarbonyl, Aroylmethoxycarbonyl, worin die Aroylgruppe vorzugsweise gegebenenfalls, z. B. durch Halogen, wie Brom, substituiertes Benzoyl darstellt, z. B. Phenacyloxycarbonyl, 2-Halogen-niederalkoxycarbonyl, z. B. 2,2,2-Trichlorethoxycarbonyl, 2-Bromethoxycarbonyl oder 2-Iodethoxycarbonyl, 2-(trisubstituiertes Silyl)-niederalkoxycarbonyl, z. B. 2-Triniederalkylsilylniederalkoxycarbonyl, wie 2-Trimethylsilylethoxycarbonyl oder 2-(Di-n-butyl-methyl-silyl)-ethoxycarbonyl, oder Triarylsilylniederalkoxycarbonyl, z. B. 2-Triphenylsilylethoxycarbonyl.

In einer Arylmethylaminogruppe, z. B. einer Mono-, Di- oder insbesondere Triarylmethylaminogruppe, sind die Arylreste insbesondere gegebenenfalls substituierte Phenylreste. Solche Gruppen sind z. B. Benzyl-, Diphenylmethyl- oder insbesondere Tritylamino.

In einer veretherten Mercaptoaminogruppe liegt die Mercaptogruppe in erster Linie als substituiertes Arylthio oder Arylniederalkylthio, worin Aryl beispielsweise gegebenenfalls z. B. durch Niederalkyl, wie Methyl oder tert.-Butyl, Niederalkoxy, wie Methoxy, Halogen, wie Chlor, und/oder Nitro substituiertes Phenyl ist, z. B. 4-Nitrophenylthio, vor.

In einem als Aminoschutzgruppe verwendbaren 2-Acyl-niederalk-1-enylrest ist Acyl z. B. der entsprechende Rest einer Niederalkancarbonsäure, einer gegebenenfalls z. B. durch Niederalkyl, wie Methyl oder tert-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende

Schutzgruppen sind in erster Linie 1-Niederalkanoyl-niederalk-1-en-2-yl, z. B. 1-Niederalkanoyl-prop-1-en-2-yl, wie 1-Acetyl-prop-1-en-2-yl, oder Niederalkoxycarbonyl-niederalk-1-en-2-yl, z. B. Niederalkoxycarbonyl-prop-1-en-2-yl, wie 1-Ethoxycarbonyl-prop-1-en-2-yl.

Eine Silylaminogruppe ist beispielsweise eine Triniederalkylsilylaminogruppe, z. B. Trimethylsilylamino oder tert-Butyl-dimethylsilylamino. Das Siliciumatom der Silylaminogruppe kann auch nur durch zwei Niederalkylgruppen, z. B. Methylgruppen, und die Aminogruppe oder Carboxylgruppe eines zweiten Moleküls der Formel I substituiert sein. Verbindungen mit solchen Schutzgruppen lassen sich z. B. mit den entsprechenden Chlorsilanen, wie Dimethylchlorsilan, als Silylierungsmittel herstellen.

Eine Aminogruppe kann auch durch Überführung in die protonierte Form geschützt werden; als entsprechende Anionen kommen in erster Linie diejenigen von starken anorganischen Säuren, wie von Schwefelsäure, Phosphorsäure oder Halogenwasserstoffsäuren, z. B. das Chlor- oder Bromanion, oder von organischen Sulfonsäuren, wie p-Toluolsulfonsäure, in Frage.

Bevorzugte Aminoschutzgruppen sind Niederalkoxycarbonyl, Phenylniederalkoxycarbonyl, Fluorenylniederalkoxycarbonyl, 2-Niederalkanoyl-niederalk-1-en-2-yl oder Niederalkoxycarbonyl-niederalk-1-en-2-yl, besonders bevorzugt tert-Butoxycarbonyl oder Benzyloxycarbonyl.

Eine Hydroxygruppe kann beispielsweise durch eine Acylgruppe, z. B. unsubstituiertes oder durch Halogen, wie Chlor, substituiertes Niederalkanoyl, wie Acetyl oder 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichlorethoxycarbonyl, 4-Nitrobenzyloxycarbonyl, Diphenylmethoxycarbonyl oder Trityl. Eine Hydroxygruppe kann ferner durch Triniederalkylsilyl, z. B. Trimethylsilyl, Triisopropylsilyl oder tert-Butyl-dimethylsilyl, eine leicht abspaltbare verethernde Gruppe, z. B. eine Alkylgruppe, wie tert-Niederalkyl, z. B. tert-Butyl, einen oxa- oder einen thiaaliphatischen oder -cycloaliphatischen, insbesondere 2-oxa- oder 2-thiaaliphatischen oder -cycloaliphatischen, Kohlenwasserstoffrest, beispielsweise 1-Niederalkoxyniederalkyl oder 1-Niederalkylthioniederalkyl, wie Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, wie 2-Tetrahydrofuryl oder 2-Tetrahydropyranyl, oder ein entsprechendes Thiaanaloges, sowie durch 1-Phenylniederalkyl, wie Benzyl, Diphenylmethyl oder Trityl, wobei die Phenylreste beispielsweise durch Halogen, z. B. Chlor, Niederalkoxy, z. B. Methoxy, und/oder Nitro substituiert sein können, geschützt sein.

Zwei in einem Molekül vorkommende, insbesondere benachbarte Hydroxylgruppen oder eine benachbarte Hydroxy- und Aminogruppe können beispielsweise durch bivalente Schutzgruppen, wie eine vorzugsweise, etwa durch ein oder zwei Niederalkylreste oder Oxo, substituierte Methylengruppe, geschützt sein, z. B. durch unsubstituiertes oder substituiertes Alkyliden, z. B. Niederalkyliden, wie Isopropyliden, Cycloalkyliden, wie Cyclohexyliden, eine Carbonylgruppe oder Benzyliden.

Eine in Nachbarstellung zu einer Carboxygruppe stehende Hydroxygruppe kann durch Bildung eines inneren Esters (Lacton), insbesondere eines $\gamma$-Lactones, geschützt sein.

Bevorzugt ist eine geschützte Hydroxygruppe durch Triniederalkylsilyl oder als Lacton geschützt, insbesondere durch tert-Butyl-dimethylsilyl oder als $\gamma$-Lacton.

Eine Mercaptogruppe, wie z. B. in Cystein, kann insbesondere durch S-Alkylierung mit gegebenenfalls substituierten Alkylresten, Silylierung, Thioacetalbildung, S-Acylierung oder durch die Bildung asymmetrischer Disulfidgruppierungen geschützt sein. Bevorzugte Mercaptoschutzgruppen sind beispielsweise gegebenenfalls im Phenylrest, z. B. durch Methoxy oder Nitro, substituiertes Benzyl, wie 4-Methoxybenzyl, gegebenenfalls im Phenylrest, z. B. durch Methoxy, substituiertes Diphenylmethyl, wie Di-(4-methoxyphenyl)-methyl, Triphenylmethyl, Pydyldiphenylmethyl, Trimethylsilyl, Benzylthiomethyl, Tetrahydropyranyl, Acylaminomethyl, wie Acetamidomethyl, iso-Butyrylacetamidomethyl oder 2-Chloracetamidomethyl, Benzoyl, Benzyloxycarbonyl oder Alkyl-, insbesondere Niederalkylaminocarbonyl, wie Ethylaminocarbonyl, sowie Niederalkylthio, wie S-Ethylthio oder S-tert-Butylthio, oder S-Sulfo.

Eine Sulfogruppe kann beispielsweise durch Niederalkyl, z. B. Methyl oder Ethyl, durch Phenyl oder als Sulfonamid, beispielsweise als Imidazolid, geschützt sein.

Als Schutzgruppe, beispielsweise Carboxyschutzgruppe, im Sinne dieser Anmeldung ist ausdrücklich auch ein in leicht abspaltbarer Weise mit der zu schützenden funktionellen Gruppe, beispielsweise Carboxygruppe, verbundener polymerer Träger zu verstehen, wie er z. B. für die Merrifield-Synthese geeignet ist. Ein solcher geeigneter polymerer Träger ist insbesondere ein durch Copolymerisation mit Divinylbenzol schwach vernetztes Polystyrolharz, das zur reversiblen Bindung geeignete Brückenglieder trägt.

Die Säuren der Formel II sind Carbonsäuren oder Sulfonsäuren.

Die Carbonsäuren der Formel II liegen entweder mit freier Carboxygruppe vor, oder als reaktionsfähiges Derivat hiervon, beispielsweise als von der freien Carboxyverbindung abgeleiteter aktivierter Ester, als reaktionsfähiges Anhydrid, oder ferner als reaktionsfähiges cyclisches Amid. Die reaktionsfähigen Derivate können

auch in situ gebildet werden.

Aktivierte Ester von Verbindungen der Formel II mit einer Carboxygruppe sind insbesondere am Verknüpfungskohlenstoffatom des veresternden Restes ungesättigte Ester, z. B. vom Vinylester-Typ, wie Vinylester (erhätlich z. B. durch Umesterung eines entsprechenden Esters mit Vinylacetat; Methode des aktivierten Vinylesters), Carbamoylester (erhältlich z. B. durch Behandeln der entsprechenden Säure mit einem Isoxazoliumreagens; 1,2-Oxazolium- oder Woodward-Methode), oder 1-Niederalkoxyvinylester (erhältlich z. B. durch Behandeln der entsprechenden Säure mit einem Niederalkoxyacetylen; Ethoxyacetylen-Methode), oder Ester vom Amidinotyp, wie N,N′-disubstituierte Amidinoester (erhältlich z. B. durch Behandeln der entsprechenden Säure mit einem geeigneten N,N′-disubstituierten Carbodiimid, z. B. N,N′-Dicyclohexylcarbodiimid; Carbodiimid-Methode), oder N,N-disubstituierte Amidinoester (erhältlich z. B. durch Behandeln der entsprechenden Säure mit einem N,N-disubstituierten Cyananmid; Cyanamid-Methode), geeignete Arylester, insbesondere durch elektronenanziehende Substituenten substituierte Phenylester (erhältlich z. B. durch Behandeln der entsprechenden Säure mit einem geeignet substituierten Phenol, z. B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N′-Dicyclohexylcarbodiimid; Methode der aktivierten Arylester), Cyanmethylester (erhältlich z. B. durch Behandeln der entsprechenden Säure mit Chloracetonitril in Gegenwart einer Base; Cyanmethylester-Methode), Thioester, insbesondere gegebenenfalls, z. B. durch Nitro, substituierte Phenylthioester (erhältlich z. B. durch Behandeln der entsprechenden Säure mit gegebenenfalls, z. B. durch Nitro, substituierten Thiophenolen, u. a. mit Hilfe der Anhydrid- oder Carbodiimid-Methode; Methode der aktivierten Thiolester), oder insbesondere Amino- oder Amidoester (erhältlich z. B. durch Behandeln der entsprechenden Säure mit einer N-Hydroxyamino- bzw. N-Hydroxyamidoverbindung, z. B. N-Hydroxysuccinimid, N-Hydroxypiperidin, N-Hydroxyphthalimid, N-Hydroxy-5-norbornen-2,3-dicarbonsäureimid, 1-Hydroxybenztriazol oder 3-Hydroxy-3,4-dihydro-1,2,3-benztriazin-4-on, z. B. nach der Anhydrid- oder Carbodiimid-Methode; Methode der aktivierten N-Hydroxyester). Auch innere Ester, z. B. γ-Lactone, sind einsetzbar.

Anhydride von Säuren können symmetrische oder vorzugsweise gemischte Anhydride dieser Säuren sein, z. B. Anhydride mit anorganischen Säuren, wie Säurehalogenide, insbesondere Säurechloride (erhältlich z. B. durch Behandeln der entsprechenden Säure mit Thionylchlorid, Phosphorpentachlorid oder Oxalylchlorid; Säurechloridmethode), Azide (erhältlich z. B. aus einem entsprechenden Säureester über das entsprechende Hydrazid und dessen Behandlung mit salpetriger Säure; Azidmethode), Anhydride mit Kohlensäurehalbestern, z. B. Kohlensäureniederalkylhalbestern (erhältlich z. B. durch Behandeln der entsprechenden Säure mit Chlorameisensäureniederalkylestern oder mit einem 1-Niederalkoxycarbonyl-2-niederalkoxy- 1,2-dihydrochinolin; Methode der gemischten O-Alkylkohlensäureanhydride), oder Anhydride mit dihalogenierter, insbesondere dichlorierter Phosphorsäure (erhältlich z. B. durch Behandeln der entsprechenden Säure mit Phosphoroxychlorid; Phosphoroxychloridmethode), Anhydride mit anderen Phosphorsäurederivaten (z. B. solchen, die man mit Phenyl-N-phenylphosphoramidochloridat erhalten kann oder durch Umsetzung von Alkylphosphorsäureamiden in Gegenwart von Sulfonsäureanhydriden und/oder racemisierungssenkenden Additiven, wie N-Hydroxybenztriazol, oder in Gegenwart von Cyanphosphonsäurediethylester) oder mit Phosphorigsäurederivaten, oder Anhydride mit organischen Säuren, wie gemischte Anhydride mit organischen Carbonsäuren (erhältlich z. B. durch Behandeln der entsprechenden Säure mit einem gegebenenfalls substituierten Niederalkan- oder Phenylniederalkancarbonsäurehalogenid, z. B. Phenylessigsäure-, Pivalinsäure- oder Trifluoressigsäurechlorid; Methode der gemischten Carbonsäureanhydride) oder mit organischen Sulfonsäuren (erhältlich z. B. durch Behandeln eines Salzes, wie eines Alkalimetallsalzes, der entsprechenden Säure mit einem geeigneten organischen Sulfonsäurehalogenid, wie Niederalkan- oder Aryl-, z. B. Methan- oder p-Toluolsulfonsäurechlorid; Methode der gemischten Sulfonsäureanhydride) sowie symmetrische Anhydride (erhältlich z. B. durch Kondensation der entsprechenden Säure in Gegenwart eines Carbodiimides oder von 1-Diethylaminopropin; Methode der symmetrischen Anhydride).

Geeignete cyclische Amide sind insbesondere Amide mit fünfgliedrigen Diazacyclen aromatischen Charakters, wie Amide mit Imidazolen, z. B. Imidazol (erhältlich z. B. durch Behandeln der entsprechenden Säure mit N,N′-Carbonyldiimidazol; Imidazol-Methode), oder Pyrazol, z. B. 3,5-Dimethylpyrazol (erhältlich z. B. über das Säurehydrazid durch Behandeln mit Acetylaceton; Pyrazolid-Methode).

Wie erwähnt, können Derivate von Carbonsäuren, die als Acylierungsmittel verwendet werden, auch in situ gebildet werden. So kann man z. B. N,N′-disubstituierte Amidinoester in situ bilden, indem man das Gemisch des Ausgangsmaterials der Formel III und der als Acylierungsmittel verwendeten Säure der Formel II in Gegenwart eines geeigneten N,N′-disubstituierten Carbodiimids, z. B. N,N′-Cyclohexylcarbodiimid, zur Reaktion bringt, beispielsweise in Gegenwart einer geeigneten Base, wie Triethylamin. Weiter kann man Amino- oder Amidoester der als Acylierungsmittel verwendeten Säuren in Gegenwart des zu acylierenden Ausgangsmaterials der Formel III bilden, indem man das Gemisch der entsprechenden Säure- und Amino-Ausgangsstoffe in Gegenwart eines N,N′-disubstituierten Carbodiimids, z. B. N,N′-Dicyclohexylcarbodiimid,

und eines N-Hydroxyamins oder N-Hydroxy-amids, z. B. N-Hydroxysuccinimid, gegebenenfalls in Anwesenheit einer geeigneten Base, z. B. 4-Dimethylamino-Pyridin, umsetzt. Ferner kann man durch Umsetzung mit N,N,N′,N′-Tetraalkyluroniumverbindungen, wie O-Benztriazol-1-yl-N,N,N′,N′-tetra-methyl-uronium-hexafluorphosphat, Aktivierung in situ erreichen. Schliesslich können Phosphorsäureanhydride der Carbonsäuren der Formel II in situ hergestellt werden, indem man ein Alkylphosphorsäureamid, wie Hexamethylphosphorsäuretriamid, in Gegenwart eines Sulfonsäureanhydrides, wie 4-Toluolsulfonsäureanhydrid, mit einem Salz, wie einem Tetrafluoroborat, z. B. Natriumtetrafluoroborat, oder mit einem anderen Abkömmling des Hexamethylphosphorsäuretriamides, wie Benzotriazol-1-yl-oxy-tris-(dimethylamino)-phosphonium-hexafluorid, vorzugsweise in Gegenwart eines racemisierungssenkenden Additives, wie N-Hydroxybenztriazol, umsetzt.

Die Aminogruppe von Verbindungen der Formel III, die an der Reaktion teilnimmt, trägt vorzugsweise mindestens ein reaktionsfähiges Wasserstoffatom, insbesondere, wenn die damit reagierende Carboxygruppe in reaktionsfähiger Form vorliegt; sie kann aber auch selbst derivatisiert sein, z. B. durch Reaktion mit einem Phosphit, wie Diethylchlorphosphit, 1,2-Phenylenchlorphosphit, Ethyldichlorphosphit, Ethylenchlorphosphit oder Tetraethylpyrophosphit. Ein Derivat einer solchen Verbindung mit einer Aminogruppe ist z. B. auch ein Carbaminsäurehalogenid, wobei die an der Reaktion teilnehmende Aminogruppe durch Halogencarbonyl, z. B. Chlorcarbonyl, substituiert ist.

Die Kondensation zur Herstellung einer Amidbindung kann in an sich bekannter Weise durchgeführt werden, beispielsweise wie in Standardwerken, wie "Houben-Weyl, Methoden der organischen Chemie", 4. Auflage, Band 15/II (1974), Band IX (1955) Band E 11 (1985), Georg Thieme Verlag, Stuttgart, "The Peptides" (E. Gross und J. Meienhofer, Hg.), Band 1 und 2, Academic Press, London und New York, 1979/1980, oder M. Bodansky, "Principles of Peptide Synthesis", Springer-Verlag, Berlin 1984, beschrieben.

Die Kondensation einer freien Carbonsäure mit dem entsprechenden Amin kann vorzugsweise in Gegenwart eines der üblichen Kondensationsmittel durchgeführt werden. Übliche Kondensationsmittel sind z. B. Carbodiimide, beispielsweise Diethyl-, Dipropyl-, N-Ethyl-N′-(3-dimethylaminopropyl)-carbodiimid oder insbesondere Dicyclohexylcarbodiimid, ferner geeignete Carbonylverbindungen, beispielsweise Carbonylimidazol, 1,2-Oxazoliumverbindungen, z. B. 2-Ethyl-5-phenyl- 1,2-oxazolium-3′-sulfonat und 2-tert-Butyl-5-methylisoxazoliumperchlorat, oder eine geeignete Acylaminoverbindung, z. B. 2-Ethoxy-1-ethoxycarbonyl- 1,2-dihydrochinolin, N,N,N′,N′-Tetraalkyluroniumverbindungen, wie O-Benztriazol-1-yl-N,N,N′,N′-tetra-methyluroniumhexafluorphosphat, ferner aktivierte Phosphorsäurederivate, z. B. Diphenylphosphorylazid, Diethylphosphorylcyanid, Phenyl-N-phenylphosphoroamidochloridat, Bis-(2-oxo-3-oxazolidinyl)phosphinsäurechlorid oder 1-Benztriazolyloxy-tris-(dimethylamino)-phosphonium-hexafluorophosphat.

In analoger Weise wie bei den für die Carbonsäuren der Formel II zur Kondensation genannten Reaktionstypen lassen sich auch die Sulfonsäuren der Formel II mit endständiger Sulfonylgruppe bei der Kondensation mit Verbindungen der Formel III zu den entsprechenden Sulfonamiden der Formel Ib umsetzen.

So können beispielsweise aktivierte Sulfonsäureester eingesetzt werden, z. B. die entsprechenden, insbesondere durch Nitrogruppen subsbtuierten, Arylester, wie Phenylester, wobei die Aminkomponente der Formel Ib auch als Alkalimetallamid, z. B. Alkalimetallarylamid, wie Natriumanilinamid, oder als Alkalimetallsalz von stickstoffhaltigen Heterocyclen, z. B. Kalium-pyrrolid, eingesetzt werden kann.

Ferner können reaktionsfähige Anhydride zum Einsatz kommen, wie etwa die entsprechenden symmetrischen (herstellbar z. B. durch Reaktion der alkylsulfonsauren Silbersalze mit Alkylsulfonylchloriden) oder vorzugsweise asymmetrischen Säureanhydride, z. B. Anhydride mit anorganischen Säuren, wie Sulfonylhalogenide, insbesondere Sulfonylchloride (erhältlich z. B. durch Umsetzung der entsprechenden Sulfonsäuren mit anorganischen Säurechloriden, z. B. Thionylchlorid, Sulfurylchlorid oder Phosphorpentachlorid), mit organischen Carbonsäuren (erhältlich z. B. durch Behandeln eines Sulfonsäurehalogenides mit dem Salz einer Carbonsäure, wie einem Alkalimetallsalz, analog der oben genannten Methode zur Herstellung der gemischten Säureanhydride), oder Azide (erhältlich z. B. aus einem entsprechenden Sulfonsäurechlorid und Natriumazid oder über das entsprechende Hydrazid und dessen Behandlung mit salpetriger Säure analog der oben genannten Azidmethode).

Gewünschtenfalls wird eine organische Base zugegeben, z. B. ein Triniederalkylamin mit voluminösen Resten, z. B. Ethyldiisopropylamin, und/oder eine heterocyclische Base, z. B. Pyridin, 4-Dimethylaminopyridin oder bevorzugt N-Methylmorpholin.

Die Kondensation aktivierter Ester, reaktionsfähiger Anhydride oder reaktionsfähiger cyclischer Amide mit den entsprechenden Aminen wird üblicherweise in Gegenwart einer organischen Base, z. B. einfachen Triniederalkylaminen, z. B. Triethylamin oder Tributylamin, oder einer der vorstehend genannten organischen Basen durchgeführt. Gewünschtenfalls wird zusätzlich noch ein Kondensationsmittel verwendet, wie für freie Carbonsäuren beschrieben ist.

Die Kondensation von Säureanhydriden mit Aminen kann z. B. in Gegenwart von anorganischen Carbonaten, z. B. Ammonium- oder Alkalimetallcarbonaten oder -hydrogencarbonaten, wie Natrium- oder Kaliumcarbonat oder -hydrogencarbonat (üblicherweise zusammen mit einem Sulfat), erfolgen, die Reaktion von Sulfonsäurehalogeniden, wie Sulfonsäurechloriden, in Gegenwart von Hydroxiden, z. B. Alkalimetallhydroxiden, wie Natriumhydroxid oder Kaliumhydroxid.

Carbonsäurechloride, beispielsweise die von der Säure der Formel II abgeleiteten Chlorkohlensäurederivate, werden mit den entsprechenden Aminen vorzugsweise in Gegenwart eines organischen Amins, z. B. der vorstehend genannten Triniederalkylamine oder heterocyclischen Basen, gegebenenfalls in Gegenwart eines Hydrogensulfates, kondensiert.

Die Kondensation wird vorzugsweise in einem inerten, aprotischen, vorzugsweise wasserfreien, Lösungsmittel oder Lösungsmittelgemisch durchgeführt, beispielsweise in einem Carbonsäureamid, z. B. Formamid oder Dimethylformamid, einem halogenierten Kohlenwasserstoff, z. B. Methylenchlorid, Tetrachlorkohlenstoff oder Chlorbenzol, einem Keton, z. B. Aceton, einem cyclischen Ether, z. B. Tetrahydrofuran, einem Ester, z. B. Essigsäureethylester, oder einem Nitril, z. B. Acetonitril, oder in einer Mischung davon, gegebenenfalls bei erniedrigter oder erhöhter Temperatur, z. B. in einem Temperaturbereich von etwa -40 °C bis etwa + 100 °C, bevorzugt von etwa - 10 °C bis etwa +50 °C, und ohne Inertgas oder unter Inertgas-, z. B. Stickstoff- oder Argonatmosphäre.

Auch wässrige, beispielsweise alkoholische, z. B. Ethanol, oder aromatische Lösungsmittel, z. B. Benzol oder Toluol, sind möglich. Bei Gegenwart von Alkalihydroxiden als Basen kann gegebenenfalls auch Aceton zugesetzt werden.

Die Kondensation kann auch gemäss der als Festphasen-Synthese bekannten Technik erfolgen, die auf R. Merrifield zurückgeht und beispielsweise in Angew. Chem. 97, 801 - 812 (1985), Naturwissenschaften 71, 252 - 258 (1984) oder in R. A. Houghten, Proc. Natl. Acad. Sci. USA 82, 5131 - 5135 ( 1985) beschrieben ist.

Die Freisetzung durch Schutzgruppen geschützter funktioneller Gruppen in den erhaltenen Verbindungen der Formel I mit geschützten Funktionen erfolgt gegebenenfalls nach einer oder mehreren der unter Verfahren f) genannten Methoden.

Verfahren b) (Herstellung einer Amidbindung)

In Ausgangsmaterialien der Formel IV und V sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Die Schutzgruppen, die freien Carbonsäuren und ihre reaktionsfähigen Derivate, die freien Amine und ihre reaktionsfähigen Derivate und die verwendeten Verfahren zur Kondensation sind vollständig analog zu den unter Verfahren a) für die Herstellung einer Amidbindung ausgehend von Verbindungen der Formel II und III beschriebenen, wenn man dort anstelle der Carbonsäuren der Formel II diejenigen der Formel IV und anstelle der Aminoverbindungen der Formel III diejenigen der Formel V einsetzt.

Die Freisetzung durch Schutzgruppen geschützter funktioneller Gruppen in den erhaltenen Verbindungen der Formel I mit geschützten Funktionen erfolgt gegebenenfalls nach einer oder mehreren der unter Verfahren f) genannten Methoden.

Verfahren c) (Herstellung einer Amidbindung)

In Ausgangsmaterialien der Formel VI und VII sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Die Schutzgruppen, die freien Carbonsäuren und ihre reaktionsfähigen Derivate, die freien Amine und ihre reaktionsfähigen Derivate und die verwendeten Verfahren zur Kondensation sind vollständig analog zu den unter Verfahren a) für die Herstellung einer Amidbindung ausgehend von Verbindungen der Formel II und III beschriebenen, wenn man dort anstelle der Carbonsäuren der Formel II diejenigen der Formel VI und anstelle der Aminoverbindungen der Formel III diejenigen der Formel VII einsetzt.

Die Freisetzung durch Schutzgruppen geschützter funktioneller Gruppen in den erhaltenen Verbindungen der Formel I mit geschützten Funktionen erfolgt gegebenenfalls nach einer oder mehreren der unter Verfahren f) genannten Methoden.

Verfahren d) (Herstellung einer Amidbindung)

In Ausgangsmaterialien der Formel VIII und IX sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Die Schutzgruppen, die freien Carbonsäuren und ihre reaktionsfähigen Derivate, die freien Amine und ihre reaktionsfähigen Derivate und die verwendeten Verfahren zur Kondensation sind vollständig analog zu den unter Verfahren a) für die Herstellung einer Amidbindung ausgehend von Verbindungen der Formel II und III beschriebenen, wenn man dort anstelle der Carbonsäuren der Formel II diejenigen der Formel VIII und anstelle der Aminoverbindungen der Formel III diejenigen der Formel IX einsetzt.

Die Freisetzung durch Schutzgruppen geschützter funktioneller Gruppen in den erhaltenen Verbindungen der Formel I mit geschützten Funktionen erfolgt gegebenenfalls nach einer oder mehreren der unter Verfahren f) genannten Methoden.

Verfahren e) (Herstellung einer Amidbindung)

In Ausgangsmaterialien der Formel X und XI sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Die Schutzgruppen, die freien Carbonsäuren und ihre reaktionsfähigen Derivate, die freien Amine und ihre reaktionsfähigen Derivate und die verwendeten Verfahren zur Kondensation sind vollständig analog zu den unter Verfahren a) für die Herstellung einer Amidbindung ausgehend von Verbindungen der Formel II und III beschriebenen, wenn man dort anstelle der Carbonsäuren der Formel II diejenigen der Formel X und anstelle der Aminoverbindungen der Formel III diejenigen der Formel XI einsetzt.

Ein reaktionsfähiges Derivat einer solchen Verbindung der Formel XI mit einer Aminogruppe ist z. B. auch ein Isocyanat, in dem die an der Reaktion teilnehmende Aminogruppe als Isocyanatgruppe abgewandelt ist, wobei im letzteren Falle nur Verbindungen der Formel I zugänglich sind, die am Stickstoffatom der durch die Reaktion gebildeten Amidgruppe ein Wasserstoffatom tragen.

Die Freisetzung durch Schutzgruppen geschützter funktioneller Gruppen in den erhaltenen Verbindungen der Formel I mit geschützten Funktionen erfolgt gegebenenfalls nach einer oder mehreren der unter Verfahren f) genannten Methoden.

Verfahren f) (Schutzgruppenabspaltung)

Die Abspaltung der Schutzgruppen, die nicht Bestandteil des gewünschten Endproduktes der Formel I sind, z. B. der Carboxy-, Amino-, Hydroxy-, Mercapto- und/oder Sulfoschutzgruppen, erfolgt in an sich bekannter Weise, z. B. mittels Solvolyse, insbesondere Hydrolyse, Alkoholyse oder Acidolyse, oder mittels Reduktion, insbesondere Hydrogenolyse oder mittels anderer Reduktionsmittel, sowie Photolyse, gegebenenfalls stufenweise oder gleichzeitig, wobei auch enzymatische Methoden verwendet werden können. Die Abspaltung der Schutzgruppen ist beispielsweise in den weiter vom im Abschnitt über " Schutzgruppen" genannten Standardwerken beschrieben.

So kann beispielsweise geschütztes Carboxy, z. B. tert-Niederalkoxycarbonyl, in 2-Stellung durch eine trisubstituierte Silylgruppe oder in 1-Stellung durch Niederalkoxy oder Niederalkylthio substituiertes Niederalkoxycarbonyl oder gegebenenfalls substituiertes Diphenylmethoxycarbonyl durch Behandeln mit einer geeigneten Säure, wie Ameisensäure, Chlorwasserstoff oder Trifluoressigsäure, gegebenenfalls unter Zugabe einer nukleophilen Verbindung, wie Phenol oder Anisol, in freies Carboxy überführt werden. Gegebenenfalls substituiertes Benzyloxycarbonyl kann z. B. mittels Hydrogenolyse, d.h. durch Behandeln mit Wasserstoff in Gegenwart eines metallischen Hydrierkatalysators, wie eines Palladiumkatalysators, freigesetzt werden. Ferner kann geeignet substituiertes Benzyloxycarbonyl, wie 4-Nitrobenzyloxycarbonyl, auch durch Reduktion, z. B. durch Behandeln mit einem Alkalimetall-, wie Natrium-dithionit, oder mit einem reduzierenden Metall, z. B. Zink, oder einem reduzierenden Metallsalz, wie einem Chrom-II-salz, z. B. Chrom-II-chlorid, üblicherweise in Gegenwart eines Wasserstoff-abgebenden Mittels, das zusammen mit dem Metall nascierenden Wasserstoff erzeugen kann, wie einer Säure, in erster Linie einer geeigneten Carbonsäure, wie einer gegebenenfalls, z. B. durch Hydroxy, substituierten Niederalkancarbonsäure, z. B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführt werden. Durch Behandeln mit einem reduzierenden Metall oder Metallsalz, wie oben beschrieben, kann auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy umgewandelt werden. Aroylmethoxycarbonyl kann ebenfalls durch Behandeln mit einem nukleophilen, vorzugsweise salzbildenden Reagens, wie Natriumthiophenolat oder Natriumiodid, gespalten werden. 2-(trisubstituiertes Silyl)-niederalkoxycarbonyl, wie 2-Triniederalkylsilylniederalkoxycarbonyl, kann auch durch Behandeln mit einem das Fluoridanion liefernden Salz der Fluorwasserstoffsäure, wie einem Alkalimetallfluorid, z. B. Natrium- oder

Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Triniederalkylarylniederalkylammoniumfluorid, z. B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Als organisches Silyloxycarbonyl, wie Triniederalkylsilyloxycarbonyl, z. B. Trimethylsilyloxycarbonyl, geschütztes Carboxy kann in üblicher Weise solvolytisch, z. B. durch Behandeln mit Wasser, einem Alkohol oder Säure, oder ausserdem Fluorid, wie oben beschrieben, freigesetzt werden. Verestertes Carboxy kann auch enzymatisch freigesetzt werden, beispielsweise durch Esterasen oder geeignete Peptidasen, z. B. verestertes Arginin oder Lysin, wie Lysinmethylester, mittels Trypsin. Als innerer Ester, wie als γ-Lacton, geschütztes Carboxy kann durch Hydrolyse in Gegenwart eines Hydroxid-haltigen Base, wie Erdalkalihydroxides oder insbesondere eines Alkalimetallhydroxides, z. B. NaOH, KOH oder LiOH, besonders LiOH, freigesetzt werden, wobei gleichzeitig die entsprechend geschützte Hydroxygruppe freigesetzt wird.

Eine geschützte Aminogruppe setzt man in an sich bekannter und je nach Art der Schutzgruppen in verschiedenartiger Weise, vorzugsweise mittels Solvolyse oder Reduktion, frei. Niederalkoxycarbonylamino, wie tert-Butoxycarbonylamino, kann in Gegenwart von Säuren, beispielsweise Mineralsäuren, z. B. Halogenwasserstoff, wie Chlorwasserstoff oder Bromwasserstoff, insbesondere Bromwasserstoff, oder von Schwefel- oder oder Phosphorsäure, vorzugsweise von Chlorwasserstoff, in polaren Lösungsmitteln, wie Wasser oder einer Carbonsäure, wie Essigsäure, oder Ethern, bevorzugt cyclischen Ethern, wie Dioxan, 2-Halogenniederalkoxycarbonylamino (gegebenenfalls nach Umwandlung einer 2-Bromniederalkoxycarbonylaminogruppe in eine 2-Iodniederalkoxycarbonylaminogruppe), Aroylmethoxycarbonylamino oder 4-Nitrobenzyloxycarbonylamino kann z. B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z. B. Natrium-dithionit, gespalten werden. Gegebenenfalls substituiertes Diphenylmethoxycarbonylamino, tert-Niederalkoxycarbonylamino oder 2-(trisubstituiertes Silyl)-niederalkoxycarbonylamino, wie 2-Triniederalkylsilylniederalkoxycarbonylamino, kann durch Behandeln mit einer geeigneten Säure, z. B. Ameisen- oder Trifluoressigsäure, gegebenenfalls substituiertes Benzyloxycarbonylamino z. B. mittels Hydrogenolyse, d.h. durch Behandeln mit Wasserstoff in Gegenwart eines geeigneten Hydrierkatalysators, wie eines Palladiumkatalysators, bevorzugt in polaren Lösungsmitteln, wie Diniederalkylniederalkanoylamiden, z. B. Dimethylformamid, Ethern, wie cyclischen Ethern, z. B. Dioxan, oder Alkoholen, wie Methanol, Ethanol oder Propanol, wobei Methanol besonders bevorzugt ist, gegebenenfalls substituiertes Triarylmethylamino oder Formylamino z. B. durch Behandeln mit einer Säure, wie Mineralsäure, z. B. Chlorwasserstoffsäure, oder einer organischen Säure, z. B. Ameisen-, Essig- oder Trifluoressigsäure, gegebenenfalls in Gegenwart von Wasser, und eine als Silylamino geschützten Aminogruppe z. B. mittels Hydrolyse oder Alkoholyse freigesetzt werden. Eine durch 2-Halogenacetyl, z. B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Substitutionssproduktes freigesetzt werden. Eine durch 2-(trisubstituiertes Silyl)-niederalkoxycarbonyl, wie 2-Triniederalkylsilylniederalkoxycarbonyl, geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z. B. durch Reduktion in freies Amino überführt, beispielsweise durch katalytische Hydrierung mit Wasserstoff in Gegenwart eines Hydrierkatalysators, wie Platinoxid, Palladium oder Raney-Nickel, durch Reduktion mittels Mercaptoverbindungen, wie Dithiothreitol oder Mercaptoethanol, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z. B. Methylenchlorid, oder auch in Wasser oder einem Gemisch von Wasser und einem organischen Lösungsmittel, wie einem Alkohol oder Dioxan, bei etwa 20 °C bis 25 °C, oder auch unter Kühlen oder Erwärmen, durchgeführt.

Eine durch eine geeignete Acylgruppe, eine Triniederalkylsilylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z. B. durch basische Hydrolyse, eine durch tert-Niederalkyl oder durch einen 2-oxa- oder 2-thiaaliphatischen oder -cycloaliphatischen Kohlenwasserstoffrest geschützte Hydroxy- bzw. Mercaptogruppe durch Acidolyse, z. B. durch Behandeln mit einer Mineralsäure oder einer starken Carbonsäure, z. B. Trifluoressigsäure, freigesetzt. Durch Pyridyldiphenylmethyl geschütztes Mercapto kann z. B. durch Quecksilber-II-salze bei pH 2-6 oder durch Zink/Essigsäure oder elektrolytische Reduktion, Acetamidomethyl und iso-Butyrylamidomethyl z. B. durch Reaktion mit Quecksilber-II-salzen bei pH 2-6, 2-Chloracetamidomethyl z. B. durch 1-Piperidinothiocarboxamid, S-Ethylthio, S-tert-Butylthio und S-Sulfo z. B. durch Thiolyse mit Thiophenol, Thioglycolsäure, Natrium-thiophenolat oder 1,4-Dithiothreitol freigesetzt werden. Zwei Hydroxygruppen oder eine benachbarte Amino- und Hydroxygruppe, die zusammen mittels einer bivalenten Schutzgruppe, vorzugsweise z. B. einer durch Niederalkyl ein- oder zweifach substituierten Methylengruppe, wie durch Niederalkyliden, z. B. Isopropyliden, Cycloyalkyliden, z. B. Cyclohexyliden, oder Benzyliden, geschützt sind, können durch saure Solvolyse, besonders in Gegenwart einer Mineralsäure oder einer starken organischen Säure, freigesetzt werden. Eine Triniederalkylsilylgruppe wird ebenfalls durch Acidolyse, z. B. durch Mineralsäure, bevorzugt Fluorwasserstoffsäure, oder eine starke Carbonsäure abgespalten. 2-Halogenniederalkoxycarbonyl wird durch die oben genannten Reduktionsmittel, z. B. reduzierendes Metall, wie Zink, reduzierende Metallsalze, wie Chrom-II-salze, oder durch Schwefelverbindungen, beispielsweise Natriumdithionit oder bevorzugt Natriumsulfid und Schwefelkohlenstoff, entfernt. Veresterte Hydroxygruppen, z. B. Niederalkanoyloxy, wie Acetyloxy, können auch durch Esterasen freigesetzt werden, acyliertes Amino beispielsweise durch geeignete Peptidasen.

Eine als Sulfonsäureester oder Sulfonamid geschützte Sulfogruppe wird beispielsweise durch saure Hydrolyse, z. B. in Gegenwart von Mineralsäure, oder bevorzugt durch basische Hydrolyse, z. B. mit Alkalimetallhydroxid oder Alkalimetallcarbonat, beispielsweise Natriumcarbonat, freigesetzt.

Die Temperaturen für die Freisetzung der geschützten funktionellen Gruppen liegen vorzugsweise zwischen -80 und 100 °C, besonders bevorzugt zwischen -20 und 50 °C, beispielsweise zwischen 10 und 35 °C, wie im Bereich der Raumtemperatur.

Beim Vorhandensein von mehreren geschützten funktionellen Gruppen können, wenn erwünscht, die Schutzgruppen so gewählt werden, dass gleichzeitig mehr als eine solche Gruppe abgespalten werden kann, beispielsweise acidolytisch, wie durch Behandeln mit Trifluoressigsäure, oder mit Wasserstoff und einem Hydrierkatalysator, wie einem Palladium-Kohle-Katalysator. Umgekehrt können die Gruppen auch so gewählt werden, dass sie nicht alle gleichzeitig, sondern in gewünschter Reihenfolge abgespalten werden können, wobei die entsprechenden Zwischenprodukte erhalten werden.

## Zusätzliche Verfahrensmassnahmen

Bei den zusätzlichen Verfahrensmassnahmen, die gewünschtenfalls durchgeführt werden, können funktionelle Gruppen der Ausgangsverbindungen, die nicht an der Reaktion teilnehmen sollen, ungeschützt oder in geschützter Form, beispielsweise durch eine oder mehrere der oben unter Verfahren a) genannten Schutzgruppen, vorliegen. Die Schutzgruppen können in den Endprodukten erhalten bleiben oder ganz oder teilweise nach einer der unter Verfahren f) genannten Methoden abgespalten werden.

Salze von Verbindungen der Formel I mit mindestens einer salzbildenden Gruppe können in an sich bekannter Weise hergestellt werden. So kann man Salze von Verbindungen der Formel I mit sauren Gruppen z. B. durch Behandeln mit Metallverbindungen, wie Alkalimetallsalzen von geeigneten organischen Carbonsäuren, z. B. dem Natriumsalz der 2-Ethyl-hexansäure, mit organischen Alkali- oder Erdalkalimetallverbindungen, wie den entsprechenden Hydroxiden, Carbonaten oder Hydrogencarbonaten, wie Natrium- und Kaliumhydroxid, -carbonat oder -hydrogencarbonat, mit entsprechenden Calciumverbindungen oder mit Ammoniak oder einem geeigneten organischen Amin bilden, wobei man vorzugsweise stöchiometrische Mengen oder nur einen kleinen Überschuss des salzbildenden Mittels verwendet. Säureadditionssalze von Verbindungen der Formel I erhält man in üblicher Weise, z. B. durch Behandeln mit einer Säure oder einem geeigneten Anionenaustauscherreagenz. Innere Salze von Verbindungen der Formel I, welche saure und basische salzbildende Gruppen enthalten, z. B. eine freie Carboxygruppe und eine freie Aminogruppe, können z.B. durch Neutralisieren von Salzen, wie Säureadditionssalzen, auf den isoelektrischen Punkt, z. B. mit schwachen Basen, oder durch Behandeln mit Ionenaustauschern gebildet werden.

Salze können in üblicher Weise in die freien Verbindungen überführt werden, Metall- und Ammoniumsalze beispielsweise durch Behandeln mit geeigneten Säuren oder sauren Ionenaustauschem, und Säureadditionssalze beispielsweise durch Behandeln mit einem geeigneten basischen Mittel oder basischen Ionenaustauschern.

Stereoisomerengemische, also Gemische von Diastereomeren und/oder Enantiomeren, wie beispielsweise racemische Gemische, können in an sich bekannter Weise durch geeignete Trennverfahren in die entsprechenden Isomeren aufgetrennt werden. So können Diastereomerengemische durch fraktionierte Kristallisation, Chromatographie, Lösungsmittelverteilung etc. in die einzelnen Diastereomeren aufgetrennt werden. Racemate können nach Überführung der optischen Antipoden in Diastereomere, beispielsweise durch Umsetzung mit optisch aktiven Verbindungen, z. B. optisch aktiven Säuren oder Basen, durch Chromatographie an

mit optisch aktiven Verbindungen belegten Säulenmaterialien oder durch enzymatische Methoden, z. B. durch selektive Umsetzung nur eines der beiden Enantiomeren, voneinander getrennt werden. Diese Trennung kann sowohl auf der Stufe eines der Ausgangsprodukte als auch bei den Verbindungen der Formel I selbst erfolgen.

In einer erhältlichen Verbindung der Formel I, worin die Hydroxygruppe frei ist, welche an das Kohlenstoffatom gebunden ist, das dem Kohlenstoffatom benachbart ist, welches den Rest $R_2$-$CH_2$- trägt, kann durch Einführung einer Schutzgruppe, wie oben unter Verfahren a) beschrieben, die freie Hydroxygruppe in eine geschützte Hydroxygruppe überführt weden, beispielsweise in eine veresterte Hydroxygruppe, beispielsweise in Niederalkanoyloxy, wie Acetyloxy. Die Veresterung erfolgt analog zu der unter Verfahren a) genannten Kondensation zu Amiden, wobei an Stelle der Aminokomponente eine Hydroxygruppe reagiert. Die Reaktion erfolgt vorzugsweise unter analogen Bedingungen, wie unter Verfahren a) beschrieben, insbesondere unter Verwendung eines Niederalkanoylanhydrides, z. B. Acetanhydrid, zur Bildung der entsprechenden Niederalkanoyloxygruppe, in einem organischen Lösungsmittel, z. B. einem cyclischen Ether, wie Tetrahydrofuran, in Gegenwart eines cyclischen tertiären Amines, wie Dimethylaminopyridin, und/oder eines Triniederalkylamines, wie Triethylamin, bei Temperaturen zwischen 0 °C und dem Siedepunkt des Reaktionsgemischen, insbesondere zwischen 10 und 30 °C.

In einer erhältlichen Verbindung der Formel I kann man eine Amino- oder Carboxamidgruppe substituieren, eine in freier oder in reaktionsfähiger Form vorliegende Carboxygruppe verestern oder amidieren bzw. eine veresterte oder amidierte Carboxygruppe in eine freie Carboxygruppe überführen.

Die Substitution einer Carboxamidgruppe oder einer anderen primären oder sekundären Aminogruppe, beispielsweise zur Herstellung der oben als Substituent von aus $R_4$ und $R_5$ zusammen mit dem bindenden Stickstoffatom gebildetem Thiomorpholino oder Morpholino genannten Carbamoylderivate Mono- oder Diniederalkylcarbamoyl, oder Mono- oder Di-hydroxyniederalkylcarbamoyl, oder unter Bildung der oben genannten Derivate des Substituenten Amino an aus $R_4$ und $R_5$ gemeinsam mit dem bindenden Stickstoffatom gebildetem Thiomorpholino oder Morpholino, in Verbindungen der Formel I, in denen der Stickstoff der umzusetzenden Aminogruppen an Wasserstoff gebunden ist, erfolgt z. B. durch Alkylierung.

Geeignete Mittel zur Alkylierung einer Carboxamidgruppe in einer Verbindung der Formel I sind beispielsweise Diazoverbindungen, z. B. Diazomethan. Man kann Diazomethan in einem inerten Lösungsmittel zersetzen, wobei das gebildete freie Methylen mit der Carboxamidgruppe in der Verbindung der Formel I reagiert. Die Zersetzung von Diazomethan erfolgt vorzugsweise katalytisch, z. B. in Gegenwart eines Edelmetalls in fein verteilter Form, z. B. Kupfer, oder eines Edelmetallsalzes, z. B. Kupfer(I)-chlorid oder Kupfer(II)-sulfat.

Alkylierungsmittel sind auch in der Deutschen Offenlegungsschrift 2 331 133 genannt, z. B. Alkylhalogenide, Sulfonsäureester, Meerweinsalze oder 1-substituierte 3-Aryltriazene, welche man unter den dort genannten Reaktionsbedingungen mit einer Verbindung der Formel I mit einer Carboxamidgruppe umsetzen kann.

Weitere Alkylierungsmittel sind ausgewählt aus entsprechenden Alkylverbindungen, die einen Substituenten X tragen, worin X eine Abgangsgruppe ist. Eine Abgangsgruppe ist insbesondere eine nukleofuge Abgangsgruppe ausgewählt aus mit einer starken anorganischen oder organischen Säure verestertem Hydroxy, wie mit einer Mineralsäure, z. B. Halogenwasserstoffsäure, wie Chlorwasserstoff-, Bromwasserstoff- oder Iodwasserstoffsäure, oder mit einer starken organischen Sulfonsäure, wie einer gegebenenfalls, z. B. durch Halogen, wie fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, Trimethansulfon- oder p-Toluolsulfonsäure, verestertes Hydroxy oder mit Stickstoffwasserstoffsäure verestertes Hydroxy.

Die Reaktion kann unter den Bedingungen einer nukleophilen Substitution erster oder zweiter Ordnung ablaufen.

Beispielsweise kann man eine der Verbindungen mit einem Substituenten X, worin X für eine Abgangsgruppe mit hoher Polarisierbarkeit der Elektronenhülle, z. B. für Brom oder Iod, steht, in einem polaren aprotischen Lösungsmittel, z. B. Aceton, Acetonitril, Nitromethan, Dimethylsulfoxid oder Dimethylformamid, umsetzen. Die Substitutionsreaktion wird gegebenenfalls bei erniedrigter oder erhöhter Temperatur, z. B. in einem Temperaturbereich von etwa -40 ° bis etwa 100 °C, bevorzugt von etwa -10 ° bis etwa 50 °C, und gegebenenfalls unter Inertgas, z. B. Stickstoff- oder Argonatmosphäre, durchgeführt.

Zur Veresterung oder Amidierung einer Carboxygruppe in einer Verbindung der Formel I, beispielsweise zur Amidierung einer freien Carboxygruppe einer Aminosäure, wie Glu oder Asp, mit Ammoniak, oder einer freien Carboxygruppe an durch $R_4$ und $R_5$ gemeinsam mit dem bindenden Stickstoffatom gebildetem Thiomorpholino oder Morpholino, kann man, wenn erwünscht, die freie Säure verwenden oder die freie Säure in eines der oben genannten reaktionsfähigen Derivate überführen und mit einem Alkohol, Ammoniak, einem primären oder einem sekundären Amin umsetzen, oder man kann zur Veresterung die freie Säure oder ein reaktionsfähiges Salz, z. B. das Cäsiumsalz, mit einem reaktionsfähigen Derivat eines Alkohols umsetzen. Beispielsweise kann man das Cäsiumsalz einer Carbonsäure mit einem dem Alkohol entsprechenden Halogenid oder Sulfonsäureester umsetzen. Die Veresterung der Carboxygruppe kann auch mit anderen üblichen Alkylierungsmitteln erfolgen, z. B. mit Diazomethan, Alkylhalogeniden, Sulfonsäureestern, Meerweinsalzen oder 1-substituierten 3-Aryltriazenen, etc.

Zur Überführung einer veresterten oder amidierten Carboxygruppe in die freie Carboxygruppe kann eine der oben bei der Abspaltung der Carboxyschutzgruppen beschriebenen Methoden oder gewünschtenfalls eine alkalische Verseifung unter üblichen, wie den im Organikum, 17. Auflage, VEB Deutscher Verlag der Wissenschaften, Berlin 1988, genannten Reaktionsbedingungen angewendet werden.

In einer Verbindung der Formel I kann man eine veresterte Carboxygruppe durch Aminolyse mit Ammoniak oder einem primären oder sekundären Amin in eine gegebenenfalls substituierte Carboxamidgruppe überführen. Die Aminolyse kann nach den üblichen, wie den im Organikum, 15. Auflage, VEB Deutscher Verlag der Wissenschaften, Berlin (Ost) 1976, für derartige Umsetzungen genannten Reaktionsbedingungen erfolgen.

In einer Verbindung der Formel I kann man eine vorhandene freie Aminogruppe acylieren, beispielsweise zur Einführung eines der für $R_1$ ausser Wasserstoff genannten Reste. Die Acylierung erfolgt nach der oben unter Verfahren a) oder einer der für Schutzgruppen genannten Methoden oder beispielsweise nach einem der im Organikum, 17. Auflage, VEB Deutscher Verlag der Wissenschaften, Berlin (Ost) 1988, genannten Verfahren.

In einer erhältlichen Verbindung der Formel I, worin die Substituenten die genannten Bedeutungen haben und mindestens eine freie Hydroxygruppe vorhanden ist und die übrigen funktionellen Gruppen in geschützter Form vorliegen, kann man die freie Hydroxygruppe acylieren oder verethern, beispielsweise die Hydroxygruppe an Thiomorpholino oder Morpholino, das aus $R_4$ und $R_5$ gemeinsam mit dem bindenden Stickstoffatom gebildet wird.

Die Acylierung kann erfolgen mit acylierenden Reagentien nach einer der unter Verfahren a) bis e) oder nach einer der für Schutzgruppen genannten Methoden oder nach einem der im Organikum, 17. Auflage, VEB Deutscher Verlag der Wissenschaften, Berlin (Ost) 1988, genannten Verfahren.

Die Veretherung kann mit den oben genannten Alkylierungsmitteln und unter den gleichen Reaktionsbedingungen erfolgen, z. B. mit Diazomethan, Alkylhalogeniden, Sulfonsäureestern, Meerweinsalzen, 1-substituierten 3-Aryltriazenen, etc.

In einer Verbindung der Formel I kann man vorhandene Schutzgruppen oder geeignete Reste $R_1$ ausser Wasserstoff nach einem der unter Verfahren f) genannten Verfahren abspalten, insbesondere durch Hydrolyse, beispielsweise in Gegenwart von Basen, wie Alkali- oder Erdalkalihydroxiden, z. B. Natriumhydroxid, oder Säuren, wie organischen Säuren oder Mineralsäuren, z. B. Halogenwasserstoff, wie Chlorwasserstoff. Die Hydrolyse erfolgt unter den üblichen Bedingungen, beispielsweise in wässriger Lösung oder in wasserfreien Lösungsmitteln, insbesondere in Ethern, wie Dioxan, bei Temperaturen zwischen -50 °C und der Rücckflusstemperatur der entsprechenden Reaktionsgemische, z. B. zwischen 0 °C und 50 °C, vorzugsweise in Gegenwart eines Schutzgases, wie Argon oder Stickstoff.

In einer Verbindung der Formel I, in der wenigstens einer der Reste $R_2$ oder $R_3$ eine Phenylgruppe bedeutet und/oder einer oder mehrere der Reste $B_1$, $A_1$ oder $A_2$ Phenylalanin bedeutet, wobei die Phenylreste auch jeweils, wie oben beschrieben, substituiert sein können, kann der oder können die entsprechenden Phenylreste selektiv zu entsprechenden Cyclohexylresten reduziert, beispielsweise hydriert, werden. Die Hydrierung erfolgt vorzugsweise in Gegenwart eines Katalysators, der die selektive Hydrierung von Doppelbindungen in Gegenwart von Peptidbindungen erlaubt, insbesondere eines Katalysators aus Schwermetalloxiden, wie eines Rh(III)/Pt(VI)-Oxidkatalysators nach Nishimura (S. Nishimura, Bull. Chem. Soc. Japan 33, 566 (1960), in geeigneten Lösungsmitteln, insbesondere Wasser, Alkoholen, wie Methanol oder Ethanol, Estern, wie Essigsäureethylester, oder Ethern, wie Dioxan, z. B.in Methanol, bei Temperaturen von 0 bis 150 °C, vorzugsweise 10 bis 50 °C, z. B. bei Raumtemperatur, und bei Wasserstoffdrucken von 1 bis 50 bar, z. B. bei Normaldruck.

Die Erfindung betrifft auch diejenigen Ausführungsformen des Verfahrens, bei denen man von einer auf irgend einer Stufe als Zwischenprodukt erhältlichen Verbindung ausgeht und die fehlenden Schritte durchführt oder das Verfahren auf irgendeiner Stufe abbricht oder einen Ausgangsstoff unter den Reaktionsbedingungen bildet oder in Form eines reaktionsfähigen Derivates oder Salzes verwendet oder eine nach dem erfindungsgemässen Verfahren erhältliche Verbindung unter den Verfahrensbedingungen erzeugt und in situ weiterverarbeitet. Dabei geht man vorzugsweise von solchen Ausgangsstoffen aus, die zu den Verbindungen führen, die oben als bevorzugt, als stärker bevorzugt, als sehr bevorzugt, als noch bevorzugter, als in erster Linie betroffen, in allererster Linie betroffen oder als ganz besonders wichtig beschrieben sind.

## Pharmazeutische Präparate:

Die Erfindung betrifft auch pharmazeutische Präparate enthaltend Verbindungen der Formel I.

Die pharmakologisch verwendbaren Verbindungen der vorliegenden Erfindung können z. B. zur Herstellung von pharmazeutischen Präparaten verwendet werden, welche eine wirksame Menge des Wirkstoffes zusammen oder im Gemisch mit einer signifikanten Menge von anorganischen oder organischen, festen oder flüssigen, pharmazeutisch verwendbaren Trägerstoffen enthalten.

Bei den erfindungsgemässen pharmazeutischen Präparaten handelt es sich um solche zur enteralen, wie nasalen, bukkalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, dem Alter und dem individuellen Zustand, individuellen pharmakokinetischen Gegebenheiten, der zu behandelnden Krankheit sowie der Applikationsweise ab.

Die Erfindung betrifft auch pharmazeutische Präparate und eine Methode zur Behandlung von durch Retroviren verursachten Krankheiten, z. B. von AIDS, insbesondere, wenn HIV-1 die Erkrankung verursacht, dadurch gekennzeichnet, dass eine therapeutisch wirksame Menge von erfindungsgemässen Verbindungen der Formel I, insbesondere an einen Warmblüter, z. B. Menschen, der wegen einer der genannten Erkrankungen, insbesondere AIDS, einer derartigen Behandlung bedarf, verabreicht wird. Die an Warmblüter, z. B. Menschen von etwa 70 kg Körpergewicht, zu verabreichenden Dosismengen liegen zwischen etwa 3 mg und etwa 10 g, vorzugsweise zwischen etwa 40 mg und etwa 4 g, z. B. bei ungefähr 300 mg bis 1,5 g pro Person und Tag, verteilt auf vorzugsweise 1 bis 3 Einzeldosen, die z. B. gleich gross sein können. Ueblicherweise erhalten Kinder die halbe Dosis von Erwachsenen.

Die pharmazeutischen Präparate enthalten von etwa 1 % bis etwa 95 %, vorzugsweise von etwa 20 % bis etwa 90 % des Wirkstoffes. Erfindungsgemässe pharmazeutische Präparate können z. B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z. B. mittels konventioneller Lösungs-, Lyophilisierungs-, Misch-, Granulier- oder Dragierverfahren, hergestellt.

Vorzugsweise verwendet man Lösungen des Wirkstoffes, daneben auch Suspensionen oder Dispersionen, und zwar insbesondere isotonische wässrige Lösungen, Dispersionen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z. B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Öl enthalten als ölige Komponente die für Injektionszwecke üblichen vegetabilen, synthetischen oder halbsynthetischen Öle. Als solche sind insbesondere flüssige Fettsäureester zu nennen, die als Säurekomponente eine langkettige Fettsäure mit 8-22, insbesondere 12-22, Kohlenstoffatomen, wie z. B. Laurinsäure, Tridecylsäure, Myristinsäure, Pentadecylsäure, Palmitinsäure, Margarinsäure, Stearinsäure, Arachidinsäure, Behensäure oder entsprechende ungesättigte Säuren, wie z. B. Ölsäure, Elaidinsäure, Erucasäure, Brasidinsäure oder Linolsäure, enthalten, gegebenenfalls unter Zusatz von Antioxidantien, wie z. B. Vitamin E, β-Carotin oder 3,5-Di-tert-butyl-4-hydroxytoluol. Die Alkoholkomponente dieser Fettsäureester hat maximal 6 Kohlenstoffatome und ist ein ein- oder mehrwertiger, z. B. ein-, zwei- oder dreiwertiger Alkohol, z. B. Methanol, Ethanol, Propanol, Butanol oder Pentanol oder deren Isomere, vor allem aber Glycol und Glycerin. Als Fettsäureester sind daher beispielsweise zu nennen: Ethyloleat, Isopropylmyristat, Isopropylpalmitat, "Labrafil M 2375 " (Polyoxyethylenglycerintrioleat der Firma Gattefossé, Paris), "Miglyol 812" (Triglycerid gesättigter Fettsäuren der Kettenlänge $C_8$ bis $C_{12}$ der Firma Hüls AG, Deutschland), besonders aber vegetabile Öle wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und, wenn erwünscht oder notwendig, nach Zugabe von geeigneten Hilfsstoffen, zu Tabletten, Dragée-Kernen oder Kapseln verarbeitet, oder durch Herstellung von Dispersionen, vorzugsweise mit Phospholipiden, die in Gläschen abgefüllt werden. Dabei kann man die Wirkstoffe auch in Kunststoffträger einbauen, die sie dosiert abgeben oder diffundieren lassen.

Geeignete Trägerstoffe sind insbesondere Füllstoffe, wie Zucker, z. B. Lactose, Saccharose, Mannit oder Sorbit, Cellulosepräparate, und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium-, oder Calciumstearat, und/oder Polyethylenglycol. Dragée-Kerne werden mit geeigneten, gegebenenfalls magensaftresistenten Überzügen versehen, wobei man u.a. konzentrierte Zuckerlösungen, welche gegebenenfalls arabischen Gummi, Talk, Polyvinylpyrrolidon, Polyethylenglycol und/oder Titandioxid enthalten, Lacklösungen in geeigneten organischen Lösungsmitteln oder, zur Herstellung von magensaftresistenten Überzügen, Lösungen von geeigneten Cellulosepräparaten, wie Ethylcellulosephthalat oder Hydroxypropylmethylcellulosephthalat, verwendet. Kapseln sind Steckkapseln aus Gelatine sowie weiche, geschlossene Kapseln aus Gelatine und einem Weichmacher, wie Glycerin oder Sorbit. Die Steckkapseln können den Wirkstoff in Form eines Granulats, z. B. mit Füllstoffen, wie Lactose, Bindemitteln, wie Stärken, und/oder Gleitmitteln, wie Talk oder Magnesiumstearat, und gegebenenfalls mit Stabilisatoren, enthalten. In weichen Kapseln ist der Wirkstoff vorzugsweise in geeigneten öligen Hilfsstoffen, wie fetten Ölen, Paraffinöl oder flüssigen Polyethylenglykolen, gelöst oder suspendiert, wobei ebenfalls Stabilisatoren und/oder antibakterielle Mittel zugefügt sein können. Den Tabletten oder Dragée-Überzügen und den Kapselhüllen können Farbstoffe oder Pigmente, z. B. zur Identifizierung oder zur Kennzeichnung verschiedener Wirkstoffdosen, beigefügt werden.

Als pharmazeutische Präparate besonders bevorzugt sind durch Phospholipide stabilisierte Dispersionen des Wirkstoffes, vorzugsweise zur oralen Applikation, enthaltend

a) ein Phospholipid oder mehrere Phospholipide der Formel

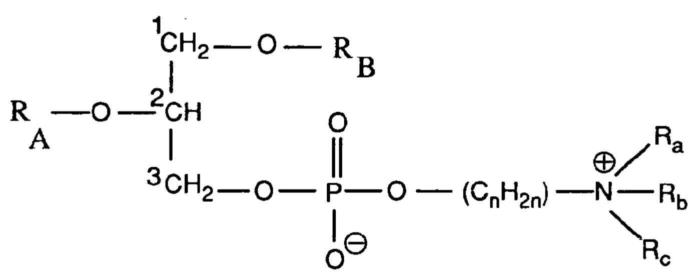

(A),

worin $R_A$ $C_{10-20}$-Acyl, $R_B$ Wasserstoff oder $C_{10-20}$-Acyl, $R_a$, $R_b$ und $R_c$ Wasserstoff oder $C_{14}$-Alkyl und n eine ganze Zahl von zwei bis vier darstellen, gewünschtenfalls b) ein weiteres Phospholipid oder mehrere weitere Phospholipide

c) den Wirkstoff und

d) eine pharmazeutisch annehmbare Trägerflüssigkeit und gewünschtenfalls weitere Hilfsstoffe und/oder Konservierungsmittel.

Das Herstellungsverfahren für diese Dispersionen ist dadurch gekennzeichnet, dass man eine Lösung oder Suspension der Komponenten a) und c) oder a), b) und c), vorzugsweise von a) und b) in einem Gewichtsverhältnis von 20: 1 bis 1 : 5, insbesondere von 5 : 1 bis 1 : 1, durch Verdünnung mit Wasser in eine Dispersion umwandelt, anschliessend das organische Lösungsmittel entfernt, beispielsweise durch Zentrifugation, Gelfiltration, Ultrafiltration oder insbesondere durch Dialyse, z. B. tangentiale Dialyse, vorzugsweise gegen Wasser, die erhaltene Dispersion, vorzugsweise nach Zugabe von Hilfsstoffen oder Konservierungsmitteln, erforderlichenfalls unter Einstellung eines annehmbaren pH-Wertes durch Zugabe von pharmazeutisch annehmbaren Puffern, wie Phosphatsalzen oder organischen Säuren (rein oder gelöst in Wasser), wie Essigssäure oder Zitronensäure, vorzugsweise zwischen pH 3 und 6, z. B. pH 4 - 5, falls sie nicht bereits die richtige Wirkstoffkonzentration hat, konzentriert, vorzugsweise auf eine Wirkstoffkonzentration von 2 bis 30 mg/ml, insbesondere von 10 bis 20 mg/ml, wobei die Konzentrierung vorzugsweise nach den zuletzt genannten Methoden zur Entfernung eines organischen Lösungsmittels erfolgt, insbesondere durch Ultrafiltration, z. B. unter Verwendung einer Apparatur zur Durchführung tangentialer Dialyse und Ultrafiltration.

Die nach diesem Verfahren herstellbare, durch Phospholipide stabilisierte Dispersion ist bei Zimmertemperatur mindestens mehrere Stunden stabil, reproduzierbar bezüglich des Mengenanteils der Komponenten und toxikologisch unbedenklich und daher insbesondere für die orale Applikation am Menschen geeignet.

Die Grössenordnung der erhaltenen Partikel in der Dispersion ist variabel und liegt vorzugsweise zwischen ca. $1,0 \times 10^{-8}$ bis ca. $1,0 \times 10^{-5}$ m, insbesondere zwischen etwa $10^{-7}$ und etwa $2 \times 10^{-6}$ m.

Die Nomenklatur der Phospholipide der Formel I und die Bezifferung der C-Atome erfolgt anhand der in

Eur. J. of Biochem. 79, 11-21 (1977) "Nomenclature of Lipids" von der IUPAC-IUB Commission on Biochemical Nomenclature (CBN) gegebenen Empfehlungen (sn-Nomenklatur, stereospecific numbering).

In einem Phospholipid der Formel A sind $R_A$ und $R_B$ mit den Bedeutungen $C_{10-20}$-Acyl vorzugsweise geradkettiges $C_{10-20}$-Alkanoyl mit einer geraden Anzahl an C-Atomen und geradkettiges $C_{10-20}$-Alkenoyl mit einer Doppelbindung und einer geraden Anzahl an C-Atomen.

Geradkettiges $C_{10-20}$-Alkanoyl $R_A$ und $R_B$ mit einer geraden Anzahl an C-Atomen sind beispielsweise n-Dodecanoyl, n-Tetradecanoyl, n-Hexadecanoyl oder n-Octadecanoyl.

Geradkettiges $C_{10-20}$-Alkenoyl $R_A$ und $R_B$ mit einer Doppelbindung und einer geraden Anzahl an C-Atomen sind beispielsweise 6-cis-, 6-trans-, 9-cis- oder 9-trans-dodecenoyl, -tetradecenoyl, -hexadecenoyl, -octadecenoyl oder -icosenoyl, insbesondere 9-cis-octadecenoyl (Oleoyl).

In einem Phospholipid der Formel A ist n eine ganze Zahl von zwei bis vier, vorzugsweise zwei. Die Gruppe der Formel $-(C_nH_{2n})-$ stellt unverzweigtes oder verzweigtes Alkylen dar, z.B. 1,1-Ethylen, 1,1-, 1,2- oder 1,3-Propylen oder 1,2-, 1,3- oder 1,4-Butylen. Bevorzugt ist 1,2-Eethylen (n=2).

Phospholipide der Formel A sind beispielsweise natürlich vorkommende Kephaline, worin $R_a$, $R_b$ und $R_c$ Wasserstoff bedeuten oder natürlich vorkommende Lecithine, worin $R_a$, $R_b$ und $R_c$ Methyl bedeuten, z.B. Kephalin oder Lecithin aus Sojabohnen, Rinderhirn, Rinderleber oder Hühnerei mit verschiedenen oder identischen Acylgruppen $R_A$ und $R_B$ oder Mischungen davon.

Bevorzugt sind synthetische, im wesentlichen reine Phospholipide der Formel A mit verschiedenen oder identischen Acylgruppen $R_A$ und $R_B$.

Der Begriff "synthetisches" Phospholipid der Formel A definiert Phospholipide, welche bezüglich $R_A$ und $R_B$ eine einheitliche Zusammensetzung haben. Solche synthetischen Phospholipide sind vorzugsweise die unten definierten Lecithine und Kephaline, deren Acylgruppen $R_A$ und $R_B$ eine definierte Struktur haben und von einer definierten Fettsäure mit einem Reinheitsgrad höher als ca. 95 % abgeleitet sind. $R_A$ und $R_B$ können gleich oder verschieden und ungesättigt oder gesättigt sein. Bevorzugt ist $R_A$ gesättigt, z.B. n-Hexadecanoyl, und $R_B$ ungesättigt, z.B. 9-cis-octadecenoyl (= Oleoyl).

Der Begriff "natürlich vorkommende" Phospholipide der Formel A definiert Phospholipide, welche bezüglich $R_A$ und $R_B$ keine einheitliche Zusammensetzung haben. Solche natürlichen Phospholipide sind ebenfalls Lecithine und Kephaline, deren Acylgruppen $R_A$ und $R_B$ strukturell undefinierbar und von natürlich vorkommenden Fettsäuregemischen abgeleitet sind.

Der Begriff "im wesentlichen reines" Phospholipid definiert einen Reinheitsgrad von mehr als 70 % (Gew.) des Phospholipids der Formel A, welcher anhand geeigneter Bestimmungsmethoden, z.B. papierchromatographisch, nachweisbar ist.

Besonders bevorzugt sind synthetische, im wesentlichen reine Phospholipide der Formel A, worin $R_A$ die Bedeutung geradkettiges $C_{10-20}$-Alkanoyl mit einer geraden Anzahl an C-Atomen und $R_B$ die Bedeutung geradkettiges $C_{10-20}$-Alkenoyl mit einer Doppelbindung und einer geraden Anzahl an C-Atomen haben. $R_a$, $R_b$ und $R_c$ sind Methyl und n ist zwei.

In einem besonders bevorzugten Phospholipid der Formel A bedeuten $R_A$ n-Dodecanoyl, n-Tetradecanoyl, n-Hexadecanoyl oder n-Octadecanoyl und $R_B$ 9-cis-Dodecenoyl, 9-cis-Tetradecenoyl, 9-cis-Hexadecenoyl, 9-cis-octadecenoyl oder 9-cis-Icosenoyl. $R_a$, $R_b$ und $R_c$ sind Methyl und n ist zwei.

Ein ganz besonders bevorzugtes Phospholipid der Formel A ist synthetisches 1-n-Hexadecanoyl-2-(9-cis-octadecenoyl)-3-sn-phosphatidylcholin mit einer Reinheit von mehr als 95 %.

Bevorzugte natürliche, im wesentlichen reine Phospholipide der Formel A sind insbesondere Lecithin (L-$\alpha$-Phosphatidylcholin) aus Sojabohnen oder Hühnerei.

Für die Acylreste in den Phospholipiden der Formeln A sind auch die in Klammern angegebenen Bezeichnungen gebräuchlich:

9-cis-Dodecenoyl (Lauroleoyl), 9-cis-Tetradecenoyl (Myristoleoyl), 9-cis-Hexadecenoyl (Palmitoleoyl), 6-cis-octadecenoyl (Petroseloyl), 6-trans-Octadecenoyl (Petroselaidoyl), 9-cis-Octadecenoyl (Oleoyl), 9-trans-Octadecenoyl (Elaidoyl), 11-cis-Octadecenoyl (Vaccenoyl), 9-cis-Icosenoyl (Gadoleoyl), n-Dodecanoyl (Lauroyl), n-Tetradecanoyl (Myristoyl), n-Hexadecanoyl (Palmitoyl), n-Octadecanoyl (Stearoyl), n-Icosanoyl (Arachidoyl).

Weitere Phospholipide sind vorzugsweise Ester von Phosphatidsäure (3-sn-Phosphatidsäure) mit den genannten Aclyresten, wie Phosphatidylserin und Phosphatidylethanolamin.

Schwerlösliche Wirkstoffe können auch als wasserlösliche, pharmazeutisch annehmbare Salze vorliegen, wie oben definiert.

In der Trägerflüssigkeit d) sind die Komponenten a), b) und c) oder a) und c) als Liposomen so enthalten, dass sich mehrere Tage bis Wochen keine Feststoffe oder feste Aggregate wie Mizellen zurückbilden und die Müssigkeit mit den genannten Komponenten, gegebenenfalls nach Filtration, vorzugsweise oral, applizierbar

ist.

In der Trägerflüssigkeit d) können pharmazeutisch annehmbare, nicht toxische Hilfsstoffe enthalten sein, z.B. wasserlösliche Hilfsstoffe welche zur Herstellung von isotonischen Bedingungen geeignet sind, z.B. ionische Zusätze wie Kochsalz oder nichtionische Zusätze (Gerüstbildner) wie Sorbit, Mannit oder Glucose oder wasserlösliche Stabilisatoren für die Liposomendispersion wie Lactose, Fructose oder Sucrose.

Zusätzlich zu den wasserlöslichen Hilfsstoffen können in der Trägeflüssigkeit für flüssige pharmazeutische Formulierungen verwendbare Emulgatoren, Netzmittel oder Tenside vorhanden sein, insbesondere Emulgatoren wie Ölsäure, nichtionische Tenside vom Typ Fettsäure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestern wie Polyoxyethylen-sorbitanmonolaurat, -oleat, -stearat, -palmitat, -tristearat oder -trioleat, Polyethylenglycol-Fettsäureester wie Polyoxyethylstearat, Polyethylenglycol-400-stearat, Polyethylenglycol-2000-stearat, insbesondere Ethylenoxid-Propylenoxid Blockpolymere vom Typ Pluronic® (Wyandotte Chem. Corp.) oder Synperonic® (ICI).

Bevorzugte Konservierungsmittel sind z. B. Antioxidantien, wie Ascorbinsäure, oder Microbizide, wie Sorbinsäure oder Benzoesäure.

Ausgangsmaterialien:

Neue Ausgangsmaterialien und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den als bevorzugt aufgeführten Verbindungen gelangt.

Bei der Herstellung aller Ausgangsmaterialien können freie funktionelle Gruppen, die nicht an der jeweiligen Reaktion teilnehmen sollen, ungeschützt oder in geschützter Form vorliegen, beispielsweise geschützt durch die oben unter Verfahren a) genannten Schutzgruppen. Diese Schutzgruppen können zu geeigneten Zeitpunkten durch die unter Verfahren f) beschriebenen Reaktionen freigesetzt werden. Die Verbindungen mit salzbildenden Gruppen können jeweils auch als Salze Verwendung finden und Salze auf jeder Stufe hergestellt oder wieder in die freien Verbindungen überführt werden.

In den Formeln wird, sofern nicht die Stereochemie asymmetrischer Kohlenstoffatome direkt durch die Wahl entsprechender Bindungssymbole definiert wird, die Konfiguration asymmetrischer Kohlenstoffatome durch die jeweils angegebene Konfigurationsbezeichnung ausgewählt aus (S), (R) und (S,R) bezeichnet.

Die Carbon- oder Sulfonsäuren der Formel II, oder reaktionsfähige Derivate hiervon, sind bekannt und sind kommerziell erhältlich oder können nach an sich bekannten Verfahren hergestellt werden.

Die Verbindungen der Formel III sind bekannt oder können nach an sich bekannten Verfahren hergestellt werden. Beispielsweise sind sie zugänglich aus Verbindungen der Formel

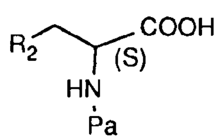

(XII),

worin $R_2$ die für Verbindungen der Formel I genannten Bedeutungen hat und Pa eine Aminoschutzgruppe, insbesondere Niederalkoxycarbonyl, wie tert-Butoxycarbonyl, oder Phenylniederalkoxycarbonyl, wie Benzyloxycarbonyl, bedeutet, durch Reduktion in eine Verbindung der Formel

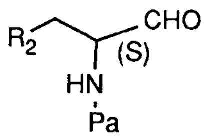

(XIII)

überführt, worin die Reste die zuletzt genannten Bedeutungen haben.

Die Reduktion von Aminosäurederivaten der Formel XII zu den entsprechenden Aldehyden XIII erfolgt beispielsweise durch Reduktion zu den entsprechenden Alkoholen und anschliessende Oxidation zu den Aldehyden der Formel XIII.

Die Reduktion zu den Alkoholen erfolgt insbesondere durch Hydrogenierung der entsprechenden Säurehalogenide oder anderweitiger, unter Verfahren a) genannter aktivierter Carbonsäurederivate, oder durch Umsetzung aktivierter Carbonsäurederivate der Verbindungen der Formel XII, insbesondere von Anhydriden mit

organischen Carbonsäuren, vorzugsweise solcher von Halogenameisensäureestern, wie Chlorameisensäureisobutylester (die vorzugsweise durch Umsetzung der Verbindungen der Formel XII in Gegenwart von basischen Aminen, z. B. Triniederalkylaminen, wie Triethylamin, in organischen Lösungsmitteln, wie cyclischen Ethern, z. B. Dioxan, bei Temperaturen zwischen -50 und 80 °C, vorzugsweise zwischen 0 und 50 °C, erhalten werden) mit komplexen Hydriden, wie Alkaliborhydriden, z. B. Natriumborhydrid, in wässriger Lösung in An- oder Abwesenheit der zuletzt verwendeten organischen Lösungsmittel bei Temperaturen zwischen -50 und 80 °C, vorzugsweise zwischen 0 und 50 °C. Die anschliessende Oxidation der erhaltenen Alkohole erfolgt vorzugsweise mit solchen Oxidationsmitteln, welche selektiv die Hydroxy- in eine Aldehydgruppe umwandeln, beispielsweise Chromsäure oder ihre Derivate, wie Pyridiniumchromat oder tert-Butylchromat, Dichromat/Schwefelsäure, Schwefeltrioxid in Gegenwart von heterocyclischen Basen, wie Pyridin/$SO_3$, Diniederalkylsulfoxiden, wie Dimethylsulfoxid, ferner Salpetersäure, Braunstein oder Selendioxid, in Wasser, wässrigen oder organischen Lösungsmitteln, wie halogenierten Lösungsmitteln, z. B. Methylenchlorid, Carbonsäureamiden, wie Dimethylformamid, und/oder cyclischen Ethern, wie Tetrahydrofuran, in Gegenwart oder Abwesenheit von basischen Aminen, z. B. Triniederalkylaminen, wie Triethylamin, bei Temperaturen zwischen -70 bis 100 °C, vorzugsweise zwischen -70 bis -50 °C oder bei -10 bis 50 °C, beispielsweise wie in der Europäischen Patentanmeldung EP-A-0 236 734 beschrieben.

Auch die direkte Reduktion der Verbindungen der Formel XII zu den Aldehyden ist möglich, beispielsweise durch Hydrierung in Gegenwart eines partiell vergifteten Palladiumkatalysators oder durch Reduktion der entsprechenden Aminosäureester, z. B. der Niederalkylester, wie Ethylester, mit komplexen Hydriden, z. B. Borhydriden, wie Natriumborhydrid, oder vorzugsweise Aluminiumhydriden, z. B. Lithiumaluminiumhydrid, Lithium-tri-(tert-butoxy)aluminiumhydrid oder insbesondere Diisobutylaluminiumhydrid, in apolaren Lösungsmitteln, z. B. in Kohlenwasserstoffen oder aromatischen Lösungsmitteln, wie Toluol, bei - 100 bis 0 °C, bevorzugt -70 bis -30 °C, und anschliessende Umsetzung zu den entsprechenden Semicarbazonen, z. B. mit den entsprechenden Säuresalzen von Semicarbazonen, wie Semicarbazidhydrochlorid, in wässrigen Lösungsmittelsystemen, wie Alkohol/Wasser, z. B. Ethanol/Wasser, bei Temperaturen zwischen -20 und 60 °C, bevorzugt 10 bis 30 °C, und Umsetzung des erhaltenen Semicarbazones mit einem reaktiven Aldehyd, z. B. Formaldehyd, in einem inerten Lösungsmittel, beispielsweise einem polaren organischen Lösungsmittel, z. B. einem Carbonsäureamid, wie Dimethylformamid, bei Temperaturen zwischen -30 und 60 °C, bevorzugt 0 bis 30 °C, und dann einer Säure, beispielsweise einer starken Mineralsäure, wie Halogenwasserstoff, in wässriger Lösung, gegebenenfalls in Gegenwart des vorher verwendeten Lösungsmittels, bei Temperaturen zwischen -40 und 50 °C, bevorzugt zwischen - 10 und 30 °C, umsetzt. Die entsprechenden Ester werden durch Umsetzung der Aminosäuren mit den entsprechenden Carbonsäuren, beispielsweise Ethanol, analog den bei der Kondensation unter Verfahren b) verwendeten Bedingungen gewonnen, beispielsweise durch Umsetzung mit anorganischen Säurehalogeniden, wie Thionylchlorid, in organischen Lösungsmittelgemischen, wie Mischungen aus aromatischen und alkoholischen Lösungsmitteln, z. B. Toluol und Ethanol, bei Temperaturen zwischen -50 und 50 °C, bevorzugt zwischen - 10 und 20 °C.

Die Herstellung der Verbindungen der Formel XIII erfolgt in besonders bevorzugter Weise unter Bedingungen analog den in J. Org. Chem. 47, 3016 (1982), J. Org. Chem. 43, 3624 (1978) oder J. Org. Chem. 51, 3921 (1986) genannten Reaktionsbedingungen.

Zur Synthese der Verbindungen der Formel III werden sodann die Verbindungen der Formel XIII mit einem reaktiven Tetraalkylsilan, vorzugsweise einem Halogenmethyltriniederalkylsilan, wie Chlormethyltrimethylsilan, in einem inerten Lösungsmittel, beispielsweise einem Ether, wie Diethylether, einem cyclischen Ether, wie Dioxan, oder einem Ester, wie Essigsäureethylester, bei Temperaturen zwischen -100 und 50 °C, bevorzugt zwischen -65 und 40 °C, umgesetzt, wobei Verbindungen der Formel

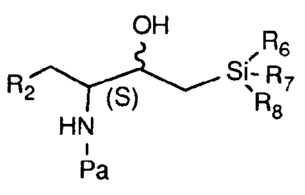

(XIV)

erhalten werden, worin $R_6$, $R_7$ und $R_8$ Niederalkyl, z. B. Methyl, bedeuten und die übrigen Reste die zuletzt genannten Bedeutungen haben, die erhaltenen Verbindungen in Gegenwart einer Lewissäure, wie Bortrifluoridethyletherat, in einem inerten Lösungsmittel, insbesondere einem halogenierten Kohlenwasserstoff, wie Methylenchlorid, mit anschliessender Nachbehandlung mit wässriger Base, z. B. Natronlauge, bei Temperaturen zwischen -30 und 80 °C, insbesondere zwischen 0 und 50 °C, unter Elimination und Schutzgruppenabspaltung in olefinische Verbindungen der Formel

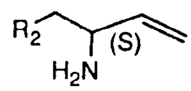

(XV)

überführt, worin $R_2$ die für Verbindungen der Formel I genannten Bedeutungen hat, in das entsprechende Olefin erneut eine Aminoschutzgruppe Pa einführt, wie unter Verfahren a) für die Einführung von Aminoschutzgruppen beschrieben, insbesondere mit Hilfe eines Säureanhydrides in einem chlorierten Kohlenwasserstoff, wie Methylenchlorid, bei Temperaturen zwischen -50 und 80 °C, insbesondere zwischen 0 und 35 °C, wobei ein geschütztes Amino-olefin der Formel

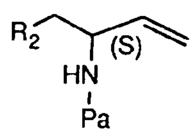

(XVI)

erhalten wird, in dem die Reste die zuletzt genannten Bedeutungen haben, die Doppelbindung in ein Oxiran umwandelt, vorzugsweise stereoselektiv unter Verwendung von Peroxiden, insbesondere Peroxycarbonsäuren, z. B. Halogeno-perbenzoesäure, wie m-Chlorperbenzoesäure, in einem inerten organischen Lösungsmittel, vorzugsweise einem halogenierten Kohlenwasserstoff, wie Methylenchlorid, bei Temperaturen zwischen -50 und 60 °C, insbesondere zwischen -10 und 25 °C, und, falls erforderlich, eine Diastereomerentrennung vornimmt, wobei Epoxide der Formel

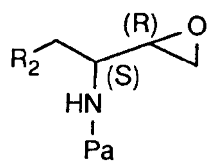

(XVII),

in denen die Reste die zuletzt genannten Bedeutungen haben, erhalten werden, an die betreffenden Olefine einen geeigneten Malonsäurediester, z. B. Malonsäuredimethylester oder Malonsäurediethylester, addiert, beispielsweise durch Aktivierung der Methylengruppe des Malonsäurediesters mittels eines Alkalimetalls, z. B. Natrium, in einem polaren wasserfreien Lösungsmittel, wie einem Alkohol, z. B. Methanol oder Ethanol, bei Temperaturen zwischen -50 und 80 °C, insbesondere zwischen 0 und 35 °C, und die Lösung mit einer Säure, wie einer Carbonsäure, z. B. Zitronensäure, behandelt, wobei ein Lacton der Formel

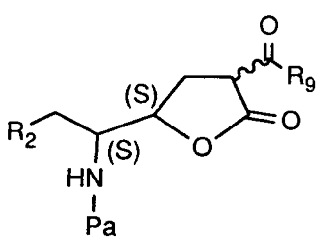

(XVIII),

worin $R_9$ Niederalkoxy, z. B. Methoxy oder Ethoxy, bedeutet und die übrigen Reste die zuletzt genannten Bedeutungen haben, erhalten wird, gewünschtenfalls in solchen Verbindungen, in denen $R_2$ Phenyl, das unsubstituiert oder wie für Verbindungen der Formel I beschrieben substituiert ist, bedeutet, diesen Rest zu Cyclohexyl reduziert, insbesondere durch Hydrierung, vorzugsweise in Gegenwart von Katalysatoren, wie Edelmetalloxiden, z. B. Gemischen von Rh(III)/Pt(VI)-Oxiden (nach Nishimura), bevorzugt in polaren Lösungsmitteln, wie Alkoholen, z. B. Methanol, bei Normaldruck oder bis zu 5 bar, bevorzugt bei Normaldruck, bei Temperaturen von -20 bis 50 °C, bevorzugt 10 bis 35 °C, die direkt oder nach der Hydrierung erhaltenen Verbindungen der Formel XVIII mit einem den Rest $R_3$-$CH_2$- einführenden Reagens, beispielsweise der Formel $R_3$-$CH_2$-W,

worin $R_3$ die für Verbindungen der Formel I genannten Bedeutungen hat und W eine nukleofuge Abgangsgruppe ausgewählt aus mit einer starken anorganischen oder organischen Säure verestertem Hydroxy, wie mit einer Mineralsäure, z. B. Halogenwasserstoffsäure, wie Chlorwasserstoff-, Bromwasserstoff- oder Iodwasserstoffsäure, oder mit einer starken organischen Sulfonsäure, wie einer gegebenenfalls, z. B. durch Halogen, wie fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, Trimethansulfon- oder p-Toluolsulfonsäure verestertes Hydroxy oder mit Stickstoffwasserstoffsäure verestertes Hydroxy, insbesondere Bromid, bedeutet, in einem wasserfreien polaren Lösungsmittel, z. B. einem Alkohol, wie Ethanol, in Gegenwart eines Alkalimetalles, z. B. Natrium, bei Temperaturen zwischen -50 und 80 °C, bevorzugt zwischen 0 und 35 °C, umsetzt unter Erhalt von Verbindungen der Formel

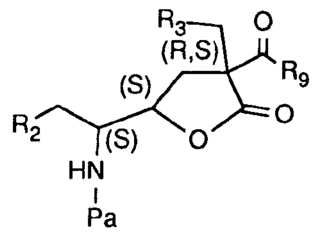

(XIX),

worin die Reste die zuletzt genannten Bedeutungen haben, die Verbindungen der Formel XIX hydrolysiert und decarboxyliert, beispielsweise durch Hydrolyse mittels einer Base, wie einem Alkalimetallhydroxid, z. B. Lithiumhydroxid, bei Temperaturen zwischen -50 und 80 °C, vorzugsweise zwischen etwa 0 und 35 °C, in einem organischen Lösungsmittel, z. B. einem Ether, wie Dimethoxyethan, und anschliessende Decarboxylierung durch Erhitzen in einem inerten Lösungsmittel, vorzugsweise einem Kohlenwasserstoff, z. B. einem aromatischen Kohlenwasserstoff, wie Toluol, auf Temperaturen zwischen 40 und 120 °C, vorzugsweise zwischen 70 und 100 °C, wobei eine Verbindung der Formel

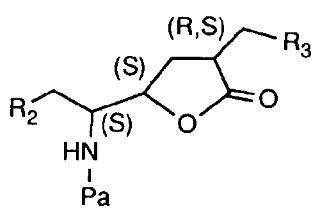

(XX),

worin die Reste die zuletzt genannten Bedeutungen haben, erhalten wird, durch Säulenchromatographie die erhaltenen (R,S,S)- und (S,S,S)-Isomeren auftrennt, das (R,S,S)-Isomere weiterverwendet und zur Öffnung des Lactonringes mit einer Base, wie einem Alkalimetallhydroxid, z. B. Lithiumhydroxid, in einem inerten Lösungsmittel, wie einem Ether, z. B. Dimethoxyethan, umsetzt unter Erhalt einer Verbindung der Formel

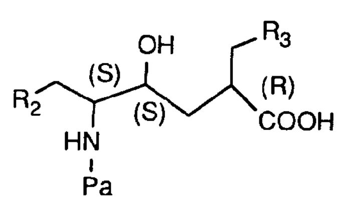

(XXI),

worin die Reste die zuletzt genannten Bedeutungen haben, in die erhaltene Verbindung eine Hydroxyschutzgruppe Py, beispielsweise eine der unter Verfahren a) genannten Hydroxyschutzgruppen unter den dort genannten Bedingungen, insbesondere eine Triniederalylsilylgruppe mit Hilfe des entsprechenden Halogen-triniederalkylsilanes, z. B. tert-Butyldimethylchlorsilanes, in einem polaren Lösungsmittel, wie einem Diniederalkyl-niederalkanoylamid, wie Dimethylformamid, in Gegenwart einer sterisch gehinderten Aminoverbindung, wie eines cyclischen Amins, z. B. Imidazol, bei Temperaturen von -50 bis 80 °C, bevorzugt von 0 bis 35 °C, einführt unter Erhalt einer Verbindung der Formel

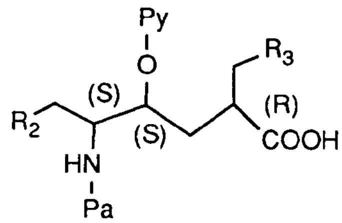

(XXII),

worin die Reste die zuletzt genannten Bedeutungen haben, und die Verbindungen der Formel III mit den unter Verfahren a) angegebenen Resten hieraus beispielsweise herstellt durch Kondensation mit einer Verbindung der Formel VII, worin die Reste die unter Verfahren c) angegebenen Bedeutungen haben, unter den für Verfahren a) angegebenen Bedingungen, insbesondere durch in-situ-Reaktion in Gegenwart eines Kondensationsmittels, wie Benzotriazol-1-yl-oxy-tris-(di-methylamino)-phosphonium-hexafluor-phosphat oder O-Benztriazol-1-yl-N,N,N′,N′-tetra-methyl-uronium-hexafluorphosphat, eines sterisch gehinderten Amins, wie N-Methylmorpholin, und einer racemisierungshindernden Verbindung, wie 1-Hydroxy-benz-triazol, in einem polaren Lösungsmittel, vorzugsweise einem Säureamid, z. B. einem Diniederalkylamino-niederalkanoylamid, wie Dimethylformamid, bei Temperaturen zwischen -50 und 80 °C, insbesondere zwischen 0 und 35 °C, und durch anschliessende Schutzgruppenabspaltung von Pa, wie unter Verfahren f) beschrieben, sofern Pa keinen dem Radikal $H-B_1$- mit den oben für Verbindungen der Formel I dargestellten Bedeutungen, ausser einer Bindung, entsprechenden Rest bedeutet, Kondensation mit einer Verbindung der Formel $H-B_1'-OH$, worin $B_1'$ die unter Verfahren b) genannten Bedeutungen hat, unter den zuletzt genannten Kondensationsbedingungen und schliesslich die Abspaltung von Py und/oder weiteren Schutzgruppen, wie unter Verfahren f) beschrieben. Zur Herstellung der Verbindungen der Formel III ist auch die sukzessive Umsetzung der Verbindungen der Formel XXII mit Verbindungen möglich, welche die Radikale $-B_1-$, $-A_1-$, $-A_2-$, $-A_1-A_2-$, $A_2-NR_4R_5$ und/oder $-NR_4R_5$ der Verbindung der Formel VII einführen.

Die vorgenannten Verbindungen der Formel XV können auch am den Rest $-NH_2$ tragenden Kohlenstoffatom in der (R,S)-Konfiguration anstelle der gezeigten (S)-Konfiguration vorliegen, die Verbindungen der Formel XII, XIII, XIV und insbesondere die der Formel XVI, XVII, XVIII, XIX, XX, XXI und/oder XXII können auch am den Rest Pa-NH- tragenden Kohlenstoffatom in der (R,S)-Konfiguration anstelle der (S)-Konfiguration vorliegen, wobei entsprechende racemische Gemische oder Diastereomerengemische auf allen Stufen auch aufgetrennt werden können.

Verbindungen der Formel XX, in denen die Reste die genannten Bedeutungen haben, werden auch aus Verbindungen der Formel XIII hergestellt, in denen die Reste die genannten Bedeutungen haben, indem man die Aldehyde der Formel XIII mit 2-Halogenpropionsäureestern, insbesondere 2-Iod-propionsäureestern, wie 2-Jod-propionsäureethylester, umsetzt, wobei man die Verbindungen der Formel

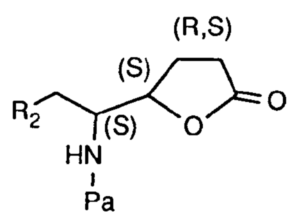

(XXIII)

erhält, worin die Reste die genannten Bedeutungen haben und worin das den Rest Pa-NH-tragende Kohlenstoffatom auch alternativ in der (R,S)-Konfiguration vorliegen kann.

Die Umsetzung erfolgt zunächst unter Bildung des Homoenolates des 2-Halogenpropionsäureesters in Gegenwart einer Mischung von Zn/Cu in einem Diniederalkyl-niederalkanoylamid, wie Dimethylacetamid, bei Temperaturen zwischen 0 und 100 °C, insbesondere zwischen 20 und 80 °C. In einem weiteren Ansatz wird, vorzugsweise unter Schutzgas, wie Stickstoff oder Argon, ein Tetraniederalkyl-orthotitanat, wie Tetraisopropylorthotitanat, in einem aromatischen Lösungsmittel, wie Toluol oder Xylol, in Gegenwart eines Halogenkohlenwasserstoffes, wie Methylenchlorid, mit einem Titantetrahalogenid, wie Titantetrachlorid, versetzt und bei 0 bis 50 ° C, insbesondere bei 20 bis 30 °C, gerührt, wobei das entsprechende Di-Halogen-titan-diniederalkylat oder vorzugsweise das Trihalogen-titan-niederalkylat, insbesondere Trichlortitan-diisopropylat, gebildet wird. Die Zn-Homoenolat-Lösung wird zu diesem bei Temperaturen zwischen - 50 und 0°C, insbesondere von -40 bis - 25 °C, zugetropft und anschliessend der Aldehyd der Formel XIII in einem Halogenkohlenwasserstoff, z. B. Methylenchlorid, zugetropft, wobei die Umsetzung bei -50 bis 30 °C, vorzugsweise bei etwa -20 bis 5 °C, stattfindet unter Bildung des Esters, insbesondere Ethylesters, der Verbindung der Formel XXIII. Dieser Ester wird dann hydrolysiert unter Bildung der Verbindung der Formel XIII, wie oben definiert, vorzugsweise in einem organischen Lösungsmittel, wie einem Aromaten, z. B. in Toluol oder Xylol, in Gegenwart einer Säure, wie einer Carbonsäure, z. B. Essigsäure, bei Temperaturen zwischen 20 °C und dem Siedepunkt des Reaktionsgemisches, insbesondere zwischen 70 und 90 °C. Falls erforderlich, wird eine Diastereomerentrennung, beispielsweise durch Chromatographie, z. B. an Kieselgel mit einem organischen Lösungsmittelgemisch, wie einem Gemisch aus Alkan und Ester, wie Niederalkan und Niederalkylniederalkanoylester, wie Hexan/Essigsäureethylester, durchgeführt.

Aus der Verbindung der Formel XXIII wird dann durch Deprotonierung mit einer starken Base unter Erhalt des am α-Kohlenstoff neben der Oxogruppe des Lactons gebildeten Carbanions und anschliessende nukleophile Substitution des Restes W einer Verbindung der Formel $R_3$-$CH_2$-W, worin $R_3$ und W wie oben bei der Herstellung von Verbindungen der Formel XIX definiert sind, die entsprechende Verbindung der Formel XX erhalten, wobei die Reaktion vorzugsweise stereoselektiv zur (R)-Konfiguration am den Rest $R_3$-$CH_2$- tragenden Kohlenstoffatom in der Verbindung der Formel XX führt. Die Umsetzung mit der starken Base, insbesondere mit einer Alkalimetall-Organosiliciumamid-Verbindung, beispielsweise einem Alkalimetall-bis-(triniederalkylsilyl)amid, wie Lithium-bis(trimethylsilyl)amid, oder ferner einem Alkalimetall-diniederalkylamid, wie Lithiumdiisopropylamid, erfolgt vorzugsweise in einem inerten organischen Lösungsmittel, insbesondere einem Ether, z. B. einem cyclischen Ether, wie Tetrahydrofuran, bei Temperaturen zwischen - 100 und 0 °C, vorzugsweise zwischen -78 und -50 °C, die nukleophile Substitution in situ durch Zugabe der Verbindung der Formel $R_3$-$CH_2$-W, im selben Lösungsmittel bei Temperaturen zwischen - 100 und 0 °C, vorzugsweise zwischen -60 und -40 °C.

Eine Verbindung der Formel XV, worin die Reste die genannten Bedeutungen haben, und worin vorzugsweise das die Gruppe -$NH_2$ tragende Kohlenstoffatom in der (R,S)-Konfiguration vorliegt, kann auch erhalten werden, indem man einen Ameisensäureester, z. B. Ameisensäureniederalkylester, wie Ameisensäureethylester, durch Reaktion mit Allylamin bei Temperaturen zwischen 20 und 70 °C, insbesondere zwischen 50 und 60 °C, umsetzt zu Ameisensäureallylamid. Dieses Amid wird dann unter Schutzgas, wie Stickstoff oder Argon, dehydratisiert, vorzugsweise mit einem Säurehalogenid, wie Phosphoroxidchlorid, Phosgen oder insbesondere einem organischen Sulfonsäurehalogenid, z. B. einem Arylsulfonsäurechlorid, wie Toluolsulfonsäurechlorid, in Gegenwart einer Base, z. B. einem Triniederalkylamin, wie Triethylamin, oder insbesondere einem mono- oder bicyclischen Amin, wie Pyridin oder Chinolin, bei Temperaturen zwischen 50 und 100 °C, insbesondere zwischen etwa 80 und etwa 100 °C. Dabei entsteht Allylisocyanid, das durch Umsetzung mit einem Organolithiumsalz, z. B. Niederalkyllithium, wie n-Butyllithium, in das entsprechende Lithiumsalz überführt wird, wobei die Reaktion vorzugsweise in einem inerten organischen Lösungsmittel, insbesondere einem Ether, wie Dioxan oder Diethylether, oder einem Alkan, z. B. Hexan, oder einem Gemisch dieser Lösungsmittel, bei Temperaturen von - 120 bis -50, insbesondere von etwa -100 bis -90 °C, durchgeführt wird. Das gebildete Lithiumsalz wird dann in situ mit einer Verbindung der Formel $R_2$-$CH_2$-W, worin $R_2$ die für Verbindungen der Formel I genannten Bedeutungen hat und W die oben für Verbindungen der Formel $R_3$-$CH_2$-W genannten Bedeutungen hat, insbesondere Brom bedeutet, umgesetzt, vorzugsweise durch Zutropfen von $R_2$-$CH_2$-W in einem organischen Lösungsnmittel, z. B. einem Ether, wie Tetrahydrofuran, bei den zuletzt genannten Temperaturen und anschliessendes Erwärmen auf 0 bis 50 °C, vorzugsweise auf 20 bis 30 °C. Dabei entsteht ein Isocyanid der Formel

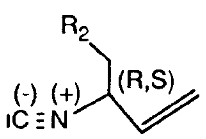

(XXIV),

worin die Reste die genannten Bedeutungen haben. Die Verbindung der Formel XXIV wird anschliessend hydrolysiert, vorzugsweise in wässriger Lösung, der eine Säure zugesetzt ist, z. B. in wässriger Halogenwasserstoffsäure, wie Salzsäure, insbesondere in konzentrierter Salzsäure, bei Temperaturen zwischen -20 und 30 °C, insbesondere zwischen etwa 0 und 10 °C, und man erhält die Verbindung der Formel XV, worin die Reste wie zuletzt definiert sind und worin vorzugsweise das die Gruppe -$NH_2$ tragende Kohlenstoffatom in der (R,S)-Konfiguration vorliegt.

Verbindungen der Formel IV sind bekannt oder nach an sich bekannten Verfahren herstellbar, beispielsweise durch Kondensation von Carbon- oder Sulfonsäuren der Formel II, oder reaktionsfähigen Derivaten hiervon, mit Aminoverbindungen der Formel H-$B_1'$-OH, worin $B_1'$ die für Verbindungen der Formel IV genannten Bedeutungen hat, wobei die Kondensation wie zuletzt beschrieben erfolgt, oder im Falle von Verbindungen

der Formel II, worin $R_1'$ N-(Heterocyclylniederalkyl)-N-niederalkyl-aminocarbonyl, wie N-(2-Pyridylmethyl)-N-methyl-aminocarbonyl, bedeutet, analog EP 0 402 646 vom 19. 12. 1990, Beispiel 218.

Verbindungen der Formel V werden beispielsweise aus Verbindungen der Formel XXII durch Kondensation mit einer Verbindung der Formel VII oder sukzessive Kondensation mit Verbindungen (z. B. H-$A_1'$-OH, H-$A_2'$-OH, H-$A_1$-$A_2$-OH, oder die Verbindung der Formel XI, worin jeweils die Reste die oben genannten Bedeutungen haben) hergestellt, welche Fragmenten der Verbindung der Formel VII entsprechen. Die Kondensationsbedingungen sind analog zu den für die Herstellung der Verbindungen der Formel III beschriebenen.

Verbindungen der Formel VI werden beispielsweise aus den Aminoverbindungen der Formel XXII, z. B. durch Einführung einer Carboxyschutzgruppe, wie unter Verfahren a) beschrieben, und Abspaltung der Schutzgruppe Pa, wie unter Verfahren f) beschrieben, hergestellt durch Kondensation mit einer Carbonsäure der Formel $R_1$-$B_1$-OH, worin die Reste die für Verbindungen der Formel I genannten Bedeutungen haben.

Verbindungen der Formel VII werden beispielsweise aus den entsprechenden Aminosäuren H-$A_1'$-OH oder H-$A_2'$-OH oder den Peptiden H-$A_1$-$A_2$-OH und den Aminkomponenten der Formel XI, worin die Reste jeweils die oben genannten Bedeutungen haben, durch Kondensation analog dem unter Verfahren a) beschriebenen Verfahren hergestellt. Zur Herstellung von Verbindungen mit reduzierter Peptidbindung zwischen $A_1$ und $A_2$ kann die Peptidbindung zwischen $A_1$ und $A_2$, vorzugsweise auf der Stufe des Dipeptides, reduziert werden, beispielsweise mit Wasserstoff in Gegenwart von Schurer- oder Edelmetallkatalysatoren, wie Platin oder Palladium, gegebenenfalls auf Trägem, wie Aktivkohle, oder durch komplexe Hydride, vorzugsweise komplexe Hydride, z. B. Lithiumaluminiumhydrid oder Disiamylboran in polaren Lösungsmitteln, wie Alkoholen, z. B. Ethanol, oder Ethern, wie cyclischen Ethern, z. B. Tetrahydrofuran, bei Temperaturen zwischen 0 und 150 °C, bevorzugt zwischen 20 °C und dem Siedepunkt des betreffenden Reaktionsgemisches. Das Amin der Formel XI ist bekannt oder wird nach an sich bekannten Methoden hergestellt.

Verbindungen der Formel VIII lassen sich beispielsweise aus Verbindungen der Formel VI durch Kondensation mit einer den Rest $A_1'$ einführenden Aminosäure herstellen. Die Umsetzung erfolgt analog den unter Verfahren a) beschriebenen Bedingungen.

Verbindungen der Formel IX werden beispielsweise aus einer Aminosäure H-$A_2'$-OH, worin $A_2'$ die unter Verfahren d) genannten Bedeutungen hat, und einem Amin der Formel XI, worin die Reste die für Verbindungen der Formel I genannten Bedeutungen haben, durch Kondensation hergestellt.

Verbindungen der Formel X werden beispielsweise aus Verbindungen der Formel VI und aus den entsprechenden Aminosäuren H-$A_1'$-OH oder H-$A_2'$-OH oder den Peptiden H-$A_1$-$A_2$-OH, worin die Reste jeweils die oben genannten Bedeutungen haben, durch Kondensation analog dem unter Verfahren a) beschriebenen Verfahren hergestellt. Zur Herstellung von Verbindungen mit reduzierter Peptidbindung zwischen $A_1$ und $A_2$ wird die Peptidbindung zwischen $A_1$ und $A_2$, vorzugsweise auf der Stufe des Dipeptides, reduziert, beispielsweise mit Wasserstoff in Gegenwart von Schwermetall- oder Edelmetallkatalysatoren, wie Platin oder Palladium, gegebenenfalls auf Trägem, wie Aktivkohle, oder durch komplexe Hydride, vorzugsweise durch komplexe Hydride, z. B. Lithiumaluminiumhydrid oder Disiamylboran in polaren Lösungsmitteln, wie Alkoholen, z. B. Ethanol, oder Ethern, wie cyclischen Ethern, z. B. Tetrahydrofuran, bei Temperaturen zwischen 0 und 150 °C, bevorzugt zwischen 20 °C und dem Siedepunkt des Reaktionsgemisches.

Das Amin der Formel XI ist bekannt, kommerziell erhältlich oder wird nach an sich bekannten Methoden hergestellt .

Die übrigen Ausgangsverbindungen sind bekannt, werden nach an sich bekannten Verfahren hergestellt oder sind käuflich.

Die folgenden Beispiele dienen zur Illustration der Erfindung, schränken deren Umfang jedoch in keiner Weise ein.

Temperaturen werden in Celsiusgraden (°C) angegeben. Sofern keine Temperatur angegeben ist, erfolgt die Reaktion bei Raumtemperatur. Die $R_f$-Werte, die das Verhältnis von Laufstrecke der jeweiligen Substanz zur Laufstrecke der Laufmittelfront angeben, werden auf Kieselgeldünnschichtplatten durch Dünnschichtchromatographie (DC) in folgenden Lösungsmittelsystemen ermittelt

| DC-Laufmittelsysteme: | | |
|---|---|---|
| A | Hexan/Essigsäureethylester | 1:1 |
| B | Essigsäureethylester | - |
| C | Hexan/Essigsäureethylester | 4:1 |
| D | Hexan/Essigsäureethylester | 2:1 |
| E | Hexan/Essigsäureethylester | 3:1 |
| F | Methylenchlorid/Methanol | 9:1 |
| G | Chloroform/Methanol/Wasser/Eisessig | 85:13:1,5:0,5 |
| H | Essigsäureethylester/Methanol | 9:1 |
| I | Hexan/Essigsäureethylester | 1:2 |
| J | Cloroform/Methanol/Essigsäure/Wasser | 75:27:5:0,5 |
| K | Essigsäureethylester/Essigsäure | 19:1 |
| L | Methylenchlorid/Methanol | 7:3 |
| M | Methylenchlorid/Ether | 49:1 |
| N | Methylenchlorid/Ether | 3:1 |

Die Abkürzung "$R_f$(A)" bedeutet beispielsweise, dass der $R_f$-Wert im Lösungsmittelsystem A ermittelt wurde. Das Mengenverhältnis von Lösungsmitteln zueinander ist stets in Volumenanteilen (v/v) angegeben. Auch bei der Definition der Fliessmittel-Systeme für die Säulenchromatographie werden die Mengenverhältnisse der verwendeten Lösungs-mittel in Volumenanteilen (v/v) angegeben.

Die weiteren verwendeten Kurzbezeichnungen und Abkürzungen haben die folgenden Bedeutungen:

| | |
|---|---|
| abs. | absolut |
| atm | physikalische Atmosphären (Druckeinheit)-1 atm entspricht 1,013 bar |
| Boc | tert-Butoxycarbonyl |
| BOP | Benzotriazol-1-yl-oxy-tri-(di-methyl-amino)-phosponium-hexafluor-phosphat |
| DC | Dünnschichtchromatographie |
| DCC | Dicyclohexylcarbodiimid |
| DMF | Dimethylformamid |
| DMSO | Dimethylsulfoxid |
| Essigester | Essigsäureethylester |
| Ether | Diethylether |
| FAB-MS | Fast-Atom-Bombardment-Massenspektroskopie |
| h | Stunde(n) |
| HBTU | O-Benztriazol-1-yl-N,N,N′,N′-tetramethyl-uronium-hexafluorphosphat |
| HOBt | 1-Hydroxy-benztriazol |
| IR | Infrarotspektroskopie |
| min | Minute(n) |
| NMM | N-Methylmorpholin |
| org. | organisch |
| Pd/C | Palladium auf Aktivkohle (Katalysator) |
| RT | Raumtemperatur |
| Smp. | Schmelzpunkt |
| Sole | gesättigte Kochsalzlösung |
| TBAF | Tetrabutylammoniumfluorid (Trihydrat) |
| Z | Benzyloxycarbonyl |

Massenspektroskopische Messwerte werden nach der "Fast-Atom-Bombardment" (FAB-MS)-Methode erhalten. Die Massenangaben beziehen sich auf das protonierte Molekülion $(M+H)^+$.

Die Werte für IR-Spektren werden in $cm^{-1}$ angegeben, in runden Klammern findet sich das jeweilige Lösungsmittel.

Zur Bezeichnung von bivalenten Radikalen von natürlichen α-Aminosäuren werden die in der Peptidchemie üblichen Abkürzungen verwendet. Die Konfiguration am α-Kohlenstoffatom wird durch Voranstellen von (L)- oder (l))- angegeben. -Cha- steht für Cyclohexylalanyl, -(p-F-Phe)- für in p-Stellung des Phenylringes mit fluor, -(p-$CH_3$O-Phe)- für in p-Stellung des Phenylringes mit einer Methoxygruppe und -(p-CN-Phe)- für in p-Stellung des Phenylringes mit einer Cyanogruppe substituiertes Phenylalanyl.

Die folgenden Kurzbezeichnungen für Reste werden durch die entsprechenden

Formelbilder und Bezeichnungen definiert:

Der Rest mit der Kurzbezeichnung -Phe[C]Phe- bedeutet das bivalente Radikal von 5(S)-Amino-2(R)-benzyl-4(S)-hydroxy-6-phenyl-hexansäure und hat die Formel

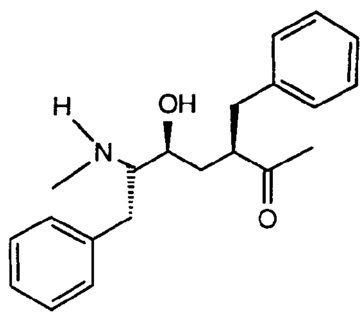

.

Der Rest mit der Kurzbezeichnung -Cha[C] (p-CN)Phe- bedeutet das bivalente Radikal von 5(S)-Amino-2(R)-(p-cyanophenylmethyl)-6-cyclohexyl-4(S)-hydroxy-hexansäure und hat die Formel

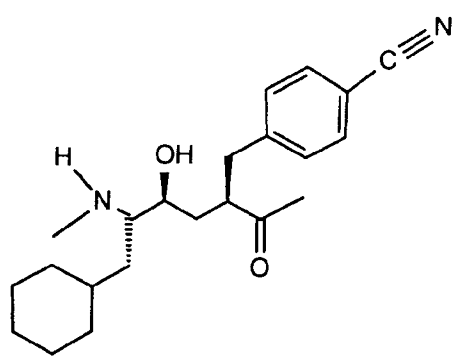

.

Der Rest mit der Kurzbezeichnung -Cha[C]Cha- bedeutet das bivalente Radikal von 5(S)-Amino-6-cyclohexyl-2(R)-cyclohexylmethyl-4(S)-hydroxy-hexansäure und hat die Formel

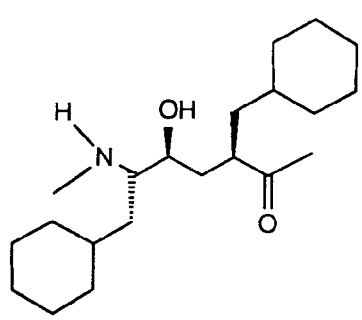

.

Der Rest mit der Kurzbezeichnung -Cha[C] (p-F)Phe- bedeutet das bivalente Radikal von 5(S)-Amino-6-cyclohexyl-2(R)-(p-fluorphenylmethyl)-4(S)-hydroxy-hexansäure und hat die Formel

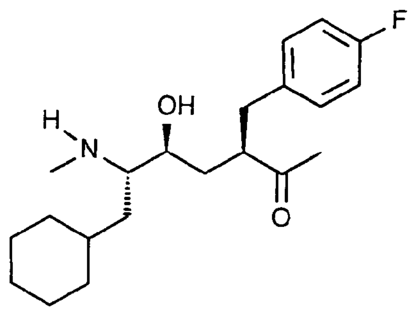 .

Der Rest mit der Kurzbezeichnung -(p-F)Phe[C]Phe- bedeutet das bivalente Radikal von 5(S)-Amino-2(R)-benzyl-6-(p-fluorphenyl)-4(S)-hydroxy-hexansäure und hat die Formel

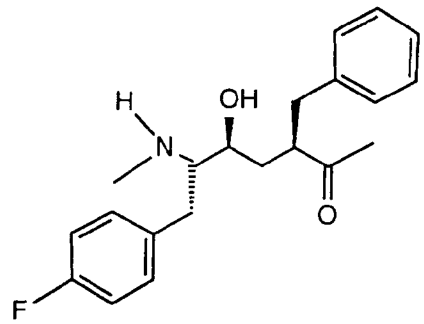 .

Die nachfolgend gezeigten weiteren Formelbilder der Zentralbausteine entsprechen den folgenden Kurzbezeichnungen, die in den nachfolgenden Beispielen verwendet werden:

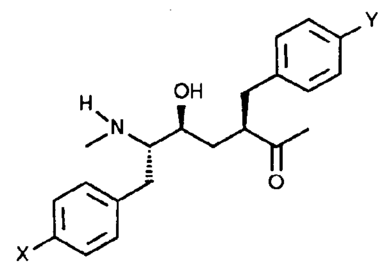

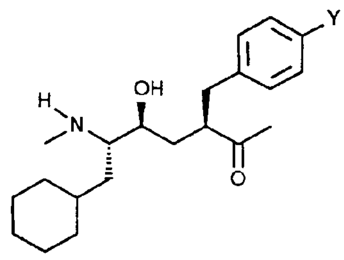

| | X | Y |
|---|---|---|
| -Phe[C](p-F)Phe- | H | F |
| -Phe[C](p-CN)Phe- | H | CN |
| -Phe[C](p-$CH_3O$)Phe- | H | $CH_3O$ |
| -Phe[C](p-$CF_3$)Phe- | H | $CF_3$ |
| -(p-F)Phe[C](p-F)Phe- | F | F |
| -(p-F)Phe[C](p-CN)Phe- | F | CN |
| -Tyr[C]Tyr- | OH | OH |
| -Tyr[C]Phe- | OH | H |
| -Phe[C]Tyr | H | OH |

| | Y |
|---|---|
| -Cha[C](p-$CH_3O$)Phe- | $CH_3O$ |
| -Cha[C](p-$CF_3$)Phe- | $CF_3$ |

Demnach entspricht -Phe[C](p-F)Phe- dem bivalenten Radikal von 5(S)-Amino-2(R)-(p-fluorphenylmethyl)-4(S)-hydroxy-6-phenyl-hexansäure; -Phe[C](p-CN)Phe- dem bivalenten Radikal von 5(S)-Amino-2(R)-(p-cyanophenylmethyl)-4(S)-hydroxy-6-phenyl-hexansäure; -Phe[C] (p-$CH_3O$)Phe- dem bivalenten Radikal von 5(S)-Amino-4(S)-hydroxy-2(R)-(p-methoxyphenylmethyl)-6-phenyl-hexansäure; -Phe[C](p-$CF_3$)Phe- dem bivalenten Radikal von 5(S)-Amino-4(S)-hydroxy-6-phenyl-2(R)-(p-trifluormethylphenylmethyl)-hexansäure; -(p-F)Phe[C](p-F)Phe- dem bivalenten Radikal von 5(S)-Amino-6-(p-fluorphenyl)-2(R)-(p-fluorphenylmethyl)-4(S)-hydroxy-hexansäure; -(p-F)Phe[C](p-CN)Phe- dem bivalenten Radikal von 5(S)-Amino-2(R)-(p-cyanophenylmethyl)-6-(p-fluorphenyl)-4(S)-hydroxy-hexansäure; -Cha[C](p-$CH_3O$)Phe- dem bivalenten Radikal von 5(S)-Amino-2(R)-(p-methoxyphenylmethyl)-6-cyclohexyl-4(S)-hydroxy-hexansäure; -Cha[C](p-$CF_3$)Phe- dem bivalenten Radikal von 5(S)-Amino-6-cyclohexyl-4(S)-hydroxy-2(R)-(p-trifluormethylphenylmethyl)-hexansäure; -Tyr[C]Tyr- dem bivalenten Radikal von 5(S)-Amino-4(S)-hydroxy-6-(p-hydroxyphenyl)-2(R)-(p-hydroxyphenylmethyl)-hexansäure; -Phe[C]Tyr- dem bivalenten Radikal von 5(S)-Amino-4(S)-hydroxy-2(R)-(p-hydroxyphenylmethyl)-6-phenylhexansäure-; und -Tyr[C]Phe- dem bivalenten Radikal von 5(S)-Amino-4(S)-hydroxy-6-(p-hydroxyphenyl)-2(R)-benzyl-hexansäure.

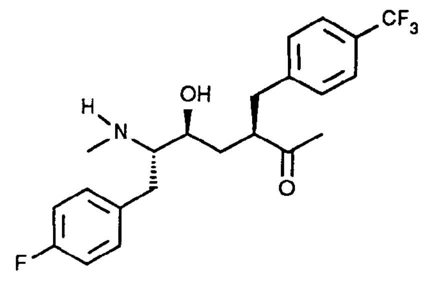

-(p-F)Phe[C](p-$CF_3$)Phe-

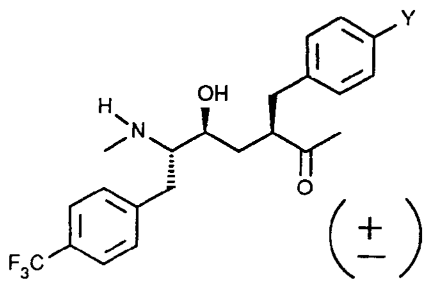

| | Y |
|---|---|
| -($CF_3$)Phe[C]Phe- | H |
| -($CF_3$)Phe[C](p-F)Phe- | F |
| -($CF_3$)Phe[C](p-$CF_3$)Phe- | $CF_3$ |

Der Rest -(p-F)Phe[C] (p-$CF_3$)Phe- entspricht demnach dem bivalenten Radikal von 5(S)-Amino-4(S)-hydroxy-6-(p-fluorphenyl)-2(R)-(p-trifluormethylphenylmethyl)-hexansäure.

Das Symbol (±) soll zum Ausdruck bringen, dass die Reste -($CF_3$)Phe[C]Phe-, -($CF_3$)Phe[C](p-F)Phe- und -($CF_3$)Phe[C](p-$CF_3$)Phe-, welche den bivalenten Radikalen von 5-Amino-2-phenyl-4-hydroxy-6- (p-trifluormethylphenyl)-hexansäure, 5-Amino-2-(p-trifluorphenyl)-4-hydroxy-6-(p-trifluormethylphenyl)-hexansäure und 5-Amino-2-(p-trifluormethylphenyl)-4-hydroxy-6-(p-trifluormethylphenyl)-hexansäure entsprechen, in den entsprechenden Beispielen als Gemisch des 2(R),4(S),5(S)- Isomeren mit dem 2(S),4(R),5(R)-Isomeren vorliegen.

**Beispiel 1: Boc-Cha[C](p-F)Phe-(L)-Val-(L)-Phe-morpholin-4-ylamid**

Eine Lösung von 160 mg 5(S)-Boc-amino-4(S)-tert-butyldimethylsilyloxy)-6-cyclohexyl-2(R)-(p-fluorphenylmethyl)-hexanoyl-(L)-Val-(L)-Phe-morpholin-4-ylamid in 1, 8 ml abs. DMF wird mit 116 mg TBAF versetzt und das Reaktionsgemisch danach 4,5 h bei RT gerührt. Die farblose Lösung wird auf 50 ml Wasser gegossen und viermal mit Essigester extrahiert. Die vereinten Extrakte werden jeweils mit zweimal 25 ml Natriumbicarbonat-Lösung, zweimal mit Wasser und einmal mit Sole gewaschen und anschliessend über Natriumsulfat getrocknet. Nach Eindampfen des Lösungsmittels wird der Rückstand aus Diisopropylether kristallisiert und die Titelverbindung erhalten. DC $R_f$ (I)= 0,14; FAB-MS $(M+H)^+$= 753.

Das Ausgangsmaterial wird wie folgt hergestellt:

**1 a) N-3(S)-(Boc-amino)-2(R,S)-hydroxy-4-phenyl-1-trimethylsilyl-butan**

24,7 g Magnesium werden in 100 ml abs. Ether vorgelegt und über 35 min mit wenig Iod und gleichzeitig mit 132,5 ml Chlormethyltrimethylsilan und 300 ml Ether versetzt, wobei die Temperatur mittels Eisbad bei 38 $^0$C gehalten wird. Das erhaltene Reaktionsgemisch wird dann 1,5 h bei RT gerührt. Nach Abkühlen auf -60 $^0$C wird mit einer Suspension von 48,6 g N-Boc-phenylalaninal (Herstellung: D. J. Kempf, J. Org. Chem. 51, 3921 (1986)) in 1,1l Ether innerhalb 40 min versetzt. Über 90 min wird das Reaktionsgemisch auf RT erhitzt und weitere 90 min bei dieser Temperatur gerührt. Danach wird auf 2l Eiswasser und 1,5l 10 %-ige wässrige Zitronensäure gegossen. Die abgetrennte wässrige Phase wird zweimal mit 500 ml Ether extrahiert. Alle Etherextrakte werden mit 500 ml einer 10 %-igen Zitronensäure-Lösung und zweimal mit Sole gewaschen. Nach Trocknen über Natriumsulfat wird unter Vakuum eingeengt und die erhaltene Titelverbindung ohne zusätzliche Reinigung weiterverwendet. DC $R_f$ (C)= 0,6; FAB-MS $(M+H)^+$= 338.

1 b) 1-Phenyl-3-buten-2(S)-amin

Eine Lösung von 18,8 g n-3(S)-(Boc-amino)-2-(R,S)-hydroxy-4-phenyl-1-trimethylsilyl-butan in 420 ml Methylenchlorid wird bei 5 °C innerhalb von 10 min mit 35,6 ml einer ungefähr 48 %-igen Lösung von Bortrifluoridethyletherat versetzt. Das Reaktionsgemisch wird dann 16 h bei RT gerührt, auf 10 °C gekühlt und innerhalb von 20 min mit 276 ml einer 4 N Natriumhydroxyd-Lösung versetzt. Die wässrige Phase wird abgetrennt und zweimal mit je 400 ml Methylenchlorid extrahiert. Die vereinigten organischen Extrakte werden mit Sole gewaschen und über Natriumsulfat getrocknet. Das Titelprodukt wird ohne zusätzliche Reinigung weiterverwendet. DC $R_f$ (G)= 0, 15 ; IR (Methylenchlorid) ($cm^{-1}$): 3370, 3020, 2920, 1640, 1605.

1 c) N-Boc-1-phenyl-3-buten-2(S)-amin

21,5 g 1-Phenyl-3-buten-2(S)-amin werden in 500 ml abs. Methylenchlorid gelöst und tropfenweise mit einer Lösung von 38,3 g Boc-Anhydrid in 250 ml Methylenchlorid versetzt. Nach 1,5 h Rühren bei RT wird auf 100 ml eingeengt, dann mit 1,51 Ether verdünnt und nacheinander zweimal mit 400 ml 10 %-iger Zitronensäure, einmal mit 400 ml Wasser, einmal mit 400 ml gesättigter wässriger Natriumbicarbonat-Lösung und zweimal mit Sole gewaschen und über Natriumsulfat getrocknet. Nach Eindampfen des Lösungsmittels wird durch Säulenchromatographie gereinigt ($SiO_2$, Hexan/Essigester: 95/5 bis 80/20) und die Titelverbindung aus Hexan auskristallisiert. Smp. 67-68 °C; DC $R_f$ (C)= 0,4; FAB-MS $(M+H)^+$= 248.

1 d) 2(R)-[1(S)-(Boc-amino)-2-phenylethyl]-oxiran

Eine Lösung von 1,45 g N-Boc-1-phenyl-3-buten-2(S)-amin in 20 ml Methylenchlorid wird innerhalb von 15 min bei 0 - 5 °C mit einer Lösung von 9,74 g m-Chlorperbenzoesäure in 50 ml Methylenchlorid versetzt. Nach 18 h Rühren bei gleicher Temperatur wird noch weitere 8 h unter Erwärmung auf RT ausgerührt und auf eiskalte 10 %-ige Natriumcarbonat-Lösung gegossen. Die wässrige Phase wird dreimal mit Ether extrahiert. Die vereinigten organischen Phasen werden nacheinander dreimal mit 10 %-iger Natriumsulfit-Lösung, dreimal mit gesättigter Natriumbicarbonat-Lösung, mit Natriumthiosulfat-Lösung und Sole gewaschen und über Natriumsulfat getrocknet. Nach Einengen der Lösungsmittel wird durch Säulenchromatographie ($SiO_2$, Hexan/Essigester: 4/1) die Titelverbindung gereinigt und aus Hexan auskristallisiert. Smp. 51 -52 °C; DC $R_f$ (C)= 0,33; FAB-MS $(M+H)^+$= 264.

1 e) 5(S)-[1(S)-(Boc-amino)-2-phenylethyl]-3(R,S)-carbethoxy-dihydrofuran-2-(3H)-on

Eine Lösung von 26 ml Malonsäure-diethylester in 260 ml abs. Ethanol wird portionsweise mit 3,4 g Natrium versetzt. Nach Verbrauch des Natriums (ca. 1,5 h) wird innerhalb von 10 min eine Lösung von 13 g 2(R)-[1(S)-(Boc-amino)-2-phenylethyl]-oxiran in 100 ml Ethanol zugetropft. Nach 5 h Rühren bei RT wird das Reaktionsgemisch auf 1,51 Eiswasser gegossen und mit 10 %-iger Zitronensäure auf pH 4 gestellt. Nach viermaligem Extrahieren mit Ether werden die vereinigten organischen Phasen nacheinander zweimal mit gesättigter wässriger Natriumbicarbonat-Lösung, einmal mit Sole, erneut mit gesättigter wässriger Natriumbicarbonat-Lösung, mit Wasser und nochmals mit Sole gewaschen. Nach Einengen des Lösungsmittels wird durch Säulenchromatographie ($SiO_2$, Hexan/Essigester: 4/1) die Titelverbindung erhalten. DC $R_f$ (C)= 0,22; FAB-MS $(M+H)^+$= 378.

1 f) 5(S)-[1(S)-(Boc-amino)-2-cyclohexylethyl]-3(R,S)-carbethoxy-dihydrofuran-2-(3H)-on

10 g 5(S)-[1(S)-(Boc-amino)-2-phenylethyl]-3(R,S)-carbethoxy-dihydrofuran-2-(3H)-on in 100 ml Ethanol werden mit 1 g Nishimura-Katalysator (Rh(III)- und Pt(VI)-Oxid (Monohydrat, Degussa) während 2 h unter Normaldruck (ca. 1 atm) hydriert. Der Katalysator wird durch Celite (Diatomeenerde, Sigma, Schweiz) abfiltriert, mit Ethanol gewaschen und das Filtrat eingedampft. DC $R_f$(C)=0,23.

1 g) 5(S)-[1(S)-(Boc-amino)-2-cyclohexylethyl]-3(R,S)-carbethoxy-3-(p-fluor-phenylmethyl)-dihydrofuran-2-(3H)-on

10.2 g 5(S)-[1(S)-(Boc-amino)-2-cyclohexylethyl]-3(R,S)-carbethoxy-dihydrofuran-2-(3H)-on werden mit 5,39 g p-Fluor-benzylbromid (Fluka, Buchs, Schweiz) und 0,68 g Natrium in 180 ml Ethanol bei RT umgesetzt. Da sich laut DC nach 1,5 h noch nicht alles Lacton umgesetzt hat, werden noch 0,2 g Natrium und 0,7 g p-Fluorbenzylbromid zugesetzt. Nach 16 h wird auf ein Gemisch von 10 %-iger Zitronensäure und Eis gegossen

und 3 mal mit Ether extrahiert. Die organischen Phasen werden 2 mal mit Wasser und mit Sole gewaschen, mit $Na_2SO_4$ getrocknet und eingedampft. Nach Zusatz von Hexan/Essigester kristallisiert das ölige Rohprodukt unter Ultraschalleinfluss teilweise aus unter Erhalt der Titelverbindung (Diastereomerenverhältnis 4:1). Säulenchromatographie ($SiO_2$, Hexan/Essigester 4:1) der Mutterlauge liefert weitere Titelverbindung (Diastereomerenverhältnis ca. 1:4). DC $R_f$(C)=0,29; FAB-MS $(M+H)^+$=492.

**1 h) 5(S)-[1(S)-(Boc-amino)-2-cyclohexylethyl]-3(R)-(p-fluor-phenylmethyl)-dihydrofuran-2-(3H)-on und5(S)-[1(S)-(Boc-amino)-2-cyclohexylethyl]-3(S)-(p-fluor-phenylmethyl)-dihydrofuran-2-(3H)-on**

10,3 g 5(S)-[1(S)-(Boc-amino)-2-cyclohexylethyl]-3(R,S)-carbethoxy-3-(p-fluor-phenylmethyl)-dihydrofuran-2-(3H)-on (Diastereomerenverhältnis ca. 1:1) werden in 174 ml 1,2-Dimethoxyethan über 5 min tropfenweise bei RT mit 91 ml 1 M Lithiumhydroxyd-Lösung versetzt und für 15 h bei RT gerührt. Nach Eindampfen des Lösungsmittels wird der erhaltene Rückstand auf 500 ml 10 %-ige Zitronensäure gegossen und dreimal mit Ether extrahiert. Die vereinigten Ether-Phasen werden einmal mit Sole gewaschen und über Natriumsulfat getrocknet. Nach Eindampfen des Lösungsmittels erhält man die rohe Carbonsäure, welche mittels nachfolgender Decarboxylierung durch 9 stündiges Erhitzen auf 90 °C in 450 ml Toluol ins Gemisch der Titelverbindungen überführt wird. Säulenchromatographie ($SiO_2$, Hexan/Essigester 9:1 $\rightarrow$ 4:1) liefert zuerst das 3 (R)-Epimere [DC $R_f$(E)=0,45], gefolgt vom 3 (S)-Epimeren [DC $R_f$(E)=0,41].

**1 i) 5(S)-(Boc-amino)-4(S)-hydroxy-6-cyclohexyl-2(R)-(p-fluor-phenylmethyl)-hexansäure**

2,05 g 5(S)-[1(S)-(Boc-amino)-2-cyclohexylethyl]-3(R)-(p-fluor-phenylmethyl)-dihydrofuran-2-(3H)-on in 78 ml Dimethoxyethan und 39 ml Wasser werden bei 20 bis 25 °C tropfenweise innerhalb von 2 min mit 19,6 ml 1 M Lithiumhydroxid-Lösung versetzt. Nach 3 h Rühren bei RT wird unter vermindertem Druck eingeengt, und der Rückstand wird in 100 ml gesättigter wässriger Ammoniumchlorid-Lösung und 5 ml 10 %-iger Zitronensäure aufgenommen und viermal mit Methylenchlorid extrahiert. Die vereinigten organischen Phasen werden mit Sole gewaschen und über Natriumsulfat getrocknet. Nach Einengen erhält man die Titelverbindung als Schaum, der ohne weitere Reinigung in der nächsten Stufe eingesetzt wird.

**1 j) 5(S)-(Boc-amino)-4(S)-(tert-butyldimethylsilyloxy)-6-cyclohexyl-2(R)-(p-fluorphenylmethyl)-hexansäure**

Eine Lösung von 2,01 g 5(S)-(Boc-amino)-4(S)-hydroxy-6-cyclohexyl-2(R)-(p-fluorphenylmethyl)-hexansäure in 6,4 ml DMF wird mit 2,73 g Imidazol und 3,39 g tert-Butyldimethylchlorsilan 18 h bei RT gerührt. Dann giesst man das Reaktionsgemisch auf Eiswasser, extrahiert mit 3 Portionen Essigester, wäscht die vereinigten organischen Phasen mit 10 %-iger Zitronensäure-Lösung, Wasser und Sole, trocknet mit Natriumsulfat und dampft sie ein. Man erhält ein Öl. Dieses Öl wird in 68 ml Methanol und 23 ml THF gelöst, bei RT mit einer Lösung von 4,1 g Kaliumcarbonat in 23 ml Wasser versetzt, 1 h gerührt und schliesslich bei RT teilweise eingedampft. Den wässrigen Rückstand giesst man auf 10 %-ige Zitronensäure-Lösung und Eis, extrahiert 3 mal mit Essigester, wäscht die organischen Phasen 2 mal mit Wasser und Sole, trocknet sie mit Natriumsulfat und dampft ein. Säulenchromatographie ($SiO_2$, Hexan/Essigester 5:1 $\rightarrow$ 2:1 ) liefert die Titelverbindung: DC $R_f$(E)=0,2; FAB-MS $(M+H)^+$=552.

**1 k) 5(S)-(Boc-amino)-4(S)-(tert-butyldimethylsilyloxy)-6-cyclohexyl-2(R)-(p-fluor-phenylmethyl)-hexanoyl-(L)-Val-(L)-Phe-morpholin-4-yl-amid**

Eine Lösung von 102 mg 5(S)-(Boc-amino)-4(S)-(tert-butyldimethylsilyloxy)6-cyclohexyl-2(R)-(p-fluorphenylmethyl)-hexansäure, 90 mg BOP und 27 mg HOBT wird 30 min bei RT in etwa 2 ml DMF gerührt und dann mit 74 mg H-(L)-Val-(L)-Phemorpholin-4-yl-amid [Herstellung siehe unter 1l) bis 1o)] versetzt. Nach 16 h bei RT dampft man ein und verteilt den Rückstand zwischen 3 Portionen Essigester, Wasser, ges. Natriumbicarbonat-Lösung, Wasser und Sole, trocknet die organischen Phasen mit Natriumsulfat und dampft sie ein. Man erhält die Titelverbindung als Rohprodukt; DC $R_f$(I)=0,57; FAB-MS $(M+H)^+$=867.

**1 l) Z-(L)-Phe-morpholin-4-ylamid**

Eine Lösung von 4,49 g Z-(L)-Phe-OH in 190 ml Methylenchlorid wird auf 0 °C gekühlt und mit 3,09 g DCC versetzt. Nach 20 min Rühren bei 0 °C wird über 15 min eine Lösung von 1,31 ml Morpholin in 10 ml Methylenchlorid zugetropft. Das Reaktionsgemisch wird weitere 24 h bei RT gerührt, und nach Abfiltrieren des ausgefallenen Dicyclohexylharnstoffes wird nacheinander mit Methylenchlorid, wässriger Natriumbicarbonat-Lösung und Sole gewaschen. Nach Trocknen über Natriumsulfat und Einengen erhält man die rohe Titelverbindung, die aus Ether auskristallisiert. DC $R_f$(B)= 0,55.

**1 m) H-(L)-Phe-morpholin-4-ylamid**

Eine Lösung von 5,5 g Z-(L)-Phe-morpholin-4-ylamid mit 1,5 g 10 %-igem Pd/C in 150 ml Methanol wird bei RT während 1 h mit der berechneten Menge Wasserstoff durch Hydrogenolyse in die Titelverbindung überführt. Nach Abfiltrieren des Katalysators wird eingeengt, und nach Verdünnen mit Essigester wird die erhaltene Lösung mit einer gesättigten Natriumbicarbonat-Lösung gewaschen, über Natriumsulfat getrocknet und unter vermindertem Druck eingeengt. Nach Säulenchromatographie (analog Beispiel 1 o)) wird die Titelverbindung in reiner Form erhalten. DC $R_f$(F)= 0,3.

**1 n) Z-(L)-Val-(L)-Phe-morpholin-4-ylamid**

Eine Lösung von 2,14 g Z-(L)-Val-OH in 80 ml abs. eisgekühltem Methylenchlorid wird mit 1,75 g DCC versetzt und nach 20 min Rühren bei dieser Temperatur tropfenweise über 15 min mit einer Lösung von 2 g H-(L)-Phe-morpholin-4-ylamid versetzt. Das Reaktionsgemisch wird weitere 24 h bei RT gerührt, und der entstandene Harnstoff wird abfiltriert. Das Filtrat wird nacheinander mit wässriger Natriumbicarbonat-Lösung und Sole gewaschen und nach Trocknen über Natriumsulfat eingeengt. Durch Verrühren mit Ether und Abfiltrieren des unlöslichen Rückstandes erhält man nach Einengen die Titelverbindung, die ohne zusätzliche Reinigung weiterverarbeitet wird. DC $R_f$(F)= 0,7.

**1 o) H-(L)-Val-(L)-Phe-morpholin-4-ylamid**

Analog Beispiel 1 m) werden 3,9 g Z-(L)-Val-(L)-Phe-morpholin-4-ylamid durch Hydrogenolyse über 0,5 g 10 %-igem Pd/C in 150 ml Methanol in die rohe Titelverbindung überführt, die durch Säulenchromatographie ($SiO_2$, Methylenchlorid bis Methylenchlorid/Methanol: 97,5 zu 2,5 (v/v)) gereinigt wird. DC $R_f$(F)= 0,4.

**Beispiel 2: Boc-Phe[C]Phe-(L)-Val-(L)-Phe-morpholin-4-ylamid**

Analog Beispiel 1 werden 330,3 mg 5(S)-Boc-amino-4(S)-(tert-butyldimethylsilyloxy)-6-phenyl-2(R)-phenylmethyl-hexanoyl-(L)-Val-(L)-Phe-morpholin-4-ylamid in 3 ml abs. DMF mit 247,2 mg TBAF in die Titelverbindung überführt, die aus Hexan auskristallisiert. DC $R_f$ (B)= 0,5; FAB-MS $(M+H)^+$= 729.

Das Ausgangsmaterial wird folgendermassen hergestellt:

**2 a) 5(S)-[(1(S)-(Boc-amino)-2-phenylethyl]-3(R,S)-carbethoxy-3-phenylmethyl-dihydrofuran-2-(3H)-on**

Eine Lösung von 23,8 g 5(S)-[1(S)-(Boc-amino)-2-phenylethyl]-3(R,S)-carbethoxy-dihydrofuran-2-(3H)-on in 410 ml abs. Ethanol und 14,4 ml Benzylbromid wird zu einer Lösung von 2,76 g Natrium in 410 ml abs. Ethanol gegeben. Das Reaktionsgemisch wird bei RT unter Argon 18 h gerührt und anschliessend auf ein Gemisch von Eis und 10 %-iger Zitronensäure gegossen. Nach dreimaligem Extrahieren mit Ether werden die vereinigten organischen Extrakte mit Wasser und Sole gewaschen und über Natriumsulfat getrocknet. Nach Einengen erhält man die Titelverbindung als farbloses Oel, das ohne zusätzliche Reinigung in die nächste Stufe eingesetzt wird. DC $R_f$ (C)= 0,4; FAB-MS $(M+H)^+$= 468.

**2 b) 5(S)-[1(S)-(Boc-amino)-2-phenylethyl]-3(R)-phenylmethyl-dihydrofuran-2-(3H)-on und5(S)-[1(S)-(Boc-amino)-2-phenylethyl]-3(S)-phenylmethyl-dihydrofuran-2-(3H)-on**

Eine Lösung von 10 g 5(S)-[(1(S)-(Boc-amino)-2-phenylethyl]-3(R,S)-carbethoxy-3-phenylmethyl-dihydrofuran-2-(3H)-on in 175 ml Dimethoxyethan wird über 5 min tropfenweise mit 81,4 ml einer 1 M wässrigen Lithiumhydroxyd-Lösung bei RT versetzt. Danach wird 15 h bei RT gerührt, und nach Eindampfen des Lösungsmittels wird der erhaltene Rückstand auf 500 ml 10 %-ige Zitronensäure gegossen und dreimal mit Ether extrahiert. Die vereinigten Ether-Phasen werden einmal mit Sole gewaschen und über Natriumsulfat getrocknet. Nach Eindampfen des Lösungsmittels erhält man 9,8 g der rohen Carbonsäure, die durch 14-stündiges Heizen auf 90 °C in 450 ml Toluol zum Titelprodukt decarboxyliert wird. Dieses wird durch Säulenchromatographie (Hexan/Essigester: 9/1) gereinigt, wobei zuerst der 5(S)-[1(S)-(Boc-amino)-2-phenylethyl]-3(R)-phenylmethyl-dihydrofuran-2-(3H)on [DC $R_f$ (C)= 0,3; FAB-MS $(M+H)^+$= 396] und danach der 5(S)-[1(S)(Boc-amino)-2-phenylethyl]-3(S)-phenylmethyl-dihydrofuran-2-(3H)on [DC $R_f$ (C)= 0,25; FAB-MS $(M+H)^+$= 396] erhalten wird.

**2 c) 5(S)-(Boc-amino)-4(S)-hydroxy-6-phenyl-2(R)-phenylmethyl- hexansäure**

Eine Lösung von 17,6 g 5(S)-[1(S)-(Boc-amino)-2-phenylethyl]-3(R)-phenylmethyl-dihydrofuran-2-(3H)on in 710 ml Ethylenglykoldimethylether und 352 ml Wasser wird tropfenweise innerhalb von 10 min mit 176 ml einer 1 M Lithiumhydroxyd-Lösung bei 20 °C versetzt. Danach wird das Reaktionsgemisch 1,5 h bei RT gerührt und das Lösungsmittel eingedampft. Der Rückstand wird auf 1l kalte 10 %-ige Zitronensäure gegossen und die saure Lösung dreimal mit je 800 ml Essigester extrahiert. Die vereinten Extrakte werden erst mit 800 ml Wasser, dann mit 800 ml Sole gewaschen. Nach Trocknen der organischen Lösung über Natriumsulfat wird das Lösungsmittel abdestilliert. Die rohe Titelverbindung wird ohne weitere Reinigung in die nächste Stufe eingesetzt. FAB-MS $(M+H)^+$= 414.

**2 d) 5(S)-(Boc-amino)-4(S)-(tert-butyldimethylsilyloxy)-6-phenyl-2(R)-phenylmethyl-hexansäure**

Eine Lösung von 6,35 g 5(S)-(Boc-amino)-4(S)-hydroxy-6-phenyl-2(R)-phenylmethyl-hexansäure in 90 ml DMF wird unter Rühren mit 8 g Imidazol und 10 g t-Butyldimethylchlorsilan versetzt. Nach 18 h Rühren bei RT wird die gelbe klare Lösung auf Eiswasser gegossen und dreimal mit je 250 ml Essigester extrahiert. Die vereinten Extrakte werden nacheinander dreimal mit 10 %-iger Zitronensäure, einmal mit Wasser, dreimal mit wässriger gesättigter Natriumbicarbonat-Lösung, einmal mit Wasser und zuletzt mit Sole gewaschen. Nach Trocknen über Natriumsulfat wird das Lösungsmittel eingedampft und der so erhaltene tert-Butyldimethylsilylether (13,5 g) in 53 ml THF gelöst und mit 53 ml Essigsäure und 20 ml Wasser behandelt. Nach 3 h Rühren bei RT wird auf Wasser gegossen und dreimal mit Ether extrahiert. Die gesammelten Ether-Extrakte werden zweimal mit Wasser und einmal mit Sole gewaschen und über Natriumsulfat getrocknet. Nach Einengen wird das Rohprodukt durch Säulenchromatographie ($SiO_2$, Hexan/Essigester: 3,5/1,5) gereinigt und die Titelverbindung erhalten. DC $R_f$ (D)= 0,37; FAB-MS $(M+H)^+$= 528.

**2 e) 5(S)-(Boc-amino)-4(S)-(tert-butyldimethylsilyloxy)-6-phenyl-2(R)-phenylmethyl-hexanoyl-(L)-Val-(L)-Phe-morpholin-4-ylamid**

Analog Beispiel 1 k) werden 250 mg 5(S)-(Boc-amino)-4(S)-tert-butyldimethylsilyloxy)-6-phenyl-2(R)-phenylmethyl-hexansäure in 3 ml DMF mit 230,5 mg BOP, 70,4 mg HOBT, 182,6 ml N-Methylmorpholin und 189,5 mg H-Val-Phe-morpholin-4-ylamid in die Titelverbindung überführt. DC RG (A)= 0,24; FAB-MS $(M+H)^+$= 843.

**Beispiel 3: Boc-Cha[C](p-CN)Phe-(L)-Val-(L)-Phe-morpholin-4-ylamid**

Analog Beispiel 1 werden 185 mg 5(S)-(Boc-amino)-4(S)-(tert-butyldimethylsilyloxy)-6-cyclohexyl-2(R)-(p-cyanophenylmethyl)-hexanoyl-(L)-Val-(L)-Phe-morpholin-4-ylamid mit 133 mg TBAF in 2,5 ml abs. DMF in die Titelverbindung überführt. DC $R_f$(B)= 0,33; FAB-MS $(M+H)^+$= 760.

Das Ausgangsmaterial wird folgendermassen hergestellt:

**3 a) 5(S)-[1(S)-(Boc-amino)-2-cyclohexylethyl]-3-carbomethoxy-dihydrofuran-2-(3H)-on**

2,5 g 5(S)-[1(S)-(Boc-amino)-2-phenylethyl]-3-carbethoxy-dihydrofuran-2-(3H)-on (Beispiel 1 e)) werden in 50 ml Methanol über 250 mg Rh(III)- und Pt(VI)-Oxid (Monohydrat)(Nishimura-Katalysator, Degussa) während 2 h bei RT unter Normaldruck (ca. 1 atm) hydriert. Nach Abfiltrieren und Nachwaschen des Katalysators mit Methanol wird eingeengt und die Titelverbindung durch Säulenchromatographie ($SiO_2$, Hexan/Essigester: 3/1 (v/v)) in reiner Form erhalten. DC $R_f$ (E)= 0,2; FAB-MS $(M+H)^+$= 370.

**3 b) 5(S)-[1(S)-(Boc-amino)-2-cyclohexylethyl]-3(R,S)-carbomethoxy-3-(p-cyanophenylmethyl)-dihydrofuran-2-(3H)-on**

Analog Beispiel 1 g) werden 2,25 g 5(S)-[1(S)-(Boc-amino)-2-cyclohexylethyl]-3(R,S)-carbomethoxy-dihydrofuran-2-(3H)-on mit 1.32 g p-Cyano-benzylbromid (Fluka, Buchs, Schweiz) und 156 mg Natrium in Methanol in die Titelverbindung überführt. Nach Aufarbeiten erhält man die Titelverbindung. DC $R_f$ (E)= 0,27 .

**3 c) 5(S)-[1(S)-(Boc-amino)-2-cyclohexylethyl]-3(R)-(p-cyanophenylmethyl)-dihydrofuran-2-(3H)-on und5(S)-[1(S)-(Boc-amino)-2-cyclohexylethyl]-3(S)-(p-cyanophenylmethyl)-dihydrofuran-2-(3H)-on**

Analog Beispiel 1 h) werden 2,95 g 5(S)-[1(S)-(Boc-amino)-2-cyclohexylethyl]-3-carbomethoxy-(p-cyanophenylmethyl)-dihydrofuran-2-(3H)-on in 55 ml 1,2-Dimethoxyethan mit 24,4 ml 1 M Lithiumhydroxyd-Lösung in die entsprechende Carbonsäure und mittels nachfolgender Decarboxylierung durch Erhitzen in 130 ml Toluol in die Titelverbindung überführt. Die Trennung durch Säulenchromatographie ($SiO_2$, Hexan/Essigester: 4/1 bis 3,5/1,5 (v/v)) ergibt zuerst die 3(R)-Form der Titelverbindung, die aus Ether/Hexan auskristallisiert [Smp. 106-108 °C, DC $R_f$ (A)= 0,53; FAB-MS $(M+H)^+$= 427] und dann die 3(S)-Form der Titelverbindung [DC $R_f$ (A)= 0,47; FAB-MS $(M+H)^+$= 427].

**3 d) 5(S)-(Boc-amino)-4(S)-hydroxy-6-cyclohexyl-2(R)-(p-cyano-phenylmethyl)-hexansäure**

Eine Lösung von 550 mg 5(S)-[1(S)-(Boc-amino)-2-cyclohexylethyl]-3(R)-(p-cyanophenylmethyl)-dihydrofuran-2-(3H)-on in 20 ml 1,2-Dimethoxyethan und 14 ml Wasser wird bei 20-25 °C tropfenweise mit 7 ml einer 1 M Lithiumhydroxyd-Lösung innerhalb von 2 min versetzt. Nach 2 h Rühren bei RT wird unter vermindertem Druck eingeengt, und der Rückstand wird in 100 ml gesättigter wässriger Ammoniumchlorid-Lösung und 5 ml 10 %-iger Zitronensäure aufgenommen und viermal mit Methylenchlorid extrahiert.

Die vereinigten organischen Phasen werden mit Sole gewaschen und über Natriumsulfat getrocknet. Nach Einengen erhält man die Titelverbindung. DC $R_f$ (F)= 0,4.

**3 e) 5(S)-(Boc-amino)-4(S)-(tert-butyldimethylsilyloxy)-6-cyclohexyl-2(R)-(p-cyanophenylmethyl)-hexansäure**

790 mg 5(S)-(Boc-amino)-4(S)-hydroxy-6-cyclohexyl-2(R)-(p-cyanophenylmethyl)hexansäure in etwa 10 ml DMF werden unter Rühren mit 1, 18 g tert-Butyldimethylchlorsilan und Imidazol versetzt. Nach 18 h Rühren bei RT wird die gelbe klare Lösung auf Eiswasser gegossen und dreimal mit je 250 ml Essigester extrahiert. Die vereinten Extrakte werden nacheinander dreimal mit 10 %-iger Zitronensäure, einmal mit Wasser, dreimal mit wässriger gesättigter Natriumbicarbonat-Lösung, einmal mit Wasser und zuletzt mit Sole gewaschen. Nach Trocknen über Natriumsulfat wird das Lösungsmittel eingedampft und der so erhaltene tert-Butyldimethylsilylether (13,5 g) in 53 ml THF gelöst und mit 53 ml Essigsäure und 20 ml Wasser behandelt. Nach 3 h Rühren bei RT wird auf Wasser gegossen und dreimal mit Ether extrahiert. Die gesammelten Ether-Extrakte werden zweimal mit Wasser und einmal mit Sole gewaschen und über Natriumsulfat getrocknet. Nach Einengen wird das Rohprodukt einer Endreinigung unterzogen. Die Endreinigung erfolgt durch Säulenchromatographie ($SiO_2$, Hexan/Essigester: 3/1 bis 1/1 (v/v)). Man erhält die Titelverbindung. DC $R_f$ (A)= 0,42; IR (Methylenchlorid) ($cm^{-1}$) 2856, 2230, 1711, 1609, 1449.

**3 f) 5(S)-(Boc-amino)-4(S)-(tert-butyldimethylsilyloxy)-6-cyclohexyl-2(R)-(p-cyanophenylmethyl)-hexanoyl-(L)-Val-(L)-Phe-morpholin-4-ylamid**

Analog Beispiel 1 k) werden 138 mg 5(S)-(Boc-amino)-4(S)-(tert-butyldimethylsilyloxy)-6-cyclohexyl-2(R)-(p-cyanophenylmethyl)-hexansäure mit 122 mg BOP, 37 mg HOBT, 0,069 ml N-Methylmorpholin und 100 mg H-(L)-Val-(L)-Phe-morpholin-4-ylamid in 3 ml DMF in die Titelverbindung überführt. Die Reinigung erfolgt durch Säulenchromatographie ($SiO_2$, Hexan/Essigester: 1/1 (v/v)). Man erhält das reine Titelprodukt. DC $R_f$(A)= 0,25; FAB-MS $(M+H)^+$= 874.

**Beispiel 4: H-Phe[C]Phe-(L)-Val-(L)-Phe-morpholin-4-ylamid**

Eine Lösung von 1,048 g Boc-Phe[C]Phe-(L)-Val-(L)-Phe-morpholin-4-ylamid (Beispiel 2) in 20 ml abs. Methylenchlorid wird bei 5 °C innerhalb von 3 min mit 20 ml Trifluoressigsäure versetzt. Nach weiteren 90 min Rühren bei RT wird eingedampft, der Rückstand mit 150 ml gesättigter wässriger Natriumbicarbonat-Lösung versetzt, und dreimal mit Essigester extrahiert. Die vereinigten Extrakte werden nacheinander mit 100 ml Wasser, 100 ml gesättigter Natriumbicarbonat-Lösung, 100 ml Wasser und mit Sole gewaschen. Nach Trocknen über Natriumsulfat wird das Lösungsmittel eingedampft, und das Rohprodukt wird durch Säulenchromatographie ($SiO_2$, Methylenchlorid/Methanol/Ammoniak: 95/5/0,1 bis 90/10/0,1 (v/v)) unter Erhalt der Titelverbindung gereinigt. DC $R_f$(G)= 0,33; FAB-MS $(M+H)^+$= 629.

**Beispiel 5: 3-Benzofuranoyl-Phe[C]Phe-(L)-Val-(L)-Phe-morpholin-4-ylamid**

Eine Lösung von 30,9 mg Benzofuran-3-carbonsäure (Herstellung gemäss Chin-Hsing Chou et al., J. Org. Chem. 51, 4208 - 4212, 1986) in 3 ml DMF wird nacheinander mit 93 mg BOP, 29 mg HOBT und 0,044 ml N-Methylmorpholin versetzt und anschliessend 30 min bei RT gerührt. Nach Zugabe von 100 mg H-Phe[C]Phe-(L)-Val-(L)-Phe-morpholin-1-ylamid (Beispiel 4) wird noch weitere 3 Stunden bei RT gerührt und danach auf 100 ml Wasser gegossen. Nach dreimaligem Extrahieren mit je 50 ml Essigester werden die vereinigten organischen Phasen nacheinander mit 100 ml Wasser, 100 ml gesättigter wässriger Natriumbicarbonat-Lösung, 100 ml Wasser und 100 ml Sole gewaschen. Nach Trocknen über Natriumsulfat und Eindampfen wird der Rückstand in Ether digeriert und die als Feststoff erhaltene Titelverbindung getrocknet. DC $R_f$(G)= 0,73; FAB-MS $(M+H)^+$= 773.

**Beispiel 6: Nicotinoyl-Phe[C]Phe-(L)-Val-(L)-Phe-morpholin-4-ylamid**

Analog Beispiel 5 werden 23,5 mg Nicotinsäure in 3 ml DMF mit 93 mg BOP, 29 mg HOBT, 0,044 ml N-Methylmorpholin und 100 mg H-Phe[C]Phe-(L)-Val-(L)-Phe-morpholin-1-ylamid in die Titelverbindung überführt. Nach Kristallisieren aus Ether und Trocknen erhält man die reine Titelverbindung. DC $R_f$(G)= 0,57; FAB-MS $(M+H)^+$= 734.

**Beispiel 7: Morpholinocarbonyl-Val-Phe[C]Phe-(L)-Val-(L)-Phe-morpholin-4-ylamid**

Analog Beispiel 5 werden 44 mg N-Morpholinocarbonyl-(L)-Val in 3 ml DMF mit 93 mg BOP, 29 mg HOBT, 0,044 ml N-Methylmorpholin und 100 mg H-Phe[C]Phe-(L)-Val-(L)-Phe-morpholin-4-ylamid (Beispiel 4) in die Titelverbindung überführt. Kristallisieren aus Ether und Trocknen führt zur Titelverbindung. DC $R_f$(G)= 0,5; FAB-MS $(M+H)^+$= 841,5.

**7 a) N-Chlorocarbonyl-morpholin**

180 ml Toluol werden innerhalb von 5 min bei RT mit 180 ml einer 20 %-igen Phosgenlösung in Toluol und danach innerhalb von 10 min unter Kühlung auf 5 bis 10 °C mit einer Lösung von 18 ml Morpholin in 180 ml Toluol versetzt. Die weisse Suspension wird 1 h bei RT und weitere 2 h unter einem Stickstoff-Strom gerührt. Nach Abnutschen des Feststoffes und Nachwaschen desselben mit Toluol wird das Filtrat eingedampft. Die so erhaltene Titelverbindung wird ohne zusätzliche Reinigung weiterverarbeitet. IR ($CH_2Cl_2$): 1730, 1400, 1205 $cm^{-1}$.

**7 b) N-Morpholinocarbonyl-(L)-Val-benzylester**

3 ml N-Chlorocarbonyl-morpholin in 210 ml $CH_2Cl_2$ werden mit 15 g des p-Toluolsulfonsalzes von (L)-Valin-Benzylester (Fluka, Buchs, Schweiz) und 15,4 ml N-Ethyldiisopropylamin versetzt. Nach 16 h Rühren bei RT wird nochmals mit 1,5 ml, nach 23 h erneut mit 0,8 ml N-Chlorocarbonyl-morpholin versetzt. Nach insgesamt 39 Stunden wird das Reaktionsgemisch aufkonzentriert, mit Essigester verdünnt und nacheinander zweimal mit 1 N Salzsäure, einmal mit Wasser, einmal mit gesättigter wässriger Natriumbicarbonat-Lösung und zweimal mit Sole gewaschen. Nach Trocknen über Natriumsulfat wird unter vermindertem Druck eingeengt. Das Rohprodukt wird durch Säulenchromatographie ($SiO_2$, Essigester) gereinigt unter Erhalt der Titelverbindung. DC $R_f$(B)= 0,5.

**7 c) N-Morpholinocarbonyl-(L)-Val**

Eine Lösung von 9,7 g N-Morpholinocarbonyl-(L)-Val-benzylester in 300 ml Essigester wird während 3 h bei RT in Gegenwart von 2 g 10 %-igem Pd/C bei Normaldruck hydriert. Nach Abfiltrieren und Nachwaschen des Katalysators mit Essigester wird die Mutterlauge eingeengt. Der Rückstand wird in Essigester aufgenommen, über Hyflo Super Cel® (Kieselgur, Fluka, Buchs, Schweiz) filtriert und unter vermindertem Druck eingeengt. Die erhaltene Titelverbindung wird ohne weitere Reinigung weiterverarbeitet.

**Beispiel 8: Boc-Cha[C]Cha-(L)-Val-(L)-Cha-morpholin-4-ylamid**

Analog Beispiel 3 a) werden 100 mg Boc-Phe[C]Phe-(L)-Val-(L)-Phe-morpholin-4-ylamid (Beispiel 2) in 30 ml Methanol in Gegenwart von 40 mg Nishimura-Katalysator über 4 h bei RT hydriert. Nach Abfiltrieren des

Katalysators und Einengen wird der Rückstand aus Hexan auskristallisiert und durch Säulenchromatographie ($SiO_2$, Hexan/Essigester: 1/2 (v/v)) gereinigt unter Erhalt der Titelverbindung. DC $R_f$(I)= 0,5; FAB-MS $(M+H)^+$= 747.

**Beispiel 9:** **Boc-Cha[C](p-F)Phe-(L)-Val-(L)-(p-F-Phe)-morpholin-4-ylamid**

Analog Beispiel 1) werden 0, 18 g 5 (S)-(Boc-amino)-4(S)-(tert-butyldimethylsilyloxy)-6-cyclohexyl-2(R)-(p-fluor-phenylmethyl)-hexanoyl-(L)-Val-(L)-(p-F-Phe)-morpholin-4-ylamid mit 114 mg TBAF in 1,8 ml DMF in die Titelverbindung überführt: DC $R_f$(B)=0,44; FAB-MS $(M+H)^+$=771.

Das Ausgangsmaterial wird wie folgt hergestellt:

**9 a) Z-(L)-p-Fluorphenylalanin**

Zu einer Lösung von 5,0 g H-(L)-(p-F-Phe)-OH [(L)-p-Fluorphenylalanin (Fluka, Buchs, Schweiz)] in 55 ml THF und 20 ml $H_2O$ wird 2 N NaOH bis zu einem pH von etwa 10 gegeben. Zur resultierenden Suspension tropft man 4,66 g Chlorameisensäure-benzylester und rührt während 4 h bei RT nach; den pH hält man durch Zugabe von 2 N NaOH bei etwa 10. Das Reaktionsgemisch wird eingedampft, der Rückstand zwischen Essigsäureethylester, 10 % Zitronensäurelösung und Sole verteilt und mit $Na_2SO_4$ getrocknet. Durch Säulenchromatographie ($SiO_2$, Dichlormethan/Methanol 7:3) erhält man die reine Titelverbindung: DC $R_f$(K)=0,50.

**9 b) Z-(L)-(p-F-Phe)-morpholin-4-ylamid**

Analog Beispiel 1 l) werden 9,01 g Z-(L)-(p-F-Phe)-OH und 2,38 g Morpholin in 350 ml Dichlormethan mit 5,62 g DCC in die Titelverbindung überführt, die nach Säulenchromatographie ($SiO_2$, Essigester) in reiner Form erhalten wird: DC $R_f$(B)=0,6.

**9 c) H-(L)-(p-F-Phe)-morpholin-4-ylamid**

Analog Beispiel 1 m) werden 0,90 g Z-(L)-(p-F-Phe)-morpholin-4-ylamid in 50 ml MeOH durch Hydrogenolyse mit 0,2 g 10 %-igem Pd/C in die Titelverbindung überführt: DC $R_f$(L)=0,4.

**9 d) Z-(L)-Val-(L)-(p-F-Phe)-morpholin-4-ylamid**

Analog Beispiel 1 n) werden 1,36 g H-(L)-(p-F-Phe)-morpholin-4-ylamid und 1,36 g Z-(L)-Val in 70 ml Dichlormethan mit 1,11 g DCC in die Titelverbindung überführt: DC $R_f$(B)=0,55.

**9 e) H-(L)-Val-(L)-(p-F-Phe)-morpholin-4-ylamid**

Analog Beispiel 1 m) werden 2,80 g Z-(L)-Val-(L)-(p-F-Phe)-morpholin-4-ylamid in 150 ml MeOH durch Hydrogenolyse mit 0,6 g 10 %-igem Pd/C in die Titelverbindung überführt, die nach Säulenchromatographie ($SiO_2$, Dichlormethan/Methanol 9:1) in reiner Form erhalten wird: DC $R_f$(F)=0,44; FAB-MS $(M+H)^+$=352.

**9 f) 5(S)-(Boc-amino)-4(S)-(tert-butyldimethylsilyloxy)-6-cyclohexyl-2(R)-(p-fluor-phenylmethyl)-hexanoyl-(L)-Val-(L)-(p-F-Phe)-morpholin-4-ylamid**

Eine Lösung von 100 mg 5(S)-(Boc-amino)-4(S)-(tert-butyldimethylsilyloxy)-6-cyclohexyl-2(R)-(p-fluorphenylmethyl)-hexansäure (Beispiel 1 j) und 70,3 mg H-(L)-Val-(L)-(p-F-Phe)-morpholin-4-ylamid in 1,7 ml NMM/$CH_3CN$ 0,25 M (0,25 M NMM in $CH_3CN$) wird mit 76 mg HBTU versetzt. Nach 16 h bei RT dampft man ein. Verteilen des Rückstands zwischen 3 Portionen Essigsäureethylester, Wasser, 2 Portionen 10 %-iger Zitronensäure-Lösung, Wasser, 2 Portionen gesättigter Natriumbicarbonat-Lösung, Wasser und zuletzt Sole, Trocknen der organischen Phasen mit Natriumsulfat und Eindampfen liefert die Titelverbindung: DC $R_f$(A)=0,20; FAB-MS $(M+H)^+$=885.

**Beispiel 10:** **Boc-Cha[C](p-F)Phe-(L)-Val-(L)-(p-$CH_3O$-Phe)-morpholin-4-ylamid**

Analog Beispiel 1) werden 0, 18 g 5(S)-(Boc-amino)-4(S)-(tert-butyldimethylsilyloxy)-6-cyclohexyl-2(R)-(p-fluor-phenylmethyl)-hexanoyl-(L)-Val-(L)-(p-$CH_3O$)Phe-morpholin-4-ylamid mit 114 mg TBAF in 1,8 ml DMF in die Titelverbindung überführt: DC $R_f$(B)=0,40; FAB-MS $(M+H)^+$=783.

Das Ausgangsmaterial wird wie folgt hergestellt:

**10 a) Z-(DL)-p-Methoxy-phenylalanin**

Analog Beispiel 9 a) werden 2,5 g DL-p-Methoxy-phenylalanin (Bachem, Bubendorf, Schweiz) in 64 ml THF und 17,9 ml $H_2O$ mit 2,3 g Chlorameisensäurebenzylester umgesetzt, wobei der pH durch Zugabe von 1 N $Na_2CO_3$-Lösung bei etwa 10 gehalten wird. Eindampfen des Reaktionsgemisches und Verteilen des Rückstandes zwischen Essigsäureethylester und verdünnter Salzsäure und Sole liefert die Titelverbindung: DC $R_f$(B)=0,3; IR ($CH_2Cl_1$): 1720, 1612, 1513 $cm^{-1}$.

**10 b) Z-(DL)-(p-$CH_3$O-Phe)-morpholin-4-ylamid**

Analog Beispiel 1l) werden 2,4 g Z-(DL)-(p-$CH_3$O-Phe)-OH (Z-(DL)-p-Methoxy-phenylalanin) in 36 ml Methylenchlorid und 0,63 g Morpholin in 36 ml Methylenchlorid mit 1,5 g DCC zur Titelverbindung umgesetzt: DC $R_f$(B)=0,5; IR ($CH_2Cl_2$): 1720, 1641, 1612, 1512 $cm^{-1}$.

**10 c) H-(DL)-(p-$CH_3$O-Phe)-morpholin-4-ylamid**

Analog Beispiel 1 m) werden 3,8 g Z-(DL)-(p-$CH_3$O-Phe)-morpholin-4-ylamid in 170 ml Methanol durch Hydrogenolyse mit 0,8 g 10 %-igem Pd/C zur Titelverbindung umgesetzt, die nach Säulenchromatographie ($SiO_2$, Methylenchlorid/Methanol 9:1) rein erhalten wird: DC $R_f$(F)=0,3; IR ($CH_2Cl_2$): 1642, 1613, 1514, 1463, 1443.

**10 d) Z-(L)-Val-(DL)-(p-$CH_3$O-Phe)-morpholin-4-ylamid**

Analog Beispiel 1 n) werden 1,80 g Z-(L)-Val-OH in 40 ml Methylenchlorid und 1,90 g H-(DL)-(p-$CH_3$O-Phe)-morpholin-4-ylamid in 40 ml Methylenchlorid mit 1,48 g DCC zur Titelverbindung umgesetzt: DC $R_f$(B)=0,12; IR ($CH_2Cl_2$): 1722, 1674, 1643, 1612, 1512, 1465, 1443.

**10 e) H-(L)-Val-(L)-(p-$CH_3$O-Phe)-morpholin-4-ylamid und H-(L)-Val-(D)-(p-$CH_3$O-Phe)-morpholin-4-ylamid**

Analog Beispiel 1 m) werden 3,6 g Z-(L)-Val-(DL)-(p-$CH_3$O-Phe)-morpholin-4-ylamid in 150 ml MeOH durch Hydrogenolyse mit 0,6 g 10 %-igem Pd/C ins Gemisch der Titelverbindungen überführt. Säulenchromatographie ($SiO_2$, Dichlormethan → Dichlormethan/Methanol 19:1 → Dichlormethan/Methanol 9:1) liefert zuerst eine Fraktion, die gemäss Aminosäureanalytik mittels GC an einer Chirasil-L-Val Säule (E. Bayer, Z. *Naturforschung.*, *B* **1983**, *38*, 1281 ) H-(L)-Val-(L)-(p-$CH_3$O-Phe)-morpholin-4-ylamid {DC $R_f$(F)=0,52; FAB-MS $(M+H)^+$=364; GC $T_{Ret.}$[(p-$CH_3$O-Phe)-Derivat]=27,65 min} enthält, gefolgt von einer Fraktion mit dem Epimeren H-(L)-Val-(D)-(p-$CH_3$O-Phe)-morpholin-4-ylamid {DC $R_f$(F)=0,37; GC $T_{Ret.}$[(p-$CH_3$O)Phe-Derivat]=27,26 min }.

**10 f) 5(S)-(Boc-amino)-4(S)-(tert-butyldimethylsilyloxy)-6-cyclohexyl-2(R)-(p-fluor-phenylmethyl)-hexanoyl-(L)-Val-(L)-(p-$CH_3$O-Phe)-morpholin-4-ylamid**

Analog Beispiel 9 f) werden 100 mg 5(S)-(Boc-amino)-4(S)-(tert-butyldimethylsilyloxy)-6-cyclohexyl-2(R)-(p-fluor-phenylmethyl)-hexansäure (Beispiel 1 j) und 72,7 mg H-(L)-Val-(L)-(p-$CH_3$O-Phe)-morpholin-4-ylamid in 1,7 ml NMM/$CH_3$CN 0,25 M und 1 ml DMF mit 76 mg HBTU zur Titelverbindung umgesetzt: DC $R_f$(A)=0,18; FAB-MS $(M+H)^+$=897.

**Beispiel 11: Boc-Cha[C](p-F)Phe-(L)-Val-(L)-Cha-morpholin-4-ylamid**

Analog Beispiel 1) werden 0,16 g 5(S)-(Boc-amino)-4(S)-(tert-butyldimethylsilyloxy)-6-cyclohexyl-2(R)-(p-fluor-phenylmethyl)-hexanoyl-(L)-Val-(L)-Cha-morpholin-4-ylamid mit 114 mg TBAF in 1,8 ml DMF in die Titelverbindung überführt: DC $R_f$(B)=0,49; FAB-MS $(M+H)^+$=759.

Das Ausgangsmaterial wird wie folgt hergestellt:

**11 a) H-(L)-Val-(L)-Cha-morpholin-4-ylamid**

Analog Beispiel 3 a) wird 1,0 g H-(L)-Val-(L)-Phe-morpholin-4-ylamid (Beispiel 1 o) in 25 ml Methanol mit

0, 15 g *Nishimura*-Katalysator zur Titelverbindung hydriert, die durch Säulenchromatographie ($SiO_2$, Methylenchlorid → Methylenchlorid/Methanol 40:1) und Digerieren aus Hexan rein erhalten wird: DC $R_f$(F)=0,50; FAB-MS $(M+H)^+$=340; IR ($CH_2Cl_2$): 1645, 1509, 1463, 1449.

**11 b) 5(S)-(Boc-amino)-4(S)-(tert-butyldimethylsilyloxy)-6-cyclohexyl-2(R)-(p-fluor-phenylmethyl)-hexanoyl-(L)-Val-(L)-Cha-morpholin-4-ylamid**

Analog Beispiel 9 f) werden 100 mg 5(S)-(Boc-amino)-4(S)-(tert-butyldimethylsilyloxy)6-cyclohexyl-2(R)-(p-fluor-phenylmethyl)-hexansäure (Beispiel 1 j) und 67,9 mg H-(L)-Val-(L)-Cha-morpholin-4-ylamid in 1,7 ml NMM/$CH_3CN$ 0,25 M mit 76 mg HBTU zur Titelverbindung umgesetzt: DC $R_f$(A)=0,47; FAB-MS $(M+H)^+$=873.

**Beispiel 12: 1,2,3,4-Tetrahydroisochinolin-3(S)-carbonyl-Val-Phe[C]Phe-(L)-Val-(L)-Phe-morpholin-4-ylamid**

Analog Beispiel 4) werden 242 mg N-Boc- 1,2,3,4-tetrahydro-isochinolin-3(S)-carbonyl-Val-Phe[C]Phe-(L)-Val-(L)-Phe-morpholin-4-ylamid in 8 ml Methylenchlorid mit 8 ml Trifluoressigsäure zur Titelverbindung gespalten: DC $R_f$(J)=0,5 ; FAB-MS $(M+H)^+$=887,5; IR (KBr): 1639, 1531, 1495, 1453.

Das Ausgangsmaterial wird wie folgt hergestellt:

**12 a) N-Boc- 1,2,3,4-tetrahydro-isochinolin-3(S)-carbonsäure**

In 400 ml Dioxan/Wasser 1:1 werden 20 g 1,2,3,4-Tetrahydro-isochinolin-3(S)-carbonsäure (Herstellung siehe: P.L. Julian, W.J. Karpel, A. Magnani and E.W. Meyer, *J. Am. Chem. Soc.* **1948**, 70, 180, jedoch ausgehend von (L)-β-Phenylalanin), 233,6 g Kaliumcarbonat und 37 g Boc-Anhydrid während 4 h bei RT gerührt. Das Reaktionsgemisch wird mit verdünnter HCl auf pH 2 angesäuert und 3 mal mit Essigsäureethylester extrahiert. Nach Waschen der organischen Phasen mit 1 N Kaliumhydrogensulfat-Lösung, Wasser und Sole, Trocknen mit $Na_2SO_4$, Eindampfen und Kristallisieren aus Methylenchlorid/Hexan erhält man die Titelverbindung: DC $R_f$(C)=0,2; $[\alpha]_D$=16 (c=1, MeOH).

**12 b) N-Boc-1,2,3,4-tetrahydro-isochinolin-3(S)-carbonyl-L-Val-benzylester**

Unter $N_2$-Atmosphäre werden 6,47 g N-Boc- 1,2,3,4-tetrahydro-isochinolin-3(S)-carbonsäure in 70 ml Methylenchlorid bei 0°C mit 4,7 g 1-Chloro-N,N,2-trimethylprop-1-enylamin (Haveaux, B., Dekoker, A., Rens, M., Sidani, A. R., Toye, J. und Ghosez, L., Org. Synth. 59, 26-34) ins entsprechende Säurechlorid überführt und nach 15 min mit 9,0 g *Hünig*-Base und einer Lösung von 6,83 g L-Val-benzylester.HCl in 54 ml Methylenchlorid versetzt. Nach 15 min bei 0°C und 16 h bei RT wird das Reaktionsgemisch mit 10 %-iger Zitronensäure-Lösung, Wasser, ges. Natriumcarbonat-Lösung, Wasser und Sole gewaschen. Die wässrigen Phasen werden mit 2 Portionen Methylenchlorid extrahiert, die vereinigten organischen Phasen mit Natriumsulfat getrocknet und eingedampft. Da laut $^1$H-NMR dabei die Boc-Schutzgruppe zum Teil abgefallen ist, wird das Rohprodukt noch mit 6,8 g Boc-Anhydrid in 160 ml Methylenchlorid und 2,7 g *Hünig*-Base umgesetzt. Säulenchromatographie ($SiO_2$, Hexan/Essigsäureethylester 3:1) liefert die reine Titelverbindung: DC $R_f$(E)=0, 15.

**12 c) N-Boc-1,2,3,4-tetrahydro-isochinolin-3(S)-carbonyl-L-Val**

Analog Beispiel 7 c) werden 1,97 g N-Boc- 1,2,3,4-tetrahydro-isochinolin-3(S)-carbonyl-L-Val-benzylester in 50 ml Essigsäureethylester mit 0,4 g 10 %-igem Pd/C zur Titelverbindung hydriert. Dieses Material wird ohne weitere Reinigung in der nächsten Stufe eingesetzt: DC $R_f$(J)=0,53; FAB-MS $(M+H)^+$=377.

**12 d) N-Boc-1,2,3,4-tetrahydro-isochinolin-3(S)-carbonyl-Val-Phe[C]Phe-(L)-Val-(L)-Phe-morpholin-4-ylamid**

Analog Beispiel 5) werden 180 mg N-Boc- 1,2,3,4-tetrahydro-isochinolin-3(S)-carbonyl-L-Val in 4 ml DMF mit 232 mg BOP, 71 mg HOBT, 0,11 ml NMM und einer Lösung von 250 mg H-Phe[C]Phe-(L)-Val-(L)-Phe-morpholin-4-ylamid (Beispiel 4) in 2 ml DMF umgesetzt. Eine Säulenchromatographie ($SiO_2$, Essigsäureethylester) liefert die Titelverbindung, die gemäss Aminosäure-Analytik (GC, Chirasil-*L*-Val: (E. Bayer, Z. *Natur-forschung.*, *B* **1983**, *38*, 1281) als Nebenprodukt ein Epimeres ca. (14 % D-Val-Anteil) enthält: DC $R_f$(B)=0,4; FAB-MS $(M+H)^+$=987; IR (KBr): 1697, 1643, 1523, 1496.

**Beispiel 13: Boc-Phe[C]Phe-(L)-Val-(L)-(p-F-Phe)-morpholin-4-ylamid**

Analog Beispiel 1 ) werden 375 mg 5(S)-(Boc-amino)-4(S)-(tert-butyldimethylsilyloxy)-6-phenyl-2(R)-phenylmethyl-hexanoyl-(L)-Val-(L)-(p-F-Phe)-morpholin-4-ylamid in 7 ml DMF mit 275 mg TBAF zur Titelverbindung desilyliert, die nach Kristallisaton aus Diethylether/Hexan rein erhalten wird: DC $R_f$(B)=0,50; FAB-MS $(M+H)^+$=747.

Das Ausgangsmaterial wird wie folgt hergestellt:

**13 a) 5(S)-(Boc-amino)-4(S)-(tert-butyldimethylsilyloxy)-6-phenyl-2(R)-phenylmethyl-hexanoyl-(L)-Val-(L)-(p-F-Phe)-morpholin-4-ylamid**

Analog Beispiel 1 k) werden 250 mg 5(S)-(Boc-amino)-4(S)-(tert-butyldimethylsilyloxy)-6-phenyl-2(R)-phenylmethyl-hexansäure (Beispiel 2 d) in 5 ml DMF mit 230,6 mg BOP, 70,4 mg HOBT, 130 μl NMM und 199,7 mg H-(L)-Val-(L)-(p-F)Phe-morpholin-4-ylamid (Herstellung siehe Beispiel 9 e) gelöst in 2 ml DMF umgesetzt. Säulenchromatographie ($SiO_2$, Essigsäureethylester/Hexan 2:1) liefert die Titelverbindung: DC $R_f$(I)=0,43; FAB-MS $(M+H)^+$=861.

**Beispiel 14: Boc-Phe[C]Phe-(L)-Val-(L)-(p-$CH_3O$-Phe)-morpholin-4-ylamid**

Analog Beispiel 1) werden 283 mg 5(S)-(Boc-amino)-4(S)-(tert-butyldimethylsilyloxy)-6-phenyl-2(R)-phenylmethyl-hexanoyl-(L)-Val-(L)-(p-$CH_3O$)Phe-morpholin-4-ylamid in 7 ml DMF mit 204,5 mg TBAF zur Titelverbindung desilyliert, die nach Digerieren aus Diethylether/Hexan 1:1 und Säulenchromatographie ($SiO_2$, Essigsäureethylester) rein erhalten wird: DC $R_f$(B)=0,37; FAB-MS $(M+H)^+$=759.

Das Ausgangsmaterial wird wie folgt hergestellt:

**14 a) 5(S)-(Boc-amino)-4(S)-(tert-butyldimethylsilyloxy)-6-phenyl-2(R)-phenylmethyl-hexanoyl-(L)-Val-(L)-(p-$CH_3O$-Phe)-morpholin-4-ylamid**

Analog Beispiel 1 k) werden 250 mg 5(S)-(Boc-amino)-4(S)-(tert-butyldimethylsilyloxy)-6-phenyl-2(R)-phenylmethyl-hexansäure (Beispiel 2 d) in 5 ml DMF mit 230,6 mg BOP, 70,4 mg HOBT, 130 μl NMM und 206,6 mg H-(L)-Val-(L)-(p-$CH_3O$)Phe-morpholin-4-ylamid (Herstellung siehe Beispiel 10 e) gelöst in 2 ml DMF umgesetzt. Säulenchromatographie ($SiO_2$, Essigsäureethylester/Hexan 2:1) liefert die Titelverbindung: DC $R_f$(I)=0,37; FAB-MS $(M+H)^+$=873.

**Beispiel 15: Boc-Phe[C]Phe-(L)-Val-(L)-Cha-morpholin-4-ylamid**

Analog Beispiel 1) werden 430 mg 5(S)-(Boc-amino)-4(S)-(tert-butyldimethylsilyloxy)-6-phenyl-2(R)-phenylmethyl-hexanoyl-(L)-Val-(L)-Cha-morpholin-4-ylamid in 5 ml DMF mit 320 mg TBAF zur Titelverbindung desilyliert, die nach Säulenchromatographie ($SiO_2$, Essigsäureethylester) und Digerieren aus Hexan rein erhalten wird: DC $R_f$(F)=0,51; FAB-MS $(M+H)^+$=735.

Das Ausgangsmaterial wird wie folgt hergestellt:

**15 a) 5(S)-(Boc-amino)-4(S)-(tert-butyldimethylsilyloxy)-6-phenyl-2(R)-phenylmethyl-hexanoyl-(L)-Val-(L)-Cha-morpholin-4-ylamid**

Analog Beispiel 1 k) werden 300 mg 5(S)-(Boc-amino)-4(S)-(tert-butyldimethylsilyloxy)-6-phenyl-2(R)-phenylmethyl-hexansäure (Beispiel 2d) in 3 ml DMF mit 277 mg BOP, 84,5 mg HOBT, 0,22 ml NMM und 231 mg H-(L)-Val-(L)-Cha-morpholin-4-ylamid (Herstellung siehe Beispiel 11 a) gelöst in 2 ml DMF umgesetzt. Säulenchromatographie ($SiO_2$, Essigsäureethylester/Hexan 1:1) liefert die Titelverbindung: DC $R_f$(A)=0,28; FAB-MS $(M+H)^+$=849.

**Beispiel 16: Boc-Phe[C]Phe-(L)-Ile-(L)-Phe-morpholin-4-ylamid**

Analog Beispiel 1) werden 329,8 mg 5(S)-(Boc-amino)-4(S)-(tert-butyldimethylsilyl-oxy)-6-phenyl-2(R)-phenylmethyl-hexanoyl-(L)-Ile-(L)-Phe-morpholin-4-ylamid in 5 ml DMF mit 242,7 mg TBAF zur Titelverbindung desilyliert, die nach Digerieren aus Hexan rein erhalten wird: DC $R_f$(B )=0,57; FAB-MS $(M+H)^+$=743.

Das Ausgangsmaterial wird wie folgt hergestellt:

**16 a) Z-(L)-Ile-(L)-Phe-morpholin-4-ylamid**

Analog Beispiel 1 n) werden 294 mg Z-(L)-Ile in 10 ml Dichlormethan mit 229 mg DCC und 260 mg H-(L)-Phe-morpholin-4-ylamid (Beispiel 1 m) in die Titelverbindung überführt, die nach Säulenchromatographie ($SiO_2$, Essigsäureethylester/Hexan 1:1)in reiner Form erhalten wird: DC $R_f$(A)=0,43; FAB-MS $(M+H)^+$=482.

**16 b) H-(L)-Ile-(L)-Phe-morpholin-4-ylamid**

Analog Beispiel 1 m) werden 0,38 g Z-(L)-Ile-(L)-Phe-morpholin-4-ylamid in 15 ml MeOH durch Hydrogenolyse mit 0, 12 g 10 %-igem Pd/C in die Titelverbindung überführt, die nach Säulenchromatographie ($SiO_2$, Dichlormethan/Methanol 9:1) in reiner Form erhalten wird: DC $R_f$(F)=0,5; FAB-MS $(M+H)^+$=348.

**16 c) 5(S)-(Boc-amino)-4(S)-(tert-butyldimethylsilyloxy)-6-phenyl-2(R)-phenylmethyl-hexanoyl-(L)-Ile-(L)-Phe-morpholin-4-ylamid**

Analog Beispiel 1 k) werden 240,5 mg 5(S)-(Boc-amino)-4(S)-(tert-butyldimethylsilyloxy)-6-phenyl-2(R)-phenylmethyl-hexansäure (Beispiel 2 d) in 5 ml DMF mit 242 mg BOP, 73,8 mg HOBT, 125,5 μl NMM und 190 mg H-(L)-Ile-(L)-Phe-morpholin-4-ylamid umgesetzt. Säulenchromatographie ($SiO_2$, Essigsäureethylester/Hexan 1:1) liefert die Titelverbindung: DC $R_f$(A)=0,21; FAB-MS $(M+H)^+$=857.

**Beispiel 17: Boc-Phe[C]Phe-(L)-Val-Gly-morpholin-4-ylamid**

Analog Beispiel 1) werden 343,7 mg 5(S)-(Boc-amino)-4(S)-(tert-butyldimethylsilyloxy)-6-phenyl-2(R)-phenylmethyl-hexanoyl-(L)-Val-Gly-morpholin-4-ylamid in 5 ml DMF mit 282,7 mg TBAF zur Titelverbindung desilyliert und schliesslich aus Diethylether digeriert: DC $R_f$(B)=0,23; FAB-MS $(M+H)^+$=639.

Das Ausgangsmaterial wird wie folgt hergestellt:

**17 a) Z-Gly-morpholin-4-ylamid**

Analog Beispiel 1l) werden 8,37 g Z-Gly-OH in 500 ml Dichlormethan mit 8,25 g DCC und 3,49 ml Morpholin in die Titelverbindung überführt: DC $R_f$(B)=0,28.

**17 b) H-Gly-morpholin-4-ylamid**

Analog Beispiel 1 m) werden 10,8 g Z-Gly-morpholin-4-ylamid in 600 ml MeOH durch Hydrogenolyse mit 3 g 10 %-igem Pd/C in die Titelverbindung überführt, die nach Abfiltrieren des Katalysators und Eindampfen des Filtrats direkt in der nächsten Stufe eingesetzt wird: DC $R_f$(F)=0,2; IR ($CH_2Cl_2$): 1654, 1461, 1440.

**17 c) Z-(L)-Val-Gly-morpholin-4-ylamid**

Analog Beispiel 1 n) werden 2,51 g Z-(L)-Val in 75 ml Dichlormethan mit 2,06 g DCC und 1,44 g H-Gly-morpholin-4-ylamid in die Titelverbindung überführt: DC $R_f$(B)=0,21.

**17 d) H-(L)-Val-Gly-morpholin-4-ylamid**

Analog Beispiel 1 m) werden 3,7 g Z-(L)-Val-Gly-morpholin-4-ylamid in 160 ml MeOH durch Hydrogenolyse mit 0,6 g 10 %-igem Pd/C in die Titelverbindung überführt, die nach Abfiltrieren des Katalysators durch Säulenchromatographie ($SiO_2$, Dichlormethan/Methanol 9:1 → 4:1) in reiner Form erhalten wird: DC $R_f$(F)=0,23; FAB-MS $(M+H)^+$=244; IR ($CH_2Cl_2$): 1650, 1508, 1467, 1439.

**17 e) 5(S)-(Boc-amino)-4(S)-(tert-butyldimethylsilyloxy)-6-phenyl-2(R)-phenylmethyl-hexanoyl-(L)-Val-Gly-morpholin-4-ylamid**

Analog Beispiel 1 k) werden 300 mg 5(S)-(Boc-amino)-4(S)-(tert-butyldimethylsilyloxy)-6-phenyl-2(R)-phenylmethyl-hexansäure (Beispiel 2 d) in 5 ml DMF mit 302 mg BOP, 92,1 mg HOBT, 156,5 μl NMM und 175,5 mg H-(L)-Val-Gly-morpholin-4-ylamid umgesetzt. Säulenchromatographie ($SiO_2$, Essigsäureethylester/Hexan 1:9 → Essigsäureethylester) liefert die Titelverbindung: DC $R_f$(B)=0,44; FAB-MS $(M+H)^+$=753.

**Beispiel 18: Boc-Phe[C]Phe-(L)-Ile-Gly-morpholin-4-ylamid**

Analog Beispiel 1) werden 362,4 mg 5(S)-(Boc-amino)-4(S)-(tert-butyldimethylsilyloxy)-6-phenyl-2(R)-phenylmethyl-hexanoyl-(L)-Ile-Gly-morpholin-4-ylamid in 5 ml DMF mit 292,7 mg TBAF zur Titelverbindung desilyliert und schliesslich aus Diethylether digeriert: DC $R_f$(B)=0,30; FAB-MS $(M+H)^+$=653.

Das Ausgangsmaterial wird wie folgt hergestellt:

**18 a) Z-(L)-Ile-Gly-morpholin-4-ylamid**

Analog Beispiel 1 n) werden 2,65 g Z-(L)-Ile in 75 ml Dichlormethan mit 2,06 g DCC und 1,44 g H-Gly-morpholin-4-ylamid (Beispiel 17 b) umgesetzt. Nach Digerieren in Diethylether erhält man die Titelverbindung: DC $R_f$(F)=0,7.

**18 b) H-(L)-Ile-Gly-morpholin-4-ylamid**

Analog Beispiel 1 m) werden 3,2 g Z-(L)-Ile-Gly-morpholin-4-ylamid in 160 ml MeOH durch Hydrogenolyse mit 0,6 g 10 %-igem Pd/C in die Titelverbindung überführt, die nach Abfiltrieren des Katalysators durch Säulenchromatographie ($SiO_2$, Dichlormethan/Methanol 9:1) in reiner Form erhalten wird: DC $R_f$(F)=0,3; FAB-MS $(M+H)^+$=258; IR ($CH_2Cl_2$): 1653, 1510, 1467, 1439.

**18 c) 5(S)-(Boc-amino)-4(S)-(tert-butyldimethylsilyloxy)-6-phenyl-2(R)-phenylmethyl-hexanoyl-(L)-Ile-Gly-morpholin-4-ylamid**

Analog Beispiel 1 k) werden 300 mg 5(S)-(Boc-amino)-4(S)-(tert-butyldimethylsilyloxy)-6-phenyl-2(R)-phenylmethyl-hexansäure (Beispiel 2 d) in 5 ml DMF mit 301,8 mg BOP, 92, 1 mg HOBT, 156,5 µl NMM und 185 mg H-(L)-Ile-Gly-morpholin-4-ylamid umgesetzt. Säulenchromatographie ($SiO_2$, Essigsäureethylester/Hexan 9:1) liefert die Titelverbindung: DC $R_f$(B)=0,40; FAB-MS $(M+H)^+$=767.

**Beispiel 19: Boc-Phe[C]Phe-(L)-Val-(L)-Val-morpholin-4-ylamid**

Analog Beispiel 1) werden 416 mg 5 (S)- (Boc-amino)-4(S)-(tert-butyldimethylsilyloxy)-6-phenyl-2(R)-phenylmethyl-hexanoyl-(L)-Val-(L)-Val-morpholin-4-ylamid in 10 ml DMF mit 330 mg TBAF zur Titelverbindung desilyliert und schliesslich aus Diethylether digeriert: DC $R_f$(B)=0,39; FAB-MS $(M+H)^+$=681.

Das Ausgangsmaterial wird wie folgt hergestellt:

**19 a) Z-(L)-Val-(L)-Val-morpholin-4-ylamid**

Analog Beispiel 1l) werden 2,0 g Z-(L)-Val-(L)-Val-OH (Bachem, Bubendorf, Schweiz) in 50 ml Dichlormethan mit 1,17 g DCC und 0,96 ml Morpholin gelöst in 50 ml Dichlormethan in die Titelverbindung überführt: DC $R_f$(B)=0,5.

**19 b) H-(L)-Val-(L)-Val-morpholin-4-ylamid**

Analog Beispiel 1 m) werden 2,3 g Z-(L)-Val-(L)-Val-morpholin-4-ylamid in 220 ml MeOH durch Hydrogenolyse mit 0,5 g 10 %-igem Pd/C in die Titelverbindung überführt, die nach Abfiltrieren des Katalysators durch Säulenchromatographie ($SiO_2$, Dichlormethan → Dichlormethan/Methanol 19:1 → 9:1) und Verrühren in Diethylether/Hexan in reiner Form erhalten wird: DC $R_f$(F)=0,74; FAB-MS $(M+H)^+$=286; IR ($CH_2Cl_2$): 1642, 1507, 1461, 1440.

**19 c) 5(S)-(Boc-amino)-4(S)-(tert-butyldimethylsilyloxy)-6-phenyl-2(R)-phenylmethyl-hexanoyl-(L)-Val-(L)-Val-morpholin-4-ylamid**

Analog Beispiel 1 k) werden 300 mg 5(S)-(Boc-amino)-4(S)-(tert-butyldimethylsilyloxy)-6-phenyl-2(R)-phenylmethyl-hexansäure (Beispiel 2d) in 5 ml DMF mit 277 mg BOP, 84,5 mg HOBT, 156,5 µl NMM und 194,6 mg H-(L)-Val-(L)-Val-morpholin-4-ylamid umgesetzt. Säulenchromatographie ($SiO_2$, Essigsäureethylester/Hexan 2:1) liefert die Titelverbindung: DC $R_f$(I)=0,27; FAB-MS $(M+H)^+$=795.

**Beispiel 20:** **Boc-Phe[C]Phe-(L)-Val-(L)-Phe-thiomorpholin-4-ylamid**

Analog Beispiel 1) werden 484 mg 5(S)-(Boc-amino)-4(S)-(tert-butyldimethylsilyloxy)-6-phenyl-2(R)-phenylmethyl-hexanoyl-(L)-Val-(L)-Phe-thiomorpholin-4-ylamid in 5 ml DMF mit 356 mg TBAF desilyliert. Säulenchromatographie ($SiO_2$, Essigsäureethylester/Hexan 2:1) und Digerieren aus Hexan ergibt die Titelverbindung: DC $R_f$(I)=0,31; FAB-MS $(M+H)^+$=745.

Das Ausgangsmaterial wird wie folgt hergestellt:

**20 a)** **Z-(L)-Val-(L)-Phe-thiomorpholin-4-ylamid**

Analog Beispiel 1l) werden 1,99 g Z-(L)-Val-(L)-Phe-OH (Bachem, Bubendorf, Schweiz) in 40 ml Dichlormethan mit 1,03 g DCC und einer Lösung von 0,52 g Thiomorpholin 40 ml Dichlormethan zur Titelverbindung umgesetzt, die nach der Extraktion als Öl anfällt: DC $R_f$(F)=0,8; IR ($CH_2Cl_2$): 1725, 1675, 1642, 1499, 1465, 1454.

**20 b)** **H-(L)-Val-(L)-Phe-thiomorpholin-4-ylamid**

Unter Eiskühlung werden 2 g Z-(L)-Val-(L)-Phe-thiomorpholin-4-ylamid mit 45 ml HBr/Essigsäure 33 % (Fluka, Buchs, Schweiz) versetzt und 1,5 h bei RT gerührt. Anschliessend dampft man das Reaktionsgemisch ein, verteilt den Rückstand zwischen 3 Portionen Essigsäureethylester, ges. Natriumhydrogencarbonat-Lösung, Wasser und Sole, trocknet die organischen Phasen mit $Na_2SO_4$ und dampft sie ein. Säulenchromatographie ($SiO_2$, Methylenchlorid/Methanol 9:1) liefert zuerst die Titelverbindung, gefolgt von einem Nebenprodukt, vermutlich einem Epimeren: DC $R_f$(F)=0,56; FAB-MS $(M+H)^+$=350; IR ($CH_2Cl_2$): 1641, 1502, 1463, 1454, 1448; Aminosäureanalytik {GC, Chirasil-*L*-Val Säule (E. Bayer, *Z. Naturforschung.*, *B* **1983**, *38*, 1281)}: $T_{Ret.}${(L)Val-Derivat}=8,36 min (ee≧99 %), $T_{Ret.}${(L)-Phe-Derivat}=22,73 min (ee=94 %).

**20 c)** **5(S)-(Boc-amino)-4(S)-(tert-butyldimethylsilyloxy)-6-phenyl-2(R)-phenylmethyl-hexanoyl-(L)-Val-(L)-Phe-thiomorpholin-4-ylamid**

Analog Beispiel 1 k) werden 300 mg 5(S)-(Boc-amino)-4(S)-(tert-butyldimethylsilyloxy)-6-phenyl-2(R)-phenylmethyl-hexansäure (Beispiel 2d) in 5 ml DMF mit 277 mg BOP, 84,5 mg HOBT, 219 µl NMM und 238,4 mg H-(L)-Val-(L)-Phe-thiomorpholin-4-ylamid gelöst in 3 ml DMF umgesetzt. Säulenchromatographie ($SiO_2$, Essigsäureethylester/Hexan 1:1) liefert die Titelverbindung: DC $R_f$(A)=0,43; FAB-MS $(M+H)^+$=859.

**Beispiel 21:**

Auf analoge Weise, wie in einem der vorstehenden Beispiele beschrieben, oder auf die nachfolgend im Detail angegebene Art und Weise, werden die folgenden Verbindungen erhalten:

A) **1)** Eine der nachstehend unter B) bis J) genannten Verbindungen, worin anstelle von -morpholin-4-ylamid der Rest -thiomorpholin-4-ylamid steht;

B) **1)** **Boc-(p-F)Phe[C](p-F)Phe-(L)-Val-(L)-Phe-morpholin-4-ylamid**

Analog Beispiel 1) werden 83 mg 5(S)-(Boc-amino)-4(S)-(tert-butyldimethylsilyloxy)-6-(p-fluor-phenyl)-2(R)-(p-fluor-phenylmethyl)-hexanoyl-(L)-Val-(L)-Phe-morpholin-4-ylamid mit 60 mg TBAF in 0,95 ml DMF in die Titelverbindung überführt: FAB-MS $(M+H)^+$=765.

Das Ausgangsmaterial wird wie folgt hergestellt:

**1) a)** **N-Boc-(p-fluorphenylalanin):**

In 0,41 Dioxan/Wasser 1:1 werden 20 g (109 mMol) p-Fluorphenylalanin (Fluka; Buchs/Schweiz) mit 35,5 g (163 mMol) Boc-anhydrid und 150 g (1,09 Mol) Kaliumcarbonat umgesetzt. Nach 4 h wird das Reaktionsgemisch mit Zitronensäurelösung angesäuert und mit 3 Portionen Essigsäureethylester extrahiert. Die organischen Phasen wäscht man mit 10 %-iger Zitronensäure, Wasser und Sole, trocknet sie mit Natriumsulfat und dampft sie ein. Lösen des Rückstands in wenig Methylenchlorid und Kristallisieren durch Zusatz von Hexan liefert die Titelverbindung.

**1) b)** **N-Boc-(p-fluorphenylalaninol):**

Bei -5°C bis -10°C versetzt man eine Lösung von 17,9 g (63 mMol) N-Boc-(p-fluorphenylalanin) in 73 ml abs. THF mit 9,66 ml (69 mMol) Triethylamin und tropft eine Lösung von 9,05 ml (69 mMol) Chlorameisensäureisobutylester in 44 ml abs. THF zu. Nach 0,5 h Rühren bei RT wird der gebildete Niederschlag abgenutscht. Das Filtrat tropft man unter Kühlen zu 4,77 g (126 mMol) Natriumborhydrid in 28 ml Wasser. Nach 4 h Rühren bei RT säuert man mit 10 %-iger Zitronensäure an, dampft das THF

am Rotationsverdampfer teilweise ab und verteilt den Rückstand zwischen 3 Portionen Essigsäureethylester, 2 Portionen 2 N Natriumhydroxidlösung, Wasser, gesättigter Natriumhydrogencarbonatlösung und Sole. Die mit Natriumsulfat getrockneten und eingedampften organischen Phasen liefern nach Lösen in wenig Methylenchlorid und Kristallisieren durch Zugabe von Hexan die Titelverbindung: DC $R_f$(A)=0,36; $^1$H-NMR (200 MHz, $CD_3OD$): 7,24 (dd, 8 und 5 Hz, 2 H), 6,98 (t, 8 Hz, 2 H), 3,73 (m, 1 H), 3,47 (d, 5 Hz, 2 H), 2,88 (dd, 13 und 6 Hz, 1 H), 2,62 (dd, 13 und 8 Hz, 1 H), 1,36 (s, 9 H).

**1) c) N-Boc-(p-fluorphenylalaninal):**

Unter $N_2$-Atmosphäre werden zu einer auf -60°C abgekühlten Lösung von 4,0 ml (46,8 mMol) Oxalylchlorid in 44 ml Methylenchlorid 4,44 ml (62,4 mMol) DMSO gelöst in 76 ml Methylenchlorid getropft. Nach 15 min Rühren fügt man der klaren Reaktionslösung 8,4 g (31,2 mMol) N-Boc-(p-fluorphenylalaninol) als Lösung in 185 ml Methylenchlorid/THF 1:1 (→ Ausfällung) zu und rührt 25 min nach. Anschliessend setzt man 17,3 ml (124,8 mMol) Triethylamin gelöst in 38 ml Methylenchlorid zu. Nach 30 min Rühren werden 278 ml einer 20 %-igen Kaliumhydrogensulfatlösung zugetropft, gefolgt von 220 ml Hexan. Man lässt auf RT aufwärmen, trennt die wässrige Phase ab und extrahiert sie mit 2 Portionen Ether. Die organischen Phasen ergeben nach Waschen mit gesättigter Natriumbicarbonatlösung, Wasser und Sole, Trocknen mit Natriumsulfat und Eindampfen die Titelverbindung, die ohne weitere Reinigung in der nächsten Stufe eingesetzt wird: $^1$H-NMR (200 MHz, $CDCl_3$): 9,63 (s, 1 H), 6,9-7,2 (2m, 4 H), 5,04 (m, 1 H), 4,42 (m, 1 H), 3,10 (m, 2 H), 1,43 (s, 9 H).

**1) d) 5(S)-[1(S)-(Boc-amino)-2-(p-fluor-phenyl)ethyl]-dihydrofuran-2-(3H)-on**

Analog Beispiel 21 D) 1) b) wird aus 16,7 g 2-Iodpropionsäureethylester in 124 ml Toluol, 8,1 g Zn/Cu und 12,4 ml Dimethylacetamid das Zn-Homoenolat gebildet. Dieses überführt man mittels Kanüle zum auf -40°C bis -25°C abgekühlten Trichlortitan-isopropylat (hergestellt aus 5,11 ml Tetraisopropyl-orthotitanat und 5,71 ml Titan-tetrachlorid in 16 ml Toluol und 88,5 ml Methylenchlorid). Man erwärmt während 5 min auf -25°C und kühlt wiederum auf -40°C ab. Anschliessend tropft man eine Lösung von 9,28 g N-Boc-(p-fluorphenylalaninal) in 33 ml Methylenchlorid zu und rühn 15 h bei ca. -20°C und schliesslich 1 h bei 0°C nach. Das Rektionsgemisch wird auf 0,4 kg Eiswasser und 0,55l tert-Butylmethylether gegossen und 10 min kräftig gerührt. Man trennt die wässrige Phase ab, extrahiert sie mit 2 Portionen Ether, wäscht die organischen Phasen mit Wasser, gesättigter Natriumhydrogencarbonat-Lösung, Wasser und Sole, trocknet sie mit Natriumsulfat und dampft sie ein. Man erhält kristallinen 5(S)-(Boc-amino)-4(S)-hydroxy-6-(p-fluorphenyl)-hexansäure-ethylester als Zwischenprodukt. Dieses Zwischenprodukt wird in 244 ml Toluol und 7,3 ml Essigsäure während 2 h auf 100°C erhitzt. Man versetzt das erkaltete Reaktionsgemisch mit 0,51 Wasser, trennt die wässrige Phase ab, extrahiert sie mit 2 Portionen Ether, wäscht die org. Phasen mit gesättigter Natriumhydrogencarbonat-Lösung, Wasser und Sole, trocknet sie mit Natriumsulfat und dampft ein. Säulenchromatographie ($SiO_2$, Hexan/Essigsäureethylester 2:1) liefert die reine Titelverbindung: DC $R_f$(D)=0,22; FAB-MS $(M+H)^+$=324. $[\alpha]^D$=20,7° (c= 1; Ethanol).

**1) e) 5(S)-[1(S)-(Boc-amino)-2-(p-fluor-phenyl)ethyl]-3(R)-(p-fluorphenylmethyl)-dihydrofuran-2-(3H)-on**

Analog Beispiel 21 D) 1) c) werden 1,0 g 5(S)-[1(S)-(Boc-amino)-2-(p-fluor-phenyl)ethyl]-dihydrofuran-2-(3H)-on gelöst in 7,9 ml THF mit 6,05 µl Lithiumbis(trimethylsilyl)amid 1 M in THF deprotoniert und mit 0,673 g p-Fluorbenzylbromid bei -75°C alkyliert (1 h). Eine Säulenchromatographie ($SiO_2$, Methylenchlorid/Ether 49:1) liefert die reine Titelverbindung: DC $R_f$(M)=0,17; $^1$H-NMR (200 MHz, $CDCl_3$): 7, 19-7,05 und 7,04-6,88 (2m, je 4 H), 4,50 (d, 10 Hz, HN), 4,11 (m, 1 H), 3,87 (qm, ca. 8 Hz, 1 H), 3, 1-2,7 (m, 5 H), 2,33-2,14 und 2,02- 1,85 (2m, je 1 H), 1,35 (s, 9 H).

**1) f) 5(S)-(Boc-amino)-4(S)-hydroxy)-6-(p-fluor-phenyl)-2(R)-(p-fluorphenylmethyl)-hexansäure**

Analog Beispiel 1i) werden 790 mg 5(S)-[1(S)-(Boc-amino)-2-(p-fluorphenyl)ethyl]-3(R)-(p-fluorphenylmethyl)-dihydrofuran-2-(3H)-on in 29 ml Dimethoxyethan und 15 ml Wasser mit 7,3 ml 1 M Lithiumhydroxid-Lösung zur Titelverbindung hydrolysiert, die direkt weiterverwendet wird.

**1) g) 5(S)-(Boc-amino)-4(S)-(tert-butyldimethylsilyloxy)-6-(p-fluorphenyl)-2(R)-(p-fluor-phenylmethyl)-hexansäure**

Analog Beispiel 1j) werden 956 mg 5(S)-(Boc-amino)-4(S)-hydroxy)-6-(p-fluor-phenyl)-2(R)-(p-fluor-phenylmethyl)-hexansäure in 2,3 ml DMF mit 1,47 g tert-Butyldimethylchlorsilan und 1,19 g Imidazol silyliert. Hydrolyse der Silylesterfunktion mit 1,76 g Kaliumcarbonat in 50 ml Methanol/THF/Wasser 3:1:1 liefert nach Säulenchromatographie ($SiO_2$, Hexan/Essigsäureethylester 2:1) die Titelverbindung: DC $R_f$(D)=0, 13; FAB-MS $(M+H)^+$=564.

**1) h) 5(S)-(Boc-amino)-4(S)-(tert-butyldimethylsilyloxy)-6-(p-fluorphenyl)-2(R)-(p-fluor-phenylmethyl)-hexanoyl-(L)-Val-(L)-Phemorpholin-4-ylamid**

Analog Beispiel 9f) werden 110 mg 5(S)-(Boc-amino)-4(S)-(tert-butyldimethylsilyloxy)-6-(p-fluor-

phenyl)-2(R)-(p-fluor-phenylmethyl)hexansäure und 71,5 mg H-(L)-Val-(L)-Phe-morpholin-4-ylamid (Beispiel 1o) in 1,83 ml NMM/$CH_3CN$ 0,25 M mit 81,5 mg HBTU umgesetzt. Die reine Titelverbindung erhält man nach Säulenchromatographie ($SiO_2$, Methylenchlorid/Ether 3:1): DC $R_f$(N)=0, 14; FAB-MS $(M+H)^+$=879.

**2) Boc-(p-F)Phe[C](p-F)Phe-(L)-Val-(L)-(p-F-Phe)-morpholin-4-ylamid**

Analog Beispiel 1) werden 150 mg 5(S)-(Boc-amino)-4(S)-(tert-butyldimethylsilyloxy)-6-(p-fluorphenyl)-2(R)-(p-fluor-phenylmethyl)hexanoyl-(L)-Val-(L)-(p-F-Phe)-morpholin-4-ylamid mit 105 mg TBAF in 2,5 ml DMF in die Titelverbindung überführt: FAB-MS $(M+H)^+$=783.

Das Ausgangsmaterial wird wie folgt hergestellt:

**2) a) 5(S)-(Boc-amino)-4(S)-(tert-butyldimethylsilyloxy)-6-(p-fluorphenyl)-2(R)-(p-fluor-phenylmethyl)-hexanoyl-(L)-Val-(L)-(p-F-Phe)morpholin-4-ylamid**

Analog Beispiel 9f) werden 100 mg 5(S)-(Boc-amino)-4(S)-(tert-butyldimethylsilyloxy)-6-(p-fluorphenyl)-2(R)-(p-fluor-phenylmethyl)hexansäure [Beispiel 21 B) 1) g)] und 68,5 mg H-(L)-Val-(L)-(p-F-Phe)morpholin-4-ylamid (Beispiel 9e) in 1,67 ml NMM/$CH_3CN$ 0,25 M mit 74 mg HBTU zur Titelverbindung umgesetzt: DC $R_f$(A)=0, 17; FAB-MS $(M+H)^+$=897.

**3) Boc-(p-F)Phe[C](p-F)Phe-(L)-Val-(L)-(p-$CH_3$O-Phe)-morpholin-4-ylamid**

Analog Beispiel 1) werden 126 mg 5(S)-(Boc-amino)-4(S)-(tert-butyldimethylsilyloxy)-6-(p-fluorphenyl)-2(R)-(p-fluor-phenylmethyl)hexanoyl-(L)-Val-(L)-(p-$CH_3$O-Phe)-morpholin-4-ylamid mit 87 mg TBAF in 2 ml DMF in die Titelverbindung überführt: FAB-MS $(M+H)^+$=795.

Das Ausgangsmaterial wird wie folgt hergestellt:

**3) a) 5(S)-(Boc-amino)-4(S)-(tert-butyldimethylsilyloxy)-6-(p-fluorphenyl)-2(R)-(p-fluor-phenylmethyl)-hexanoyl-(L)-Val-(L)-(p-$CH_3$O-Phe)-morpholin-4-ylamid**

Analog Beispiel 9f) werden 80 mg 5(S)-(Boc-amino)-4(S)-(tert-butyldimethylsilyloxy)-6-(p-fluorphenyl)-2(R)-(p-fluor-phenylmethyl)hexansäure [Beispiel 21 B) 1) g)] und 56,7 mg H-(L)-Val-(L)(p-$CH_3$O-Phe)-morpholin-4-ylamid (Beispiel 10e) in 1,3 ml NMM/$CH_3CN$ 0,25 M mit 59,2 mg HBTU zur Titelverbindung umgesetzt: FAB-MS $(M+H)^+$=909.

**4) Boc-(p-F)Phe[C](p-F)Phe-(L)-Val-(L)-Cha-morpholin-4-ylamid**

Analog Beispiel 1) werden 230 mg 5(S)-(Boc-amino)-4(S)-(tert-butyldimethylsilyloxy)-6-(p-fluorphenyl)-2(R)-(p-fluor-phenylmethyl)-hexanoyl-(L)-Val-(L)-Cha-morpholin-4-ylamid mit 164 mg TBAF in 3,8 ml DMF in die Titelverbindung überführt: FAB-MS $(M+H)^+$=771.

Das Ausgangsmaterial wird wie folgt hergestellt:

**4) a) 5(S)-(Boc-amino)-4(S)-(tert-butyldimethylsilyloxy)-6-(p-fluorphenyl)-2(R)-(p-fluor-phenylmethyl)-hexanoyl-(L)-Val-(L)-Chamorpholin-4-ylamid**

Analog Beispiel 9f) werden 150 mg 5(S)-(Boc-amino)-4(S)-(tert-butyldimethylsilyloxy)-6-(p-fluorphenyl)-2(R)-(p-fluor-phenylmethyl)hexansäure [Beispiel 21 B) 1) g)] und 99,4 mg H-(L)-Val-(L)-Chamorpholin-4-ylamid (Beispiel 11a) in 2,4 ml NMM/$CH_3CN$ 0,25 M mit 111 mg HBTU zur Titelverbindung umgesetzt: FAB-MS $(M+H)^+$=885.

**5)** Boc-(p-F)Phe[C] (p-F)Phe-(L)-Ile-(L)-Phe-morpholin-4-ylamid

C)

1) Boc-(p-F)Phe[C] (p-CN)Phe-(L)-Val-(L)-Phe-morpholin-4-ylamid
2) Boc-(p-F)Phe[C](p-CN)Phe-(L)-Val-(L)-(p-F-Phe)-morpholin-4-ylamid
3) Boc-(p-F)Phe[C] (p-CN)Phe-(L)-Val-(L)-(p-$CH_3$O-Phe)-morpholin-4-ylamid
4) Boc-(p-F)Phe[C] (p-CN)Phe-(L)-Val-(L)-Cha-morpholin-4-ylamid
5) Boc-(p-F)Phe[C](p-CN)Phe-(L)-Ile-(L)-Phe-morpholin-4-ylamid

D) **1) Boc-Phe[C](p-F)Phe-(L)-Val-(L)-Phe-morpholin-4-ylamid**

Analog Beispiel 1) werden 0, 31 g 5 (S)-(Boc-amino)-4(S)-(tert-butyldimethylsilyloxy)-6-phenyl-2(R)-(p-fluor-phenylmethyl)-hexanoyl-(L)-Val-(L)-Phe-morpholin-4-ylamid mit 226 mg TBAF in 3,0 ml DMF zur Titelverbindung entschützt, die nach Säulenchromatographie ($SiO_2$, Essigsäureethylester) rein erhalten wird: DC $R_f$(B)=0,55; FAB-MS $(M+H)^+$=747.

Das Ausgangsmaterial wird wie folgt hergestellt:

**1) a) 2-Iodpropionsäureethylester**

Eine Suspension von 170 ml 2-Brompropionsäureethylester (Fluka; Buchs/Schweiz) und 950 g Natriumiodid in 1,8l Aceton wird 20 h bei 60 °C gerührt. Das Reaktionsgemisch wird filtriert, das Filtrat teilweise eingedampft, auf ca. 2,5l Ether gegossen, mit 1,011 %-iger Natriumthiosulfat-Lösung und schliesslich Sole gewaschen, mit Natriumsulfat getrocknet und eingedampft. Destillation (83°C; 20 mbar) liefert die reine Titelverbindung: MS $(M)^+$=228; $^1$H-NMR (200 MHz, $CDCl_3$): 4,17 (q, 7 Hz, 2 H), 3,34 und 2,97 (2t, 7 Hz, 2x 2H), 1,28 (t, 7 Hz, 3 H).

**1) b) 5(S)-[1(S)-(Boc-amino)-2-phenylethyl]-dihydrofuran-2-(3H)-on**

(analog A.E. DeCamp, A.T. Kawaguchi, R.P. Volante, and I. Shinkai, Tetrahedron Lett. 32, 1867 (1991)). Unter $N_2$-Atmosphäre gibt man zu einer Lösung von 17,4 g 2-Iodpropionsäureethylester in 130 ml Toluol 8,03 g Zn/Cu (Herstellung: R.D. Smith, H.E. Simmons, W.E. Parham, M.D. Bhavsar, Org. Synth., Coll. Vol 5, 855 (1973)) und 12,96 ml Dimethylacetamid und rührt kräftig während 1 h bei RT und 4 h bei 80°C nach (→ Zn-Homoenolatlösung). In einer zweiten Apparatur ($N_2$-Atmosphäre) wird eine Lösung von 5,58 ml (18,9 mMol) Tetraisopropyl-orthotitanat in 16,4 ml Toluol und 91,8 ml Methylenchlorid unter leichtem Kühlen mit 5,90 ml (53,8 mMol) Titan-tetrachlorid versetzt, 15 min bei RT gerührt (→ gelbe Lösung) und auf -40°C abgekühlt (→ teilweise Kristallisation des Trichlor-titan-isopropylats). Mittels Kanüle dekantiert man die auf RT abgekühlte Zn-Homoenolatlösung vom metallischen Festkörper und tropft sie zum Trichlor-titanisopropylat, wobei die Temperatur bei -40°C bis -30°C gehalten wird (→ tiefrote Lösung), erwärmt während 5 min auf -25°C und kühlt wiederum auf -40°C ab. Anschliessend tropft man eine Lösung von 9,0 g N-Boc-phenylalaninal (Herstellung: D.J. Kempf, J. Org. Chem. 51, 3921 (1986)) in 32,8 ml Methylenchlorid zu und rührt 15 h bei ca. -20°C und schliesslich 1 h bei 0°C nach. Das Reaktionsgemisch wird auf 0,5 kg Eiswasser und 0,5l Ether gegossen und 10 min kräftig gerührt. Man trennt die wässrige Phase ab, extrahiert sie mit 2 Portionen Ether; wäscht die organischen Phasen mit 2 Portionen Wasser, gesättigter Natriumhydrogencarbonat-Lösung und Sole, trocknet sie mit Natriumsulfat und dampft sie ein. Man erhält als Zwischenprodukt kristallinen 5(S)-(Boc-amino)-4(S)-hydroxy-6-phenyl-hexansäureethylester. Dieses Zwischenprodukt wird in 295 ml Toluol und 9 ml Essigsäure während 2,5 h auf 80°C erhitzt. Man versetzt das Reaktionsgemisch mit 0,5l Wasser, trennt die wässrige Phase ab, extrahiert sie mit 2 Portionen Ether, wäscht die org. Phasen mit gesättigter Natriumhydrogencarbonat-Lösung, Wasser und Sole und trocknet sie mit Natriumsulfat. Teilweises Eindampfen der organischen Phasen und Versetzen mit Hexan liefert die kristalline Titelverbindung, die laut Analytik ca. 10 % des (4R)-Epimeren (DC $R_f$(E)=0,08) enthält. Säulenchromatographie ($SiO_2$, Hexan/Essigsäureethylester 2:1) liefert die reine Titelverbindung: DC $R_f$(E)=0,14; $[\alpha]^D$=17,7° (c=1; Ethanol).

**1) c) 5(S)-[1(S)-(Boc-amino)-2-phenylethyl]-3(R)-(p-fluor-phenylmethyl)-dihydrofuran-2-(3H)-on**

(analog A.K. Ghosh, S.P. McKee, and W.J. Thompson, J. Org. Chem. 56, 6500 (1991)). Unter $N_2$-Atmosphäre wird eine Lösung von 300 mg (0,982 mMol) 5(S)-[1(S)-(Boc-amino)-2-phenylethyl]-dihydrofuran-2-(3H)-on in 6 ml THF bei -75°C mit 1,92 ml Lithiumbis(trimethylsilyl)amid 1 M in THF (Aldrich) versetzt und 15 min bei dieser Temperatur nachgerührt. Dann tropft man 132 µl (1,077 mMol) p-Fluorbenzylbromid (Fluka; Buchs/Schweiz) zu und rührt während 30 min bei -50°C aus. Nach erneutem Abkühlen auf -75°C werden 0,3 ml Propionsäure und dann 0,3 ml Wasser zugesetzt. Man wärmt auf 0°C auf, verdünnt mit Essigsäureethylester, wäscht mit 10 %-iger Zitronensäure-Lösung, gesättigter Natriumbicarbonat-Lösung und Sole, trocknet über Natriumsulfat und dampft ein. Säulenchromatographie ($SiO_2$, Hexan/Essigsäureethylester 4:1) liefert die reine Titelverbindung: DC $R_f$(D)=0,54; FAB-MS $(M+H)^+$=414.

**1) d) 5(S)-(Boc-amino)-4(S)-hydroxy-6-phenyl-2(R)-(p-fluor-phenylmethyl)-hexansäure**

Analog Beispiel 1i) werden 1,46 g 5(S)-[1(S)-(Boc-amino)-2-phenylethyl]-3(R)-(p-fluor-phenylmethyl)-dihydrofuran-2-(3H)-on in 57 ml Dimethoxyethan und 29 ml Wasser mit 14,1 ml 1 M LithiumhydroxidLösung zur Titelverbindung hydrolysiert, die ohne weitere Reinigung weiterverwendet wird.

**1) e) 5(S)-(Boc-amino)-4(S)-(tert-butyldimethylsilyloxy)-6-phenyl-2(R)-(p-fluor-phenylmethyl)-hexansäure**

Analog Beispiel 1j) werden 0,9 g 5(S)-(Boc-amino)-4(S)-hydroxy)-6-phenyl-2(R)-(p-fluor-phenylmethyl)-hexansäure in 4 ml DMF mit 1,49 g tert-Butyldimethylchlorsilan und 1,2 g Imidazol silyliert. Hydrolyse der Silylesterfunktion mit 1,9 g Kaliumcarbonat in 50 ml Methanol/THF/Wasser 3:1:1 liefert nach Ansäuern mit Zitronensäure-Lösung und Extraktion mit Essigsäureethylester die Titelverbindung: DC $R_f$(D)=0,2.

**1) f) 5(S)-(Boc-amino)-4(S)-(tert-butyldimethylsilyloxy)-6-phenyl-2(R)(p-fluor-phenylmethyl)-hexanoyl-(L)-Val-(L)-Phe-morpholin-4-ylamid**

Analog Beispiel 9f) werden 200 mg 5(S)-(Boc-amino)-4(S)-(tert-butyldimethylsilyloxy)-6-phenyl-2(R)-(p-fluor-phenylmethyl)-hexansäure und 134 mg H-(L)-Val-(L)-Phe-morpholin-4-ylamid (Beispiel 1o) in 3,6 ml NMM/$CH_3CN$ 0,25 M mit 153 mg HBTU umgesetzt. Nach Kristallisation aus Hexan erhält man die reine Titelverbindung: DC $R_f$(A)=0,25.

**2)** Boc-Phe[C](p-F)Phe-(L)-Val-(L)-(p-F-Phe)-morpholin-4-ylamid

**3)** Boc-Phe[C](p-F)Phe-(L)-Val-(L)-(p-$CH_3O$-Phe)-morpholin-4-ylamid

**4) Boc-Phe[C](p-F)Phe-(L)-Val-(L)-Cha-morpholin-4-ylamid**

Analog Beispiel 1) werden 120 mg 5(S)-(Boc-amino)-4(S)-(tert-butyldimethylsilyloxy)-6-phenyl-2(R)-(p-fluor-phenylmethyl)hexanoyl-(L)-Val-(L)-Cha-morpholin-4-ylamid mit 87 mg TBAF in 1,4 ml DMF zur Titelverbindung entschützt: DC $R_f$(B)=0,61; FAB-MS $(M+H)^+$=753.

Das Ausgangsmaterial wird wie folgt hergestellt:

**4) a)** **5(S)-(Boc-amino)-4(S)-(tert-butyldimethylsilyloxy)-6-phenyl-2(R)-(p-fluor-phenylmethyl)-hexanoyl-(L)-Val-(L)-Cha-morpholin-4-ylamid**

Analog Beispiel 9f) werden 100 mg 5(S)-(Boc-amino)-4(S)-(tert-butyldimethylsilyloxy)-6-phenyl-2(R)-(p-fluor-phenylmethyl)-hexansäure [Beispiel 21 D) 1) e)] und 68 mg H-(L)-Val-(L)-Cha-morpholin-4-ylamid (Beispiel 11a) in 1,8 ml NMM/$CH_3CN$ 0,25 M mit 76,4 mg HBTU umgesetzt: DC $R_f$(A)=0,50.

**5)** Boc-Phe[C](p-F)Phe-(L)-Ile-(L)-Phe-morpholin-4-ylamid

E) **1) Boc-Phe[C](p-CN)Phe-(L)-Val-(L)-Phe-morpholin-4-ylamid** Analog Beispiel 1) werden 60 mg 5(S)-(Boc-amino)-4(S)-(tert-butyldimethylsilyloxy)-6-phenyl-2(R)-(p-cyano-phenylmethyl)-hexanoyl(L)-Val-(L)-Phe-morpholin-4-ylamid mit 64,3 mg TBAF in 1,0 ml DMF zur Titelverbindung entschützt. Säulenchromatographie ($SiO_2$, Essigsäureethylester) liefert die reine Titelverbindung: DC $R_f$(B)=0,26; FAB-MS $(M+H)^+$=754.

Das Ausgangsmaterial wird wie folgt hergestellt:

**1) a)** **5(S)-[1(S)-(Boc-amino)-2-phenylethyl]-3(R)-(p-cyano-phenylmethyl)-dihydrofluran-2-(3H)-on**

Analog Beispiel 21 D) 1) c) werden 1,5 g 5(S)-[1(S)-(Boc-amino)2-phenylethyl]-dihydrofuran-2-(3H)-on [Beispiel 21 D) 1) b)] gelöst in 32 ml THF mit 9,8 ml Lithiumbis(trimethylsilyl)amid 1 M in THF deprotoniert und mit 1,0 g 4-Brommethylbenzonitril (Fluka; Buchs/Schweiz) gelöst in 3 ml THF alkyliert. Eine Säulenchromatographie ($SiO_2$, Hexan/Essigsäureethylester 1:1) liefert die reine Titelverbindung: DC $R_f$(D)=0,33.

**1) b)** **5(S)-(Boc-amino)-4(S)-hydroxy)-6-phenyl-2(R)-(p-cyano-phenylmethyl)-hexansäure**

Analog Beispiel 1i) werden 0,50 g 5(S)-[1(S)-(Boc-amino)-2-phenylethyl]-3(R)-(p-cyano-phenylmethyl)-dihydrofuran2-(3H)-on in 19 ml Dimethoxyethan und 10 ml Wasser mit 4,8 ml 1 M Lithiumhydroxid-Lösung zur Titelverbindung hydrolysiert: DC $R_f$(F)=0,3.

**1) c)** **5(S)-(Boc-amino)-4(S)-(tert-butyldimethylsilyloxy)-6-phenyl-2(R)-(p-cyano-phenylmethyl)-hexansäure**

Analog Beispiel 1j) werden 0,62 g 5(S)-(Boc-amino)-4(S)-hydroxy)-6-phenyl-2(R)-(p-cyano-phenylmethyl)-hexansäure in 6,2 ml DMF mit 0,98 g tert-Butyldimethylchlorsilan und 0,79 g Imidazol silyliert. Hydrolyse der Silylesterfunktion mit 1,2 g Kaliumcarbonat in 31 ml Methanol/THF/Wasser 3:1:1 liefert nach Ansäuem mit Zitronensäure-Lösung und Extraktion mit Essigsäureethylester die Titelverbindung: DC $R_f$(D)=0,29;
FAB-MS $(M+H)^+$=553.

**1) d)** **5(S)-(Boc-amino)-4(S)-(tert-butyldimethylsilyloxy)-6-phenyl-2(R)-(p-cyano-phenylmethyl)-hexanoyl-(L)-Val-(L)-Phe-morpholin-4-ylamid**

Analog Beispiel 9f) werden 72 mg 5 (S)-(Boc-amino)-4(S)-(tert-butyldimethylsilyloxy)-6-phenyl-2(R)-(p-cyano-phenylmethyl)-hexansäure und 43,3 mg H-(L)-Val-(L)-Phe-morpholin-4-ylamid (Beispiel 1o) in 1,14 ml NMM/$CH_3CN$ 0,25 M mit 50 mg HBTU zur Titelverbindung umgesetzt: DC $R_f$(A)=0, 19.

**2)** Boc-Phe[C](p-CN)Phe-(L)-Val-(L)-(p-F-Phe)-morpholin-4-ylamid

**3)** Boc-Phe[C](p-CN)Phe-(L)-Val-(L)-(p-$CH_3O$-Phe)-morpholin-4-ylamid

**4)** Boc-Phe[C](p-CN)Phe-(L)-Val-(L)-Cha-morpholin-4-ylamid

**5)** **Boc-Phe[C](p-CN)Phe-(L)-Ile-(L)-Phe-morpholin-4-ylamid**

Analog Beispiel 1) werden 510 mg 5(S)-(Boc-amino)-4(S)-(tert-butyldimethylsilyloxy)-6-phenyl-2(R)-(p-cyano-phenylmethyl)hexanoyl-(L)-Ile- (L)-Phe-morpholin-4-ylamid mit 362,3 mg TBAF in 10 ml DMF desilyliert. Das Reaktionsgemisch giesst man auf Eiswasser, extrahiert mit 3 Portionen Methylenchlorid, wäscht die organischen Phasen mit gesättigter Natriumbicarbonat-Lösung, Wasser und Sole nach, trocknet sie mit Natriumsulfat und dampft sie ein. Säulenchromatographie ($SiO_2$, Essigsäureethylester) liefert die reine Titelverbindung: DC $R_f$(B)=0,51; FAB-MS $(M+H)^+$=768.

Das Ausgangsmaterial wird wie folgt hergestellt:

**5) a)** **5(S)-(Boc-amino)-4(S)-(tert-butyldimethylsilyloxy)-6-phenyl-2(R)-(p-cyano-phenylmethyl)-hexanoyl-(L)-Ile-(L)-Phe-morpholin-4-ylamid**

Analog Beispiel 9f) werden 360 mg 5 (S)-(Boc-amino)-4(S)-(tert-butyldimethylsilyloxy)-6-phenyl-2(R)-(p-cyano-phenylmethyl)-hexansäure [Beispiel 21 E) 1) d)] und 253 mg H-(L)-Ile-(L)-Phe-morpholin-4-ylamid (Beispiel 16b) in 6,36 ml NMM/$CH_3CN$ 0,25 M mit 276 mg HBTU umgesetzt. Verteilen des Eindampfrückstands zwischen Methylenchlorid, 10 % Zitronensäure-Lösung, Wasser, gesättigter Natriumbicarbonat-Lösung, Wasser und Sole, Trocknen der organischen Phasen mit Natriumsulfat und Eindampfen liefert die Titelverbindung: DC $R_f$(D)=0,07.

F)

**1)** Boc-Phe[C](p-$CH_3O$)Phe-(L)-Val-(L)-Phe-morpholin-4-ylamid
**2)** Boc-Phe[C](p-$CH_3O$)Phe-(L)-Val-(L)-(p-F-Phe)-morpholin-4-ylamid
**3)** Boc-Phe[C](p-$CH_3O$)Phe-(L)-Val-(L)-(p-$CH_3O$-Phe)-morpholin-4-ylamid
**4)** Boc-Phe[C](p-$CH_3O$)Phe-(L)-Val-(L)-Cha-morpholin-4-ylamid
**5)** Boc-Phe[C](p-$CH_3O$)Phe-(L)-Ile-(L)-Phe-morpholin-4-ylamid

G)

**1)** Boc-Phe[C](p-$CF_3$)Phe-(L)-Val-(L)-Phe-morpholin-4-ylamid
**2)** Boc-Phe[C](p-$CF_3$)Phe-(L)-Val-(L)-(p-F-Phe)-morpholin-4-ylamid
**3)** Boc-Phe[C](p-$CF_3$)Phe-(L)-Val-(L)-(p-$CH_3O$-Phe)-morpholin-4-ylamid
**4)** Boc-Phe[C](p-$CF_3$)Phe-(L)-Val-(L)-Cha-morpholin-4-ylamid
**5)** Boc-Phe[C](p-$CF_3$)Phe-(L)-Ile-(L)-Phe-morpholin-4-ylamid

H)

**1)** Boc-Cha[C] (p-CN)Phe-(L)-Val-(L)-(p-F-Phe)-morpholin-4-ylamid
**2)** Boc-Cha[C](p-CN)Phe-(L)-Val-(L)-(p-$CH_3O$-Phe)-morpholin-4-ylamid
**3)** Boc-Cha[C](p-CN)Phe-(L)-Val-(L)-Cha-morpholin-4-ylamid
**4)** Boc-Cha[C](p-CN)Phe-(L)-Ile-(L)-Phe-morpholin-4-ylamid

I)

**1)** Boc-Cha[C](p-$CH_3O$)Phe-(L)-Val-(L)-Phe-morpholin-4-ylamid
**2)** Boc-Cha[C] (p-$CH_3O$)Phe-(L)-Val-(L)-(p-F-Phe)-morpholin-4-ylamid
**3)** Boc-Cha[C](p-$CH_3O$)Phe-(L)-Val-(L)-(p-$CH_3O$-Phe)-morpholin-4-ylamid
**4)** Boc-Cha[C](p-$CH_3O$)Phe-(L)-Val-(L)-Cha-morpholin-4-ylamid
**5)** Boc-Cha[C] (p-$CH_3O$)Phe-(L)-Ile-(L)-Phe-morpholin-4-ylamid

J)

**1)** Boc-Cha[C] (p-$CF_3$)Phe-(L)-Val-(L)-Phe-morpholin-4-ylamid
**2)** Boc-Cha[C] (p-$CF_3$)Phe-(L)-Val-(L)-(p-F-Phe)-morpholin-4-ylamid
**3)** Boc-Cha[C](p-$CF_3$)Phe-(L)-Val-(L)-(p-$CH_3O$-Phe)-morpholin- 4-ylamid
**4)** Boc-Cha[C](p-$CF_3$)Phe-(L)-Val-(L)-Cha-morpholin-4-ylamid
5) Boc-Cha[C] (p-$CF_3$)Phe-(L)-Ile-(L)-Phe-morpholin-4-ylamid

**Beispiel 22:** Analog einem der vorgenannten Beispiele, oder auf die jeweils im Detail angegebene Weise, werden durch Wahl entsprechender Ausgangsverbindungen hergestellt:

**A)** Boc-(L)-Val-Phe[C]Phe-(L)-Val-(L)-Phe-morpholin-4-ylamid;
**B)** H-(L)-Val-Phe[C]Phe-(L)-Val-(L)-Phe-morpholin-4-ylamid;
**C) Boc-Cha[C](p-F)Phe-(L)-Ile-(L)-Phe-morpholin-4-ylamid**

Analog Beispiel 1) werden 0, 15 g 5(S)-(Boc-amino)-4(S)-(tert-butyldimethylsilyl-oxy)-6-cyclohexyl-2(R)-(p-fluor-phenylmethyl)-hexanoyl-(L)-Ile-(L)-Phe-morpholin-4-ylamid mit 117 mg TBAF in 1,7 ml DMF in die Titelverbindung überführt: DC $R_f$(I)=0,18; FAB-MS $(M+H)^+$=767.

Das Ausgangsmaterial wird wie folgt hergestellt:

**C) a) 5(S)-(Boc-amino)-4(S)-(tert-butyldimethylsilyloxy)-6-cyclohexyl-2(R)-(p-fluor-phenylmethyl)-hexanoyl-(L)-Ile-(L)-Phe-morpholin-4-ylamid**

Analog Beispiel 9f) werden 102 mg 5(S)-(Boc-amino)-4(S)-(tert-butyldimethylsilyloxy)6-cyclohexyl-2(R)-(p-fluor-phenylmethyl)-hexansäure (Beispiel 1j) und 70,8 mg H-(L)-Ile-(L)-Phe-morpholin-4-ylamid (Beispiel 16b) in 1,77 ml NMM/$CH_3CN$ 0,25 M mit 77,4 mg HBTU zur Titelverbindung umgesetzt: DC $R_f$(A)=0,17; FAB-MS $(M+H)^+$=881.

**D)** Boc-Cha[C](p-F)Phe-(L)-Val-(L)-Cha-morpholin-4-ylamid;
**E)** Boc-Phe[C]Phe-(L)-Val-(D)-Phe-morpholin-4-ylamid;
**F)** Boc-Phe[C]Phe-(L)-Val(red)-(L)-Phe-morpholin-4-ylamid;
**G)** Isobutyloxycarbonyl-Phe[C]Phe-(L)-Val-(L)-Phe-morpholin-4-ylamid;
**H)** Boc-Cha[C](p-CN)Phe-(L)-Val-(L)-Phe-thiomorpholin-4-ylamid;
**I) Boc-Cha(Cl(p-F)Phe-(L)-Val-(L)-Phe-thiomorpholin-4-ylamid**

Analog Beispiel 1) werden 0,16g 5(S)-(Boc-amino)-4(S)-(tert-butyldimethylsilyl-oxy)-6-cyclohexyl-2(R)-(p-fluor-phenylmethyl)-hexanoyl-(L)-Val-(L)-Phe-thiomorpholin-4-ylamid mit 114 mg TBAF in 1,8 ml DMF in die Titelverbindung überführt: DC $R_f$(I)=0,38; FAB-MS $(M+H)^+$=769.

Das Ausgangsmaterial wird wie folgt hergestellt:

**I) a) 5(S)-(Boc-amino)-4(S)-(tert-butyldimethylsilyloxy)-6-cyclohexyl-2(R)-(p-fluor-phenylmethyl)-hexanoyl-(L)-Val-(L)-Phe-thiomorpholin-4-ylamid**

Analog Beispiel 9f) werden 100 mg 5(S)-(Boc-amino)-4(S)-(tert-butyldimethylsilyloxy)-6-cyclohexyl-2(R)-(p-fluor-phenylmethyl)-hexansäure (Beispiel 1j) und 70 mg H-(L)-Val-(L)-Phe-thiomorpholin-4-ylamid (Beispiel 20b) in 1,7 ml NMM/$CH_3CN$ 0,25 M mit 76 mg HBTU umgesetzt. Nach 18 h bei RT wir ein Teil der Titelverbindung direkt durch Filtration aus dem Reaktionsgemisch gewonnen. Weitere Titelverbindung erhält man durch Verteilen des Eindampfrückstandes des Filtrats zwischen 3 Portionen Essigsäureethylester, Wasser, 2 Portionen 10 % Zitronensäure-Lösung, Wasser, 2 Portionen gesättigter Natriumbicarbonat-Lösung, Wasser und Sole, Trocknen der organischen Phasen mit Natriumsulfat und Eindampfen: DC $R_f(A)=0,55$; FAB-MS $(M+H)^+=883$.

**J)** Die Verbindungen aus Beispiel 22 A) bis 22 G), worin anstelle von -morpholin-4-ylamid der Rest -thiomorpholin-4-ylamid steht.

## Beispiel 23

Analog einem der vorstehenden Verfahren werden hergestellt:
a) Boc-Phe[C]Phe-(L)-Val-(L)-Phe-(4-methylpiperazin-1-yl)amid
b) Boc-Phe[C]Phe-(L)-Val-(L)-Phe-piperidin-1-yl-amid
c) Boc-Phe[C]Phe-(L)-Val-(L)-Phe-pyrrolidin-1-ylamid

## Beispiel 24: Gelatine-Lösung

Eine sterilfiltrierte wässrige Lösung eines der in den vorausgehenden oder nachfolgenden Beispielen 1 bis 23 und 33 bis 41 genannten Wirkstoffe der Formel I, die zusätzlich 20 % Cyclodextrin enthält, und eine sterile, mit Phenol konservierte Gelatinelösung werden unter Erwärmen unter aseptischen Bedingungen so vermischt, dass 1,0 ml Lösung folgender Zusammensetzung resultiert:

| | |
|---|---|
| Wirkstoff | 3 mg |
| Gelatine | 150,0 mg |
| Phenol | 4,7 mg |
| dest. Wasser mit 20 % Cyclodextrinen | 1,0 ml |

## Beispiel 25: Sterile Trockensubstanz zur Injektion

Man löst 5 mg einer der in den vorausgehenden oder nachfolgenden Beispielen 1 bis 23 und 33 bis 41 genannten Verbindungen der Formel I als Wirkstoff in 1 ml einer wässrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

## Beispiel 26: Nasenspray

In einer Mischung von 3,5 ml Myglyol 812® und 0,08 g Benzylalkohol werden 500 mg fein gemahlenes (<5,0 gm) Pulver einer der in den vorausgehenden oder nachfolgenden Beispielen 1 bis 23 und 33 bis 41 genannten Verbindungen der Formel I als Wirkstoff suspendiert. Diese Suspension wird in einen Behälter mit Dosierventil eingefüllt. Es werden 5,0 g Freon 12® unter Druck durch das Ventil in einen Behälter abgefüllt. Durch Schütteln wird das "Freon" in der Myglyol-Benzylalkoholmischung gelöst. Dieser Spraybehälter enthält ca. 100 Einzeldosen, die einzeln appliziert werden können.

## Beispiel 27: Lacktabletten

Für die Herstellung von 10 000 Tabletten enthaltend je 100 mg Wirkstoff werden folgende Bestandteile verarbeitet:
Wirkstoff
1000 g

Maisstärke
680 g
Kolloidale Kieselsäure
200 g
Magnesiumstearat
20 g
Stearinsäure
50 g
Natriumcarboxymethylstärke
250 g
Wasser
quantum satis

Ein Gemisch von einer der in den vorausgehenden oder nachfolgenden Beispielen 1 bis 23 und 33 bis 41 genannten Verbindungen der Formel I als Wirkstoff, 50 g Maisstärke und kolloidaler Kieselsäure wird mit Stärkekleister aus 250 g Maisstärke und 2,2 kg entmineralisiertem Wasser zu einer feuchten Masse verarbeitet. Diese wird durch ein Sieb von 3 mm Maschenweite getrieben und bei 45 ° während 30 Min. im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

**Beispiel 28: Oral verabreichbare Dispersion 1**

625 mg einer der in den vorausgehenden oder nachfolgenden Beispielen 1 bis 23 und 33 bis 41 genannten Verbindungen der Formel 1, z. B. Boc-Phe[C]Phe-(L)-Val-(L)-Phe-morpholin-4-ylamid, als Wirkstoff und 625 mg POPC (1-Palmitoyl-2-oleoyl-phosphatidylcholin = 1 -Hexadecanoyl-2-(9-cis-octadecenoyl)-3-sn-phosphatidylcholin) werden in 25 ml Ethanol gelöst. Die Lösung wird mit der zehnfachen Menge Wasser verdünnt. Hierzu wird die ethanolische Lösung bei Raumtemperatur mit einer Rate von 10 ml/min zu der vorgelegten Menge Wasser zugetropft. Das Ethanol wird aus der Mischung durch tangentiale Dialyse ("Cross Flow Filtration") gegen 1750 ml Wasser (System: Minitan®, 700 $cm^2$- Polyethersulphon-Membran mit einer Ausschlussgrenze von 100 kD, der Firma Millipore (USA)) entfernt. Die Mischung wird unter Verwendung desselben Systems auf 15 mg Wirkstoff konzentriert durch Ultrafiltration. Nach Zugabe von 1,24 mg/ml Zitronensäure und 1,24 mg/ml Dinatriumhydrogenphosphat.2 $H_2O$ zur Einstellung auf pH 4,2 und von 1 mg/ml Sorbinsäure als antimikrobiellem Konservierungsmittel wird die Dispersion erneut auf 15 mg/ml aufkonzentriert und in Gläschen, beispielsweise mit 20 ml Inhalt, abgefüllt. Die Dispersionspartikel haben einen Durchmesser von 0,1 - 2 μm. Sie sind bei +2 bis 8 °C mindestens ein halbes Jahr lang stabil und geegnet zur oralen Verabreichung.

**Beispiel 29: Oral verabreichbare Dispersion 2**

Die Herstellung erfolgt analog Beispiel 28, jedoch unter Verwendung von 25 mg Wirkstoff und 50 mg POPC zur Herstellung der ethanolischen Lösung.

**Beispiel 30: Oral verabreichbare Dispersion 3**

Die Herstellung erfolgt analog Beispiel 28, jedoch unter Verwendung von 25 mg Wirkstoff und 125 mg POPC zur Herstellung der ethanolischen Lösung.

**Beispiel 31: Oral verabreichbare Dispersion 4**

Die Herstellung erfolgt analog Beispiel 28, jedoch unter Verwendung von 50 mg Wirkstoff und 50 mg POPC zur Herstellung der ethanolischen Lösung.

**Beispiel 32: Oral verabreichbare Dispersion 5**

Die Herstellung erfolgt analog einem der Beispiele 28 bis 31, jedoch unter Verwendung von Wirkstoff und Phosphatidylcholin aus Soja oder Phosphatidylcholin aus Eigelb (70 - 100 % rein) anstelle von POPC zur Herstellung der ethanolischen Lösung. Gewünschtenfalls wird ein Antioxidans, wie Ascorbinsäure, in einer Konzentration von 5 mg/ml zugegeben.

**Beispiel 33: Boc-(p-F)Phe[C](p-$CF_3$)Phe-(L)-Val-(L)-Phe-morpholin-4-ylamid**

Analog Beispiel 1) werden 265 mg 5(S)-(Boc-amino)-4(S)-(tert-butyldimethylsilyloxy)-6-(p-fluor-phenyl)-2(R)-(p-trifluormethyl-phenylmethyl)-hexanoyl-(L)-Val-(L)-Phemorpholin-4-ylamid mit 180 mg TBAF in 4,2 ml DMF in die Titelverbindung überführt: DC $R_f$(I)= 0,3; FAB-MS $(M+H)^+$=815.

Das Ausgangsmaterial wird wie folgt hergestellt:

**33a) 5(S)-[1(S)-(Boc-amino)-2-(p-fluor-phenyl)ethyl]-3(R)-(p-trifluormethyl-phenylmethyl)-dihydrofuran-2-(3H)-on**

Analog Beispiel 21 D) 1) c) werden 1,0 g 5(S)-[1(S)-(Boc-amino)-2-(p-fluor-phenyl)ethyl]-dihydrofuran-2-(3H)-on gelöst in 6,3 ml THF mit 6,05 ml Lithiumbis(tri-methylsilyl)amid 1 M in THF deprotoniert und mit 0,739 g p-Trifluormethyl-benzylbromid (Fluka; Buchs/Schweiz) bei -75°C alkyliert (40 min). Eine Säulenchromatographie ($SiO_2$, Hexan/Essigsäureethylester 2:1) liefert die reine Titelverbindung: DC $R_f$(D)=0,48; FAB-MS $(M+H)^+$=482.

**33b) 5(S)-(Boc-amino)-4(S)-hydroxy)-6-(p-fluor-phenyl)-2(R)-(p-trifluormethyl-phenylmethyl)-hexansäure**

Analog Beispiel 1i) werden 1,05 g 5(S)-[1(S)-(Boc-amino)-2-(p-fluor-phenyl)ethyl]-3(R)-(p-trifluormethyl-phenylmethyl)-dihydrofuran-2-(3H)-on in 35,5 ml Dimethoxyethan und 17,9 ml Wasser mit 8,7 ml 1 M Lithiumhydroxid-Lösung zur Titelverbindung hydrolysiert, die direkt weiterverwendet wird.

**33c) 5(S)-(Boc-amino)-4(S)-(tert-butyldimethylsilyloxy)-6-(p-fluor-phenyl)-2(R)-(p-trifluormethyl-phenylmethyl)-hexansäure**

Analog Beispiel 1j) werden 1,06 g 5(S)-(Boc-amino)-4(S)-hydroxy)-6-(p-fluorphenyl)-2(R)-(p-trifluormethyl-phenylmethyl)-hexansäure in 2,3 ml DMF mit 1,47 g tert-Butyldimethylchlorsilan und 1, 18 g Imidazol silyliert. Hydrolyse der Silylesterfunktion mit 1,76 g Kaliumcarbonat in 50 ml Methanol/THF/Wasser 3:1:1 liefert nach Säulenchromatographie ($SiO_2$, Hexan/Essigsäureethylester 2:1) die Titelverbindung: DC $R_f$(D)=0,15; $^1$H-NMR (200 MHz, $CD_3OD$): 7,59 und 7,39 (2d, 8 Hz, je 2 H), 7,19 und 6,98 (2m, je 2 H), 6,47 und 5,90 (d, ca. 9 Hz, zusammen 1 H), 3,9-3,65 (m, 2 H), 3,15-2,8 (m, 4 H), 2,53-2,37, 2,07-1,9 und 1,6-1,4 (3m, je 1 H), 1,37-1,22 (m, 9 H), 0,94 (s, 9 H), 0,2-0, 1 (m, 6 H).

**33d) 5(S)-(Boc-amino)-4(S)-(tert-butyldimethylsilyloxy)-6-(p-fluor-phenyl)-2(R)-(p-trifluormethyl-phenylmethyl)-hexanoyl-(L)-Val-(L)-Phe-morpholin-4-ylamid**

Analog Beispiel 9f) werden 180 mg 5 (S)-(Boc-amino)-4(S)-(tert-butyldimethylsilyloxy)6-(p-fluor-phenyl)-2(R)-(p-trifluormethyl-phenylmethyl)-hexansäure und 107,5 mg H-(L)-Val-(L)-Phe-morpholin-4-ylamid (Beispiel 1o) in 2,8 ml NMM/$CH_3CN$ 0,25 M mit 122 mg HBTU umgesetzt: FAB-MS $(M+H)^+$=929.

**Beispiel 34: Boc-(p-F)Phe[C](p-$CF_3$)Phe-(L)-Val-(L)-(p-F-Phe)-morpholin-4-ylamid**

Analog Beispiel 1) werden 270 mg 5(S)-(Boc-amino)-4(S)-(tert-butyldimethylsilyloxy)-6-(p-fluor-phenyl)-2(R)-(p-trifluormethyl-phenylmethyl)-hexanoyl-(L)-Val-(L)-(p-F-Phe)-morpholin-4-ylamid mit 180 mg TBAF in 4,2 ml DMF in die Titelverbindung überführt: DC $R_f$(I)=0,2; FAB-MS $(M+H)^+$=833.

Das Ausgangsmaterial wird wie folgt hergestellt:

**34a) 5(S)-(Boc-amino)-4(S)-(tert-butyldimethylsilyloxy)-6-(p-fluor-phenyl)-2(R)-(p-trifluormethyl-phenylmethyl)-hexanoyl-(L)-Val-(L)-(p-F-Phe)-morpholin-4-ylamid**

Analog Beispiel 9f) werden 180 mg 5 (S)-(Boc-amino)-4(S)-(tert-butyldimethylsilyloxy)6-(p-fluor-phenyl)-2(R)-(p-trifluormethyl-phenylmethyl)-hexansäure (Beispiel 33c) und 113,2 mg H-(L)-Val-(L)-(p-F-Phe)-morpholin-4-ylamid (Beispiel 9e) in 2,8 ml NMM/$CH_2CN$ 0,25 M mit 122 mg HBTU umgesetzt: FAB-MS $(M+H)^+$=947.

**Beispiel 35: Boc-(p-F)Phe[C](p-$CF_3$)Phe-(L)-Val-(L)-(p-$CH_3O$-Phe)-morpholin-4-ylamid**

Analog Beispiel 1) werden 193 mg 5(S)-(Boc-amino)-4(S)-(tert-butyldimethylsilyloxy)-6-(p-fluor-phenyl)-2(R)-(p-trifluormethyl-phenylmethyl)-hexanoyl-(L)-Val-(L)-(p-$CH_3O$-Phe)-morpholin-4-ylamid mit 127 mg TBAF in 3 ml DMF in die Titelverbindung überführt: DC $R_f$(I)=0,47; FAB-MS $(M+H)^+$=845.

Das Ausgangsmaterial wird wie folgt hergestellt:

**35a) 5(S)-(Boc-amino)-4(S)-(tert-butyldimethylsilyloxy)-6-(p-fluor-phenyl)-2(R)-(p-trifluormethyl-phenylmethyl)-hexanoyl-(L)-Val-(L)-(p-$CH_3O$-Phe)-morpholin-4-ylamid**

Analog Beispiel 9f) werden 180 mg 5(S)-(Boc-amino)-4(S)-(tert-butyldimethylsilyloxy)-6-(p-fluor-phenyl)-2(R)-(p-fluormethyl-phenylmethyl)-hexansäure (Beispiel 33c) und 117,2 mg H-(L)-Val-(L)-(p-$CH_3O$-Phe)-morpholin-4-ylamid (Beispiel 10e) in 2,8 ml NMM/$CH_3CN$ 0,25 M mit 122 mg HBTU umgesetzt: FAB-MS $(M+H)^+$=959.

**Beispiel 36: Morpholinosulfonyl-(L)-Val-Phe[C]Phe-(L)-Val-(L)-Phe-morpholin-4-ylamid**

Analog Beispiel 5) werden 102 mg N-Morpholinosulfonyl-(L)-Val in 4 ml DMF mit 186 mg BOP, 57 mg HOBT und 0,09 ml NMM aktiviert und mit 200 mg H-Phe[C]Phe-(L)-Val-(L)-Phe-morpholin-4-ylamid (Beispiel 4) in 1 ml DMF zur Titelverbindung umgesetzt. Das Reaktionsgemisch wird auf Wasser gegossen, mit viel Essigsäureethylester (schlecht löslich) extrahiert und mit Wasser, gesättigter Natriumbicarbonat-Lösung, Wasser und Sole gewaschen. Verrühren des Rohproduktes in Ether liefert die reine Titelverbindung: FAB-MS $(M+H)^+$=877.

Das Ausgangsmaterial wird wie folgt hergestellt:

**36a) N-Chlorosulfonyl-morpholin**

Unter intensivem Kühlen gibt man zu 23,5 ml Morpholin bei ca. 0 °C 32,7 ml Sulfurylchlorid. Anschliessend erwärmt man die Suspension vorsichtig auf 60°C, wobei HCl-Entwicklung einsetzt. Nach 5 h bei 60 °C ist die HCl-Entwicklung beendet. Das erkaltete braune Reaktionsgemisch wird auf Eis gegossen, das sich dabei ausscheidende Öl mit Ether extrahiert, mit Wasser, 5 %-iger Natriumbicarbonat-Lösung und Wasser gewaschen und mit Natriumsulfat getrocknet. Destillation bei erhöhter Temperatur und vermindertem Druck (90°C; 1 mbar) der eingedampften organischen Phasen liefert die Titelverbindung: $^1$H-NMR (200 MHz, DMSO-$d_6$): 3,80 und 3,29 (2t, 5 Hz, je 4 H).

**36b) N-Morpholinosulfonyl-(L)-Val**

Zu 6,3 g N-Chlorosulfonyl-morpholin in 20 ml THF tropft man 2 g (L)-Valin gelöst in 50 ml 1 N NaOH und rührt 17 h bei RT aus. Die gelbe Emulsion wird mit 15 ml 1 N NaOH versetzt und mit Ether extrahiert. Die wässrige Phase säuert man mit 2 N HCl an und extrahiert sie mit Essigsäureethylester. Die Essigsäureethylester-Phase wird mit Natriumsulfat getrocknet und eingedampft. Kristallisation aus Ether liefert laut $^1$H-NMR-Spektrum ein Nebenprodukt. Die reine Titelverbindung wird aus dem Eindampfrückstand des Filtrats durch Kristallisation aus Hexan erhalten: DC $R_f$(F)=0,25.

**Beispiel 37: Morpholinosulfonyl-Phe[C]Phe-(L)-Val-(L)-Phe-morpholin-4-ylamid**

Eine Lösung von 200 mg H-Phe[C]Phe-(L)-Val-(L)-Phe-morpholin-4-ylamid (Beispiel 4) in 5 ml DMF wird mit 0, 132 ml Triethylamin und 71 mg N-Chlorosulfonyl-morpholin (Beispiel 36a) in 1 ml DMF versetzt. Da nach 2 h bei RT gemäss DC noch viel H-Phe[C]Phe-(L)-Val-(L)-Phe-morpholin-4-ylamid vorliegt, werden nochmals 71 mg N-Chlorosulfonyl-morpholin zugesetzt. Nach 18 h wird auf Wasser gegossen, mit 3 Portionen Essigsäureethylester extrahiert, mit gesättigter Natriumbicarbonat-Lösung, Wasser und Sole gewaschen, mit Natriumsulfat getrocknet und eingedampft. Eine Säulenchromatographie ($SiO_2$, Methylenchlorid/Methanol 9:1) liefert die reine Titelverbindung: DC $R_f$(F)=0,60; FAB-MS $(M+H)^+$=778.

**Beispiel 38: N-(N-(2-Pyridylmethyl)-N-methyl-aminocarbonyl)-(L)-Val-Phe[C]Phe(L)-Val-(L)-Phe-morpholin-4-ylamid**

Analog Beispiel 5) werden 152 mg N-(N-(2-pyridylmethyl)-N-methyl-aminocarbonyl)-(L)-valin (Herstellung siehe EP 402646 A1, 19. Dez. 1990) in 5 ml DMF mit 279 mg BOP, 85 mg HOBT und 0, 132 ml NMM aktiviert und mit 300 mg H-Phe[C]Phe-(L)-Val-(L)-Phe-morpholin-4-ylamid (Beispiel 4) zur Titelverbindung umgesetzt. Eine Säulenchromatographie ($SiO_2$, Essigsäureethylester/Aceton 9:1 → Aceton) liefert nach Digerieren in Ether die reine Titelverbindung: DC $R_f$(F)=0,28; FAB-MS $(M+H)^+$=876.

**Beispiel 39: 5(S)-(Boc-amino)-4(S)-(acetoxy)-6-phenyl-2(R)-phenylmethylhexanoyl-(L)-Val-(L)-Phe-morpholin-4-ylamid**

Zu 400 mg Boc-Phe[C]Phe-(L)-Val-(L)-Phe-morpholin-4-ylamid (Beispiel 2) in 8 ml THF gibt man 0, 114 ml Triethylamin, 1 mg Dimethylaminopyridin und 0,08 ml Acetanhydrid. Nach 30 min bei RT wird die farblose Lösung auf Wasser gegossen und mit 3 Portionen Essigsäureethylester extrahiert. Die organischen Phasen ergeben nach Waschen mit Wasser, gesättigter Natriumbicarbonat-Lösung, Wasser und Sole, Trocknen mit Natriumsulfat und Eindampfen die Titelverbindung, die nach Säulenchromatographie ($SiO_2$, Hexan/Essigsäureethylester 1:2) rein anfällt: DC $R_f$(B)=0,59; FAB-MS $(M+H)^+$=771.

**Beispiel 40:**

Auf analoge Weise, wie in einem der vorstehenden Beispiele beschrieben, werden die folgenden Verbindungen erhalten:

A) Boc-(p-$CF_3$)Phe[C]Phe-(L)-Val-(L)-Phe-morpholin-4-ylamid
B) Boc-(p-$CF_3$)Phe[C]Phe-(L)-Val-(L)-(p-F)Phe-morpholin-4-ylamid
C) Boc-(p-$CF_3$)Phe[C]Phe-(L)-Val-(L)-(p-$CH_3O$)Phe-morpholin-4-ylamid
D) Boc-(p-$CF_3$)Phe[C]Phe-(L)-Val-(L)-Cha-morpholin-4-ylamid
E) Boc-(p-$CF_3$)Phe[C]Phe-(L)-Ile-(L)-Phe-morpholin-4-ylamid
F) Boc-(p-$CF_3$)Phe[C] (p-F)Phe-(L)-Val-(L)-Phe-morpholin-4-ylamid
G) Boc-(p-$CF_3$)Phe[C](p-F)Phe-(L)-Val-(L)-(p-F)Phe-morpholin-4- ylamid
H) Boc-(p-$CF_3$)Phe[C] (p-F)Phe-(L)-Val-(L)-Cha-morpholin-4-ylamid
I) Boc-(p-$CF_3$)Phe[C] (p-F)Phe-(L)-Val-(L)-(p-$CH_3O$)Phe-morpholin-4-ylamid
J) Boc-(p-$CF_3$)Phe[C](p-F)Phe-(L)-Ile-(L)-Phe-morpholin-4-ylamid
K) Boc-(p-$CF_3$)Phe[C](p-$CF_3$)Phe-(L)-Val-(L)-Phe-morpholin-4-ylamid
L) Boc-(p-$CF_3$)Phe[C] (p-$CF_3$)Phe-(L)-Val-(L)-(p-F)Phe-morpholin- 4- ylamid
M) Boc-(p-$CF_3$)Phe[C](p-$CF_3$)Phe-(L)-Ile-(L)-Phe-morpholin-4-ylamid
N) Boc-(p-$CF_3$)Phe[C] (p-$CF_3$)Phe-(L)-Val-(L)-(p-$CH_3O$)Phe-morpholin-4-ylamid
O) Boc-(p-$CF_3$)Phe[C](p-$CF_3$)Phe-(L)-Val-(L)-Cha-morpholin-4-ylamid
P) Die Verbindungen gemäss den vorstehenden Beispielen A) bis 0), worin der Rest -morpholin-4-ylamid durch den Rest thiomorpholin-4-ylamid ersetzt ist.

Das Ausgangsmaterial wird wie folgt hergestellt:

**40a) N-Allylformamid**

Eine Lösung von 300 ml Allylamin in 1288 ml Ameisensäureethylester wird während 8 h auf 60°C erwärmt. Das Reaktionsgemisch wird am Rotationsverdampfer eingeengt und der Rückstand über eine Vigreux Kolonne destilliert (77°C; 1 mbar): $^1$H-NMR (200 MHz, $CDCl_3$): 8,2-7,95 (m, 1 H), 6,5-5,8 (sb, 1 H), 5,9-5,7 (m, 1 H), 5,3-5,05 (m, 2 H), 3,95-3,75 (m, 2 H).

**40b) Allylisocyanid**

(U. Schöllkopf, R. Jentsch, K. Madawinata und R. Harms, Liebigs Ann. Chem., (1976) 2105). Unter $N_2$-Atmosphäre werden 517 g Chinolin und 286 g p-Toluolsulfonsäurechlorid bei 90°C vorgelegt. Man legt ein Vakuum von 2-4 mbar an und tropft 85 g N-Allylformamid zu, wobei das entstehende Isocyanid bei einer Innentemperatur von 85-95°C kontinuierlich über eine Vigreux Kolonne in die Kühlfalle (Aceton/Trockeneis) abdestilliert. Nach beendeter Umsetzung wird das Destillat unverzüglich nochmals über eine Vigreux Kolonne destilliert ($N_2$-Atmosphäre Normaldruck; 100°C): $^1$H-NMR (200 MHz, $CDCl_3$): 5,9-5,7 (m, 1 H), 5,45 (d, 16 Hz, 1 H), 5,32 (d, 10 Hz, 1 H), 4,05 (m, 2 H); IR ($CH_2Cl_2$): 2150, 1650.

**40c) rac. 1-(p-Trifluormethyl-phenyl)-3-buten-2-amin**

Unter $N_2$-Atmosphäre werden 4,5 g Allylisocyanid in 100 ml THF/Ether/Pentan abs 4:1:1 gelöst und auf -100°C abgekühlt. Man tropft bei -100 bis -90°C 42 ml n-Butyllithium (1,6 M in Hexan) zu, wobei erst eine Gelbfärbung auftritt und sich kurz vor Ende der Zugabe ein Festkörper ausscheidet. Langsam lässt man das Reaktionsgemisch auf -70°C aufwärmen und kühlt dann wieder auf - 100°C ab. Bei - 100 bis -85°C wird eine Lösung von 16 g p-Trifluormethyl-benzylbromid (Fluka; Buchs/Schweiz) in 10 ml THF zugetropft und langsam auf RT aufgewärmt. Das Reaktionsgemisch wird am Rotationsverdampfer (80 mbar; 30 °C) eingedampft, der

Rückstand auf 150 ml Eiswasser gegossen und 3 mal mit Ether extrahiert. Die Ether-Phasen dampft man ein, versetzt den braunen Rückstand bei 0°C mit 85 ml Methanol und 17 ml konz. Salzsäure und lässt über Nacht im Kühlschrank stehen. Das Gemisch wird am Rotationsverdampfer eingedampft und der Rückstand verteilt zwischen 2 x 150 ml 2 M Salzsäure und 2 x 200 ml Ether. Die vereinigten wässrigen Phasen stellt man unter Kühlen mit festem Natriumhydroxid alkalisch und extrahiert sie mit 3 Portionen Essigsäureethylester. Waschen der organischen Phasen mit Sole, Trocknen mit Natriumsulfat, Eindampfen und Destillation am Kugelrohr (0,1 mbar; 170 °C) liefert die reine Titelverbindung: $^1$H-NMR (200 MHz, $CDCl_3$): 7,56 und 7,32 (2d, 8 Hz, je 2 H), 5,96-5,78 (m, 1 H), 5, 19-5,02 (m, 2 H), 3,68-3,55 (m, 1 H), 2,87 und 2,71 (AB x d, $J_{ab}$= 13 Hz, J1=6 Hz, $J_2$= 8 Hz, 2 H), 1,4 (sb, 2 H).

Die weitere Umsetzung analog Beispiel 1 d) bis 1 k) und 1), 9 f) und 9, 10 f) und 10, 15 a) und 15 oder 16 c) und 16 führt zu den oben unter a) bis o) genannten Verbindungen.

**Beispiel 41:**

Analog einem der vorstehenden Beispiele werden hergestellt:

a) Boc-Phe[C]Phe-(L)-Val-(L)-Tyr-morpholin-4-ylamid;
b) Boc-Tyr[C]Phe-(L)-Val-(L)-Phe-morpholin-4-ylamid;
c) Boc-Tyr[C]Phe-(L)-Val-(L)-Tyr-morpholin-4-ylamid;
d) Boc-Phe[C]Tyr-(L)-Val-(L)-Phe-morpholin-4-ylamid;
e) Boc-Phe[C]Tyr-(L)-Val-(L)-Tyr-morpholin-4-ylamid;
f) Boc-Tyr[C]Tyr-(L)-Val-(L)-Phe-morpholin-4-ylamid;
g) Boc-Tyr[C]Tyr-(L)-Val-(L)-Tyr-morpholin-4-ylamid;
h) die Verbindungen gemäss den vorstehend genannten Beispielen a) bis h), worin der Rest -morpholin-4-ylamid durch den Rest thiomorpholin-4-ylamid ersetzt ist.

**Patentansprüche**

**1.** Verbindungen der Formel

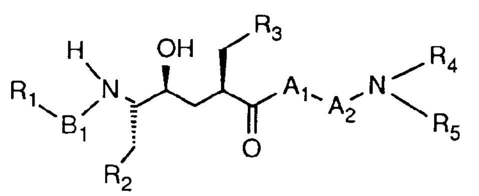

(I),

worin $R_1$ Wasserstoff, Niederalkoxycarbonyl, Heterocyclylcarbonyl, Benzyloxycarbonyl, welches unsubstituiert oder durch bis zu drei unabhängig voneinander aus Fluor, Haloniederalkyl, Niederalkanoyl, Sulfo, Niederalkylsulfonyl und Cyano ausgewählte Reste substituiert ist, Heterocyclyloxycarbonyl, worin Heterocyclyl über ein Kohlenstoffatom gebunden ist, einen der genannten Carbonylreste, worin die bindende Carbonylgruppe durch eine Thiocarbonylgruppe ersetzt ist, Heterocyclylsulfonyl, Niederalkylsulfonyl oder N-(Heterocyclylniederalkyl)-N-niederalkyl-aminocarbonyl bedeutet, $B_1$ eine Bindung oder ein bivalentes Radikal einer α-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl, wobei diese Reste unsubstituiert oder durch ein bis drei unabhängig aus Hydroxy, Niederalkoxy, Halo, Haloniederalkyl, Sulfo, Niederalkylsulfonyl, Cyano und Nitro ausgewählte Reste substituiert sind, bedeuten, $A_1$ eine Bindung zwischen -C=O und $A_2$ oder ein bivalentes Radikal einer α-Aminosäure bedeutet, welches N-terminal mit der Gruppe -C=O und C-terminal mit $A_2$ verbunden ist, $A_2$ ein bivalentes Radikal einer α-Aminosäure bedeutet, welches N-terminal mit $A_1$ und C-terminal mit der Gruppe $NR_4R_5$ verbunden ist, oder $A_1$ und $A_2$ zusammen ein bivalentes Radikal eines Dipeptides bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom unsubstituiertes oder substituiertes Thiomorpholino oder Morpholino bedeuten, oder Salze dieser Verbindungen, sofern salzbildende Gruppen vorliegen, oder hydroxygeschützte Derivate dieser Verbindungen oder deren Salze.

2. Verbindungen der Formel I gemäss Anspruch 1, worin $R_1$ Wasserstoff, tert-Butoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, mit bis zu drei unabhängig voneinander aus Fluor, Haloniederalkyl, Niederalkanoyl, Sulfo, Niederalkylsulfonyl und Cyano ausgewählten Resten substituiertes Benzyloxycarbonyl, Heterocyclyloxycarbonyl, worin Heterocyclyl über ein Kohlenstoffatom gebunden ist und ausgewählt ist aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, ß-Carbolinyl und einem ganz oder teilweise gesättigten Derivat dieser Reste, Morpholinosulfonyl oder N-(2-Pyridylmethyl)-N-methyl-aminocarbonyl bedeutet, $B_1$ eine Bindung oder ein zweiwertiges Radikal einer $\alpha$-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl, wobei diese Reste unsubstituiert oder durch ein bis zwei unabhängig voneinander aus Hydroxy, Methoxy, Fluor, Sulfo, Niederalkylsulfonyl, Trifluormethyl und Cyano ausgewählte Reste substituiert sind, bedeuten, $A_1$ ein bivalentes Radikal einer hydrophoben $\alpha$-Aminosäure bedeutet, welches N-terminal mit der Gruppe -C=O und C-terminal mit $A_2$ verbunden ist, $A_2$ ein bivalentes Radikal einer hydrophoben $\alpha$-Aminosäure bedeutet, welches N-terminal mit $A_1$ verbunden ist und C-terminal mit dem Rest $NR_4R_5$ verbunden ist, oder $A_1$ und $A_2$ zusammen ein bivalentes Radikal eines Dipeptides aus zwei hydrophoben $\alpha$-Aminosäuren bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom Thiomorpholino oder Morpholino bedeuten, oder pharmazeutisch verwendbare Salze dieser Verbindungen, sofern salzbildende Gruppen vorliegen; wobei die Hydroxygruppe in Verbindungen der Formel an dem Kohlenstoffatom, welches dem Kohlenstoffatom benachbart ist, das den Rest $R_2$-$CH_2$- trägt, frei oder in durch Niederalkanoyl geschützter Form vorliegt.

3. Verbindungen der Formel I gemäss Anspruch 1, worin $R_1$ Wasserstoff, tert-Butoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, Morpholinosulfonyl oder N-(2-Pyridylmethyl)-N-methyl-aminocarbonyl bedeutet, $B_1$ eine Bindung oder ein bivalentes Radikal der $\alpha$-Aminosäure Valin bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl bedeuten, wobei diese Reste unsubstituiert oder durch ein bis zwei unabhängig voneinander aus Hydroxy, Methoxy, Fluor, Sulfo, Niederalkylsulfonyl, Trifluormethyl und Cyano ausgewählte Reste substituiert sind, $A_1$ ein bivalentes Radikal einer der $\alpha$-Aminosäuren Glycin, Valin oder Isoleucin bedeutet, welches N-terminal mit der Gruppe -C=O und C-terminal mit $A_2$ verbunden ist, $A_2$ ein bivalentes Radikal einer der $\alpha$-Aminosäuren Glycin, Valin, Phenylalanin, Tyrosin, Cyclohexylalanin, p-Methoxy-phenylalanin oder p-Fluorphenylalanin bedeutet, welches N-terminal mit $A_1$ und C-terminal mit der Gruppe $NR_4R_5$ verbunden ist, oder $A_1$ und $A_2$ zusammen ein bivalentes Radikal eines Dipeptides mit reduzierter zentraler Peptidbindung bedeuten, welches aus einem N-terminalen Aminosäureradikal ausgewählt aus Gly(red), Val(red) oder Ile(red) und einem C-terminalen Aminosäureradikal ausgewählt aus Glycin, Phenylalanin, Tyrosin, Cyclohexylalanin, p-Methoxy-phenylalanin oder p-Fluor-phenylalanin besteht, und welches N-terminal mit der Gruppe -C=O und C-terminal mit der Gruppe $NR_4R_5$ verbunden ist, wie es oben für $A_1$ und $A_2$ definiert ist, und $R_4$ und $R_5$ gemeinsam mit dem bindenden Stickstoffatom Thiomorpholino oder Morpholino bedeutet, oder pharmazeutisch verwendbare Salze dieser Verbindungen, sofern salzbildende Gruppen vorliegen.

4. Verbindungen der Formel I gemäss Anspruch 1, worin $R_1$ Wasserstoff, tertButoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, Morpholinosulfonyl oder N-(2-Pyridylmethyl)-N-methyl-aminocarbonyl bedeutet, $B_1$ eine Bindung oder ein bivalentes Radikal der $\alpha$-Aminosäure Valin bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl bedeuten, wobei diese Reste unsubstituiert oder durch ein bis zwei unabhängig voneinander aus Hydroxy, Methoxy, Fluor, Trifluormethyl und Cyano ausgewählte Reste substituiert sind, $A_1$ und $A_2$ zusammen ein bivalentes Radikal eines Dipeptides der Formel Val-Phe, Ile-Phe, Val-Cha, Ile-Cha, Ile-Gly, Val-Val, Val-Gly, Val-(p-F-Phe), Val-(p-$CH_3O$-Phe), Gly-(p-F-Phe), Val-Tyr oder eines Derivates hiervon mit reduzierter zentraler Amidbindung der Formel Val(red)-Phe bilden, das N-terminal mit der Gruppe -C=O und C-terminal mit der Gruppe $NR_4R_5$ verbunden ist, und $R_4$ und $R_5$ gemeinsam mit dem bindenden Stickstoffatom Thiomorpholino oder Morpholino bedeuten, oder pharmazeutisch verwendbare Salze dieser Verbindungen, sofern salzbildende Gruppen vorliegenden wobei die Hydroxygruppe in Verbindungen der Formel an dem Kohlenstoffatom, welches dem Kohlenstoffatom benachbart ist, das den Rest $R_2$-$CH_2$- trägt, frei oder in durch Acetyl geschützter Form vorliegt.

5. Die Verbindung der Formel I gemäss Anspruch 1, worin $R_1$ tert-Butoxycarbonyl, $B_1$ eine Bindung, $R_2$ und $R_3$ Phenyl, $A_1$ Valin, $A_2$ Phenylalanin, und $R_4$ und $R_5$ zusammen mit dem bindenden Stickstoffatom Morpholino bedeuten.

6. Eine Verbindung der Formel I gemäss Anspruch 1, ausgewählt aus den Verbindungen mit den Bezeichnungen
Boc-Cha[C](p-F)Phe-(L)-Ile-(L)-Phe-morpholin-4-ylamid
Boc-(p-F)Phe[C](p-F)Phe-(L)-Val-(L)-Phe-morpholin-4-ylamid,
Boc-(p-F)Phe[C](p-F)Phe-(L)-Val-(L)-(p-F-Phe)-morpholin-4-ylamid,
Boc-(p-F)Phe[C](p-F)Phe-(L)-Val-(L)-(p-$CH_3$O-Phe)-morpholin-4-ylamid,
Boc-(p-F)Phe[C](p-F)Phe-(L)-Val-(L)-Cha-morpholin-4-ylamid,
Boc-(p-F)Phe[C](p-F)Phe-(L)-Ile-(L)-Phe-morpholin-4-ylamid,
Boc-(p-F)Phe[C](p-CN)Phe-(L)-Val-(L)-Phe-morpholin-4-ylamid,
Boc-(p-F)Phe[C](p-CN)Phe-(L)-Val-(L)-(p-F-Phe)-morpholin-4-ylamid,
Boc-(p-F)Phe[C](p-CN)Phe-(L)-Val-(L)-(p-$CH_3$O-Phe)-morpholin-4-ylamid,
Boc-(p-F)Phe[C](p-CN)Phe-(L)-Val-(L)-Cha-morpholin-4-ylamid,
Boc-(p-F)Phe[C](p-CN)Phe-(L)-Ile-(L)-Phe-morpholin-4-ylamid,
Boc-Phe[C](p-F)Phe-(L)-Val-(L)-Phe-morpholin-4-ylamid,
Boc-Phe[C](p-F)Phe-(L)-Val-(L)-(p-F-Phe)-morpholin-4-ylamid,
Boc-Phe[C](p-F)Phe-(L)-Val-(L)-(p-$CH_3$O-Phe)-morpholin-4-ylamid,
Boc-Phe[C](p-F)Phe-(L)-Val-(L)-Cha-morpholin-4-ylamid,
Boc-Phe[C](p-F)Phe-(L)-Ile-(L)-Phe-morpholin-4-ylamid,
Boc-Phe[C](p-CN)Phe-(L)-Val-(L)-Phe-morpholin-4-ylamid,
Boc-Phe[C](p-CN)Phe-(L)-Val-(L)-(p-F-Phe)-morpholin-4-ylamid,
Boc-Phe[C](p-CN)Phe-(L)-Val-(L)-(p-$CH_3$O-Phe)-morpholin-4-ylamid,
Boc-Phe[C](p-CN)Phe-(L)-Val-(L)-Cha-morpholin-4-ylamid,
Boc-Phe[C](p-CN)Phe-(L)-Ile-(L)-Phe-morpholin-4-ylamid,
Boc-Phe[C](p-$CH_3$O)Phe-(L)-Val-(L)-Phe-morpholin-4-ylamid,
Boc-Phe[C](p-$CH_3$O)Phe-(L)-Val-(L)-(p-F-Phe)-morpholin-4-ylamid,
Boc-Phe[C](p-$CH_3$O)Phe-(L)-Val-(L)-(p-$CH_3$O-Phe)-morpholin-4-yl amid,
Boc-Phe[C] (p-$CH_3$O)Phe-(L)-Val-(L)-Cha-morpholin-4-ylamid,
Boc-Phe[C](p-$CH_3$O)Phe-(L)-Ile-(L)-Phe-morpholin-4-ylamid,
Boc-Phe[C](p-$CF_3$)Phe-(L)-Val-(L)-Phe-morpholin-4-ylamid,
Boc-Phe[C](p-$CF_3$)Phe-(L)-Val-(L)-(p-F-Phe)-morpholin-4-ylamid,
Boc-Phe[C](p-$CF_3$)Phe-(L)-Val-(L)-(p-$CH_3$O-Phe)-morpholin-4-ylamid,
Boc-Phe[C](p-$CF_3$)Phe-(L)-Val-(L)-Cha-morpholin-4-ylamid,
Boc-Phe[C](p-$CF_3$)Phe-(L)-Ile-(L)-Phe-morpholin-4-ylamid,
Boc-Cha[C](p-CN)Phe-(L)-Val-(L)-Phe-morpholin-4-ylamid,
Boc-Cha[C](p-CN)Phe-(L)-Val-(L)-(p-F-Phe)-morpholin-4-ylamid,
Boc-Cha[C](p-CN)Phe-(L)-Val-(L)-(p-$CH_3$O-Phe)-morpholin-4-ylamid,
Boc-Cha[C](p-CN)Phe-(L)-Val-(L)-Cha-morpholin-4-ylamid,
Boc-Cha[C](p-CN)Phe-(L)-Ile-(L)-Phe-morpholin-4-ylamid,
Boc-Cha[C](p-$CH_3$O)Phe-(L)-Val-(L)-Phe-morpholin-4-ylamid,
Boc-Cha[C](p-$CH_3$O)Phe-(L)-Val-(L)-(p-F-Phe)-morpholin-4-ylamid,
Boc-Cha[C](p-$CH_3$O)Phe-(L)-Val-(L)-(p-$CH_3$O-Phe)-morpholin-4-ylamid,
Boc-Cha[C](p-$CH_3$O)Phe-(L)-Val-(L)-Cha-morpholin-4-ylamid,
Boc-Cha[C](p-$CH_3$O)Phe-(L)-Ile-(L)-Phe-morpholin-4-ylamid,
Boc-Cha[C](p-$CF_3$)Phe-(L)-Val-(L)-Phe-morpholin-4-ylamid,
Boc-Cha[C](p-$CF_3$)Phe-(L)-Val-(L)-(p-F-Phe)-morpholin-4-ylamid,
Boc-Cha[C](p-$CF_3$)Phe-(L)-Val-(L)-(p-$CH_3$O-Phe)-morpholin- 4-ylamid,
Boc-Cha[C](p-$CF_3$)Phe-(L)-Val-(L)-Cha-morpholin-4-ylamid,
Boc-Cha[C](p-$CF_3$)Phe-(L)-Ile-(L)-Phe-morpholin-4-ylamid,
Boc-(L)-Val-Phe[C]Phe-(L)-Val-(L)-Phe-morpholin-4-ylamid,
H-(L)-Val-Phe[C]Phe-(L)-Val-(L)-Phe-morpholin-4-ylamid,
Boc-Phe[C]Phe-(L)-Val-(D)-Phe-morpholin-4-ylamid,
Boc-Phe[C]Phe-(L)-Val(red)-(L)-Phe-morpholin-4-ylamid,
Isobutyloxycarbonyl-Phe[C]Phe-(L)-Val-(L)-Phe-morpholin-4-ylamid,
Boc-Cha[C] (p-CN)Phe-(L)-Val-(L)-Phe-thiomorpholin-4-ylamid,

Boc-Cha[C](p-F)Phe-(L)-Val-(L)-Phe-thiomorpholin-4-ylamid,
Boc-(p-$CF_3$)Phe[C]Phe-(L)-Val-(L)-Phe-morpholin-4-ylamid,
Boc-(p-$CF_3$)Phe[C]Phe-(L)-Val-(L)-(p-F)Phe-morpholin-4-ylamid,
Boc-(p-$CF_3$)Phe[C]Phe-(L)-Val-(L)-(p-$CH_3O$)Phe-morpholin-4-ylamid,
Boc-(p-$CF_3$)Phe[C]Phe-(L)-Val-(L)-Cha-morpholin-4-ylamid,
Boc-(p-$CF_3$)Phe[C]Phe-(L)-Ile-(L)-Phe-morpholin-4-ylamid,
Boc-(p-$CF_3$)Phe[C](p-F)Phe-(L)-Val-(L)-Phe-morpholin-4-ylamid,
Boc-(p-$CF_3$)Phe[C](p-F)Phe-(L)-Val-(L)-(p-F)Phe-morpholin-4-ylamid,
Boc-(p-$CF_3$)Phe[C](p-F)Phe-(L)-Val-(L)-Cha-morpholin-4-ylamid,
Boc-(p-$CF_3$)Phe[C](p-F)Phe-(L)-Val-(L)-(p-$CH_3O$)Phe-morpholin-4-ylamid,
Boc-(p-$CF_3$)Phe[C](p-F)Phe-(L)-Ile-(L)-Phe-morpholin-4-ylamid,
Boc-(p-$CF_3$)Phe[C](p-$CF_3$)Phe-(L)-Val-(L)-Phe-morpholin-4-ylamid,
Boc-(p-$CF_3$)Phe[C](p-$CF_3$)Phe-(L)-Val-(L)-(p-F)Phe-morpholin-4-ylamid,
Boc-(p-$CF_3$)Phe[C](p-$CF_3$)Phe-(L)-Ile-(L)-Phe-morpholin-4-ylamid,
Boc-(p-$CF_3$)Phe[C] (p-$CF_3$)Phe-(L)-Val-(L)-(p-$CH_3O$)Phe-morpholin-4-ylamid,
Boc-(p-$CF_3$)Phe[C](p-$CF_3$)Phe-(L)-Val-(L)-Cha-morpholin-4-ylamid,
Boc-Cha[C](p-F)Phe-(L)-Val-(L)-Phe-morpholin-4-ylamid,
Boc-Cha[C](p-CN)Phe-(L)-Val-(L)-Phe-morpholin-4-ylamid,
H-Phe[C]Phe-(L)-Val-(L)-Phe-morpholin-4-ylamid,
3-Benzofuranoyl-Phe[C]Phe-(L)-Val-(L)-Phe-morpholin-4-ylamid,
Nicotinoyl-Phe[C]Phe-(L)-Val-(L)-Phe-morpholin-4-ylamid,
Morpholinocarbonyl-Val-Phe[C]Phe-(L)-Val-(L)-Phe-morpholin-4-ylamid,
Boc-Cha[C]Cha-(L)-Val-(L)-Cha-morpholin-4-ylamid,
Boc-Cha[C](p-F)Phe-(L)-Val-(L)-(p-F-Phe)-morpholin-4-ylamid,
Boc-Cha[C](p-F)Phe-(L)-Val-(L)-(p-$CH_3O$-Phe)-morpholin-4-ylamid,
Boc-Cha[C](p-F)Phe-(L)-Val-(L)-Cha-morpholin-4-ylamid,
1,2,3,4-Tetrahydroisochinolin-3(S)-carbonyl-Val-Phe[C]Phe-(L)-Val-(L)-Phe-morpholin-4-ylamid,
Boc-Phe[C]Phe-(L)-Val-(L)-(p-F-Phe)-morpholin-4-ylamid,
Boc-Phe[C]Phe-(L)-Val-(L)-(p-$CH_3O$-Phe)-morpholin-4-ylamid,
Boc-Phe[C]Phe-(L)-Val-(L)-Cha-morpholin-4-ylamid,
Boc-Phe[C]Phe-(L)-Ile-(L)-Phe-morpholin-4-ylamid,
Boc-Phe[C]Phe-(L)-Val-Gly-morpholin-4-ylamid,
Boc-Phe[C]Phe-(L)-Ile-Gly-morpholin-4-ylamid,
Boc-Phe[C]Phe-(L)-Val-(L)-Val-morpholin-4-ylamid,
Boc-Phe[C]Phe-(L)-Val-(L)-Phe-thiomorpholin-4-ylamid,
Boc-(p-F)Phe[C](p-$CF_3$)Phe-(L)-Val-(L)-Phe-morpholin-4-ylamid,
Boc-(p-F)Phe[C](p-$CF_3$)Phe-(L)-Val-(L)-(p-F-Phe)-morpholin-4-ylamid,
Boc-(p-F)Phe[C] (p-$CF_3$)Phe-(L)-Val-(L)-(p-$CH_3O$-Phe)-morpholin-4-ylamid,
Morpholinosulfonyl-(L)-Val-Phe[C]Phe-(L)-Val-(L)-Phe-morpholin-4-ylamid,
Morpholinosulfonyl-Phe[C]Phe-(L)-Val-(L)-Phe-morpholin-4-ylamid,
N-(N-(2-Pyridylmethyl)-N-methyl-aminocarbonyl)-(L)-Val-Phe[C]Phe-(L)-Val-(L)-Phe-morpholin-4-ylamid,
5(S)-(Boc-amino)-4(S)-(acetoxy)-6-phenyl-2(R)-phenylmethylhexanoyl-(L)-Val-(L)-Phe-morpholin-4-ylamid,
Boc-Phe[C]Phe-(L)-Val-(L)-Tyr-morpholin-4-ylamid,
Boc-Tyr[C]Phe-(L)-Val-(L)-Phe-morpholin-4-ylamid,
Boc-Tyr[C]Phe-(L)-Val-(L)-Tyr-morpholin-4-ylamid,
Boc-Phe[C]Tyr-(L)-Val-(L)-Phe-morpholin-4-ylamid,
Boc-Phe[C]Tyr-(L)-Val-(L)-Tyr-morpholin-4-ylamid,
Boc-Tyr[C]Tyr- (L)-Val-(L)-Phe-morpholin-4-ylamid und
Boc-Tyr[C]Tyr-(L)-Val-(L)-Tyr-morpholin-4-ylamid,

oder eine der genannten Verbindungen mit dem Rest -morpholin-4-ylamid, worin anstelle von -morpholin-4-ylamid der Rest -thiomorpholin-4-ylamid steht, oder Salze davon, sofern salzbildende Gruppen vorliegen.

7. Verbindungen der Formel I, oder pharmazeutisch verwendbare Salze davon, sofern salzbildende Gruppen vorliegen, oder hydroxygeschützte Derivate dieser Verbindungen oder deren Salze, gemäss einem der Ansprüche 1 - 6 zur Anwendung in einem Verfahren zur therapeutischen Behandlung des menschlichen oder tierischen Körpers.

8. Pharmazeutische Präparate enthaltend Verbindungen der Formel I gemäss einem der Ansprüche 1 - 7, oder ein pharmazeutisch verwendbares Salz einer solchen Verbindung mit mindestens einer salzbildenden Gruppe, oder hydroxygeschützte Derivate dieser Verbindungen oder deren Salze, zusammen mit pharmazeutisch verwendbarem Trägermaterial.

9. Verwendung einer der in den Ansprüchen 1 - 6 genannten Verbindungen der Formel I, oder eines pharmazeutisch verwendbaren Salzes einer solchen Verbindung mit mindestens einer salzbildenden Gruppe, oder eines hydroxygeschützten Derivates dieser Verbindungen oder Salze davon, zur Herstellung von pharmazeutischen Präparaten zur Anwendung zur Behandlung von AIDS.

10. Verfahren zur Herstellung einer Verbindung der Formel I nach Anspruch 1 oder von dessen hydroxygeschütztem Derivat, und von Salzen von solchen Verbindungen mit mindestens einer salzbildenden Gruppe, dadurch gekennzeichnet, dass man
a) zur Herstellung von Verbindungen der Formel

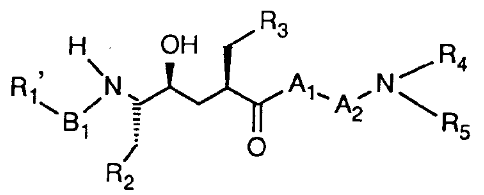
(Ib),

worin $R_1'$ die für Verbindungen der Formel I genannten Bedeutungen von $R_1$ ausser Wasserstoff hat, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I genannten Bedeutungen haben, eine Säure der Formel

$R_1'$ -OH (II)

oder ein reaktionsfähiges Säurederivat davon, worin $R_1'$ ausser Wasserstoff dieselben Bedeutungen hat wie $R_1$ in Verbindungen der Formel I, mit einer Aminoverbindung der Formel

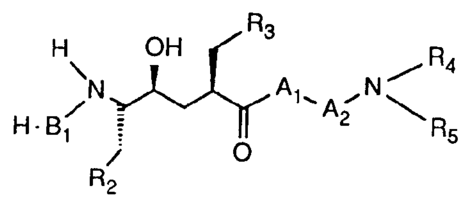
(III)

oder einem reaktionsfähigen Derivat hiervon, worin die Reste die für Verbindungen der Formel genannten Bedeutungen haben, kondensiert, wobei in den Ausgangsmaterialien der Formeln II und III freie funktionelle Gruppen mit Ausnahme der an der Reaktion beteiligten gegebenenfalls in geschützter Form vorliegen, und gewünschtenfalls vorhandene Schutzgruppen abspaltet, oder
b) zur Herstellung von Verbindungen der Formel

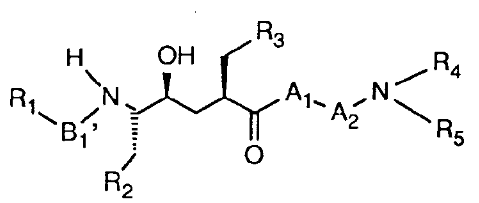
(Ic),

worin $B_1'$ dieselben Reste wie $B_1$ in Verbindungen der Formel I ausser einer Bindung bedeutet, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel genannten Bedeutungen haben, eine Carbonsäure der Formel

$R_1$-$B_1'$ -OH (IV)

oder ein reaktionsfähiges Säurederivat davon, worin $R_1$ die für Verbindungen der Formel I genannten Bedeutungen hat und $B_1'$ die zuletzt genannten Bedeutungen hat, mit einer Aminoverbindung der Formel

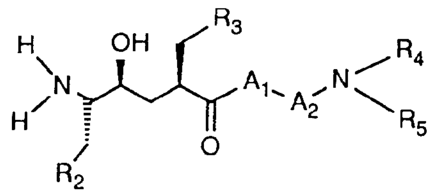

(V),

oder einem reaktionsfähigen Derivat davon, worin die Reste die für Verbindungen der Formel I genannten Bedeutungen haben, kondensiert, wobei freie funktionelle Gruppen in den Ausgangsmaterialien der Formeln IV und V mit Ausnahme der an der Reaktion beteiligten gegebenenfalls in geschützter Form vorliegen, und gewünschtenfalls vorhandene Schutzgruppen abspaltet, oder

c) eine Carbonsäure der Formel

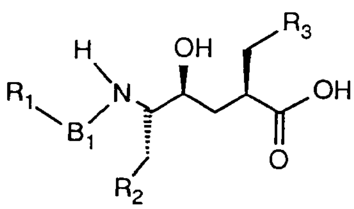

(VI),

oder ein reaktionsfähiges Derivat davon, worin die Reste die für Verbindungen der Formel I genannten Bedeutungen haben, mit einer Aminoverbindung der Formel

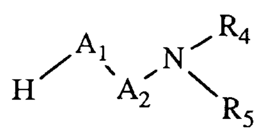

(VII)

oder einem reaktionsfähigen Derivat davon, worin die Reste die für Verbindungen der Formel I genannten Bedeutungen haben, kondensiert, wobei freie funktionelle Gruppen in den Ausgangsmaterialien der Formeln VI und VII mit Ausnahme der an der Reaktion beteiligten gegebenenfalls in geschützter Form vorliegen, und gewünschtenfalls vorhandene Schutzgruppen abspaltet, oder

d) zur Herstellung einer Verbindung der Formel

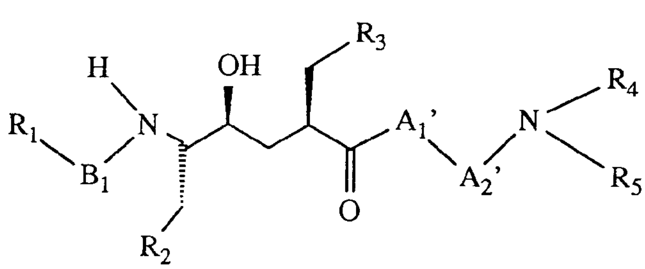

(Id),

worin $A_1'$ und $A_2'$ die Bedeutungen von $A_1$ und $A_2$ in Verbindungen der Formel I haben, wobei $A_1'$ jedoch keine Bindung bedeutet und die Peptidbindung zwischen $A_1'$ und $A_2'$ nicht in reduzierter Form vorliegt, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I genannten Bedeutungen haben, eine Carbonsäure der Formel

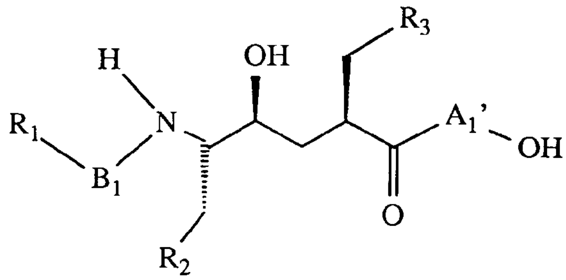

(VIII)

oder ein reaktionsfähiges Derivat davon, worin die Reste die zuletzt genannten Bedeutungen haben, mit einer Aminoverbindung der Formel

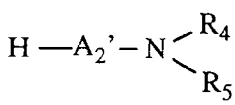

(IX)

oder einem reaktionsfähigen Derivat davon, worin die Reste die zuletzt genannten Bedeutungen haben, kondensiert, wobei freie funktionelle Gruppen in den Ausgangsmaterialien der Formeln VIII und IX mit Ausnahme der an der Reaktion beteiligten gegebenenfalls in geschützter Form vorliegen, und gewünschtenfalls vorhandene Schutzgruppen abspaltet, oder

e) eine Carbonsäure der Formel

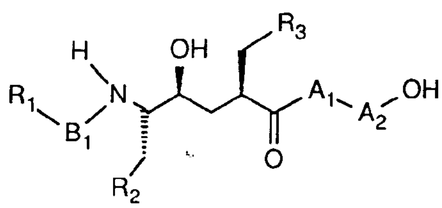

(X)

oder ein reaktionsfähiges Derivat davon, worin die Reste die für Verbindungen der Formel I genannten Bedeutung haben, mit einer Aminoverbindung der Formel

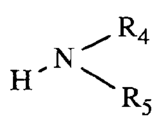

(XI)

oder einem reaktionsfähigen Derivat hiervon, wobei die Reste die für Verbindungen der Formel I genannten Bedeutungen haben, kondensiert, wobei freie funktionelle Gruppen in den Ausgangsmaterialien der Formeln X und XI mit Ausnahme der an der Reaktion beteiligten gegebenenfalls in geschützter Form vorliegen, und gewünschtenfalls vorhandene Schutzgruppen abspaltet, oder

f) in einer Verbindung der Formel I, worin die Substituenten die oben genannten Bedeutungen haben mit der Massgabe, dass in der betreffenden Verbindung der Formel I mindestens eine funktionelle Gruppe durch Schutzgruppen geschützt ist, vorhandene Schutzgruppen abspaltet,

und/oder gewünschtenfalls eine nach einem der vorstehenden Verfahren a) bis f) erhaltene Verbindung der Formel I mit mindestens einer salzbildenden Gruppe in ihr Salz und/oder ein erhältliches Salz in die freie Verbindung oder in ein anderes Salz überführt und/oder gegebenenfalls erhältliche Isomerengemische von Verbindungen der Formel I auftrennt und/oder eine erfindungsgemässe Verbindung der Formel in eine andere erfindungsgemässe Verbindung der Formel umwandelt.

**Patentansprüche für forgende Vertragsstaaten : ES, GR**

1. Verfahren zur Herstellung von Verbindungen der Formel

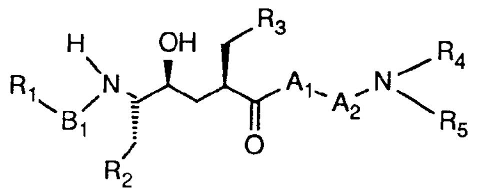

(I),

worin $R_1$ Wasserstoff, Niederalkoxycarbonyl, Heterocyclylcarbonyl, Benzyloxycarbonyl, welches unsubstituiert oder durch bis zu drei unabhängig voneinander aus Fluor, Haloniederalkyl, Niederalkanoyl, Sulfo, Niederalkylsulfonyl und Cyano ausgewählte Reste substituiert ist, Heterocyclyloxycarbonyl, worin Heterocyclyl über ein Kohlenstoffatom gebunden ist, einen der genannten Carbonylreste, worin die bindende Carbonylgruppe durch eine Thiocarbonylgruppe ersetzt ist, Heterocyclylsulfonyl, Niederalkylsulfonyl oder N-(Heterocyclylniederalkyl)-N-niederalkyl-aminocarbonyl bedeutet, $B_1$ eine Bindung oder ein bivalentes Radikal einer α-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl, wobei diese Reste unsubstituiert oder durch ein bis drei unabhängig aus Hydroxy, Niederalkoxy, Halo, Haloniederalkyl, Sulfo, Niederalkylsulfonyl, Cyano und Nitro ausgewählte Reste substituiert sind, bedeuten, $A_1$ eine Bindung zwischen -C=O und $A_2$ oder ein bivalentes Radikal einer α-Aminosäure bedeutet, welches N-terminal mit der Gruppe -C=O und C-terminal mit $A_2$ verbunden ist, $A_2$ ein bivalentes Radikal einer α-Aminosäure bedeutet, welches N-terminal mit $A_1$ und C-terminal mit der Gruppe $NR_4R_5$ verbunden ist, oder $A_1$ und $A_2$ zusammen ein bivalentes Radikal eines Dipeptides bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom unsubstituiertes oder substituiertes Thiomorpholino oder Morpholino bedeuten, oder von Salzen dieser Verbindungen, sofern salzbildende Gruppen vorliegen, oder von hydroxygeschützten Derivaten dieser Verbindungen oder deren Salzen; dadurch gekennzeichnet, dass man

a) zur Herstellung von Verbindungen der Formel

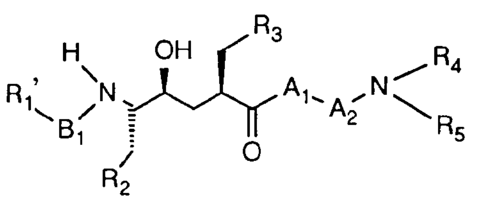

(Ib),

worin $R_1'$ die für Verbindungen der Formel I genannten Bedeutungen von $R_1$ ausser Wasserstoff hat, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I genannten Bedeutungen haben, eine Säure der Formel

$R_1'$-OH (II)

oder ein reaktionsfähiges Säurederivat davon, worin $R_1'$ ausser Wasserstoff dieselben Bedeutungen hat wie $R_1$ in Verbindungen der Formel I, mit einer Aminoverbindung der Formel

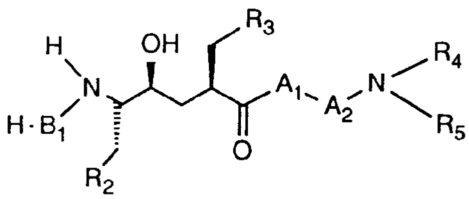

(III)

oder einem reaktionsfähigen Derivat hiervon, worin die Reste die für Verbindungen der Formel I genannten Bedeutungen haben, kondensiert, wobei in den Ausgangsmaterialien der Formeln II und III freie funktionelle Gruppen mit Ausnahme der an der Reaktion beteiligten gegebenenfalls in geschützter Form vorliegen, und gewünschtenfalls vorhandene Schutzgruppen abspaltet, oder

b) zur Herstellung von Verbindungen der Formel

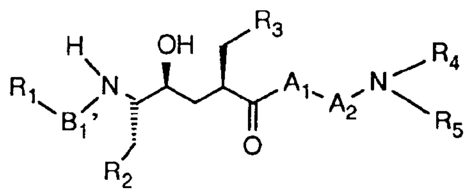

(Ic),

worin $B_1'$ dieselben Reste wie $B_1$ in Verbindungen der Formel I ausser einer Bindung bedeutet, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I genannten Bedeutungen haben, eine Carbonsäure der Formel

$R_1$-$B_1'$-OH (IV)

oder ein reaktionsfähiges Säurederivat davon, worin $R_1$ die für Verbindungen der Formel I genannten Bedeutungen hat und $B_1'$ die zuletzt genannten Bedeutungen hat, mit einer Aminoverbindung der Formel

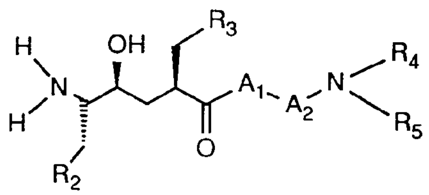

(V),

oder einem reaktionsfähigen Derivat davon, worin die Reste die für Verbindungen der Formel I genannten Bedeutungen haben, kondensiert, wobei freie funktionelle Gruppen in den Ausgangsmaterialien der Formeln IV und V mit Ausnahme der an der Reaktion beteiligten gegebenenfalls in geschützter Form vorliegen, und gewünschtenfalls vorhandene Schutzgruppen abspaltet, oder

c) eine Carbonsäure der Formel

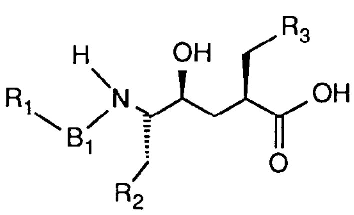

(VI),

oder ein reaktionsfähiges Derivat davon, worin die Reste die für Verbindungen der Formel I genannten Bedeutungen haben, mit einer Aminoverbindung der Formel

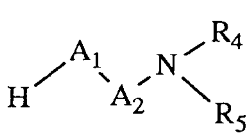

(VII)

oder einem reaktionsfähigen Derivat davon, worin die Reste die für Verbindungen der Formel I genannten Bedeutungen haben, kondensiert, wobei freie funktionelle Gruppen in den Ausgangsmaterialien der Formeln VI und VII mit Ausnahme der an der Reaktion beteiligten gegebenenfalls in geschützter Form vorliegen, und gewünschtenfalls vorhandene Schutzgruppen abspaltet, oder

d) zur Herstellung einer Verbindung der Formel

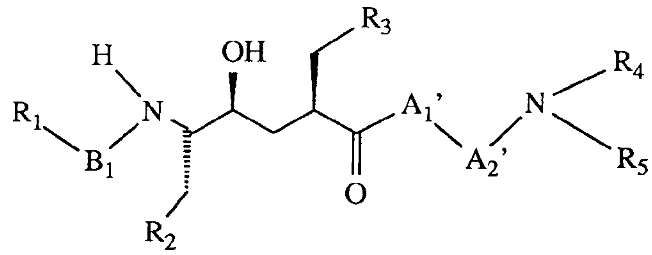

(Id),

worin $A_1'$ und $A_2'$ die Bedeutungen von $A_1$ und $A_2$ in Verbindungen der Formel I haben, wobei $A_1'$ jedoch keine Bindung bedeutet und die Peptidbindung zwischen $A_1'$ und $A_2'$ nicht in reduzierter Form vorliegt, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel genannten Bedeutungen haben, eine Carbonsäure der Formel

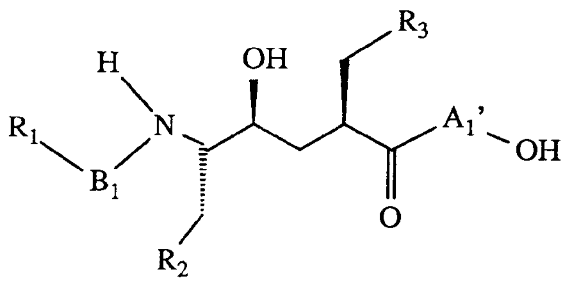

(VIII)

oder ein reaktionsfähiges Derivat davon, worin die Reste die zuletzt genannten Bedeutungen haben, mit einer Aminoverbindung der Formel

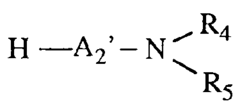

(IX)

oder einem reaktionsfähigen Derivat davon, worin die Reste die zuletzt genannten Bedeutungen haben, kondensiert, wobei freie funktionelle Gruppen in den Ausgangsmaterialien der Formeln VIII und IX mit Ausnahme der an der Reaktion beteiligten gegebenenfalls in geschützter Form vorliegen, und gewünschtenfalls vorhandene Schutzgruppen abspaltet, oder

e) eine Carbonsäure der Formel

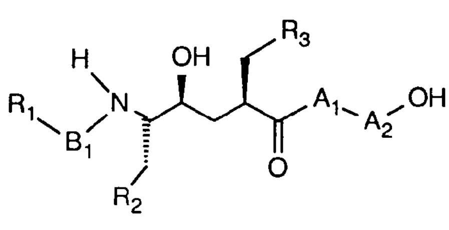

(X)

oder ein reaktionsfähiges Derivat davon, worin die Reste die für Verbindungen der Formel I genannten Bedeutung haben, mit einer Aminoverbindung der Formel

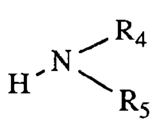

(XI)

oder einem reaktionsfähigen Derivat hiervon, wobei die Reste die für Verbindungen der Formel I genannten Bedeutungen haben, kondensiert, wobei freie funktionelle Gruppen in den Ausgangsmaterialien der Formeln X und XI mit Ausnahme der an der Reaktion beteiligten gegebenenfalls in geschützter Form vorliegen, und gewünschtenfalls vorhandene Schutzgruppen abspaltet, oder

f) in einer Verbindung der Formel I, worin die Substituenten die oben genannten Bedeutungen haben mit der Massgabe, dass in der betreffenden Verbindung der Formel mindestens eine funktionelle Gruppe durch Schutzgruppen geschützt ist, vorhandene Schutzgruppen abspaltet,

und/oder gewünschtenfalls eine nach einem der vorstehenden Verfahren a) bis f) erhaltene Verbindung der Formel I mit mindestens einer salzbildenden Gruppe in ihr Salz und/oder ein erhältliches Salz in die freie Verbindung oder in ein anderes Salz überführt und/oder gegebenenfalls erhältliche Isomerengemische von Verbindungen der Formel I auftrennt und/oder eine erfindungsgemässe Verbindung der Formel in eine andere erfindungsgemässe Verbindung der Formel umwandelt.

2. Verfahren gemäss Anspruch 1 zur Herstellung von Verbindungen der Formel I, worin $R_1$ Wasserstoff, Niederalkoxycarbonyl, Heterocyclylcarbonyl, Benzyloxycarbonyl, welches unsubstituiert oder durch bis zu drei unabhängig voneinander aus Fluor, Haloniederalkyl, Niederalkanoyl, Sulfo, Niederalkylsulfonyl und Cyano ausgewählte Reste substituiert ist, oder Heterocyclyloxycarbonyl, worin Heterocyclyl über ein Kohlenstoffatom gebunden ist, bedeutet, oder einen der genannten Carbonylreste, worin die bindende Carbonylgruppe durch eine Thiocarbonylgruppe ersetzt ist, $B_1$ eine Bindung oder ein bivalentes Radikal einer α-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl, wobei diese Reste unsubstituiert oder durch ein bis drei unabhängig aus Halo, Haloniederalkyl, Sulfo, Niederalkylsulfonyl, Cyano und Nitro ausgewählte Reste substituiert sind, bedeuten, $A_1$ eine Bindung zwischen -C=O und $A_2$ oder ein bivalentes Radikal einer α-Aminosäure bedeutet, welches N-terminal mit der Gruppe -C=O und C-terminal mit $A_2$ verbunden ist, $A_2$ ein bivalentes Radikal einer α-Aminosäure bedeutet, welches N-terminal mit $A_1$ und C-terminal mit der Gruppe $NR_4R_5$ verbunden ist, oder $A_1$ und $A_2$ zusammen ein bivalentes Radikal eines Dipeptides bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom unsubstituiertes oder substituiertes Thiomorpholino oder Morpholino bedeuten, oder von Salzen dieser Verbindungen, sofern salzbildende Gruppen vorliegen.

3. Verfahren gemäss Anspruch 1 zur Herstellung von Verbindungen der Formel I, worin $R_1$ Heterocyclylsulfonyl, Niederalkylsulfonyl oder N-(Heterocyclylniederalkyl)-N-niederalkyl-aminocarbonyl bedeutet und die übrigen Reste die genannten Bedeutungen haben, oder von Salzen davon, sofern salzbildende Gruppen vorliegen.

4. Verfahren gemäss Anspruch 2 zur Herstellung von Verbindungen der Formel I, worin $R_1$ Wasserstoff, tert-Butoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, mit bis zu drei unabhängig voneinander aus Fluor, Haloniederalkyl, Niederalkanoyl, Sulfo, Niederalkylsulfonyl und Cyano ausgewählten Resten substituiertes Benzyloxycarbonyl, oder Heterocyclyloxycarbonyl bedeutet, worin Heterocyclyl über ein Kohlenstoffatom gebunden ist und ausgewählt ist aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, ß-Carbolinyl und einem ganz oder teilweise gesättigten Derivat dieser Reste, $B_1$ eine Bindung oder ein zweiwertiges Radikal einer α-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl, wobei diese Reste unsubstituiert oder durch ein bis zwei unabhängig voneinander aus Fluor, Sulfo, Niederalkylsulfonyl und Cyano ausgewählte Reste substituiert sind, bedeuten, $A_1$ ein bivalentes Radikal einer hydrophoben α-Aminosäure bedeutet, welches N-terminal mit der Gruppe -C=O und C-terminal mit $A_2$ verbunden ist, $A_2$ ein bivalentes Radikal einer hydrophoben α-Aminosäure bedeutet, welches N-terminal mit $A_1$ verbunden ist und C-terminal mit dem Rest $NR_4R_5$ verbunden ist, oder $A_1$ und $A_2$ zusammen ein bivalentes Radikal eines Dipeptides aus zwei hydrophoben α-Aminosäuren bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom Morpholino bedeuten, oder von pharmazeutisch verwendbaren Salzen dieser Verbindung, sofern salzbildende Gruppen vorliegen.

5. Verfähren gemäss Anspruch 1 zur Herstellung von Verbindungen der Formel I, worin $R_1$ Wasserstoff, tert-Butoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, mit bis zu drei unabhängig voneinander aus Fluor, Haloniederalkyl, Niederalkanoyl, Sulfo, Niederalkylsulfonyl und Cyano ausgewählten Resten substituiertes Benzyloxycarbonyl, Heterocyclyloxycarbonyl, worin Heterocyclyl über ein Kohlenstoffatom gebunden ist und ausgewählt ist aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, β-Carbolinyl und einem ganz oder teilweise gesättigten Derivat dieser Reste, Morpholinosulfonyl oder N-(2-Pyridylmethyl)-N-methyl-aminocarbonyl bedeutet, $B_1$ eine Bindung oder ein zweiwertiges Radikal einer α-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl, wobei diese Reste unsubstituiert oder durch ein bis zwei unabhängig voneinander aus Hydroxy, Methoxy, Fluor, Sulfo, Niederalkylsulfonyl, Trifluormethyl und Cyano ausgewählte Reste substituiert sind, bedeuten, $A_1$ ein bivalentes Radikal einer hydrophoben α-Aminosäure bedeutet, welches N-terminal mit der Gruppe -C=O und C-terminal mit $A_2$ verbunden ist, $A_2$ ein bivalentes Radikal einer hydrophoben α-Aminosäure bedeutet, welches N-terminal mit $A_1$ verbunden ist und C-terminal mit dem Rest $NR_4R_5$ verbunden ist, oder $A_1$ und $A_2$ zusammen ein bivalentes Radikal eines Dipeptides aus zwei hydrophoben α-Aminosäuren bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom Thiomorpholino oder Morpholino bedeuten, oder von pharmazeutisch verwendbaren Salzen dieser Verbindungen, sofern salzbildende Gruppen vorliegen; wobei die Hydroxygruppe in Verbindungen der Formel an dem Kohlenstoffatom, welches dem Kohlenstoffatom benachbart ist, das den Rest $R_2$-$CH_2$- trägt, frei oder in durch Niederalkanoyl geschützter Form vorliegt.

6. Verfahren gemäss Anspruch 1 zur Herstellung einer Verbindung der Formel I gemäss Anspruch 1, worin $R_1$ Wasserstoff, tert-Butoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, Morpholinosulfonyl oder N-(2-Pyridylmethyl)-N-methyl-aminocarbonyl bedeutet, $B_1$ eine Bindung oder ein bivalentes Radikal der α-Aminosäure Valin bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl bedeuten, wobei diese Reste unsubstituiert oder durch ein bis zwei unabhängig voneinander aus Hydroxy, Methoxy, Fluor, Sulfo, Niederalkylsulfonyl, Trifluormethyl und Cyano ausgewählte Reste substituiert sind, $A_1$ ein bivalentes Radikal einer der α-Aminosäuren Glycin, Valin oder Isoleucin bedeutet, welches N-terminal mit der Gruppe -C=O und C-terminal mit $A_2$ verbunden ist, $A_2$ ein bivalentes Radikal einer der α-Aminosäuren Glycin, Valin, Phenylalanin, Tyrosin, Cyclohexylalanin, p-Methoxy-phenylalanin oder p-Fluorphenylalanin bedeutet, welches N-terminal mit $A_1$ und C-terminal mit der Gruppe $NR_4R_5$ verbunden ist, oder $A_1$ und $A_2$ zusammen ein bivalentes Radikal eines Dipeptides mit reduzierter zentraler Peptidbindung bedeuten, welches aus einem N-terminalen Aminosäureradikal ausgewählt aus Gly(red), Val(red) oder Ile(red) und einem C-terminalen Aminosäureradikal ausgewählt aus Glycin, Phenylalanin, Tyrosin, Cyclohexylalanin, p-Methoxy-phenylalanin oder p-Fluor-phenylalanin besteht, und welches N-terminal mit der Gruppe -C=O und C-terminal mit der Gruppe $NR_4R_5$ verbunden ist, wie es oben für $A_1$ und $A_2$ definiert ist, und $R_4$ und $R_5$ gemeinsam mit dem bindenden Stickstoffatom Thiomorpholino oder Morpholino bedeutet, oder von pharmazeutisch verwendbaren Salzen dieser Verbindung, sofern salzbildende Gruppen vorliegen.

7. Verfahren gemäss Anspruch 1 zur Herstellung von Verbindungen der Formel I, worin $R_1$ Wasserstoff, tertButoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, Morpholinosulfonyl oder N-(2-Pyridylmethyl)-N-methyl-aminocarbonyl bedeutet, $B_1$ eine Bindung oder ein bivalentes Radikal der α-Aminosäure Valin bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl bedeuten, wobei diese Reste unsubstituiert oder durch ein bis zwei unabhängig voneinander aus Hydroxy, Methoxy, Fluor, Trifluormethyl und Cyano ausgewählte Reste substituiert sind, $A_1$ und $A_2$ zusammen ein bivalentes Radikal eines Dipeptides der Formel Val-Phe, Ile-Phe, Val-Cha, Ile-Cha, Ile-Gly, Val-Val, Val-Gly, Val-(p-F-Phe), Val-(p-$CH_3O$-Phe), Gly-(p-F-Phe), Val-Tyr oder eines Derivates hiervon mit reduzierter zentraler Amidbindung der Formel Val(red)-Phe bilden, das N-terminal mit der Gruppe -C=O und C-terminal mit der Gruppe $NR_4R_5$ verbunden ist, und $R_4$ und $R_5$ gemeinsam mit dem bindenden Stickstoffatom Thiomorpholino oder Morpholino bedeuten, oder von pharmazeutisch verwendbaren Salzen dieser Verbindungen, sofern salzbildende Gruppen vorliegen; wobei die Hydroxygruppe in Verbindungen der Formel I an dem Kohlenstoffatom, welches dem Kohlenstoffatom benachbart ist, das den Rest $R_2$-$CH_2$-trägt, frei oder in durch Acetyl geschützter Form vorliegt.

8. Verfahren gemäss Anspruch 1 zur Herstellung einer Verbindung der Formel I, worin $R_1$ tert-Butoxycarbonyl, $B_1$ eine Bindung, $R_2$ und $R_3$ Phenyl, $A_1$ Valin, $A_2$ Phenylalanin, und $R_4$ und $R_5$ zusammen mit dem bindenden Stickstoffatom Morpholino bedeuten.

9. Verfahren gemäss Anspruch 1 zur Herstellung einer Verbindung der Formel ausgewählt aus den Verbindungen mit den Bezeichnungen
Boc-Cha[C](p-F)Phe-(L)-Ile-(L)-Phe-morpholin-4-ylamid
Boc-(p-F)Phe[C](p-F)Phe-(L)-Val-(L)-Phe-morpholin-4-ylamid,
Boc-(p-F)Phe[C](p-F)Phe-(L)-Val-(L)-(p-F-Phe)-morpholin-4-ylamid,
Boc-(p-F)Phe[C](p-F)Phe-(L)-Val-(L)-(p-$CH_3$O-Phe)-morpholin-4-ylamid,
Boc-(p-F)Phe[C)(p-F)Phe-(L)-Val-(L)-Cha-morpholin-4-ylamid,
Boc-(p-F)Phe[C)(p-F)Phe-(L)-Ile-(L)-Phe-morpholin-4-ylamid,
Boc-(p-F)Phe[C](p-CN)Phe-(L)-Val-(L)-Phe-morpholin-4-ylamid,
Boc-(p-F)Phe[C](p-CN)Phe-(L)-Val-(L)-(p-F-Phe)-morpholin-4-ylamid,
Boc-(p-F)Phe[C](p-CN)Phe-(L)-Val-(L)-(p-$CH_3$O-Phe)-morpholin-4-ylamid,
Boc-(p-F)Phe[C](p-CN)Phe-(L)-Val-(L)-Cha-morpholin-4-ylamid,
Boc-(p-F)Phe[C](p-CN)Phe-(L)-Ile-(L)-Phe-morpholin-4-ylamid,
Boc-Phe[C](p-F)Phe-(L)-Val-(L)-Phe-morpholin-4-ylamid,
Boc-Phe[C](p-F)Phe-(L)-Val-(L)-(p-F-Phe)-morpholin-4-ylamid,
Boc-Phe[C](p-F)Phe-(L)-Val-(L)-(p-$CH_3$O-Phe)-morpholin-4-ylamid,
Boc-Phe[C](p-F)Phe-(L)-Val-(L)-Cha-morpholin-4-ylamid,
Boc-Phe[C](p-F)Phe-(L)-Ile-(L)-Phe-morpholin-4-ylamid,
Boc-Phe[C](p-CN)Phe-(L)-Val-(L)-Phe-morpholin-4-ylamid,
Boc-Phe[C](p-CN)Phe-(L)-Val-(L)-(p-F-Phe)-morpholin-4-ylamid,
Boc-Phe[C](p-CN)Phe-(L)-Val-(L)-(p-$CH_3$O-Phe)-morpholin-4-ylamid,
Boc-Phe[C](p-CN)Phe-(L)-Val-(L)-Cha-morpholin-4-ylamid,
Boc-Phe[C](p-CN)Phe-(L)-Ile-(L)-Phe-morpholin-4-ylamid,
Boc-Phe[C](p-$CH_3$O)Phe-(L)-Val-(L)-Phe-morpholin-4-ylamid,
Boc-Phe[C](p-$CH_3$O)Phe-(L)-Val-(L)-(p-F-Phe)-morpholin-4-ylamid,
Boc-Phe[C](p-$CH_3$O)Phe-(L)-Val-(L)-(p-$CH_3$O-Phe)-morpholin-4-yl amid,
Boc-Phe[C](p-$CH_3$O)Phe-(L)-Val-(L)-Cha-morpholin-4-ylamid,
Boc-Phe[C](p-$CH_3$O)Phe-(L)-Ile-(L)-Phe-morpholin-4-ylamid,
Boc-Phe[C](p-$CF_3$)Phe-(L)-Val-(L)-Phe-morpholin-4-ylamid,
Boc-Phe[C](p-$CF_3$)Phe-(L)-Val-(L)-(p-F-Phe)-morpholin-4-ylamid,
Boc-Phe[C](p-$CF_3$)Phe-(L)-Val-(L)-(p-$CH_3$O-Phe)-morpholin-4-ylamid,
Boc-Phe[C](p-$CF_3$)Phe-(L)-Val-(L)-Cha-morpholin-4-ylamid,
Boc-Phe[C](p-$CF_3$)Phe-(L)-Ile-(L)-Phe-morpholin-4-ylamid,
Boc-Cha[C](p-CN)Phe-(L)-Val-(L)-Phe-morpholin-4-ylamid,
Boc-Cha[C](p-CN)Phe-(L)-Val-(L)-(p-F-Phe)-morpholin-4-ylamid,
Boc-Cha[C](p-CN)Phe-(L)-Val-(L)-(p-$CH_3$O-Phe)-morpholin-4-ylamid,
Boc-Cha[C](p-CN)Phe-(L)-Val-(L)-Cha-morpholin-4-ylamid,
Boc-Cha[C](p-CN)Phe-(L)-Ile-(L)-Phe-morpholin-4-ylamid,
Boc-Cha[C](p-$CH_3$O)Phe-(L)-Val-(L)-Phe-morpholin-4-ylamid,
Boc-Cha[C](p-$CH_3$O)Phe-(L)-Val-(L)-(p-F-Phe)-morpholin-4-ylamid,
Boc-Cha[C](p-$CH_3$O)Phe-(L)-Val-(L)-(p-$CH_3$O-Phe)-morpholin-4-ylamid,
Boc-Cha[C](p-$CH_3$O)Phe-(L)-Val-(L)-Cha-morpholin-4-ylamid,
Boc-Cha[C](p-$CH_3$O)Phe-(L)-Ile-(L)-Phe-morpholin-4-ylamid,
Boc-Cha[C](p-$CF_3$)Phe-(L)-Val-(L)-Phe-morpholin-4-ylamid,
Boc-Cha[C](p-$CF_3$)Phe-(L)-Val-(L)-(p-F-Phe)-morpholin-4-ylamid,
Boc-Cha[C](p-$CF_3$)Phe-(L)-Val-(L)-(p-$CH_3$O-Phe)-morpholin-4-ylamid,
Boc-Cha[C](p-$CF_3$)Phe-(L)-Val-(L)-Cha-morpholin-4-ylamid,
Boc-Cha[C](p-$CF_3$)Phe-(L)-Ile-(L)-Phe-morpholin-4-ylamid,
Boc-(L)-Val-Phe[C]Phe-(L)-Val-(L)-Phe-morpholin-4-ylamid,
H-(L)-Val-Phe[C]Phe-(L)-Val-(L)-Phe-morpholin-4-ylamid,
Boc-Phe[C]Phe-(L)-Val-(D)-Phe-morpholin-4-ylamid,
Boc-Phe[C]Phe-(L)-Val(red)-(L)-Phe-morpholin-4-ylamid,
Isobutyloxycarbonyl-Phe[C]Phe-(L)-Val-(L)-Phe-morpholin-4-ylamid,
Boc-Cha[C](p-CN)Phe-(L)-Val-(L)-Phe-thiomorpholin-4-ylamid,

Boc-Cha[C](p-F)Phe-(L)-Val-(L)-Phe-thiomorpholin-4-ylamid,
Boc-(p-$CF_3$)Phe[C]Phe-(L)-Val-(L)-Phe-morpholin-4-ylamid,
Boc-(p-$CF_3$)Phe[C]Phe-(L)-Val-(L)-(p-F)Phe-morpholin-4-ylamid,
Boc-(p-$CF_3$)Phe[C]Phe-(L)-Val-(L)-(p-$CH_3O$)Phe-morpholin-4-ylamid,
Boc-(p-$CF_3$)Phe[C]Phe-(L)-Val-(L)-Cha-morpholin-4-ylamid,
Boc-(p-$CF_3$)Phe[C]Phe-(L)-Ile-(L)-Phe-morpholin-4-ylamid,
Boc-(p-$CF_3$)Phe[C](p-F)Phe-(L)-Val-(L)-Phe-morpholin-4-ylamid,
Boc-(p-$CF_3$)Phe[C](p-F)Phe-(L)-Val-(L)-(p-F)Phe-morpholin-4-ylamid,
Boc-(p-$CF_3$)Phe[C](p-F)Phe-(L)-Val-(L)-Cha-morpholin-4-ylamid,
Boc-(p-$CF_3$)Phe[C](p-F)Phe-(L)-Val-(L)-(p-$CH_3O$)Phe-morpholin-4 -ylamid,
Boc-(p-$CF_3$)Phe[C](p-F)Phe-(L)-Ile-(L)-Phe-morpholin-4-ylamid,
Boc-(p-$CF_3$)Phe[C](p-$CF_3$)Phe-(L)-Val-(L)-Phe-morpholin-4-ylamid,
Boc-(p-$CF_3$)Phe[C](p-$CF_3$)Phe-(L)-Val-(L)-(p-F)Phe-morpholin-4-ylamid,
Boc-(p-$CF_3$)Phe[C](p-$CF_3$)Phe-(L)-Ile-(L)-Phe-morpholin-4-ylamid,
Boc-(p-$CF_3$)Phe[C](p-$CF_3$)Phe-(L)-Val-(L)-(p-$CH_3O$)Phe-morpholin-4-ylamid,
Boc-(p-$CF_3$)Phe[C](p-$CF_3$)Phe-(L)-Val-(L)-Cha-morpholin-4-ylamid,
Boc-Cha[C](p-F)Phe-(L)-Val-(L)-Phe-morpholin-4-ylamid,
Boc-Cha[C](p-CN)Phe-(L)-Val-(L)-Phe-morpholin-4-ylamid,
H-Phe[C]Phe-(L)-Val-(L)-Phe-morpholin-4-ylamid,
3-Benzofuranoyl-Phe[C]Phe-(L)-Val-(L)-Phe-morpholin-4-ylamid,
Nicotinoyl-Phe[C]Phe-(L)-Val-(L)-Phe-morpholin-4-ylamid,
Morpholinocarbonyl-Val-Phe[C]Phe-(L)-Val-(L)-Phe-morpholin-4-ylamid,
Boc-Cha[C]Cha-(L)-Val-(L)-Cha-morpholin-4-ylamid,
Boc-Cha[C](p-F)Phe-(L)-Val-(L)-(p-F-Phe)-morpholin-4-ylamid,
Boc-Cha[C](p-F)Phe-(L)-Val-(L)-(p-$CH_3O$-Phe)-morpholin-4-ylamid,
Boc-Cha[C](p-F)Phe-(L)-Val-(L)-Cha-morpholin-4-ylamid,
1,2,3,4-Tetrahydroisochinolin-3(S)-carbonyl-Val-Phe[C]Phe-(L)Val-(L)-Phe-morpholin-4-ylamid,
Boc-Phe[C]Phe-(L)-Val-(L)-(p-F-Phe)-morpholin-4-ylamid,
Boc-Phe[C]Phe-(L)-Val-(L)-(p-$CH_3O$-Phe)-morpholin-4-ylamid,
Boc-Phe[C]Phe-(L)-Val-(L)-Cha-morpholin-4-ylamid,
Boc-Phe[C]Phe-(L)-Ile-(L)-Phe-morpholin-4-ylamid,
Boc-Phe[C]Phe-(L)-Val-Gly-morpholin-4-ylamid,
Boc-Phe[C]Phe-(L)-Ile-Gly-morpholin-4-ylamid,
Boc-Phe[C]Phe-(L)-Val-(L)-Val-morpholin-4-ylamid,
Boc-Phe[C]Phe-(L)-Val-(L)-Phe-thiomorpholin-4-ylamid,
Boc-(p-F)Phe[C](p-$CF_3$)Phe-(L)-Val-(L)-Phe-morpholin-4-ylamid,
Boc-(p-F)Phe[C](p-$CF_3$)Phe-(L)-Val-(L)-(p-F-Phe)-morpholin-4-ylamid,
Boc-(p-F)Phe[C](p-$CF_3$)Phe-(L)-Val-(L)-(p-$CH_3O$-Phe)-morpholin-4-ylamid,
Morpholinosulfonyl-(L)-Val-Phe[C]Phe-(L)-Val-(L)-Phe-morpholin-4-ylamid,
Morpholinosulfonyl-Phe[C]Phe-(L)-Val-(L)-Phe-morpholin-4-ylamid,
N-(N-(2-pyridylmethyl)-N-methyl-aminocarbonyl)-(L)-Val-Phe[C]Phe-(L)-Val-(L)-Phe-morpholin-4-ylamid,
5(S)-(Boc-amino)-4(S)-(acetoxy)-6-phenyl-2(R)-phenylmethylhexanoyl-(L)-Val-(L)-Phe-morpholin-4-ylamid,
Boc-Phe[C]Phe-(L)-Val-(L)-Tyr-morpholin-4-ylamid,
Boc-Tyr[C]Phe-(L)-Val-(L)-Phe-morpholin-4-ylamid,
Boc-Tyr[C]Phe-(L)-Val-(L)-Tyr-morpholin-4-ylamid,
Boc-Phe[C]Tyr-(L)-Val-(L)-Phe-morpholin-4-ylamid,
Boc-Phe[C]Tyr-(L)-Val-(L)-Tyr-morpholin-4-ylamid,
Boc-Tyr[C]Tyr-(L)-Val-(L)-Phe-morpholin-4-ylamid und
Boc-Tyr[C]Tyr-(L)-Val-(L)-Tyr-morpholin-4-ylamid,
oder von einer der genannten Verbindungen mit dem Rest -morpholin-4-ylamid, worin anstelle von -morpholin-4-ylamid der Rest -thiomorpholin-4-ylamid steht, oder von Salzen davon, sofern salzbildende Gruppen vorliegen.

**10.** Verfahren zur Herstellung eines pharmazeutischen Präparates enthaltend eine gemäss einem der Ansprüche 1 bis 9 hergestellte Verbindung der Formel I, oder ein pharmazeutisch annehmbares Salz davon, sofern salzbildende Gruppen vorliegen, oder ein hydroxygeschütztes Derivat oder ein Salz davon, dadurch gekennzeichnet, dass man die Verbindung der Formel I, ein Salz davon, oder ein hydroxygeschütztes Derivat davon oder dessen Salz mit mindestens einem pharmazeutisch annehmbaren Trägermaterial versetzt.